# EUROPEAN PATENT APPLICATION

(11) **EP 2 361 902 A1**
(43) Date of publication of application: **31.08.2011**
(21) Application number: 09827627.2
(22) Date of filing: 20.11.2009
(51) Int. Cl.: C07D 213/82, A61K 31/44, A61K 31/4433, A61K 31/4439, A61K 31/444, A61K 31/455, A61K 31/497, A61K 31/501, A61K 31/506, A61K 31/5377, A61K 31/55, A61K 31/551, A61K 31/553, A61P 1/04

(54) **4,6-DIAMINONICOTINAMIDE COMPOUND**

(30) Priority: 21.11.2008 JP 2008297770
(71) Applicant: Astellas Pharma Inc., Tokyo 103-8411 (JP)
(72) Inventor: SHIRAKAMI Shohei, Tokyo 103-8411 (JP); TAKAHASHI Fumie, Tokyo 103-8411 (JP); NAKAJIMA Yutaka, Tokyo 103-8411 (JP); OMURA Hirofumi, Tokyo 103-8411 (JP); AOYAMA Naohiro, Tokyo 103-8411 (JP); SASAKI Hiroshi, Tokyo 103-8411 (JP); HONDO Takeshi, Tokyo 103-8411 (JP); TOMINAGA Hiroaki, Tokyo 103-8411 (JP)
(74) Representative: Gille Hrabal Struck Neidlein Prop Roos
(86) International application number: PCT/JP2009/069731
(87) International publication number: WO 2010/058846

(57) **Abstract**

[Problem]

The present invention provides a 4,6-diaminonicotinamide compound which is useful as an active ingredient of a pharmaceutical composition, in particular, a pharmaceutical composition for treating diseases caused by undesirable and/or abnormal cytokine signal transduction.

[Means for Solution]

The present inventors have extensively studied compounds having a JAK3 inhibitory action, and as a result, they have found that a 4,6-diaminonicotinamide compound which is the compound of the present invention has an excellent JAK3 inhibitory action and is useful as an agent for preventing or treating diseases caused by undesirable and/or abnormal cytokine signal transduction, thereby completing the present invention.

## Description

### Technical Field

The present invention relates to a 4,6-diaminonicotinamide compound which is useful as an active ingredient of a pharmaceutical composition, in particular, a pharmaceutical composition for treating diseases caused by undesirable and/or abnormal cytokine signal transduction.

### Background Art

Janus kinase 3 (hereafter referred to as JAK3) is a member of the Janus family which are members of protein kinases. Although kinases in this family, other than JAK3, are expressed in a wide range of tissues, JAK3 is expressed locally in hematopoietic cells. This does not contradict the fact that JAK3 plays an important role in signal transduction via various receptors, such as interleukin (hereafter referred to as IL) -2, IL-4, IL-7, IL-9, IL-15, and IL-21 by noncovalent binding with the common γ chain (Non-Patent Reference 1 and Non-Patent Reference 2).
XSCID (X-linked severe combined immunodeficiency) patient groups have been identified with reduced levels of JAK3 protein or with genetic defects in a common γ chain, suggesting that immunosupression occurs due to blocking signaling through the JAK3 pathway (Non-Patent References 3 and 4). Animal experiments have suggested that JAK3 not only plays an important role in maturation of B- and T-lymphocytes but also in maintaining the function of T-cells. Hence, it is expected that diseases involving proliferation abnormality of T-cells, such as rejection upon organ transplantation and autoimmune diseases, can be treated by controlling immune response through this mechanism.

On the other hand, it is known that a pyridine derivative represented by the formula (A) has a PKC-theta inhibitory action and is useful for immunodeficiency, autoimmune diseases, inflammatory diseases, rejection accompanying organ or bone marrow transplantation, or other diseases mediated by T-cells, or the like (Patent Reference 1).

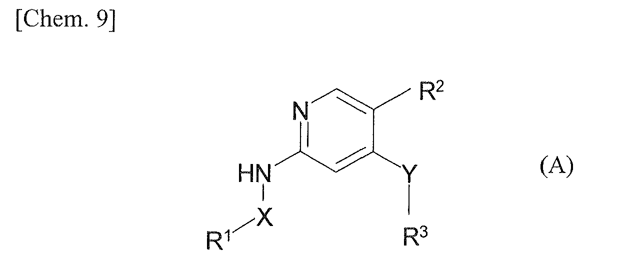

(wherein R³ represents: 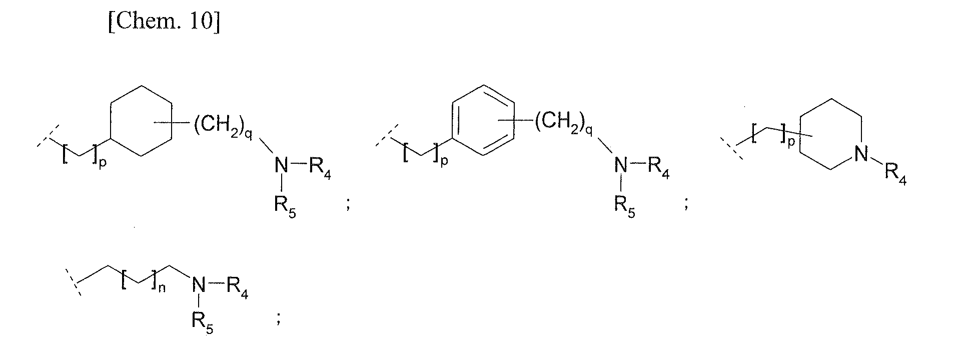 p represents 1 to 3, and n represents 0 to 5. For the other symbols, refer to the corresponding patent publications).
This compound is characterized in that in the side chain R³, various substituents are bonded to Y via a straight alkylene group having one or more C.

Furthermore, a pyrrolopyrimidine derivative represented by the formula (B) is known to have a glucokinase activity and be useful for diseases such as diabetes, impaired glucose tolerance, blood sugar disorders during fasting, and the like (Patent Reference 2).

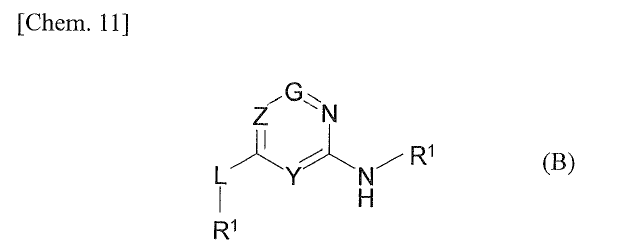

(for the symbols in the formulas, refer to the corresponding patent publications).
The compound of the formula (B) described in the corresponding Patent Reference is characterized in that R¹ is a 5-membered heteroaryl ring containing at least one nitrogen atom.

Next, there is known a compound represented by the formula (C) which is useful for diseases associated with cell proliferation, such as cancer, inflammation, and the like (Patent Reference 3).

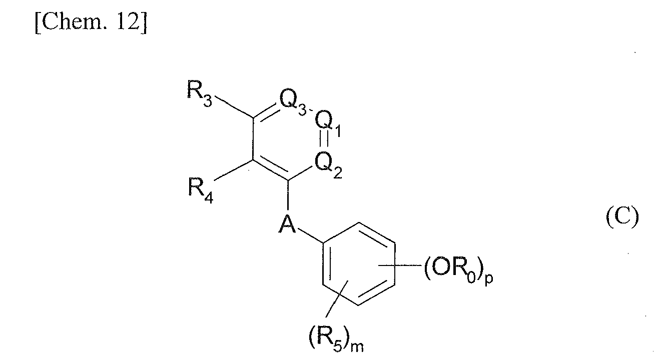

(wherein A represents a bond, O, S(O)q, N(R_{N}), C(O), or CH(R_{c}). For the other symbols, refer to the corresponding patent publications).
The compound represented by the formula (C) described in the corresponding Patent Reference is characterized in that two aromatic rings are directly bonded to A.

Further, there is known a compound represented by the formula (D), which is useful for diseases in which immune response participates (Patent Reference 4).

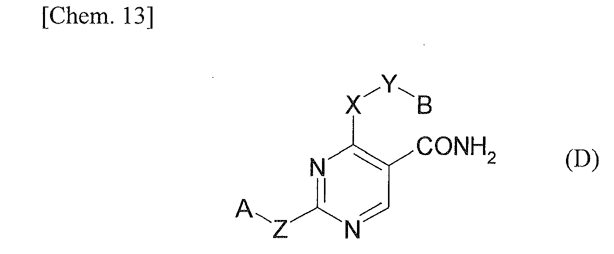

(for the symbols in the formula, refer to the corresponding patent publications.)
The compound of the formula (D) described in the corresponding Patent Reference is structurally characterized in that a carboxamide group is bonded to a pyrimidine ring.

In addition, a compound represented by the formula (E) useful for diseases such as cancer and the like is disclosed in an International Publication Pamphlet which was published after the priority date of the present application (Patent Reference 5).

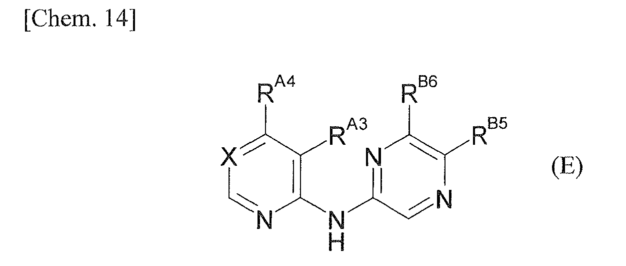

(wherein X represents -CR^{A5}= or -N=. For the other symbols, refer to the corresponding patent publications).
The compound of the formula (E) described in the corresponding Patent Reference is structurally characterized in that a pyrimidine ring or a pyridine ring is bonded to a pyrazine ring via an amino group.
In any reference, the compound of the present invention is not disclosed specifically.

Non-Patent Reference 1: J. J. O'shea, et al., Cell, Vol. 109 (suppl.), S121, 2002
Non-Patent Reference 2: K. Ozaki, et al., Science, Vol. 298, p. 1630, 2002
Non-Patent Reference 3: P. Macchi, et al., Nature, Vol. 377, p. 65, 1995
Non-Patent Reference 4: S. M. Russell, et al., Science, Vol. 270, p. 797, 1995 Patent Document
Patent Reference 1: Pamphlet of International Publication WO 2006/105023
Patent Reference 2: Pamphlet of International Publication WO 2007/089512
Patent Reference 3: Pamphlet of International Publication WO 2008/024963
Patent Reference 4: Pamphlet of International Publication WO 99/31073
Patent Reference 5: Pamphlet of International Publication WO 2009/044162

### Disclosure of Invention

### Problems to Be Solved by the Invention

The present invention provides a 4,6-diaminonicotinamide compound which is useful as an active ingredient of a pharmaceutical composition, in particular, a pharmaceutical composition for treating diseases caused by undesirable and/or abnormal cytokine signal transduction.

### Means for Solving the Problem

The present inventors have assiduously studied compounds having a JAK3 inhibitory action, and as a result, they have found that a 4,6-diaminonicotinamide compound which is the compound of the present invention has an excellent JAK3 inhibitory action and is useful as an agent for preventing or treating diseases caused by undesirable and/or abnormal cytokine signal transduction, thereby completing the present invention.

The present invention relates to a compound of the formula (I) or a salt thereof: 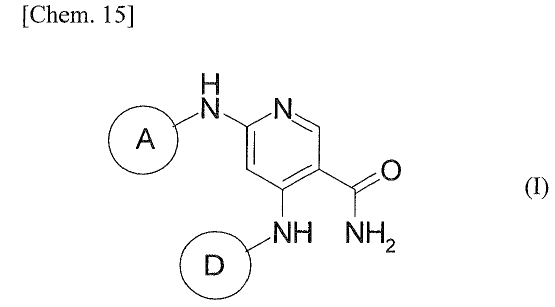 (wherein
A represents H, or lower alkyl, aryl, or a nitrogen-containing hetero ring, which may each be substituted,
D represents the following group: 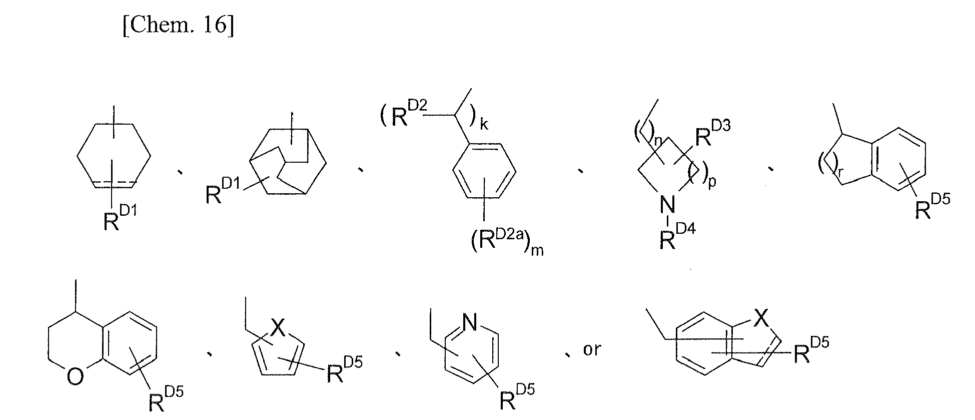
R^{D1} represents H, halogen, lower alkyl, OH, or CN; or -O-lower alkyl or a nitrogen-containing hetero ring, which may each be substituted,
R^{D2} may be the same as or different from each other and represent H, or lower alkyl,
R^{D2a} may be the same as or different from each other and represent H, halogen, OH, -S-lower alkyl, -S(=O)₂-lower alkyl, -N(lower alkyl)₂, -N(lower alkyl) -S(=O)₂-lower alkyl, -CN, or a nitrogen-containing hetero ring; or lower alkyl or -O-lower alkyl, which may each be substituted,
k represents 0, 1, or 2,
m represents 1, 2, or 3,
R^{D3} represents H, halogen, or lower alkyl which may be substituted,
R^{D4} represents H, CN, or protected carboxy; or lower alkyl, aryl, a nitrogen-containing hetero ring, -C(=O)-lower alkyl, or -S(=O)₂-aryl, which may each be substituted,
n represents 0, or 1,
p represents 1, 2, 3, or 4,
X represents NH, N-lower alkyl, or S,
R^{D5} represents H, halogen, -N(lower alkyl)₂, or lower alkyl which may be substituted, and
r represents 1 or 2.
wherein the broken line portion in the formula above together with an adjacent bond represents a single bond or a double bond).
Furthermore, unless specifically described otherwise, in the case where the symbols in any of the formulas in the present specification are also used in other formulas, the same symbols denote the same meanings.

The present invention relates to a pharmaceutical composition containing the compound of the formula (I) or a salt thereof and an excipient.
Further, the present invention relates to a pharmaceutical composition, particularly, a pharmaceutical composition for preventing or treating diseases caused by undesirable cytokine signal transduction or diseases caused by abnormal cytokine signal transduction, including the compound of the formula (I) or a salt thereof and an excipient.
In addition, the present invention relates to use of the compound of the formula (I) or a salt thereof for preparation of a pharmaceutical composition for preventing or treating diseases caused by undesirable cytokine signal transduction or diseases caused by abnormal cytokine signal transduction, use of the compound of the formula (I) or a salt thereof for preventing or treating diseases caused by undesirable cytokine signal transduction or diseases caused by abnormal cytokine signal transduction, and a method for preventing or treating diseases caused by undesirable cytokine signal transduction or diseases caused by abnormal cytokine signal transduction, including administering to a patient an effective amount of the compound of the formula (I) or a salt thereof.

### Effects of the Invention

The compound of the formula (I) or a salt thereof has a JAK3 inhibitory action and can be used as an agent for preventing or treating undesirable and/or abnormal diseases caused by abnormal cytokine signal transduction.

### Best Mode for Carrying Out the Invention

The present invention will be explained in more detail herein below. Further, "the compound of the formula (I) or a salt thereof" may be denoted as "the compound (I) of the present invention" or "the compound (I)" below in some cases.

In the present specification, the "lower alkyl" is a straight or branched alkyl having 1 to 6 carbon atoms (hereinafter simply referred to as C₁₋₆), for example, methyl, ethyl, n-propyl, isopropyl, n-butyl, isobutyl, sec-butyl, tert-butyl, n-pentyl, n-hexyl, and the like. In another embodiment, it is C₁₋₄ alkyl, and in a further embodiment, C₁₋₃ alkyl.

The "lower alkylene" is a straight or branched C₁₋₆ alkylene, for example methylene, ethylene, trimethylene, tetramethylene, pentamethylene, hexamethylene, propylene, methyl methylene, ethyl ethylene, 1,2-dimethyl ethylene, 1,1,2,2-tetramethyl ethylene, and the like. In another embodiment, it is C₁₋₄ alkylene, and in a further other embodiment, C₁₋₃ alkylene.

The "cycloalkyl" is a C₃₋₁₀ saturated hydrocarbon ring group, which may have a bridge. It is, for example, cyclopropyl, cyclobutyl, cyclopentyl, cyclohexyl, cycloheptyl, cyclooctyl, adamantyl, and the like, in another embodiment, C₃₋₈ cycloalkyl, and in a further embodiment, C₃₋₆ cycloalkyl.

The "C₅₋₈ cycloalkene" is a C₅₋₈ monocyclic unsaturated hydrocarbon ring, which excludes a C₅₋₈ monocyclic aromatic hydrocarbon ring, for example, cyclopentene, cyclopentadiene, cyclohexene, cyclohexadiene, cycloheptene, cycloheptadiene, cyclooctene, cyclooctadiene, and the like.

The "aryl" is a C₆₋₁₄ monocyclic to tricyclic aromatic hydrocarbon ring group, and examples thereof include C₅₋₈ cycloalkene and a fused ring group wherein C₅₋₈ cycloalkene is fused at a site of the double bond thereof. It is, for example, phenyl, naphthyl, 5-tetrahydronaphthyl, 4-indenyl, 1-fluorenyl, and the like.

The "hetero ring" means a ring group containing i) a monocyclic 3- to 8-membered, and in another embodiment, 5- to 7-membered hetero ring, containing 1 to 4 hetero atoms selected from oxygen, sulfur, and nitrogen, and ii) a bi- to tricyclic hetero ring (in which the bi- to tricyclic heterocyclic ring includes a spiro ring) containing 1 to 5 hetero atoms selected from oxygen, sulfur, and nitrogen, formed by condensation of the monocyclic hetero ring with one or two rings selected from the group consisting of a monocyclic hetero ring, a benzene ring, C₅₋₈ cycloalkane, and C₅₋₈ cycloalkene. The ring atom, sulfur or nitrogen, may be oxidized to form an oxide or a dioxide.

Examples of the "hetero ring" include the following embodiments:
(1) Monocyclic saturated hetero ring
   (a) those containing 1 to 4 nitrogen atoms, for example, azepanyl, diazepanyl, aziridinyl, azetidinyl, pyrrolidinyl, imidazolidinyl, piperidyl, pirazolidinyl, piperazinyl, azocanyl, hexamethyleneimino, homopiperazinyl, and the like;
   (b) those containing 1 to 3 nitrogen atoms and 1 to 2 sulfur atoms and/or 1 to 2 oxygen atoms, for example, thiomorpholinyl, thiazolidinyl, isothiazolidinyl, oxazolidinyl, morpholinyl, and the like;
   (c) those containing 1 to 2 sulfur atoms, for example, tetrahydrothiopyranyl and the like;
   (d) those containing 1 to 2 sulfur atoms and 1 to 2 oxygen atoms, for example, oxathiolanyl and the like; and
   (e) those containing 1 to 2 oxygen atoms, for example, oxiranyl, oxetanyl, dioxolanyl, tetrahydrofuranyl, tetrahydropyranyl, 1,4-dioxanyl, and the like;

(2) Monocyclic unsaturated hetero ring group
   (a) those containing 1 to 4 nitrogen atoms, for example, pyrrolyl, 2-pyrrolinyl, imidazolyl, 2-imidazolinyl, pyrazolyl, 2-pyrazolinyl, pyridyl, dihydropyridyl, tetrahydropyridinyl, pyrimidinyl, pyrazinyl, pyridazinyl, triazolyl, tetrazolyl, triazinyl, dihydrotriazinyl, azepinyl, and the like;
   (b) those containing 1 to 3 nitrogen atoms and 1 to 2 sulfur atoms and/or 1 to 2 oxygen atoms, for example, thiazolyl, isothiazolyl, thiadiazolyl, dihydrothiazinyl, oxazolyl, isoxazolyl, oxadiazolyl, oxazinyl, and the like;
   (c) those containing 1 to 2 sulfur atoms, for example, thienyl, thiepinyl, dihydrodithiopyranyl, dihydrodithionyl, 2H-thiopyranyl, and the like;
   (d) those containing 1 to 2 sulfur atoms and 1 to 2 oxygen atoms, for example, dihydroxythiopyranyl and the like; and
   (e) those containing 1 to 2 oxygen atoms, for example, furyl, dihydrofuryl, pyranyl, 2HC-pyranyl, oxepinyl, dioxolyl, and the like;

(3) Fused polycyclic saturated hetero ring group
   (a) those containing 1 to 5 nitrogen atoms, for example, quinuclidinyl, 7-azabicyclo[2.2.1]heptyl, 3-azabicyclo[3.2.2]nonanyl, 2,8-diazaspiro[4.5]deca-8-yl, 2,3,6,8-tetraazaspiro[4.5]decan-8-yl, and the like;
   (b) those containing 1 to 4 nitrogen atoms and 1 to 3 sulfur atoms and/or 1 to 3 oxygen atoms, for example, trithiadiazaindenyl, dioxoloimidazolidinyl, 6-oxa-2,8-diazaspiro[4.5]decan-8-yl, 6-thia-2,8-diazaspiro[4.5]decan-8-yl, and the like; and
   (c) those containing 1 to 3 sulfur atoms and/or 1 to 3 oxygen atoms, for example, 2,6-dioxabicyclo[3.2.2]octo-7-yl, 2-oxa-6-thiaspiro[4.5]decan-8-yl, and the like;

(4) Fused polycyclic unsaturated hetero ring group
   (a) those containing 1 to 5 nitrogen atoms, for example, indolyl, isoindolyl, indolinyl, indolidinyl, benzoimidazolyl, dihydrobenzoimidazolyl, tetrahydrobenzoimidazolyl, quinolyl, tetrahydroquinolyl, isoquinolyl, tetrahydroisoquinolyl, indazolyl, imidazopyridyl, benzotriazolyl, tetrazolopyridazinyl, carbazolyl, acridinyl, quinoxalinyl, dihydroquinoxalinyl, tetrahydroquinoxalinyl, phthalazinyl, dihydroindazolyl, benzopyrimidinyl, naphthyridinyl, quinazolinyl, cinnolinyl, pyridopyrrolidinyl, triazolopiperidinyl, 9,10-dihydroacridine, 2,8-diazaspiro[4.5]deca-3-en-8-yl, 2,3,6,8-tetraazaspiro[4.5]deca-1-en-8-yl, and the like;
   (b) those containing 1 to 4 nitrogen atoms and 1 to 3 sulfur atoms and/or 1 to 3 oxygen atoms, for example, benzothiazolyl, dihydrobenzothiazolyl, benzothiadiazolyl, imidazothiazolyl, imidazothiadiazolyl, benzoxazolyl, dihydrobenzoxazolyl, dihydrobenzoxazinyl, benzoxadiazolyl, benzoisothiazolyl, benzoisoxazolyl, thiazolopiperidinyl, 10H-phenothiazine, 6-oxa-2,8-diazaspiro[4.5]deca-3-en-8-yl, 6-thia-2,8-diazaspiro[4.5]deca-3-en-8-yl, and the like;
   (c) those containing 1 to 3 sulfur atoms, for example, benzothienyl, benzodithiopyranyl, chromanyl, dibenzo[b,d]thienyl, and the like;
   (d) those containing 1 to 3 sulfur atoms and 1 to 3 oxygen atoms, for example, benzoxathiopyranyl, phenoxazinyl, 2-oxa-6-thia-spiro[4.5]deca-3-en-8-yl, and the like; and
   (e) those containing 1 to 3 oxygen atoms, for example, benzodioxolyl, benzofuranyl, dihydrobenzofuranyl, isobenzofuranyl, chromanyl, chromenyl, isochromenyl, dibenzo[b,d]furanyl, methylenedioxyphenyl, ethylenedioxyphenyl, xanthenyl, and the like;
      etc.

Further, the "hetero ring" in (1) to (4) above is described to be a monovalent group, but this may represent a divalent or higher group in some cases.

The "nitrogen-containing hetero ring" refers to one containing at least one nitrogen atom, as in (1)(a), (1)(b), (2)(a), (2)(b), (3)(a), (3)(b), (4)(a), (4)(b), and the like, among the "hetero rings" above.

The "nitrogen-containing saturated hetero ring" includes, for example, (1)(a), (1)(b), (3)(a), and (3)(b), among the "nitrogen-containing hetero rings" above.

The "aromatic hetero ring" refers to a hetero ring group having an aromatic property, among (2) and (4) of the "hetero rings". Examples thereof include monocyclic aromatic hetero rings such as pyridyl, pyrrolyl, pyrazinyl, pyrimidinyl, pyridazinyl, imidazolyl, triazolyl, triazinyl, tetrazolyl, thiazolyl, pyrazolyl, isothiazolyl, oxazolyl, isoxazolyl, thiadiazolyl, oxadiazolyl, thienyl, furyl, and the like, bicyclic aromatic hetero rings such as indolyl, isoindolyl, benzoimidazolyl, indazolyl, quinolyl, isoquinolyl, quinazolinyl, quinoxalinyl, phthalazinyl, benzothiazolyl, benzoisothiazolyl, benzothiadiazolyl, benzoxazolyl, benzoisoxazolyl, benzofuranyl, benzothienyl, and the like, and tricyclic aromatic hetero rings such as carbazolyl, dibenzo[b,d]furanyl, dibenzo[b,d]thienyl, and the like.

The "nitrogen-containing aromatic hetero ring" refers to those having at least one nitrogen atom, among the "aromatic hetero rings" above. Examples thereof include monocyclic aromatic hetero rings such as pyridyl, pyrrolyl, pyrazinyl, pyrimidinyl, pyridazinyl, imidazolyl, triazolyl, triazinyl, tetrazolyl, thiazolyl, pyrazolyl, isothiazolyl, oxazolyl, isoxazolyl, thiadiazolyl, oxadiazolyl, and the like, bicyclic aromatic hetero rings such as indolyl, isoindolyl, benzoimidazolyl, indazolyl, quinolyl, isoquinolyl, quinazolinyl, quinoxalinyl, phthalazinyl, benzothiazolyl, benzoisothiazolyl, benzothiadiazolyl, benzoxazolyl, benzoisoxazolyl, and the like, and tricyclic aromatic hetero rings such as carbazolyl, and the like.

The "nitrogen-containing aromatic 6-membered hetero ring" refers to monocyclic and bicyclic heteroaryl having a 6-membered ring, among the "nitrogen-containing aromatic hetero rings" above. Examples thereof include monocyclic hetero rings such as pyridyl, pyrazinyl, pyrimidinyl, pyridazinyl, and the like, and bicyclic hetero rings such as quinolyl, isoquinolyl, quinazolinyl, quinoxalinyl, and the like.

The "unsaturated hetero ring having no aromatic property" refers to a ring group excluding the "aromatic hetero ring" among (2) and (4) of the "hetero rings" above. Examples thereof include monocyclic unsaturated hetero rings such as 2-pyrrolinyl, 2-imidazolinyl, 2-pyrazolinyl, dihydropyridyl, tetrahydropyridinyl, dihydrotriazinyl, dihydrothiazinyl, 2H-thiopyranyl, dihydrodithiopyranyl, dihydrodithionyl, dihydroxathiopyranyl, dihydrofuryl, 2H-pyranyl, and the like, bicyclic unsaturated hetero rings such as indolinyl, isoindolinyl, benzoimidazolinyl, indazolinyl, indolidinyl, tetrahydroquinolyl, tetrahydroisoquinolyl, chromanyl, chromenyl, benzodithiopyranyl, thiochromanyl, isochromenyl, and the like, and tricyclic unsaturated hetero rings such as 9,10-dihydroacridine, xanthenyl, 10H-phenothiazine, and the like.

The "nitrogen-containing unsaturated hetero ring having no aromatic property" refers to one having at least one nitrogen atom among the "unsaturated hetero rings having no aromatic property" above. Examples thereof include monocyclic unsaturated hetero rings such as 2-pyrrolinyl, 2-imidazolinyl, 2-pyrazolinyl, dihydropyridyl, tetrahydropyridinyl, dihydrotriazinyl, dihydrothiazinyl, and the like, bicyclic unsaturated hetero rings such as indolinyl, isoindolinyl, benzoimidazolinyl, indazolinyl, indolidinyl, tetrahydroquinolyl, tetrahydroisoquinolyl, and the like, and tricyclic unsaturated hetero rings such as 9,10-dihydroacridine, 10H-phenothiazine, and the like.

The "nitrogen-containing hetero ring having at least one NH on a ring-forming atom" refers to one having a hydrogen atom on a ring-forming nitrogen atom, among the "nitrogen-containing hetero rings" above, and examples thereof include monocyclic saturated hetero rings such as azepanyl, diazepanyl, aziridinyl, azetidinyl, pyrrolidinyl, imidazolidinyl, piperidyl, pirazolidinyl, piperazinyl, azocanyl, hexamethyleneimino, homopiperazinyl, thiomorpholinyl, thiazolidinyl, isothiazolidinyl, oxazolidinyl, morpholinyl, and the like, monocyclic unsaturated hetero rings such as pyrrolyl, 2-pyrrolinyl, imidazolyl, 2-imidazolinyl, pyrazolyl, 2-pyrazolinyl, dihydropyridyl, tetrahydropyridinyl, triazolyl, tetrazolyl, triazinyl, dihydrotriazinyl, azepinyl, thiazolyl, isothiazolyl, thiadiazolyl, dihydrothiazinyl, oxazolyl, isoxazolyl, oxadiazolyl, oxazinyl, and the like, fused polycyclic saturated hetero rings such as quinuclidinyl, 7-azabicyclo[2.2.1]heptyl, 3-azabicyclo[3.2.2]nonyl, 2,8-diazaspiro[4.5]decan-8-yl, 2,3,6,8-tetraazaspiro[4.5]decan-8-yl, trithiadiazaindenyl, dioxoloimidazolidinyl, 6-oxa-2,8-diazaspiro[4.5]decan-8-yl, 6-thia-2,8-diazaspiro[4.5]decan-8-yl, and the like, and fused polycyclic unsaturated hetero rings such as indolyl, isoindolyl, indolinyl, benzoimidazolyl, dihydrobenzoimidazolyl, tetrahydrobenzoimidazolyl, tetrahydroquinolyl, tetrahydroisoquinolyl, indazolyl, imidazopyridyl, benzotriazolyl, tetrazolopyridazinyl, carbazolyl, dihydroquinoxalinyl, tetrahydroquinoxalinyl, dihydroindazolyl, pyridopyrrolidinyl, triazolopiperidinyl, 9,10-dihydroacridine, 2,8-diazaspiro[4.5]deca-3-en-8-yl, 2,3,6,8-tetraazaspiro[4.5]deca-1-en-8-yl, dihydrobenzothiazolyl, benzothiadiazolyl, imidazothiazolyl, imidazothiadiazolyl, dihydrobenzoxazolyl, dihydrobenzoxazinyl, benzoxadiazolyl, thiazolopiperidinyl, 10H-phenothiazine, 6-oxa-2,8-diazaspiro[4.5]deca-3-en-8-yl, 6-thia-2,8-diazaspiro[4.5]deca-3-en-8-yl, and the like.

The "halogen" means F, Cl, Br, or I, and preferably F.

Examples of the "protected carboxy" group can include the following groups:
(1) Esterified carboxy group. Specific examples thereof include -CO-O-lower alkyl, -CO-O-aryl, -CO-O-hetero ring, -CO-O-lower alkylene-O-lower alkyl, -CO-O-lower alkylene-aryl, -CO-O-lower alkylene-O-aryl, and the like. Further, these groups may be substituted with CN, nitrogen-containing hetero rings, -OH, or -O-lower alkyl; or cycloalkyl which may each be substituted with one or two lower alkyl or -C(=O)-lower alkyl.
(2) Amidated carboxy group. Specific examples thereof include -CO-NH₂, -CO-NH-lower alkyl, -CO-NH-cycloalkyl, -CO-NH-hetero ring, -CO-N(lower alkyl)₂, -CON(lower alkyl)-aryl, -CO-N(lower alkyl)-cycloalkyl, -CO-N(lower alkyl)-(lower alkylene-aryl), -CO-NH-lower alkylene-OH, -CO-NH-lower alkylene-CO₂H, and the like. Further, these groups may be substituted with CN, a nitrogen-containing hetero ring, -OH, or -O-lower alkyl; or cycloalkyl which may each be substituted with one or two lower alkyl or -C(=O)-lower alkyl.

In the present specification, the expression "which may be substituted" represents "which is not substituted" or "which is substituted with 1 to 5 substituents". Further, if it has a plurality of substituents, the substituents may be the same as or different from each other. For example, -N(lower alkyl)₂ includes an ethylmethylamino group.

In the present specification, in D, D¹, and D², 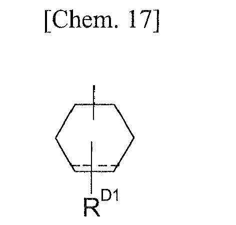 the broken line portion forms, together with an adjacent bond, a single bond or a double bond, and represents:

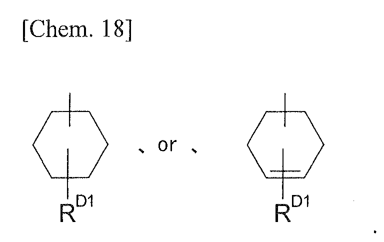

In the present specification, in D, D¹, and D³, 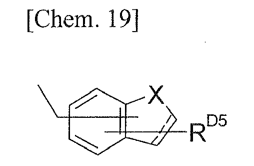 the broken line portion forms, together with an adjacent bond, a single bond or a double bond, and represents:

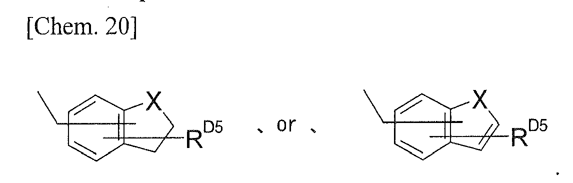

Examples of the "diseases caused by undesirable cytokine signal transduction or diseases caused by abnormal cytokine signal transduction" include organ transplantation such as kidney transplantation, liver transplantation, heart transplantation, pancreas transplantation, lung transplantation, and the like, xenotransplantation, autoimmune diseases such as Type I diabetes, rheumatoid arthritis, multiple sclerosis, systemic lupus erythematosus (SLE), myasthenia gravis, and the like, Alzheimer's disease, Crohn's disease, psoriasis, asthma, atopic dermatitis, erythrocytosis, leukemia and tumors such as hormone-refractory prostate cancer and the like, multiple myeloma, bone marrow fibrosis, thrombocythemia, and the like.

Examples of the acceptable substituent used in the present specification include the groups shown in (a) to (1) below:
(a) Halogen.
(b) -OH, -O-lower alkyl (wherein the lower alkyl may be substituted with 1 to 3 halogens).
(c) Amino which may be substituted with 1 or 2 lower alkyl, nitro.
(d) -SO₂-lower alkyl, -SO₂-cycloalky -SO₂-monocyclic saturated hetero ring, -SO₂-aryl, sulfamoyl which may be substituted with 1 or 2 lower alkyl.
(e) -CHO, -CO-lower alkyl, -CO-cycloalkyl (wherein the cycloalkyl may be substituted with at least one -O-lower alkyl), -CO-monocyclic saturated hetero ring, cyano.
(f) Protected carboxy.
(g) Aryl or cycloalkyl. Further, these groups may each be substituted with at least one group selected from the group consisting of -OH, -O-lower alkyl, amino which may be substituted with 1 or 2 lower alkyl, -CO₂H, carbamoyl which may be substituted with 1 or 2 lower alkyl, aryl, a hetero ring, halogen, and CN.
(h) Nitrogen-containing aromatic hetero ring or monocyclic saturated hetero ring.
   Further, this monocyclic saturated hetero ring may be substituted with at least one group selected from the group consisting of oxo (=O), -lower alkyl, -O-lower alkyl, and amino which may be substituted with 1 or 2 lower alkyl.
(i) Lower alkyl which may be substituted with at least one group selected from the substituents shown in (a) to (h) above.
(j) -CO-monocyclic saturated hetero ring substituted with a group selected from -OH, oxo (=O), lower alkyl, -O-lower alkyl, -C(=O)-lower alkyl, NH₂, NH(lower alkyl), N(lower alkyl)₂, cycloalkyl, aryl, a hetero ring (wherein the hetero ring may be substituted with oxo (=O) or a hetero ring), and -NH-S(=O)₂-lower alkyl. Further, the "lower alkyl", "cycloalkyl", "aryl", and "hetero ring" of this lower alkyl, -O-lower alkyl, -C(=O)-lower alkyl, cycloalkyl, aryl, a hetero ring, or -NH-S(=O)₂-lower alkyl may be substituted with at least one group selected from the substituents shown in (a) to (i) above.
(k) Lower alkyl which may be substituted with at least one group selected from the substituents shown in (a) to (h) and (j) above.
(l) Oxo (=O).

Examples of the embodiments regarding the acceptable substituent in the "aryl which may be substituted" in A include the groups shown in (a) to (k) above. Alternatively, in another embodiment of the substituent, examples of the embodiments include the groups shown in (a) to (i) above. Furthermore, in another embodiment of the substituent of the "monocyclic saturated hetero ring" of the "-CO-monocyclic saturated hetero ring" in (j), examples of the embodiments include -OH, oxo (=O), lower alkyl, -O-lower alkyl, -C(=O)-lower alkyl, cycloalkyl, aryl, a hetero ring, and -NH-S(=O)₂-lower alkyl. In addition, in (j), the "lower alkyl", "cycloalkyl", "aryl", and "hetero ring" of lower alkyl, -O-lower alkyl, -C(=O)-lower alkyl, cycloalkyl, aryl, a hetero ring, or -NH-S(=O)₂-lower alkyl may be substituted with at least one group selected from the substituents shown in (a) to (i) above.

Examples of the acceptable substituent in the "lower alkyl which may be substituted" in A include the groups shown in (g) above.

Examples of the acceptable substituent of the "nitrogen-containing hetero ring which may be substituted" in A, and the "nitrogen-containing aromatic 6-membered hetero ring which may be substituted", "nitrogen-containing unsaturated hetero ring having no aromatic property, which may be substituted", and "nitrogen-containing saturated hetero ring which may be substituted" in A¹ include the groups shown in (a) to (c), (e) to (k), and (1) above. Alternatively, in another embodiment of these substituents, examples of the substituent include the groups shown in (a) to (c), (e) to (i), and (1) above.

Examples of the acceptable substituent in the "-O-lower alkyl which may be substituted" and "nitrogen-containing hetero ring which may be substituted" in R^{D1} include the groups shown in (e) and (j) above. Alternatively, in another embodiment of these substituents, examples of the substituent include the groups shown in (e) above.

Examples of the acceptable substituent in the "-O-lower alkyl which may be substituted" in R^{D2a} include the groups shown in (b) above.

Examples of the acceptable substituent in the "-O-lower alkyl which may be substituted" in R^{D2a} include the groups shown in (a), (e), and (j) above. Alternatively, in another embodiment of these substituents, examples of the substituent include the groups shown in (a) and (e) above.

Examples of the acceptable substituent in the "lower alkyl which may be substituted" in R^{D3} include the groups shown in (b) above.

Examples of the acceptable substituent in the "lower alkyl which may be substituted" in R^{D4} include the groups shown in (b) and (g) above.

Examples of the acceptable substituent in the "aryl which may be substituted" in R^{D4} include the groups shown in (a), (b), (e), and (i) to (k) above. Alternatively, in another embodiment of these substituents, examples of the substituent include the groups shown in (a), (b), (e), and (i) above.

Examples of the acceptable substituent in the "nitrogen-containing hetero ring which may be substituted" in R^{D4} include the groups shown in (e), (f), and (j) above. Alternatively, in another embodiment of these substituents, examples of the substituent include the groups shown in (e) and (f) above.

Examples of the acceptable substituent in the "lower alkyl which may be substituted" in R^{D5} include the groups shown in (a) above.

Embodiments regarding the compound of the present invention (I) will be presented below.
(1) The compound, wherein A is H, or a nitrogen-containing aromatic 6-membered hetero ring, a nitrogen-containing unsaturated hetero ring having no aromatic property, or a nitrogen-containing saturated hetero ring, which may each be substituted.
(2) The compound, wherein A is a nitrogen-containing aromatic 6-membered hetero ring which may be substituted.
(3) The compound, wherein A is a nitrogen-containing aromatic 6-membered hetero ring which may be substituted with a group of lower alkyl, a monocyclic saturated hetero ring which may be substituted with oxo (=O), -CO-monocyclic saturated hetero ring (wherein the monocyclic saturated hetero ring may be substituted with a group selected from lower alkyl, -OH, -O-lower alkyl), -SO₂-cycloalkyl, and -SO₂-monocyclic saturated hetero ring.
(4) The compound, wherein A is pyridin-2-yl, pyrazin-2-yl, pyrimidin-4-yl, 5-methylpyrazin-2-yl, 2-methylpyrimidin-4-yl, 5-(2-oxopyrrolidin-1-yl)pyridin-2-yl, 5-methoxymethylpyridin-2-yl, 5-fluoropyridin-2-yl, 5-(trifluoromethyl)pyridin-2-yl, 5-(2-oxopiperidin-1-yl)pyridin-2-yl, 5-(4-methyl-2-oxopiperazin-1-yl)pyridin-2-yl, 5-(4-methyl-7-oxo-1,4-diazepan-1-yl)pyridin-2-yl, 5-(2-oxoazepan-1-yl)pyridin-2-yl, 5-(3-oxomorpholin-4-yl)pyridin-2-yl, 5-(3-oxo-2-azabicyclo[2,2,1]hept-2-yl)pyridin-2-yl, 5-(5-oxo-1,4-oxazepan-4-yl)pyridin-2-yl, 5-[(4-methylpiperazin-1-yl)carbonyl]pyridin-2-yl, 5-[(4-methyl-1,4-diazepan-1-yl)carbonyl]pyridin-2-yl, 5-(pyrrolidin-1-ylcarbonyl)pyridin-2-yl, 5-(3,3,4-trimethyl-2,5-dioxoimidazolidin-1-yl)pyridin-2-yl, or 5-[(4-methoxypiperidin-1-yl)carbonyl]pyridin-2-yl.
(5) The compound, wherein D is 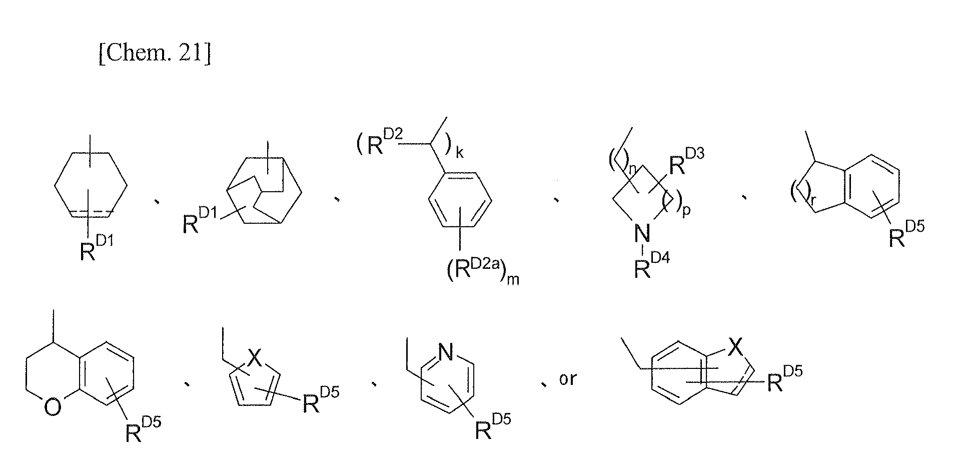 (wherein
   R^{D1} represents H, halogen, lower alkyl, OH, or CN; or -O-lower alkyl or a nitrogen-containing hetero ring, which may each be substituted,
   R^{D2} may be the same as or different from each other and represent H or lower alkyl,
   R^{D2a} may be the same as or different from each other and represent H, halogen, OH, -S-lower alkyl, -S(=O)₂-lower alkyl, -N(lower alkyl) -S(=O)₂-lower alkyl, or CN; or lower alkyl or -O-lower alkyl, which may each be substituted,
   k represents 1 or 2,
   m represents 1, 2, or 3,
   R^{D3} represents H, halogen, or lower alkyl which may be substituted,
   R^{D4} represents H, CN, or protected carboxy; or lower alkyl, aryl, a nitrogen-containing hetero ring, -C(=O)-lower alkyl, or -S(=O)₂-aryl, which may each be substituted,
   n represents 0,
   p represents 1, 2, 3, or 4,
   X represents NH, N-lower alkyl, or S,
   R^{D5} represents H, halogen, -N(lower alkyl)₂, or lower alkyl which may be substituted, and
   r represents 1 or 2.
   Further, the broken line portion in the formula above together with an adjacent bond represents a single bond or a double bond).
(6) The compound, wherein D is 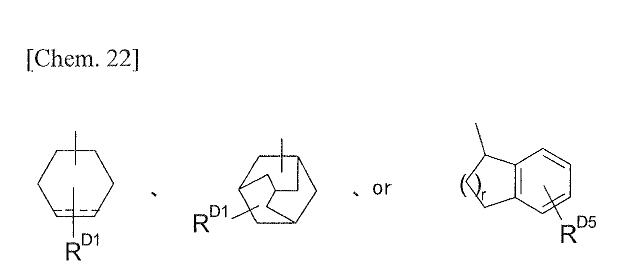
   R^{D1} represents H, halogen, lower alkyl, OH, or CN; or -O-lower alkyl or a nitrogen-containing hetero ring, which may each be substituted,
   R^{D5} represents H, halogen, -N(lower alkyl)₂, or lower alkyl which may be substituted, and
   r represents 1 or 2.
   Further, the broken line portion in the formula above together with an adjacent bond represents a single bond or a double bond).
(7) The compound, wherein D is 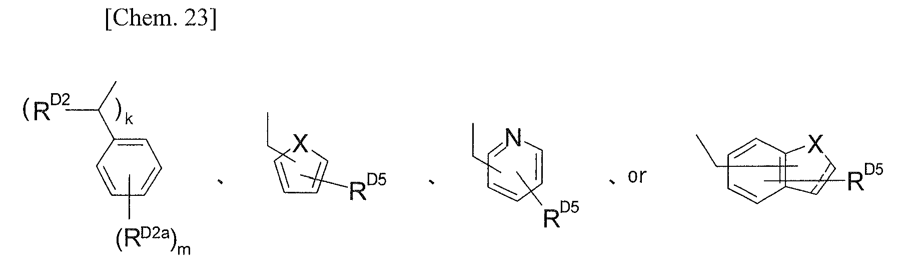
   R^{D2} may be the same as or different from each other and represent H, or lower alkyl,
   R^{D2a} may be the same as or different from each other and represent H, halogen, OH, -S-lower alkyl, -S(=O)₂-lower alkyl, -N(lower alkyl) -S(=O)₂-lower alkyl, or CN; or lower alkyl or -O-lower alkyl, which may each be substituted,
   k represents 1 or 2,
   m represents 1, 2, or 3,
   X represents NH, N-lower alkyl, or S, and
   R^{D5} represents H, halogen, -N(lower alkyl)₂, or lower alkyl which may be substituted.
   Further, the broken line portion in the formula above together with an adjacent bond represents a single bond or a double bond).
(8) The compound, wherein D is 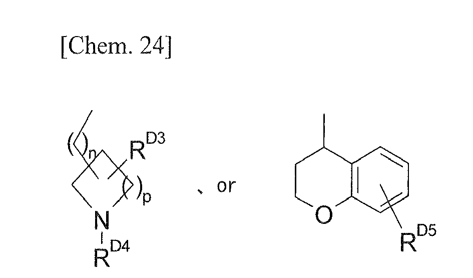
   R^{D3} represents H, halogen, or lower alkyl which may be substituted,
   R^{D4} represents H, CN, or protected carboxy; or lower alkyl, aryl, a nitrogen-containing hetero ring, -C(=O)-lower alkyl or -S(=O)₂-aryl, which may each be substituted,
   n represents 0,
   p represents 1, 2, 3, or 4, and
   R^{D5} represents H, halogen, -N(lower alkyl)₂, or lower alkyl which may be substituted).
(9) The compound, wherein D is 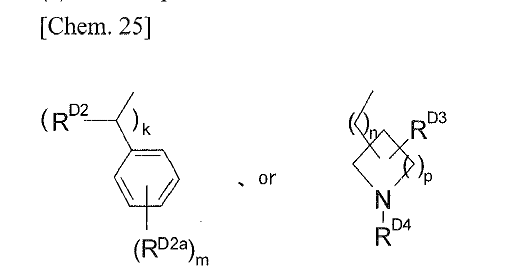 (wherein
   R^{D2} may be the same as or different from each other and represent H or lower alkyl,
   R^{D2a} may be the same as or different from each other and represent H, halogen, OH, -S-lower alkyl, -S(=O)₂-lower alkyl, -N(lower alkyl) -S(=O)₂-lower alkyl, or CN; or lower alkyl or -O-lower alkyl, which may each be substituted,
   k represents 1 or 2,
   m represents 1, 2, or 3,
   R^{D3} represents H, halogen, or lower alkyl which may be substituted,
   R^{D4} represents H, CN, or protected carboxy; or lower alkyl, aryl, a nitrogen-containing hetero ring, -C(=O)-lower alkyl, or -S(=O)₂-aryl, which may each be substituted,
   n represents 0, and
   p represents 1, 2, 3, or 4).
(10) The compound, wherein D is 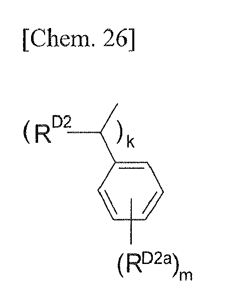
   (wherein R^{D2} may be the same as or different from each other and represent H, or lower alkyl,
   R^{D2a} may be the same as or different from each other and represent H, halogen, OH, -S-lower alkyl, -S(=O)₂-lower alkyl, -N(lower alkyl) -S(=O)₂-lower alkyl, or CN; or lower alkyl or -O-lower alkyl, which may each be substituted,
   k represents 1 or 2, and
   m represents 1, 2, or 3).
(11) The compound, wherein D is 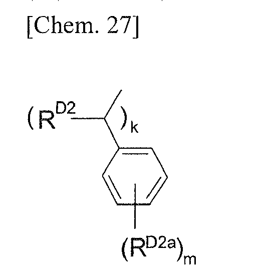 (wherein R^{D2} represents H,
   R^{D2a} may be the same as or different from each other and represent H, halogen, -S-lower alkyl, -S(=O)₂-lower alkyl, -N(lower alkyl) -S(=O)₂-lower alkyl, or CN; or lower alkyl or -O-lower alkyl, which may each be substituted with halogen,
   k represents 1, and
   m represents 1, 2, or 3).
(12) The compound, wherein D is 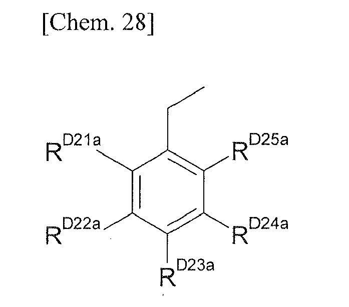 (wherein R^{D21a}, R^{D22a}, R^{D23a}, R^{D24a}, and R^{D25a} may be the same as or different from each other and represent H, F, CN, methoxy, trifluoromethoxy, methylthio, methylsulfonyl, methyl(methylsulfonyl)amino, or pyrazol-1-yl).
(13) The compound, wherein D is 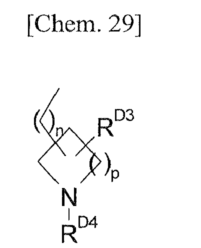 (wherein R^{D3} represents H, halogen, or lower alkyl which may be substituted,
   R^{D4} represents H, CN, or protected carboxy; or lower alkyl, aryl, a nitrogen-containing hetero ring, -C(=O)-lower alkyl, or -S(=O)₂-aryl, which may each be substituted,
   n represents 0, and
   p represents 1, 2, 3, or 4).
(14) The compound, wherein D is 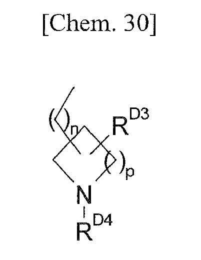
   (wherein R^{D3} represents H, halogen, or lower alkyl which may be substituted with OH,
   R^{D4} represents H, CN, or protected carboxy; or aryl or a nitrogen-containing hetero ring, which may each be substituted with cyano, -O-benzyl, OH, or F,
   n represents 0, and
   p represents 1, 2, 3, or 4).
(15) The compound, wherein D is 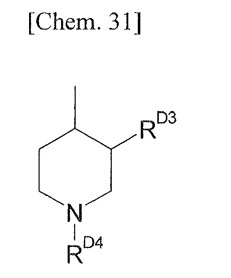
   (wherein R^{D3} represents H, F, methoxy, methyl, or hydroxymethyl,
   R^{D4} represents 4-cyanophenyl, 4-benzyloxyphenyl, 4-hydroxyphenyl, 4-fluorophenyl, 5-[(cyanomethyl)carbamoyl]pyridin-2-yl, 5-[(cyanomethyl)(methyl)carbamoyl]pyridin-2-yl, 5-cyanopyridin-2-yl, 6-cyanopyridin-3-yl, 6-cyanopyridazin-3-yl, 4-cyanomethoxyphenyl, or 5-cyanopyrazin-2-yl).
(16) The compound, wherein D is 2,6-difluorobenzyl or 2,3,6-trifluorobenzyl.
(17) The compound, wherein D is 1-(5-cyanopyridin-2-yl)piperidin-4-yl, 1-(6-cyanopyridin-3-yl)piperidin-4-yl, 1-(6-cyanopyridazin-3-yl)piperidin-4-yl, 1-(5-cyanopyridin-2-yl)-3-methylpiperidin-4-yl, 1-(5-cyanopyridin-2-yl)-3-fluoropiperidin-4-yl, 1-(5-cyanopyridin-2-yl)-3-(hydroxymethyl)piperidin-4-yl, 1-(5-cyanopyridin-2-yl)-3-methoxypiperidin-4-yl, 1-(6-cyanopyridazin-3-yl)-3-fluoropiperidin-4-yl, 1-(5-cyanopyrazin-2-yl)-3-fluoropiperidin-4-yl, 1-(4-cyanophenyl)piperidin-4-yl, 1-(4-fluorophenyl)piperidin-4-yl, 1-(4-hydroxyphenyl)piperidin-4-yl, 1-(4-benzyloxyphenyl)piperidin-4-yl, 1-(4-cyanomethoxyphenyl)piperidin-4-yl, 1-{5-[(cyanomethyl)carbamoyl]pyridin-2-yl}piperidin-4-yl, or 1-{5-[(cyanomethyl)(methyl)carbamoyl]pyridin-2-yl}piperidin-4-yl.
   Furthermore, in another embodiment, the compounds formed from the combinations of two or more groups of the groups described in (1) to (17) above are preferred, and specific examples thereof include the following compounds.
(18) A compound of the formula (Ia): 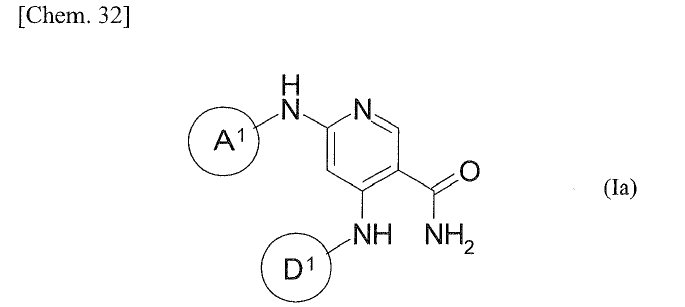 (wherein
   A¹ represents H, or a nitrogen-containing aromatic 6-membered hetero ring, a nitrogen-containing unsaturated hetero ring having no aromatic property, or a nitrogen-containing saturated hetero ring, which may each be substituted,
   D¹ represents the following group: 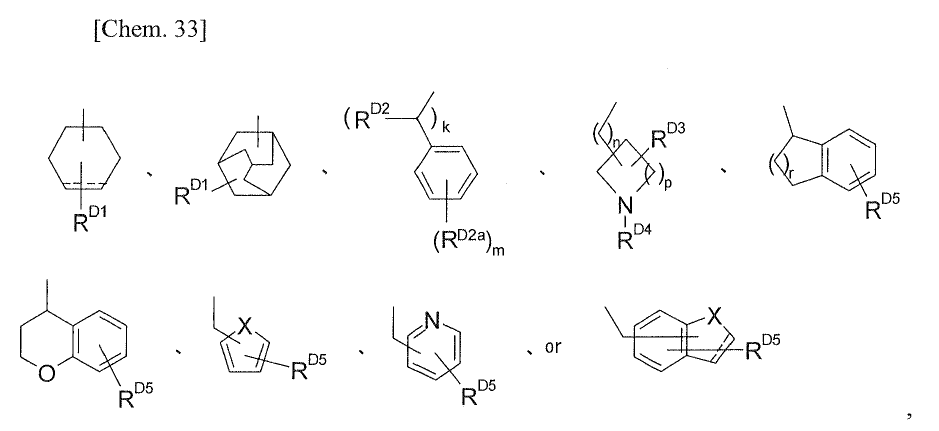
   R^{D1} represents H, halogen, lower alkyl, OH, or CN; or -O-lower alkyl or a nitrogen-containing hetero ring, which may each be substituted,
   R^{D2} may be the same as or different from each other and represent H or lower alkyl,
   R^{D2a} may be the same as or different from each other and represent H, halogen, OH, -S-lower alkyl, -S(=O)₂-lower alkyl, -N(lower alkyl) -S(=O)₂-lower alkyl, or CN; or lower alkyl or -O-lower alkyl, which may each be substituted,
   k represents 1 or 2,
   m represents 1, 2, or 3,
   R^{D3} represents H, halogen, or lower alkyl which may be substituted,
   R^{D4} represents H, CN, or protected carboxy; or lower alkyl, aryl, a nitrogen-containing hetero ring, -C(=O)-lower alkyl, or -S(=O)₂-aryl, which may each be substituted,
   n represents 0,
   p represents 1, 2, 3, or 4,
   X represents NH, N-lower alkyl, or S,
   R^{D5} represents H, halogen, -N(lower alkyl)₂, or lower alkyl which may be substituted, and
   r represents 1 or 2.
   Further, the broken line portion in the formula above together with an adjacent bond represents a single bond or a double bond).
(19) A compound of the formula (Ib): 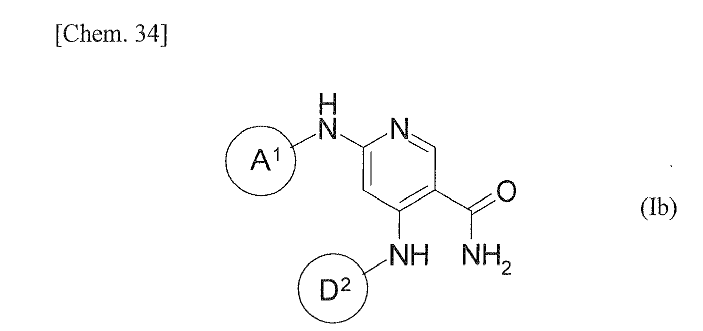 (wherein
   A¹ represents H, or a nitrogen-containing aromatic 6-membered hetero ring, a nitrogen-containing unsaturated hetero ring having no aromatic property, or a nitrogen-containing saturated hetero ring, which may each be substituted,
   D² represents the following group: 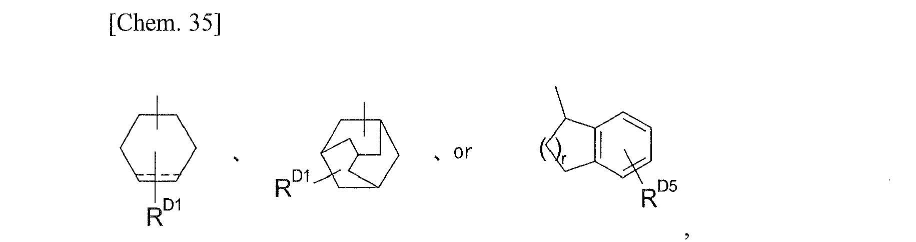
   R^{D1} represents H, halogen, lower alkyl, OH, or CN; or -O-lower alkyl or a nitrogen-containing hetero ring, which may each be substituted,
   R^{D5} represents H, halogen, -N(lower alkyl)₂, or lower alkyl which may be substituted, and
   r represents 1 or 2.
   Further, the broken line portion in the formula above together with an adjacent bond represents a single bond or a double bond).
(20) A compound of the formula (Ic): 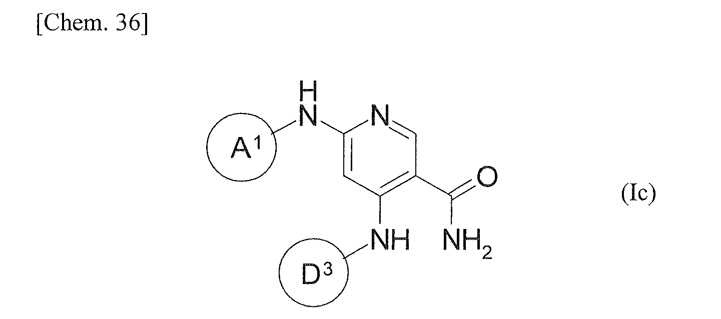 (wherein
   A¹ represents H, or a nitrogen-containing aromatic 6-membered hetero ring, a nitrogen-containing unsaturated hetero ring having no aromatic property, or a nitrogen-cotaining saturated hetero ring, which may each be substituted,
   D³ represents the following group: 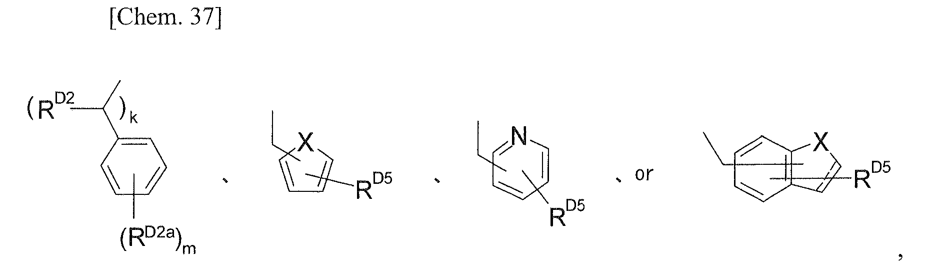
   R^{D2} may be the same as or different from each other and represent H or lower alkyl,
   R^{D2a} may be the same as or different from each other and represent H, halogen, OH, -S-lower alkyl, -S(=O)₂-lower alkyl, -N(lower alkyl) -S(=O)₂-lower alkyl, or CN; or lower alkyl, or -O-lower alkyl, which may each be substituted,
   k represents 1 or 2,
   m represents 1, 2, or 3,
   X represents NH, N-lower alkyl, or S, and
   R^{D5} represents H, halogen, -N(lower alkyl)₂, or lower alkyl which may be substituted.
   Further, the broken line portion in the formula above with an adjacent bond represents a single bond or a double bond together).
(21) A compound of the formula (Id): 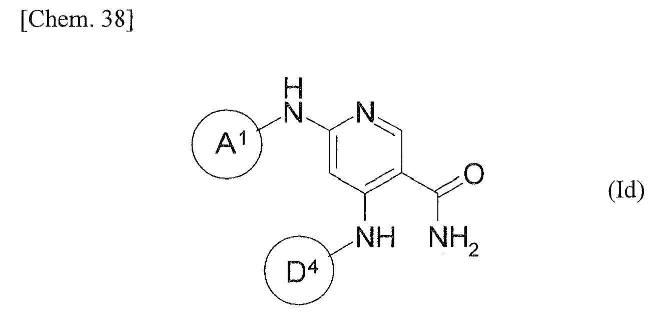 (wherein
   A¹ represents H, or a nitrogen-containing aromatic 6-membered hetero ring, a nitrogen-containing unsaturated hetero ring having no aromatic property, or a nitrogen-containing saturated hetero ring, which may each be substituted,
   D⁴ represents the following group: 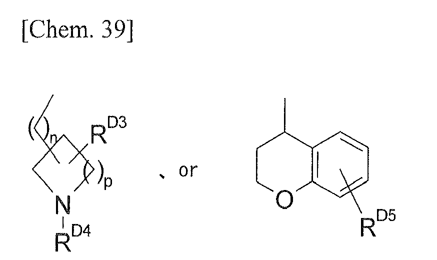
   R^{D3} represents H, halogen, or lower alkyl which may be substituted,
   R^{D4} represents H, CN, or protected carboxy; or lower alkyl, aryl, a nitrogen-containing hetero ring, -C(=O)-lower alkyl, or -S(=O)₂-aryl, which may each be substituted,
   n represents 0,
   p represents 1, 2, 3, or 4, and
   R^{D5} represents H, halogen, -N(lower alkyl)₂, or lower alkyl which may be substituted).
(22) The compound as described in (18), wherein D¹ is: 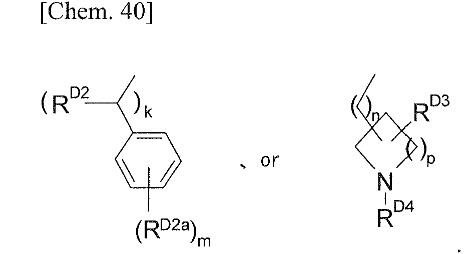
(23) The compound as described in (22), wherein A¹ is a nitrogen-containing aromatic 6-membered hetero ring which may be substituted.
(24) The compound as described in (23), wherein D¹ is: 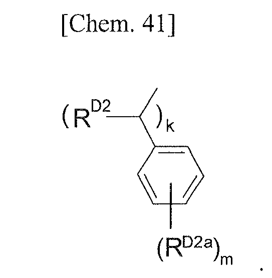
(25) The compound as described in (24), wherein R^{D2} is H,
   R^{D2a} may be the same as or different from each other and are H, halogen, -S-lower alkyl, -S(=O)₂-lower alkyl, -N(lower alkyl) -S(=O)₂-lower alkyl, or CN; or lower alkyl or -O-lower alkyl, which may be each substituted with halogen,
   k is 1, and
   m is 1, 2, or 3.
(26) The compound as described in (23), wherein D¹ is: 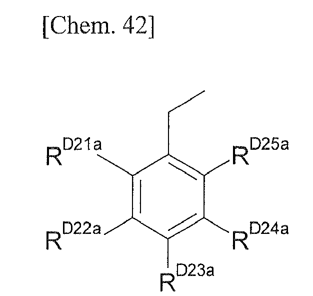 (wherein R^{D21a}, R^{D22a}, R^{D23a}, R^{D24a}, and R^{D25a} may be the same as or different from each other and represent H, F, CN, methoxy, trifluoromethoxy, methylthio, methylsulfonyl, methyl(methylsulfonyl)amino, or pyrazol-1-yl).
(27) The compound as described in (23), wherein D¹ is 2,6-difluorobenzyl or 2,3,6-trifluorobenzyl.
(28) The compound as described in (23), wherein D¹ is: 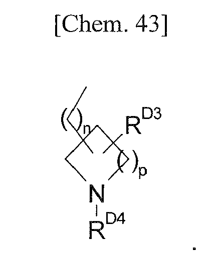
(29) The compound as described in (28), wherein R^{D3} is H, halogen, or lower alkyl which may be substituted with OH,
   R^{D4} is H, CN, or protected carboxy; or aryl or a nitrogen-containing hetero ring, which may each be substituted with cyano, -O-benzyl, OH, or F,
   n is 0, and
   p is 1, 2, 3, or 4.
(30) The compound as described in (23), wherein D¹ is: 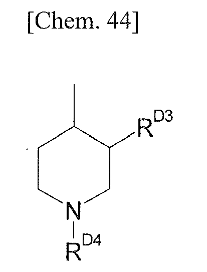
   (wherein R^{D3} represents H, F, methoxy, methyl, or hydroxymethyl, and
   R^{D4} represents 4-cyanophenyl, 4-benzyloxyphenyl, 4-hydroxyphenyl, 4-fluorophenyl, 5-[(cyanomethyl)carbamoyl]pyridin-2-yl, 5-[(cyanomethyl)(methyl)carbamoyl]pyridin-2-yl, 5-cyanopyridin-2-yl, 6-cyanopyridin-3-yl, 6-cyanopyridazin-3-yl, 4-cyanomethoxyphenyl, or 5-cyanopyrazin-2-yl).
(31) The compound as described in (23), wherein D¹ is 1-(5-cyanopyridin-2-yl)piperidin-4-yl, 1-(6-cyanopyridin-3-yl)piperidin-4-yl, 1-(6-cyanopyridazin-3-yl)piperidin-4-yl, 1-(5-cyanopyridin-2-yl)-3-methylpiperidin-4-yl, 1-(5-cyanopyridin-2-yl)-3-fluoropiperidin-4-yl, 1-(5-cyanopyridin-2-yl)-3-(hydroxymethyl)piperidin-4-yl, 1-(5-cyanopyridin-2-yl)-3-methoxypiperidin-4-yl, 1-(6-cyanopyridazin-3-yl)-3-fluoropiperidin-4-yl, 1-(5-cyanopyrazin-2-yl)-3-fluoropiperidin-4-yl, 1-(4-cyanophenyl)piperidin-4-yl, 1-(4-fluorophenyl)piperidin-4-yl, 1-(4-hydroxyphenyl)piperidin-4-yl, 1-(4-benzyloxyphenyl)piperidin-4-yl, 1-(4-cyanomethoxyphenyl)piperidin-4-yl, 1-{5-[(cyanomethyl)carbamoyl]pyridin-2-yl}piperidin-4-yl, or 1-{5-[(cyanomethyl)(methyl)carbamoyl]pyridin-2-yl}piperidin-4-yl.
(32) The compound as described in (22), wherein A¹ is pyridin-2-yl, pyrazin-2-yl, pyrimidin-4-yl, 5-methylpyrazin-2-yl, 2-methylpyrimidin-4-yl, 5-(2-oxopyrrolidin-1-yl)pyridin-2-yl, 5-methoxymethylpyridin-2-yl, 5-fluoropyridin-2-yl, 5-(trifluoromethyl)pyridin-2-yl, 5-(2-oxopiperidin-1-yl)pyridin-2-yl, 5-(4-methyl-2-oxopiperazin-1-yl)pyridin-2-yl, 5-(4-methyl-7-oxo-1,4-diazepan-1-yl)pyridin-2-yl, 5-(2-oxoazepan-1-yl)pyridin-2-yl, 5-(3-oxomorpholin-4-yl)pyridin-2-yl, 5-(3-oxo-2-azabicyclo[2,2,1]hept-2-yl)pyridin-2-yl, 5-(5-oxo-1,4-oxazepan-4-yl)pyridin-2-yl, 5-[(4-methylpiperazin-1-yl)carbonyl]pyridin-2-yl, 5-[(4-methyl-1,4-diazepan-1-yl)carbonyl]pyridin-2-yl, 5-(pyrrolidin-1-ylcarbonyl)pyridin-2-yl, 5-(3,3,4-trimethyl-2,5-dioxoimidazolidin-1-yl)pyridin-2-yl, or 5-[(4-methoxypiperidin-1-yl)carbonyl]pyridin-2-yl.
(33) The compound as described in (32), wherein D¹ is: 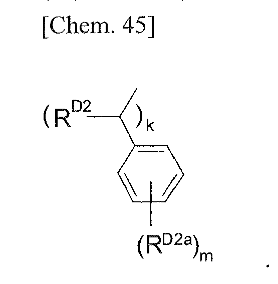
(34) The compound as described in (33), wherein
   R^{D2} is H,
   R^{D2a} may be the same as or different from each other and represent H, halogen, -S-lower alkyl, -S(=O)₂-lower alkyl, -N(lower alkyl) -S(=O)₂-lower alkyl, or CN; or lower alkyl or -O-lower alkyl, which may each be substituted with halogen,
   k is 1, and
   m is 1, 2, or 3.
(35) The compound as described in (32), wherein D¹ is: 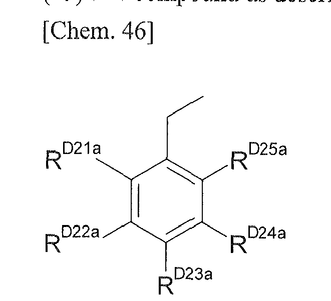 (wherein R^{D21a}, R^{D22a}, R^{D23a}, R^{D24a}, and R^{D25a} may be the same as or different from each other and represent H, F, CN, methoxy, trifluoromethoxy, methylthio, methylsulfonyl, methyl(methylsulfonyl)amino, or pyrazol-1-yl).
(36) The compound as described in (32), wherein D¹ is 2,6-difluorobenzyl or 2,3,6-trifluorobenzyl.
(37) The compound as described in (32), wherein D¹ is 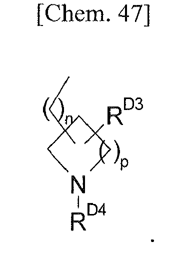
(38) The compound as described in (37), wherein
   R^{D3} is H, halogen, or lower alkyl which may be substituted with OH,
   R^{D4} is H, CN, or protected carboxy; or aryl or a nitrogen-containing hetero ring, which may each be substituted with cyano, -O-benzyl, OH, or F,
   n is 0, and
   pis 1, 2, 3, or 4.
(39) The compound as described in (32), wherein D¹ is: 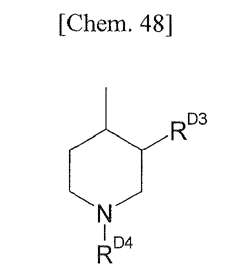
   (wherein R^{D3} represents H, F, methoxy, methyl, or hydroxymethyl, and
   R^{D4} represents 4-cyanophenyl, 4-benzyloxyphenyl, 4-hydroxyphenyl, 4-fluorophenyl, 5-[(cyanomethyl)carbamoyl]pyridin-2-yl, 5-[(cyanomethyl)(methyl)carbamoyl]pyridin-2-yl, 5-cyanopyridin-2-yl, 6-cyanopyridin-3-yl, 6-cyanopyridazin-3-yl, 4-cyanomethoxyphenyl, or 5-cyanopyrazin-2-yl).
(40) The compound as described in (32), wherein D¹ is 1-(5-cyanopyridin-2-yl)piperidin-4-yl, 1-(6-cyanopyridin-3-yl)piperidin-4-yl, 1-(6-cyanopyridazin-3-yl)piperidin-4-yl, 1-(5-cyanopyridin-2-yl)-3-methylpiperidin-4-yl, 1-(5-cyanopyridin-2-yl)-3-fluoropiperidin-4-yl, 1-(5-cyanopyridin-2-yl)-3-(hydroxymethyl)piperidin-4-yl, 1-(5-cyanopyridin-2-yl)-3-methoxypiperidin-4-yl, 1-(6-cyanopyridazin-3-yl)-3-fluoropiperidin-4-yl, 1-(5-cyanopyrazin-2-yl)-3-fluoropiperidin-4-yl, 1-(4-cyanophenyl)piperidin-4-yl, 1-(4-fluorophenyl)piperidin-4-yl, 1-(4-hydroxyphenyl)piperidin-4-yl, 1-(4-benzyloxyphenyl)piperidin-4-yl, 1-(4-cyanomethoxyphenyl)piperidin-4-yl, 1-{5-[(cyanomethyl)carbamoyl]pyridin-2-yl}piperidin-4-yl, or 1-{5-[(cyanomethyl)(methyl)carbamoyl]pyridin-2-yl}piperidin-4-yl.
(41) The compound (Ia), wherein A¹ is pyridin-2-yl and D¹ is 1-(5-cyanopyridin-2-yl)piperidin-4-yl.
(42) The compound (Ia), wherein A¹ is pyrazin-2-yl and D¹ is 1-(5-cyanopyridin-2-yl)piperidin-4-yl.
(43) The compound (Ia), wherein A¹ is pyrimidin-4-yl and D¹ is 1-(5-cyanopyridin-2-yl)piperidin-4-yl.
(44) The compound (Ia), wherein A¹ is pyrazin-2-yl and D¹ is 1-(5-cyanopyridin-2-yl)-3-fluoropiperidin-4-yl.
(45) The compound (Ia), wherein A¹ is 2-methylpyrimidin-4-yl and D¹ is 1-(5-cyanopyridin-2-yl)piperidin-4-yl.
(46) The compound (Ia), wherein A¹ is pyrimidin-4-yl and D¹ is 1-(5-cyanopyridin-2-yl)-3-fluoropiperidin-4-yl.
(47) The compound (Ia), wherein A¹ is pyrazin-2-yl and D¹ is 1-(6-cyanopyridazin-3-yl)-3-fluoropiperidin-4-yl.
(48) The compound (Ia), wherein A¹ is pyrazin-2-yl and D¹ is 1-(6-cyanopyridazin-3-yl)piperidin-4-yl.
(49) The compound (Ia), wherein A¹ is pyrimidin-4-yl and D¹ is 1-(5-cyanopyrazin-2-yl)-3 -fluoropiperidin-4-yl.
(50) The compound (Ia), wherein A¹ is pyrimidin-4-yl and D¹ is 1-(6-cyanopyridazin-3-yl)piperidin-4-yl.
(51) The compound (Ia), wherein A¹ is 5-methylpyrazin-2-yl and D¹ is 1-(6-cyanopyridazin-3-yl)piperidin-4-yl.
(52) The compound (Ia), wherein A¹ is 5-methylpyrazin-2-yl and D¹ is 1-(5-cyanopyridin-2-yl)piperidin-4-yl.
(53) The compound (Ia), wherein A¹ is pyrazin-2-yl and D¹ is 1-(4-benzyloxyphenyl)piperidin-4-yl.
(54) The compound (Ia), wherein A¹ is pyrazin-2-yl and D¹ is 1-(4-hydroxyphenyl)piperidin-4-yl.
(55) The compound (Ia), wherein A¹ is pyrazin-2-yl and D¹ is 1-(4-fluorophenyl)piperidin-4-yl.
(56) The compound (Ia), wherein A¹ is pyrimidin-4-yl and D¹ is 1-(6-cyanopyridin-3-yl)piperidin-4-yl.
(57) The compound (Ia), wherein A¹ is 2-methylpyrimidin-4-yl and D¹ is 1-(5-cyanopyridin-2-yl)-3-fluoropiperidin-4-yl.
(58) The compound (Ia), wherein A¹ is 2-methylpyrimidin-4-yl and D¹ is 1-(6-cyanopyridazin-3-yl)-3-fluoropiperidin-4-yl.
(59) The compound (Ia), wherein A¹ is pyrazin-2-yl and D¹ is 1-(4-cyanomethoxyphenyl)piperidin-4-yl.
(60) The compound (Ia), wherein A¹ is pyrazin-2-yl and D¹ is 1-[(4-cyanomethoxy)phenyl]piperidin-4-yl.
(61) The compound (Ia), wherein A¹ is pyrazin-2-yl and D¹ is 1-{5-[(cyanomethyl)carbamoyl]pyridin-2-yl}piperidin-4-yl.
(62) The compound (Ia), wherein A¹ is pyridin-2-yl and D¹ is 1-(5-cyanopyridin-2-yl)-3-(hydroxymethyl)piperidin-4-yl.
(63) The compound (Ia), wherein A¹ is 5-(2-oxopyrrolidin-1-yl)pyridin-2-yl and D¹ is 1-(5-cyanopyridin-2-yl)piperidin-4-yl.
(64) The compound (Ia), wherein A¹ is pyridin-2-yl and D¹ is 1-(5-cyanopyridin-2-yl)-3-(hydroxymethyl)piperidin-4-yl.
(65) The compound (Ia), wherein A¹ is 5-methoxymethylpyridin-2-yl and D¹ is 1-(5-cyanopyridin-2-yl)-3-(hydroxymethyl)piperidin-4-yl.
(66) The compound (Ia), wherein A¹ is 5-fluoropyridin-2-yl and D¹ is 1-(5-cyanopyridin-2-yl)-3-(hydroxymethyl)piperidin-4-yl.
(67) The compound (Ia), wherein A¹ is 5-(trifluoromethyl)pyridin-2-yl and D¹ is 1-(5-cyanopyridin-2-yl)-3-(hydroxymethyl)piperidin-4-yl.
(68) The compound (Ia), wherein A¹ is 5-(2-oxopiperidin-1-yl)pyridin-2-yl and D¹ is 1-(5-cyanopyridin-2-yl)-3-fluoropiperidin-4-yl.
(69) The compound (Ia), wherein A¹ is pyrazin-2-yl and D¹ is 1-{5-[(cyanomethyl)(methyl)carbamoyl]pyridin-2-yl}piperidin-4-yl.
(70) The compound (Ia), wherein A¹ is 5-(2-oxoazepan-1-yl)pyridin-2-yl and D¹ is 1-(5-cyanopyridin-2-yl)-3-fluoropiperidin-4-yl.
(71) The compound (Ia), wherein A¹ is 5-(2-oxopiperidin-1-yl)pyridin-2-yl and D¹ is 1-(5-cyanopyridin-2-yl)-3-methylpiperidin-4-yl.
(72) The compound (Ia), wherein A¹ is 5-(3-oxo-2-azabicyclo[2.2.1]hept-2-yl)pyridin-2-yl and D¹ is 1-(5-cyanopyridin-2-yl)piperidin-4-yl.
(73) The compound (Ia), wherein A¹ is 5-methoxymethylpyridin-2-yl and D¹ is 1-(4-cyanophenyl)piperidin-4-yl.
(74) The compound (Ia), wherein A¹ is pyrazin-2-yl and D¹ is 1-(4-cyanophenyl)piperidin-4-yl.
(75) The compound (Ia), wherein A¹ is pyrazin-2-yl and D¹ is 1-(5-cyanopyridin-2-yl)-3-(hydroxymethyl)piperidin-4-yl.
(76) The compound (Ia), wherein A¹ is 2-methylpyrimidin-4-yl and D¹ is 1-(4-cyanophenyl)piperidin-4-yl.
(77) The compound (Ia), wherein A¹ is pyrazin-2-yl and D¹ is 2,3,6-trifluorobenzyl.
(78) The compound (Ia), wherein A¹ is 2-methylpyrimidin-4-yl and D¹ is 2,3,6-trifluorobenzyl.
(79) The compound (Ia), wherein A¹ is 5-(2-oxopiperidin-1-yl)pyridin-2-yl and D¹ is 2,3,6-trifluorobenzyl.
(80) The compound (Ia), wherein A¹ is 5-(4-methyl-2-oxopiperazin-1-yl)pyridin-2-yl and D¹ is 2,3,6-trifluorobenzyl.
(81) The compound (Ia), wherein A¹ is 5-(4-methyl-7-oxo-1,4-diazepan-1-yl)pyridin-2-yl and D¹ is 2,3,6-trifluorobenzyl.
(82) The compound (Ia), wherein A¹ is 5-(3-oxomorpholin-4-yl)pyridin-2-yl and D¹ is 2,3,6-trifluorobenzyl.
(83) The compound (Ia), wherein A¹ is 5-(3-oxo-2-azabicyclo[2.2.1]hept-2-yl)pyridin-2-yl and D¹ is 2,3,6-trifluorobenzyl.
(84) The compound (Ia), wherein A¹ is 5-(2-oxoazepan-1-yl)pyridin-2-yl and D¹ is 2,3,6-trifluorobenzyl.
(85) The compound (Ia), wherein A¹ is 5-(3-oxo-2-azabicyclo[2.2.1]hept-2-yl)pyridin-2-yl and D¹ is 2,3,6-trifluorobenzyl.
(86) The compound (Ia), wherein A¹ is 5-(5-oxo-1,4-oxazepan-4-yl)pyridin-2-yl and D¹ is 2,3,6-trifluorobenzyl.
(87) The compound (Ia), wherein A¹ is 5-(3,3,4-trimethyl-2,5-dioxoimidazolidin-1-yl)pyridin-2-yl and D¹ is 2,3,6-trifluorobenzyl.
(88) The compound (Ia), wherein A¹ is 5-(pyrrolidin-1-ylcarbonyl)pyridin-2-yl and D¹ is 2,6-difluorobenzyl.
(89) The compound (Ia), wherein A¹ is 5-[(4-methoxypiperidin-1-yl)carbonyl]pyridin-2-yl and D¹ is 2,6-difluorobenzyl.
(90) The compound (Ia), wherein A¹ is 5-[(4-methylpiperazin-1-yl)carbonyl]pyridin-2-yl and D¹ is 2,6-difluorobenzyl.
(91) The compound (Ia), wherein A¹ is 5-[(4-methyl-1,4-diazepan-1-yl)carbonyl]pyridin-2-yl and D¹ is 2,6-difluorobenzyl.
(92) The compound (Ia), wherein A¹ is 5-(4-methyl-7-oxo-1,4-diazepan-1-yl)pyridin-2-yl and D¹ is 2,6-difluorobenzyl.
   Further embodiments of the compound of the present invention are presented below.
(93) The compound as described in (23) to (27), wherein A¹ is a nitrogen-containing aromatic 6-membered hetero ring which may be substituted with a group selected from lower alkyl, a monocyclic saturated hetero ring which may be substituted with oxo (=O), -CO-monocyclic saturated hetero ring (wherein the monocyclic saturated hetero ring may be substituted with -OH, oxo (=O), lower alkyl, -O-lower alkyl, -C(=O)-lower alkyl, NH₂, NH(lower alkyl), N(lower alkyl)₂, cycloalkyl, aryl, a hetero ring (wherein the hetero ring may be substituted with oxo (=O) or a hetero ring) or -NH-S(=O)₂-lower alkyl), -SO₂-cycloalkyl, and -SO₂-monocyclic saturated hetero ring.
(94) The compound as described in (23) to (27), wherein A¹ is a nitrogen-containing aromatic 6-membered hetero ring which may be substituted with a group selected from -CO-monocyclic saturated hetero ring (wherein the monocyclic saturated hetero ring may be substituted with -OH, oxo (=O), or lower alkyl (wherein the lower alkyl may be substituted with OH), -O-lower alkyl, -C(=O)-lower alkyl, NH₂, NH(lower alkyl), N(lower alkyl)₂, cycloalkyl, aryl, hetero ring (wherein the hetero ring may be substituted with oxo (=O) or hetero ring), or -NH-S(=O)₂-lower alkyl) and -SO₂-monocyclic saturated hetero ring.
(95) The compound as described in (23) to (27), wherein A¹ is a nitrogen-containing aromatic 6-membered hetero ring which may be substituted, wherein the nitrogen-containing aromatic 6-membered hetero ring may be substituted with pyrrolidine, piperidine or piperazine, wherein the pyrrolidine may be substituted with N(lower alkyl)₂ or oxo (=O), the piperidine may be substituted with lower alkyl (wherein the lower alkyl may be substituted with OH), O-lower alkyl, a nitrogen-containing aromatic hetero ring (wherein the nitrogen-containing aromatic hetero ring may be substituted with a nitrogen-containing aromatic 6-membered hetero ring), or oxo (=O), and the piperazine may be substituted with lower alkyl.
(96) The compound as described in (23) to (27), wherein A¹ is pyridin-2-yl which may be substituted, the pyridin-2-yl may be substituted with pyrrolidine, piperidine, or piperazine, wherein the pyrrolidine may be substituted with dimethylamino or oxo (=O), the piperidine may be substituted with oxadiazoline which may be substituted with pyridine, or 2-hydroxyethyl, methoxy, or oxo (=O), and the piperazine may be substituted with methyl.
(97) The compound as described in (18), wherein A¹ is a nitrogen-containing aromatic 6-membered hetero ring which may be substituted, and
   D¹ is: 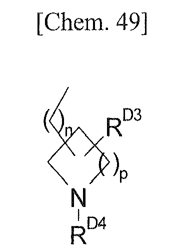
   (wherein R^{D3} represents H or halogen; or lower alkyl which may be substituted with OH,
   R^{D4} represents aryl or a nitrogen-containing hetero ring, which may each be substituted with cyano, -O-benzyl, OH, or F,
   n represents 0, and
   p represents 1, 2, 3, or 4).
(98) The compound as described in (97), wherein D¹ is: 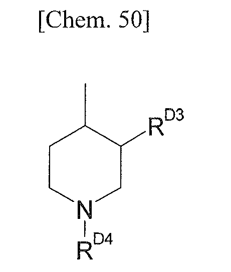
   (wherein R^{D3} represents H, F, methoxy, methyl, or hydroxymethyl, and
   R^{D4} represents aryl or a nitrogen-containing hetero ring, which may each be substituted with cyano, -O-benzyl, OH, or F).
(99) The compound as described in (98), wherein R^{D4} is 4-cyanophenyl, 4-benzyloxyphenyl, 4-hydroxyphenyl, 4-fluorophenyl, 5-[(cyanomethyl)carbamoyl]pyridin-2-yl, 5-[(cyanomethyl)(methyl)carbamoyl]pyridin-2-yl, 5-cyanopyridin-2-yl, 6-cyanopyridin-3-yl, 6-cyanopyridazin-3-yl, 4-cyanomethoxyphenyl, or 5-cyanopyrazin-2-yl).
(100) The compound as described in (98), wherein R^{D4} is 5-[(cyanomethyl)carbamoyl]pyridin-2-yl, 5-[(cyanomethyl)(methyl)carbamoyl]pyridin-2-yl, 5-cyanopyridin-2-yl, 6-cyanopyridin-3-yl, 6-cyanopyridazin-3-yl, or 5-cyanopyrazin-2-yl.
(101) The compound as described in (97), wherein D¹ is 1-(5-cyanopyridin-2-yl)piperidin-4-yl, 1-(6-cyanopyridin-3-yl)piperidin-4-yl, 1-(6-cyanopyridazin-3-yl)piperidin-4-yl, 1-(5-cyanopyridin-2-yl)-3-methylpiperidin-4-yl, 1-(5-cyanopyridin-2-yl)-3-fluoropiperidin-4-yl, 1-(5-cyanopyridin-2-yl)-3-(hydroxymethyl)piperidin-4-yl, 1-(5-cyanopyridin-2-yl)-3-methoxypiperidin-4-yl, 1-(6-cyanopyridazin-3-yl)-3-fluoropiperidin-4-yl, 1-(5-cyanopyrazin-2-yl)-3-fluoropiperidin-4-yl, 1-(4-cyanophenyl)piperidin-4-yl, 1-(4-fluorophenyl)piperidin-4-yl, 1-(4-hydroxyphenyl)piperidin-4-yl, 1-(4-benzyloxyphenyl)piperidin-4-yl, 1-(4-cyanomethoxyphenyl)piperidin-4-yl, 1-{5-[(cyanomethyl)carbamoyl]pyridin-2-yl}piperidin-4-yl, or 1-{5-[(cyanomethyl)(methyl)carbamoyl]pyridin-2-yl}piperidin-4-yl.
(102) The compound as described in (97), wherein D¹ is 1-(5-cyanopyridin-2-yl)piperidin-4-yl, 1-(6-cyanopyridin-3-yl)piperidin-4-yl, 1-(6-cyanopyridazin-3-yl)piperidin-4-yl, 1-(5-cyanopyridin-2-yl)-3-methylpiperidin-4-yl, 1-(5-cyanopyridin-2-yl)-3-fluoropiperidin-4-yl, 1-(5-cyanopyridin-2-yl)-3-(hydroxymethyl)piperidin-4-yl, 1-(5-cyanopyridin-2-yl)-3-methoxypiperidin-4-yl, 1-(6-cyanopyridazin-3-yl)-3-fluoropiperidin-4-yl, 1-(5-cyanopyrazin-2-yl)-3-fluoropiperidin-4-yl, 1-{5-[(cyanomethyl)carbamoyl]pyridin-2-yl}piperidin-4-yl, or 1-{5-[(cyanomethyl)(methyl)carbamoyl]pyridin-2-yl}piperidin-4-yl.
(103) The compound as described in (97) to (102), wherein A¹ is pyrazine which may be substituted.
(104) The compound as described in (97) to (102), wherein A¹ is a nitrogen-containing aromatic 6-membered hetero ring which may be substituted (provided that pyrazine is excluded).
(105) The compound as described in (97) to (102), wherein A¹ is pyrazin-2-yl or 5-methylpyrazin-2-yl.
(106) The compound as described in (97) to (102), wherein A¹ is pyridin-2-yl, pyrimidin-4-yl, 2-methylpyrimidin-4-yl, 5-(2-oxopyrrolidin-1-yl)pyridin-2-yl, 5-methoxymethylpyridin-2-yl, 5-fluoropyridin-2-yl, 5-(trifluoromethyl)pyridin-2-yl, 5-(2-oxopiperidin-1-yl)pyridin-2-yl, 5-(4-methyl-2-oxopiperazin-1-yl)pyridin-2-yl, 5-(4-methyl-7-oxo-1,4-diazepan-1-yl)pyridin-2-yl, 5-(2-oxoazepan-1-yl)pyridin-2-yl, 5-(3-oxomorpholin-4-yl)pyridin-2-yl, 5-(3-oxo-2-azabicyclo[2,2,1]hept-2-yl)pyridin-2-yl, 5-(5-oxo-1,4-oxazepan-4-yl)pyridin-2-yl, 5-[(4-methylpiperazin-1-yl)carbonyl]pyridin-2-yl, 5-[(4-methyl-1,4-diazepan-1-yl)carbonyl]pyridin-2-yl, 5-(pyrrolidin-1-ylcarbonyl)pyridin-2-yl, 5-(3,3,4-trimethyl-2,5-dioxoimidazolidin-1-yl)pyridin-2-yl, or 5-[(4-methoxypiperidin-1-yl)carbonyl]pyridin-2-yl.
(107) The compound as described in (97) to (102), wherein A¹ is pyridin-2-yl, 5-(2-oxopyrrolidin-1-yl)pyridin-2-yl, 5-methoxymethylpyridin-2-yl, 5-fluoropyridin-2-yl, 5-(trifluoromethyl)pyridin-2-yl, 5-(2-oxopiperidin-1-yl)pyridin-2-yl, 5-(4-methyl-2-oxopiperazin-1-yl)pyridin-2-yl, 5-(4-methyl-7-oxo-1,4-diazepan-l-yl)pyridin-2-yl, 5-(2-oxoazepan-1-yl)pyridin-2-yl, 5-(3-oxomorpholin-4-yl)pyridin-2-yl, 5-(3-oxo-2-azabicyclo[2,2,1]hept-2-yl)pyridin-2-yl, 5-(5-oxo-1,4-oxazepan-4-yl)pyridin-2-yl, 5-[(4-methylpiperazin-1-yl)carbonyl]pyridin-2-yl, 5-[(4-methyl-1,4-diazepan-1-yl)carbonyl]pyridin-2-yl, 5-(pyrrolidin-1-ylcarbonyl)pyridin-2-yl, 5-(3,3,4-trimethyl-2,5-dioxoimidazolidin-1-yl)pyridin-2-yl, or 5-[(4-methoxypiperidin-1-yl)carbonyl]pyridin-2-yl.

Examples of the specific compounds included in the present invention include:
(1) 4-{[1-(5-cyanopyridin-2-yl)piperidin-4-yl]amino-6-(pyridin-2-ylamino)nicotinamide,
(2) 4-{[1-(5-cyanopyridin-2-yl)piperidin-4-yl]amino}-6-(pyrazin-2-ylamino)nicotinamide,
(3) 4-{[1-(4-cyanophenyl)piperidin-4-yl]amino}-6-(pyrazin-2-yl)nicotinamide,
(4) 4-{[1-(5-cyanopyridin-2-yl)piperidin-4-yl]amino -6-(pyrimidin-4-ylamino)nicotinamide,
(5) 4-{[1-(5-cyanopyridin-2-yl)piperidin-4-yl]amino}-6-[(2-methylpyrimidin-4-yl)amino]nicotinamide,
(6) 4-{[(3S,4R)-1-(5-cyanopyridin-2-yl)-3-fluoropiperidin-4-yl]amino}-6-(pyrimidin-4-ylamino)nicotinamide,
(7) 4-{[(3S,4R)-1-(6-cyanopyridazin-3-yl)-3-fluoropiperidin-4-yl]amino} -6-(pyrazin-2-ylamino)nicotinamide,
(8) 4-{[1-(6-cyanopyridazin-3-yl)piperidin-4-yl]amino}-6-(pyrazin-2-ylamino)nicotinamide,
(9) 4-{[(3S,4R)-1-(5-cyanopyrazin-2-yl)-3-fluoropiperidin-4-yl]amino}-6-(pyrimidin-4-ylamino)nicotinamide,
(10) 4-{[1-(6-cyanopyridazin-3-yl)piperidin-4-yl]amino-6-(pyrimidin-4-ylamino)nicotinamide,
(11) 4-{[1-(6-cyanopyridazin-3-yl)piperidin-4-yl]amino}-6-[(5-methylpyrazin-2-yl)amino]nicotinamide,
(12) 4-{[1-(5-cyanopyridin-2-yl)piperidin-4-yl]amino}-6-[(5-methylpyrazin-2-yl)amino]nicotinamide,
(13)4-({1-[4-(benzyloxy)phenyl]piperidin-4-yl}amino)-6-(pyrazin-2-ylamino)nicotinamide,
(14) 4-[1-(4-hydroxyphenyl)piperidin-4-yl] amino}-6-(pyrazin-2-ylamino)nicotinamide,
(15) 4-{[1-(4-fluorophenyl)piperidin-4-yl]amino} -6-(pyrazin-2-ylamino)nicotinamide,
(16) 4- {[1-(6-cyanopyridin-3-yl)piperidin-4-yl]amino}-6-(pyrimidin-4-ylamino)nicotinamide,
(17) 4-{[(3S,4R)-1-(5-cyanopyridin-2-yl)-3-fluoropiperidin-4-yl]amino} -6-[(2-methylpyrimidin-4-yl)amino]nicotinamide,
(18) 4-{[(3S,4R)-1-(6-cyanopyridazin-3-yl)-3-fluoropiperidin-4-yl]amino -6-[(2-methylpyrimidin-4-yl)amino]nicotinamide,
(19) 4-({1-[4-(cyanomethoxy)phenyl]piperidin-4-yl}amino)-6-(pyrazin-2-ylamino)nicotinamide,
(20) 4-[(1-{5-[(cyanomethyl)carbamoyl]pyridin-2-yl}piperidin-4-yl)amino]-6-(pyrazin-2-ylamino)nicotinamide,
(21)6-(4-{[5-carbamoyl-2-(pyrazin-2-ylamino)pyridin-4-yl] amino }piperidin-1-yl) -N-(cyanomethyl)-N-methylnicotinamide,
(22) 4-{[1-(5-cyanopyridin-2-yl)piperidin-4-yl]amino}-6-{[5-(2-oxopyrrolidin-1-yl)pyridin-2-yl] amino}nicotinamide,
(23) 4-{[(3S,4R)-1-(5-cyanopyridin-2-yl)-3-(hydroxymethyl)piperidin-4-yl]amino}-6-(pyridin-2-ylamino)nicotinamide,
(24) 4-{[1-(4-cyanophenyl)piperidin-4-yl]amino} -6- {[5-(methoxymethyl)pyridin-2-yl]amino}nicotinamide,
(25) 4-{[(3S,4R)-1-(5-cyanopyridin-2-yl)-3-(hydroxymethyl)piperidin-4-yl]amino}-6-[(pyrazin-2-yl)] aminonicotinamide,
(26) 4- [(3S,4R)-1-(5-cyanopyridin-2-yl)-3-(hydroxymethyl)piperidin-4-yl]amino}-6-{[5-(methoxymethyl)pyridin-2-yl] amino nicotinamide,
(27) 4-{[(3S,4R)-1-(5-cyanopyridin-2-yl)-3-(hydroxymethyl)piperidin-4-yl]amino}-6-[(5-fluoropyridin-2-yl)amino]nicotinamide,
(28) 4- {[(3S,4R)-1-(5-cyanopyridin-2-yl)-3-(hydroxymethyl)piperidin-4-yl]amino}-6-{[5-(trifluoromethyl)pyridin-2-yl]amino}nicotinamide,
(29) 4-{[(3S,4R)-1-(5-cyanopyridin-2-yl)-3-fluoropiperidin-4-yl]amino} -6- {[5-(2-oxopiperidin-1-yl)pyridin-2-yl]amino}nicotinamide,
(30) 4-{[(3R,4S)-1-(5-cyanopyridin-2-yl)-3-fluoropiperidin-4-yl]amino} -6- {[55-(2-oxopiperidin-1-yl)pyridin-2-yl]amino}nicotinamide,
(31) 4-{[(3S,4R)-1-(5-cyanopyridin-2-yl)-3-fluoropiperidin-4-yl]amino}-6-{[5-(2-oxoazepan-1-yl)pyridin-2-yl]amino}nicotinamide,
(32) 4-{[(3R,4S)-1-(5-cyanopyridin-2-yl)-3-methylpiperidin-4-yl]amino}-6-{[5-(2-oxopiperidin-1-yl)pyridin-2-yl]amino}nicotinamide,
(33) 4-{[1-(5-cyanopyridin-2-yl)piperidin-4-yl]amino}-6-({5-[(1R,4S)-3-oxo-2-azabicyclo[2.2.1]hept-2-yl]pyridin-2-yl}amino)nicotinamide,
(34) 4-{[1-(5-cyanopyridin-2-yl)piperidin-4-yl]amino}-6-({5-[(1S,4R)-3-oxo-2-azabicyclo[2.2.1]hept-2-yl]pyridin-2-yl}amino)nicotinamide,
(35) 4-{[(3S,4R)-1-(5-cyanopyridin-2-yl)-3-fluoropiperidin-4-yl]amino}-6-(pyrazin-2-ylamino)nicotinamide,
(36) 4-{[1-(4-cyanophenyl)piperidin-4-yl]amino}-6-[(2-methylpyrimidin-4-yl)amino]nicotinamide,
(37) 6-(pyrazin-2-ylamino)-4-[(2,3,6-trifluorobenzyl)amino]nicotinamide,
(38)6-[(2-methylpyrimidin-4-yl)amino]-4-[(2,3,6-trifluorobenzyl)amino]nicotinamide,
(39) 6-{[S-(2-oxopiperidin-1-yl)pyridin-2-yl]amino}-4-[(2,3,6-trifluorobenzyl)amino]nicotinamide,
(40) 6-{[5-(4-methyl-2-oxopiperazin-1-yl)pyridin-2-yl]amino}-4-[(2,3,6-trifluorobenzyl)amino]nicotinamide,
(41) 6-{[5-(4-methyl-7-oxo-1,4-diazepan-1-yl)pyridin-2-yl]amino}-4-[(2,3,6-trifluorobenzyl)amino]nicotinamide,
(42) 6-{[5-(3-oxomorpholin-4-yl)pyridin-2-yl]amino}-4-[(2,3,6-trifluorobenzyl)amino]nicotinamide,
(43) 6-({5-[(1S,4R)-3-oxo-2-azabicyclo[2.2.1]hept-2-yl]pyridin-2-yl}amino)-4-[(2,3,6-trifluorobenzyl)amino]nicotinamide,
(44) 6-{[5-(2-oxoazepan-1-yl)pyridin-2-yl]amino}-4-[(2,3,6-trifluorobenzyl)amino)]nicotinamide,
(45) 6-({5-[(1R,4S)-3-oxo-2-azabicyclo[2.2.1]hept-2-yl]pyridin-2-yl}amino)-4-[(2,3,6-trifluorobenzyl)amino]nicotinamide,
(46) 6-{[5-(5-oxo-1,4-oxyazepan-4-yl)pyridin-2-yl]amino}-4-[(2,3,6-trifluorobenzyl)amino]nicotinamide,
(47) 4-[(2,3,6-trifluorobenzyl)amino]-6-{[5-(3,4,4-trimethyl-2,5-dioxoimidazolidin-1-yl)pyridin-2-yl]amino}nicotinamide,
(48) 4-[(2,6-difluorobenzyl)amino]-6-{[5-(pyrrolidin-1-ylcarbonyl)pyridin-2-yl]amino}nicotinamide,
(49) 4-[(2,6-difluorobenzyl)amino]-6-({5-[(4-methylpiperazin-1-yl)carbonyl]pyridin-2-yl}amino)nicotinamide,
(50) 4-[(2,6-difluorobenzyl)amino]-6-({5-[(4-methyl-1,4-diazepan-1-yl)carbonyl]pyridin-2-yl}amino)nicotinamide,
(51) 4-[(2,6-difluorobenzyl)amino]-6-{[5-(4-methyl-7-oxo-1,4-diazepan-1-yl)pyridin-2-yl]amino}nicotinamide, and
(52) 4-[(2,6-difluorobenzyl)amino]-6-({5-[(4-methoxypiperidin-1-yl)carbonyl]pyridin-2-yl} amino)nicotinamide.

The compound of the formula (I) may exist in the form of tautomers or geometrical isomers depending on the kind of substituents. In the present specification, the compound of the formula (I) may be described in only one form of isomer, but the present invention includes these isomers as well as isolated forms of the isomers, or a mixture thereof.
In addition, the compound of the formula (I) may have asymmetric carbon atoms or axial asymmetry in some cases, and correspondingly, it may exist in the form of optical isomers. The present invention includes both an isolated form of the optical isomers of the compound of the formula (I) or a mixture thereof.

Furthermore, the present invention also includes a pharmaceutically acceptable prodrug of the compound represented by the formula (I). The pharmaceutically acceptable prodrug is a compound having a group that can be converted into an amino group, a hydroxyl group, a carboxyl group, or the like through solvolysis or under physiological conditions. Examples of the group forming the prodrug include the groups described in Prog. Med., 5, 2157-2161 (1985) and "Iyakuhin No Kaihatsu (Development of Medicines)" (Hirokawa Pub. Co., 1990), Vol. 7, Molecular Design, 163-198.

Moreover, the salt of the compound of the formula (I) is a pharmaceutically acceptable salt of the compound of the formula (I) and may form an acid addition salt or a salt with a base depending on the kind of substituents. Specific examples thereof include acid addition salts with inorganic acids such as hydrochloric acid, hydrobromic acid, hydroiodic acid, sulfuric acid, nitric acid, phosphoric acid, and the like, and with organic acids such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditolyltartaric acid, citric acid, methanesulfonic acid, ethanesulfonic acid, benzenesulfonic acid, p-toluenesulfonic acid, aspartic acid, glutamic acid, and the like, and salts with inorganic bases such as sodium, potassium, magnesium, calcium, aluminum, and the like or organic bases such as methylamine, ethylamine, ethanolamine, lysine, ornithine, and the like, salts with various amino acids or amino acid derivatives such as acetylleucine and the like, ammonium salts, etc.

In addition, the present invention also includes various hydrates or solvates, and polymorphic crystal substances of the compound of the formula (I) and a salt thereof. In addition, the present invention also includes compounds labeled with various radioactive or non-radioactive isotopes.

### (Preparation Methods)

The compound of the formula (I) and a salt thereof can be prepared using the characteristics based on the basic structure or the type of substituent thereof and by applying various known synthesis methods. During the preparation, protecting the relevant functional group with a suitable protective group or replacing the relevant functional group with a group that can be easily converted into the functional group at the stage from material to an intermediate may be effective depending on the type of the functional group in production technology in some cases. The protective group for such a functional group may include, for example, the protective groups described in "Greene's Protective Groups in Organic Synthesis (4th Ed, 2006)" written by P. G. M. Wuts and T. W. Greene, and one of these should only be selected and used as necessary depending on reaction conditions. In this kind of method, a desired compound can be obtained by introducing the protective group, by carrying out reaction and by eliminating the protective group as necessary.
In addition, the prodrug of the compound of the formula (I) can be produced by introducing a specific group or by carrying out the reaction using the resulting compound of the formula (I) at the stage from a starting material to an intermediate, just as in the case of the above-mentioned protective group. The reaction can be carried out using methods known to those skilled in the art, such as ordinary esterification, amidation, dehydration, and the like.
Hereinbelow, the representative preparation methods for the compound of the formula (I) will be described. Each of the production processes may also be carried out with reference to the References appended in the present description. Further, the preparation methods of the present invention are not limited to the examples as shown below.

### (Production Process 1)

### Amidation

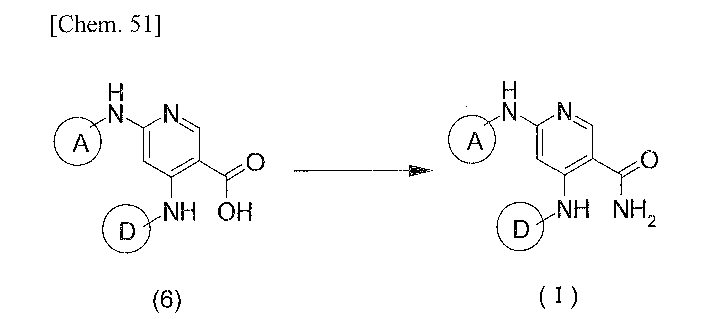

The compound of the present invention (I) can be obtained by an amidation reaction of a carboxylic acid derivative (6) with NH₃.
In the case where a carboxylic acid is used as a carboxylic acid derivative, a carboxylic acid (6) and NH₃ in an equivalent amount or in an excess amount are used, and a mixture thereof is stirred under any temperature condition from cooling to heating, preferably at -20°C to 60°C, usually for 0.1 hours to 5 days, in a solvent which is inert to the reaction, in the presence of a condensing agent. The solvent as used herein is not particularly limited, but examples thereof include aromatic hydrocarbons such as benzene, toluene, xylene, and the like, halogenated hydrocarbons such as dichloromethane, 1,2-dichloroethane, chloroform, and the like, ethers such as diethyl ether, tetrahydrofuran, dioxane, dimethoxyethane, and the like, N,N-dimethylformamide, dimethylsulfoxide, ethyl acetate, acetonitrile, or water and a mixture thereof. Examples of the condensation agent include 1-(3-dimethylaminopropyl)-3-ethylcarbodiimide, dicyclohexylcarbodiimide, 1,1'-carbonyldiimidazole, diphenylphosphoric azide, and phosphorus oxychloride, but are not limited thereto. It may be preferable for the reaction in some cases to use an additive agent (for example, 1-hydroxybenzotriazole). It may be advantageous in some cases for the smooth progress of the reaction to carry out the reaction in the presence of an organic base such as triethylamine, N,N-diisopropylethylamine, N-methylmorpholine, and the like, or an inorganic base such as potassium carbonate, sodium carbonate, potassium hydroxide, sodium hydroxide, and the like.
Furthermore, a method in which a reactive derivative of the carboxylic acid (6) is used and reacted with NH₃ can also be used. Here, examples of the reactive derivative of the carboxylic acid include acid halides that can be obtained by the reaction of a halogenating agent such as phosphorus oxychloride, thionyl chloride, and the like, mixed acid anhydrides that can be obtained by the reaction of isobutyl chloroformate or the like, active esters obtained by the condensation with 1-hydroxybenzotriazole or the like, etc. The reaction of the reactive derivative and NH₃ can be carried out under any temperature condition from cooling to heating, preferably at -20°C to 60°C, in a solvent which is inert to the reaction, such as halogenated hydrocarbons, aromatic hydrocarbons, ethers, and the like.

### [References]

"Organic Functional Group Preparations", written by S. R. Sandler and W. Karo, 2nd edition, Vol. 1, Academic Press Inc., 1991
"Courses in Experimental Chemistry (5th edition)", edited by The Chemical Society of Japan, Vol. 16 (2005) (Maruzen)

### (Production Process 2)

### Ipso substitution

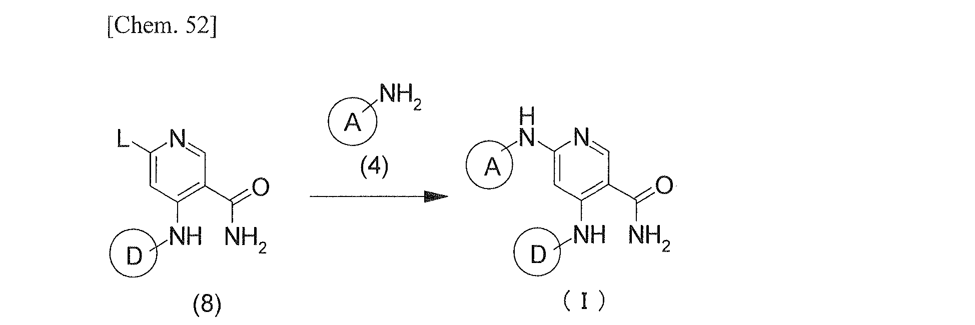

(wherein L represents a leaving group).
The compound of the present invention (I) can be obtained by reacting a compound (8) with an amine (4). Here, examples of the leaving group X include halogen, a methanesulfonyloxy group, a p-toluenesulfonyloxy group, and the like.
The compound (8) and the amine (4) in an equivalent amount or excess amount are used and a mixture thereof is stirred under any temperature condition from under cooling to under heating and refluxing, preferably at 0°C to 200°C, and more preferably at 150°C to 200°C, usually for 0.1 hours to 5 days in a solvent which is inert to the reaction or without a solvent. It may be advantageous in some cases for the smooth progress of the reaction to carry out the reaction under microwave irradiation. Here, the solvent is not particularly limited, but examples thereof include alcohols such as methanol, ethanol, tert-butanol, and the like, aromatic hydrocarbons such as benzene, toluene, xylene, and the like, ethers such as diethyl ether, tetrahydrofuran, dioxane, dimethoxyethane, and the like, halogenated hydrocarbons such as dichloromethane, 1,2-dichloroethane, chloroform, and the like, N,N-dimethylformamide, dimethylsulfoxide, ethyl acetate, acetonitrile, and a mixture thereof. It may be advantageous in some cases for the smooth progress of the reaction to carry out the reaction in the presence of an organic base such as triethylamine, N,N-diisopropylethylamine, N-methylmorpholine, and the like, or an inorganic base such as sodium tert-butoxide, potassium carbonate, bis(methylsilyl)sodium amide, sodium carbonate, or potassium hydroxide, and the like.
Furthermore, the reaction may be carried out using a catalyst which is not particularly limited, but includes catalysts used for an Ullmann reaction, a Buchwald-Hartwig reaction, or the like. The catalyst as used herein is not particularly limited, but a suitable combination of tris(dibenzylideneacetone)palladium, tetrakis(triphenylphosphine) palladium, or the like with 4,5-bis(diphenylphosphino)-9,9'-dimethylxanthene (Xantphos), 2-dicyclohexylphosphino-2',6'-dimethoxybiphenyl (SPhos), 2-dicyclohexylphosphino-2',4',6'-triisopropylbiphenyl (XPhos), and the like can be used.

### [References]

"Organic Functional Group Preparations", written by S. R. Sandler and W. Karo, 2nd edition, Vol. 1, Academic Press Inc., 1991
"Courses in Experimental Chemistry (5^{th} edition)", edited by The Chemical Society of Japan, Vol. 14 (2005) (Maruzen)
Synthesis 2006, 4, 629-632

### (Other Production Processes)

Furthermore, several substituents in the formula (I) can also be eqsily converted to other functional groups by using the compound of the present invention (I) as a starting material by means of the reaction apparent to a skilled person in the art, or modified methods thereof. The reaction can be carried out by any combination of the processes that can be usually employed by a skilled person in the art, such as hydrolyis, alkylation, halogenation, hydrogenation, and the like. Several examples thereof are presented below.

### Ipso substitution

(wherein Z represents a leaving group, 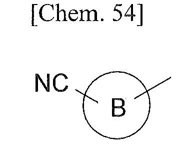 represents aryl or a hetero ring group substituted with CN, 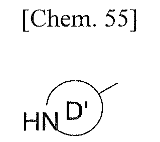 represents a nitrogen-containing hetero ring group having at least NH on a ring-forming atom).
The compound (I-2) of the present invention can be obtained by reacting a cyclic amino compound (I-1) with a compound (12) in the same method as in Production Process 2 described above. Here, examples of the leaving group Z include halogen, methanesulfonyloxy, p-toluenesulfonyloxy groups, and the like.

### Amidation

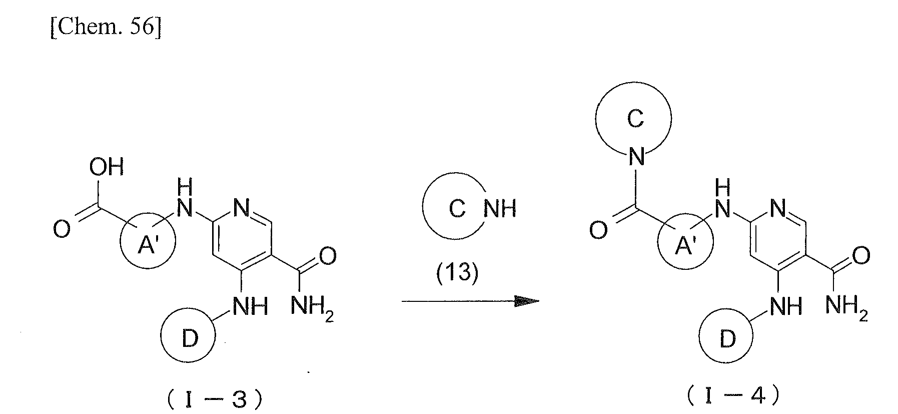

(wherein 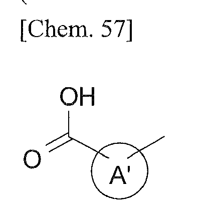 represents lower alkylene substituted with COOH, aryl, or a nitrogen-containing hetero ring group, 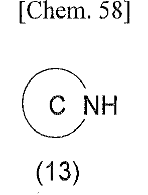 represents a nitrogen-containing hetero ring group having at least one NH on a ring-forming atom).
The compound (I-4) of the present invention can be obtained by an amidation reaction of a carboxylic acid derivative (I-3) with an amine (13) in the same method as in Production Process 1 described above.

### (Starting Material Synthesis 1)

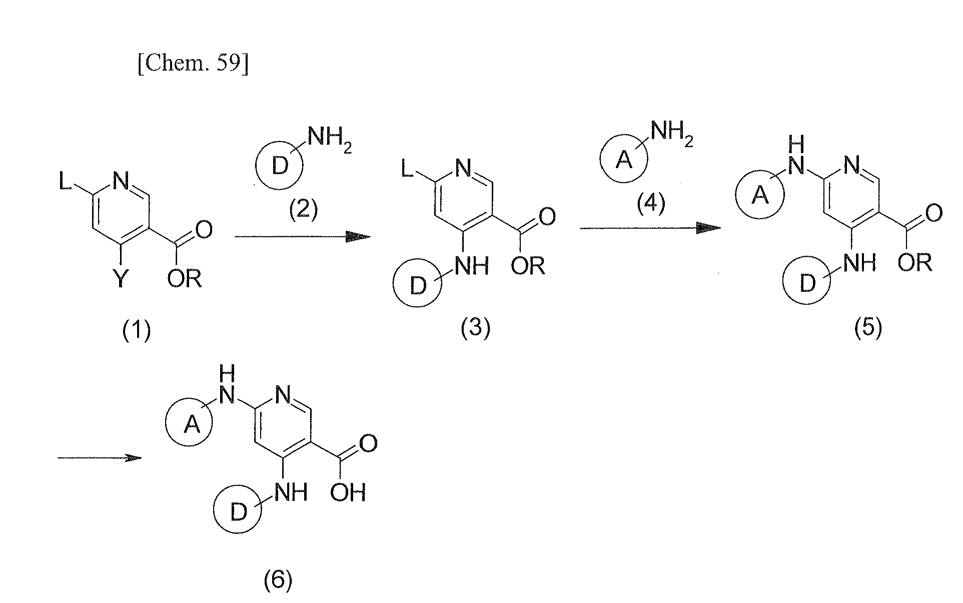

(wherein L and Y represent leaving groups, and R represents lower alkyl).
The compound (3) can be obtained by reacting the compound (1) with an amine (2). Here, examples of the leaving group Y include halogen, methanesulfonyloxy, p-toluenesulfonyloxy groups, and the like. The present reaction can be carried out by the same method as in Production Process 1 described above.
Next, the compound (5) can be obtained by reacting the compound (3) with an amine. The present reaction can be carried out by the same method as in Production Process 2 described above.
Moreover, the carboxylic acid derivative (6) can be obtained by a hydrolysis reaction of the compound (5).
Here, the hydrolysis reaction can be carried out with reference to the Reference of Greene et al. described above.

### (Starting Material Synthesis 2)

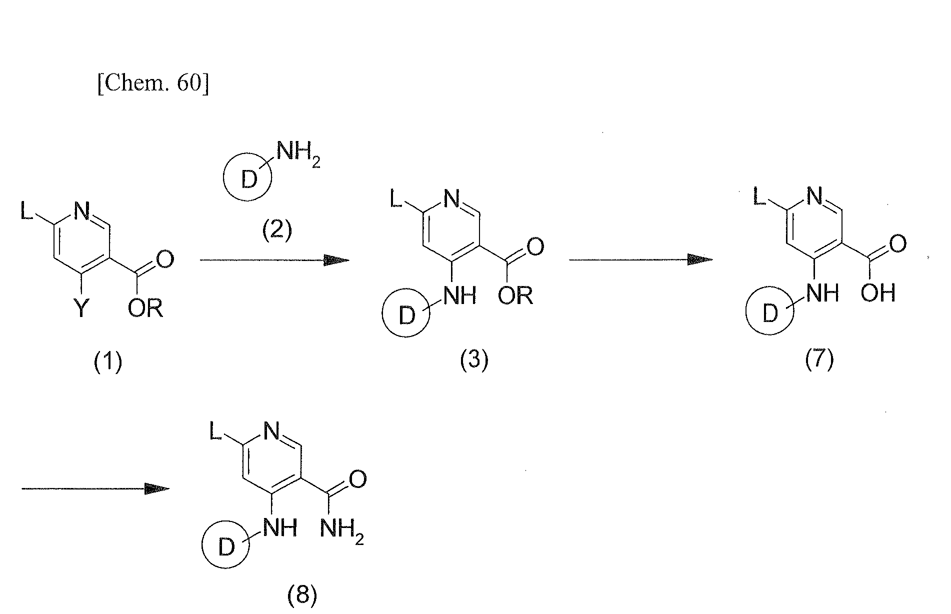

The compound (8) can be prepared in three steps from the compound (1) by carrying out each of the ipso substitution reaction of (preparation method) Production Process 2, the hydrolysis reaction described above and the same method as the amidation reaction of (preparation method) Production Process 1 stepwise.

### (Starting Material Synthesis 3)

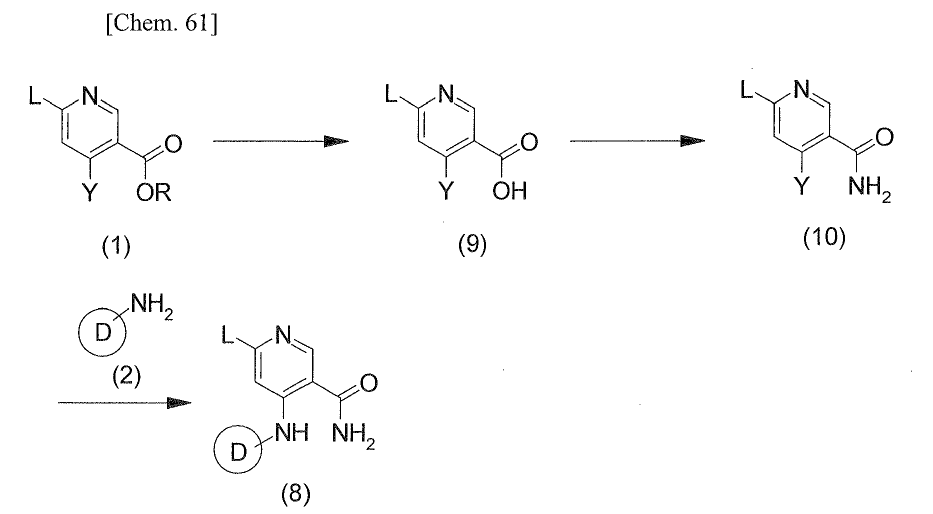

The compound (8) can be prepared in three steps from the compound (1) by carrying out, respectively, the hydrolysis reaction, the amidation reaction of (preparation method) Production Process 1, and the same method as the ipso substitution reaction of (preparation method) Production Process 2 stepwise.

The compounds of the formula (I) can be isolated and purified as their free compounds, salts, hydrates, solvates, or polymorphic crystal substances thereof. The salts of the compound of the formula (I) can be prepared after carrying out to treat a conventional salt forming reaction.
Isolation and purification are carried out by employing ordinary chemical operations such as extraction, fractional crystallization, various types of fractional chromatography, and the like.
Various isomers can be separated by selecting an appropriate starting compound or by using the difference in the physicochemical properties between the isomers. For example, the optical isomers can be obtained by means of general method for designing optical resolution of racemic products (for example, fractional crystallization for inducing diastereomer salts with optically active bases or acids, chromatography using a chiral column or the like, and others), and further, the isomers can also be prepared from an appropriate optically active starting compound.

The pharmacological activity of the compound of the formula (I) according to the present invention was verified by the following test.

### Test Example 1: JAK3 inhibition test

### (1) Preparation of human JAK3

Purified human JAK3 kinase domain was purchased from Carna Biosciences, Inc. (Kobe, Japan). This was afforded as described below. His-tag (41 kDa) was attached to the N-terminal of the 796-1124 (C-terminal) fragment of the human JAK3 protein (accession number #NM_000215), expressed using a baculovirus expression system, and then purified using Ni-NTA affinity column chromatography.

### (2) Measurement of JAK3 activity

As substrates, Biotin-Lyn-Substrate-2 (Biotin-XEQED EPEGF YFEWL EPE), X=ε-Acp (Peptide Institute, Inc., Osaka, Japan) and ATP were used. As an assay buffer, 15 mM Tris-HCl, pH 7.5, containing 0.01% Tween 20, and 2 mM DTT (dithiothreitol) was used. Normally, 20 µL of a substrate solution (an assay buffer containing 627 nM Biotin-Lyn-Substrate-2, 20 µM ATP, and 25 mM MgCl₂), an assay buffer containing 10 µL of a compound to be tested, and 20 µL of an enzyme solution were added to a microplate, and stirred sufficiently.
After incubation at room temperature for one hour, the plate was washed with a cleaning buffer (50 mM Tris-HCl pH 7.5, 150 mM NaCl, 0.02% Tween 20), and a blocking buffer (a cleaning buffer containing 0.1 % Bovine Serum Albumin) was added to the plate. After incubation at room temperature for 30 minutes, the blocking buffer was removed, and an HRP-PY-20 solution (afforded by diluting the HRP-PY-20 solution with the blocking buffer 500 times) was added. After incubation at room temperature for 30 minutes, the plate was washed four times, and a TMB substrate solution (Sigma) was added to the plate. After incubation at room temperature for 4 minutes, 1 M sulfuric acid was added to stop the reaction. Enzyme activity was measured as an absorbance at 450 nm. The JAK3 inhibitory activity of the test compound was calculated assuming that the concentration of the test compound inhibiting JAK3 activity by 50% is an IC₅₀ value.
As a result, the Example Compounds of the present invention shown in Table 1 exhibited the following IC₅₀ values.

**[Table 1]**

| Ex | IC₅₀ (nM) | | Ex | IC₅₀ (nM) |
|---|---|---|---|---|
| 5-7 | 0.21 | | 8-6 | 0.74 |
| 5-8 | 0.80 | | 8-7 | 0.80 |
| 5-9 | 0.36 | | 8-8 | 1.0 |
| 5-10 | 0.29 | | 8-9 | 1.6 |
| 5-11 | 0.34 | | 12-50 | 1.0 |
| 5-15 | 1.0 | | 12-54 | 0.69 |
| 5-18 | 1.1 | | 12-73 | 0.58 |
| 5-22 | 2.0 | | 12-74 | 0.74 |
| 5-26 | 0.39 | | 12-76 | 0.43 |
| 5-25 | 0.24 | | 12-78 | 0.69 |
| 5-46 | 0.86 | | 12-79 | 0.79 |
| 5-55 | 0.74 | | 12-80 | 0.69 |
| 5-67 | 0.90 | | 12-81 | 0.56 |
| 5-72 | 0.14 | | 12-91 | 0.44 |
| 5-73 | 0.24 | | 12-97 | 0.25 |

### Test Example 2: Rat heart transplantation

Abdominal heterotopic cardiac transplantation in rats was performed according to the reference. Male ACI rats were used as donor and male Lewis rats were used as receipient.
In addition, Lewis rats were fasted the day before the transplantion. The test drugs were orally administered once daily for 14 days from the date of transplantion. In the combination study, FK506 was administered intramuscularly. For judgement of heart transplantion rejection, the heart transplantation was observed daily by palpation for 29 days after transplantation, and stop of beating was judged as rejection. The survival period was counted to one day before the rejection.

### References

1. K. One, E. S Lindsey: Improved technique of heart transplantation in rats. J. Thorac. Cardioras. Surg, 57:225, 1969
2. Manual for organ transplantation experiments Nozawa Masumi, Inc., Shujunsya, 1999

As a result of the tests above, it was confirmed that the compound of the formula (I) has a JAK3 inhibitory activity and an inhibitory effect on rejection upon transplantion. Therefore, the compound of the formula (I) can be used for treatment of organ transplantation such as kidney transplantation, liver transplantation, heart transplantation, pancreas transplantation, lung transplantation, and the like, xenotransplantation, autoimmune diseases such as Type I diabetes, rheumatoid arthritis, multiple sclerosis, systemic lupus erythematosus (SLE), myasthenia gravis, and the like, Alzheimer's disease, Crohn's disease, psoriasis, asthma, atopic dermatitis, erythrocytosis, and cancers such as hormone-refractory prostate cancer, and the like, leukemia, multiple myeloma, bone marrow fibrosis, thrombocythemia, and the like.

The pharmaceutical composition containing one or two or more kinds of the compound represented by the formula (I) or salts thereof as an active ingredient can be prepared using excipients that are usually used in the art, that is, excipients for pharmaceutical preparation, carriers for pharmaceutical preparation, and the like.
Administration can be accomplished either by oral administration via tablets, pills, capsules, granules, powders, solutions, and the like, or parenteral administration, such as intraarticular, intravenous, or intramuscular injections, and the like, suppositories, ophthalmic solutions, eye ointments, percutaneous liquid preparations, ointments, transdermal patches, transmucosal liquid preparations, transmucosal patches, inhalations, and the like.

The solid composition for oral administration is used in the form of tablets, powders, granules, or the like. In such a solid composition, one or more active ingredient(s) are mixed with at least one inactive excipient. According to a usual method, the composition may contain inactive additives, such as lubricants, disintegrating agents, stabilizing agents, and solubilization assisting agents. If necessary, tablets or pills may be coated with sugar or a film of a gastric or enteric coating substance.
The liquid composition for oral administration contains pharmaceutically acceptable emulsions, solutions, suspensions, syrups, elixirs, or the like, and also contains generally used inert diluents, for example, purified water or ethanol. In addition to the inert diluent, the liquid composition may also contain auxiliary agents, such as a solubilization assisting agent, a moistening agent, and a suspending agent, as well as sweeteners, flavors, aromatics, and antiseptics.

The injections for parenteral administration contain aseptic aqueous or non-aqueous solutions, suspensions, or emulsions. Examples of the aqueous solvent include distilled water for injection use and physiological saline. Examples of the non-aqueous solvent include alcohols such as ethanol. Such a composition may further contain additive agents such as a tonicity agent, an antiseptic agent, a moistening agent, an emulsifying agent, a dispersing agent, a stabilizing agent, and a solubilization assisting agent. These are sterilized by filtration through a bacteria retaining filter, blending of a germicide, or irradiation. Furthermore, they may also be prepared in the form of sterile solid compositions and dissolved or suspended in sterile water or a sterile solvent for injecting prior to their use.

Examples of the drug for external use include ointments, plasters, creams, jellies, patches, sprays, lotions, eye-drops, eye ointments, and the like. The drug contains generally used ointment bases, lotion bases, aqueous or non-aqueous solutions, suspensions, emulsions, or the like.

The transmucosal agents such as inhalers, transnasal agents, and the like are used in the form of solids, liquids, or semisolids and can be prepared according to conventional known methods. For example, known excipients, and further, pH adjusters, antiseptics, surfactants, lubricants, stabilizing agents, thickeners, or the like may also be added where appropriate. For administration, suitable devices for inhalation or insufflation can be used. For example, using known devices and sprayers such as measuring inhalation devices, the compound can be administered independently or in the form of prescribed mixture powders. Furthermore, the compound combined with pharmaceutically acceptable carriers can also be administered in the form of solutions or suspensions. Dry powder inhalers and the like may be devices for single or multiple administrations, and dry powders or capsules containing powders can also be used. Still further, the devices may be in the form of a pressure aerosol spray or the like that uses suitable ejection agents such as chlorofluoroalkane, or a suitable gas such as carbon dioxide and the like.

Usually, in the case of oral administration, the daily dose is suitably from about 0.001 to 100 mg/kg per body weight, preferably from 0.1 to 30 mg/kg, and more preferably from 0.1 to 10 mg/kg, and this is administered in one portion or dividing it into 2 to 4 portions. In the case of intravenous administration, the daily dose is suitably from about 0.0001 to 10 mg/kg per body weight, and this is administered once a day or two or more times a day. In addition, a transmucosal agent is administered at a dose from about 0.001 to 100 mg/kg per body weight, and this is administered once a day or two or more times a day. The dose is appropriately decided in response to an individual case by taking the symptoms, the age, the gender, and the like into consideration.

The compound of the formula (I) can be used in combination with various therapeutic or prophylactic agents for the diseases, in which the compound of the formula (I) is considered effective, as described above. The combined preparation may be administered simultaneously or separately and continuously, or at a desired time interval. The preparations to be co-administered may be a blend or prepared individually.

### [Examples]

Hereinbelow, the preparation methods for the compound of the formula (I) will be described in more detail with reference to Examples. Further, the present invention is not limited to the preparation methods described in the specific Examples and Preparation Examples as described below, but the compound of the formula (I) can be prepared by any combination of the preparation methods or the methods that are apparent to a skilled person in the art.

Furthermore, the following symbols are used in the Examples, Preparation Examples, and Tables as described below.
Pr: Preparation Example No.,
Ex: Example No.,
Data: Physicochemical data,
ESI+: m/z value in ESI-MS (positive ion)
ESI-: m/z value in ESI-MS (negative ion)
NMR-DMSO-d₆: δ (ppm) in ¹H-NMR in DMSO-d₆,
NMR-CDCl₃: δ (ppm) in ¹H-NMR in CDCl₃,
NMR-D₂O: δ (ppm) in ¹H-NMR in D₂O,
Structure: Structural formula (the following symbols in the structural formulas represent the following meanings. HCl: Hydrochloride, 2HCl: Dihydrochloride, 3HCl: Trihydrochloride, 4HCl: Tetrahydrochloride, HBr: Hydrobromide, TFA: Trifluoroacetate, 3TFA: Tri-trifluoroacetate, *: Racemic compound with the compound shown in the structural formula and an enantiomer thereof),
DMSO: dimethylsulfoxide,
THF: Tetrahydrofuran,
DIBOC: Di-tert-butyldicarbonate,
LAH: Lithium aluminum hydride,
EtOAc: Ethyl acetate,
MgSO₄: Anhydrous magnesium sulfate,
DMF: N,N-Dimethylformamide,
MsCl: Methanesulfonylchloride,
brine: Saturated brine,
Na₂SO₄: Anhydrous sodium sulfate,
MeOH: Methanol,
CHCl₃: Chloroform,
CDI: Carbonyldiimidazole.

### Preparation Example 1

2-Aminobenzylamine (2.1 g) was dissolved in THF (43 mL), and DIBOC (4.2 g) was added thereto at room temperature. After stirring for 1 hour, the solvent was evaporated under reduced pressure to obtain oily tert-butyl (2-aminobenzyl)carbamate (3.9 g) of dark orange color as a crude product. (Reference: Organic Letters, 2002, 4, 63-66).

In the same manner as in Preparation Example 1, the compounds of Preparation Example 1-1 to Preparation Example 1-3 shown in the Tables as shown below were prepared.

### Preparation Example 2

4- {[(1R,2s,3S,5s,7s)-5-Hydroxyadamantan-2-yl]amino}-6-(pyrazin-2-ylamino)nicotinamide (90 mg) and 48% hydrobromic acid (2 mL) were mixed and allowed to undergo a reaction at 150°C for 30 minutes in a microwave reaction device. After cooling, the resulting precipitate was collected by filteration and washed with diisopropyl ether to obtain 4-{[(1R,2s,3S,5s,7s)-5-bromoadamantan-2-yl]amino}-6-(pyrazin-2-ylamino)nicotinamide hydrobromide (120 mg) as a pale yellow solid.

In the same manner as in Preparation Example 2, the compound of Preparation Example 2-1 shown in the Tables as described later was prepared.

### Preparation Example 3

In the same manner as in Example 1 as described later, the compounds of Preparation Example 3 and Preparation Example 3-1 shown in the Tables as described later were prepared.

### Preparation Example 4

Under a nitrogen gas atmosphere, to a mixed liquid of 1-tert-butyl-3-methyl(3S,4R)-4-{[(1R)-1-phenylethyl]amino}piperidine-1,3-dicarboxylate (350 mg) in THF (10 mL) was added LAH (73 mg) at 4°C. The mixture was stirred at 4°C for 1 hour. To the reaction liquid was added EtOAc to stop the reaction, followed by stirring for 20 minutes. The mixture was extracted with CHCl₃, followed by washing with water. The organic layer was dried over MgSO₄ and then filtered, and the filtrate was concentrated under reduced pressure. The residue was purified by amino-silica gel column chromatography (EtOAc/hexane = from 1/3 to 1/2) to obtain tert-butyl (3S,4R)-3-(hydroxymethyl)-4-{[(1R)-1-phenylethyl]amino}piperidine-1-carboxylate (290 mg) as a colorless oily substance.

In the same manner as in Preparation Example 4, the compound of Preparation Example 4-1 shown in the Tables as described later was prepared.

### Preparation Example 5

To a solution of 6-chloro-4-[(1H-indol-4-yl methyl)amino]nicotinamide (100 mg) in DMF (1 mL) was added 60% sodium hydride (17 mg) at 4°C. The reaction liquid was stirred for 30 minutes at the same temperature, and then methyl iodide (31 µL) was added thereto. The reaction liquid was stirred at room temperature at 1 hour, and then CHCl₃ and water were added thereto. The mixture was extracted with CHCl₃, followed by washing with water. The organic layer was dried over MgSO₄ and then filtered, and the filtrate was concentrated under reduced pressure. The residue was purified by silica gel column chromatography (CHCl₃/MeOH = from 100/0 to 95/5) to obtain 6-chloro-4-{[(1-methyl-1H-indol-4-yl)methyl]amino}nicotinamide (90 mg) as a pale brown solid.

In the same manner as in Preparation Example 5, the compound of Preparation Example 5-1 shown in the Tables as described later was prepared.

### Preparation Example 6

Sodium hydride (60% dispersion in paraffin, 420 mg) was suspended in petroleum ether (10 mL) and left to stand still. Then, the supernatant was removed by decantation, followed drying under reduced pressure. The residue was suspended in THF (30 mL), and a solution of [6-(tritylamino)pyridin-3-yl]methanol (3.2 g) in DMF (30 mL) was added thereto at 0°C. After stirring for 30 minutes as it was, the solution was warmed to room temperature and further stirred for 30 minutes. Thereafter, the reaction mixture was cooled to 0°C again, and MsCl (811 µL) was added dropwise thereto. After stirring at 0°C for 30 minutes as it was, dimethylamine (2.0 M solution in THF, 44 mL) was added dropwise to the mixture. Thereafter, the mixture was stirred at 0°C for 30 minutes, warmed to room temperature, and further stirred overnight. The reaction mixed liquid was concentrated under reduced pressure and the residue was purified by basic silica gel column chromatography (CHCl₃/MeOH = from 100/0 to 96/4) to obtain 5-[(dimethylamino)methyl]-N-tritylpyridin-2-amine (1.129 g) as a white solid.

In the same manner as in Preparation Example 6, the compounds of Preparation Example 6-1 and Preparation Example 6-2 shown in the Tables as described later were prepared.

### Preparation Example 7

Sodium hydride (60% dispersion in paraffin, 240 mg) was suspended in 20 mL of THF and a solution of [6-(tritylamino)pyridin-3-yl]methanol (2.0 g) in DMF (20 mL) was added thereto at 0°C. After 30 minutes, the reaction mixture was warmed to room temperature and further stirred for 30 minutes. The reaction mixture was cooled to 0°C again and iodomethane (442 µL) was slowly added dropwise thereto. After addition dropwise, the mixture was stirred at 0°C for 1 hour and further at room temperature for 2 hours. The reaction mixture was concentrated under reduced pressure, and water and EtOAc were added thereto under cooling to 0°C. The organic layer was extracted twice with EtOAc, and the resulting organic layer was combined, followed by drying over Na₂SO₄. After filtration, concentration was performed under reduced pressure and the resulting residue was purified by silica gel column chromatography (hexane /EtOAc = from 90/10 to 75/25) to obtain 5-(methoxymethyl)-N-tritylpyridin-2-amine (1.52 g) as a white solid.

### Preparation Example 8

4-{ [(1R,2s,3 S,5s,7s)-5-Bromoadamantan-2-yl]amino}-6-(pyrazin-2-ylamino)nicotinamide hydrobromide (60 mg), triethylamine (80 µL), and 3-hydroxypropanenitrile (1 mL) were mixed and allowed to undergo a reaction at 160°C for 30 minutes in a microwave reaction device. The mixture was purified by silica gel column chromatography (CHCl₃/MeOH = from 100/0 to 85/15) to obtain 4-{[(1R,2s,3S,5s,7s)-5-(2-cyanoethoxy)adamantan-2-yl]amino}-6-(pyrazin-2-ylamino)nicotinamide (25 mg) as a white solid.

### Preparation Example 9

Dimethylformamide (20 mL) was added to 4-nitrobenzenethiol (2.0 g) and dissolved therein, followed by addition of potassium carbonate (2.7 g), tributylphosphine (0.64 mL), and bromocyclopentane (1.7 mL) thereto, followed by stirring at 80°C for 1 hour. After cooling to room temperature, water was added thereto, followed by extraction with EtOAc. The organic layer was washed with brine, then dried over Na₂SO₄, filtered, and concentrated under reduced pressure to obtain a crude product. The crude product was purified by silica gel column chromatography (hexane /EtOAc = from 100/0 to 90/10) to obtain 1-(cyclopentylsulfanyl)-4-nitrobenzene (2.9 g) as a brown oily substance.

### Preparation Example 10

1-Cyclopentylsulfanyl-4-nitrobenzene (2.9 g) was dissolved in 1,2-dichloroethane (50 mL) and cooled in an ice-water bath. Under stirring, meta-chlorobenzoic acid (4.9 g) and sodium hydrogen carbonate (1.2 g) were added thereto, followed by stirring at room temperature for 30 hours. 1,2-Dichloroethane was added for dilution, followed by washing with a saturated aqueous sodium thiosulfate solution. The organic layer was washed with a saturated aqueous sodium hydrogen carbonate solution and brine, dried over Na₂SO₄, and filtered, and the solvent was evaporated under reduced pressure. The resulting crude product was purified by silica gel column chromatography (hexane /EtOAc = from 100/0 to 90/10) to obtain 1-(cyclopentylsulfonyl)-4-nitrobenzene (31. g) as a white solid.

In the same manner as in Preparation Example 10, the compound of Preparation Example 10-1 shown in the Tables as described later was prepared.

### Preparation Example 11

To a solution of tert-butyl (3S,4R)-4-[(5-carbamoyl-2-chloropyridin-4-yl)amino]-3-(hydroxymethyl)piperidine-1-carboxylate (95 mg) in DMF (1.4 mL) were added tert-butyl(chloro)dimethylsilane (37 mg) and imidazole (17 mg), followed by stirring at room temperature for 1.5 hours. To the reaction liquid were added tert-butyl(chloro)dimethylsilane (74 mg) and imidazole (34 mg), followed by stirring at room temperature for 1.5 hours and stirring further at 60°C for 1.5 hours. To the reaction liquid was added water, and then the mixture was extracted with CHCl₃, followed by washing with water. The organic layer was dried over MgSO₄ and then filtered, and the filtrate was concentrated under reduced pressure. The residue was purified by silica gel column chromatography (CHCl₃/MeOH = from 100/0 to 98/2) to obtain tert-butyl (3S,4R)-3-({[tert-butyl(dimethyl)silyl]oxy}methyl)-4-[(5-carbamoyl-2-chloropyridin-4-yl)amino]piperidine-1-carboxylate (90 mg) as a white amorphous substance.

### Preparation Example 12

To a solution of (3RS,4SR)-1-benzyl-N-(2,4-dimethoxybenzyl)-3-methylpiperidin-4-amine (14 g) in dichloroethane (210 mL) were added triethylamine (22 mL) and trifluoroacetic anhydride (8.4 mL) at 4°C. The reaction liquid was stirred at the same temperature for 2 hours. To the reaction liquid was added water, followed by extraction with CHCl₃ and washing with water. The organic layer was dried over MgSO₄ and then filtered, and the filtrate was concentrated under reduced pressure. The residue was purified by silica gel column chromatography (EtOAc/n-hexane = from 1/5 to 1/4) to obtain N-[(3RS,4SR)-1-benzyl-3-methylpiperidin-4-yl]-N-(2,4-dimethoxybenzyl)-2,2,2-trifluoroacetamide (15.1 g) as a white amorphous substance.

### Preparation Example 13

Under a nitrogen gas atmosphere, to a mixed liquid of tert-butyl(4-oxocyclohexyl)carbamate (1 g) in THF (30 mL) was added dropwise a 0.5 M solution of hexamethyldisilazane potassium in toluene (14.7 mL) at -78°C. The reaction liquid was stirred at the same temperature for 1 hour, and then a solution of N-phenyl bis(trifluoromethane)sulfonimide (2 g) in THF (15 mL) was added thereto. The reaction liquid was stirred at the same temperature for 2 hours and a saturated aqueous ammonium chloride solution was added thereto, followed by extraction with EtOAc and washing with a saturated aqueous sodium hydrogen carbonate solution and brine. The organic layer was dried over MgSO4 and then filtered, and the filtrate was concentrated under reduced pressure. The residue was purified by silica gel column chromatography (EtOAc/hexane = 10/90) to obtain 4-[(tert-butoxycarbonyl)amino]cyclohex-1-en-1-yltrifluoromethanesulfonate (605 mg) as a colorless oily substance.

### Preparation Example 14

To a mixture of 4,6-dichloronicotinamide (300 mg) and N-methyl-2-pyrrolidinone (1.5 mL) were added N,N-diisopropylethylamine (1.09 mL) and 2,6-difluoro-4-methoxybenzylamine (326 mg), followed by microwave irradiation and stirring at 140°C for 1 hour.
After cooling the reaction liquid, the reaction liquid was added to water, followed by stirring at room temperature for 30 minutes. The precipitated solid was collected by filtration, washed with diisopropyl ether, and then dried under reduced pressure to obtain 6-chloro-4-[(2,6-difluoro-4-methoxybenzyl)amino]nicotinamide (514 mg).

In the same manner as in Preparation Example 14, the compounds of Preparation Examples 14-1 to 14-36 and Preparation Examples 14-38 to 14-59 and 14-62 to 14-74 shown in the Tables as described later were prepared.

### Preparation Example 15

A mixture of ethyl 4,6-dichloronicotinate (500 mg), 2-(methylsulfanyl)aniline (0.244 mL), tetraethylammonium chloride (37.7 mg), and N-methyl-2-pyrrolidinone (2.5 mL) was stirred at 120°C overnight. After cooling to room temperature, the mixture was diluted with water and extracted with EtOAc. The organic layer was dried over Na₂SO₄ and then filtered, and the solvent was evaporated under reduced pressure. The residue was purified by silica gel column chromatography (EtOAc/hexane = from 0/100 to 10/90) to obtain ethyl 6-chloro-4-{[2-(methylsulfanyl)phenyl]amino}nicotinate (94.5 mg).

In the same manner as in Preparation Example 15, the compound of Preparation Example 15-1 shown in the Tables as described later was prepared.

### Preparation Example 16

tert-Butyl(2-aminobenzyl)carbamate (3.9 g) and pyridine (15 mL) were dissolved in 1,2-dichloroethane, followed by addition of MsCl (1.63 g) under ice-cooling and stirring at room temperature for 3 hours. The organic solvent was evaporated under reduced pressure, followed by addition of CHCl₃ and washing with 1 M hydrochloric acid and water in this order. The organic phase was dried over MgSO₄, and then white solid was filtered off. The solvent of the filtrate was evaporated under reduced pressure and then the resulting solid was washed with EtOAc and hexane to obtain tert-butyl{2-[(methylsulfonyl)amino]benzyl}carbamate (3.68 g) as a pale brown solid.

In the same manner as in Preparation Example 16, the compound of Preparation Example 16-1 shown in the Tables as described later was prepared.

### Preparation Example 17

(6-Aminopyridin-3-yl)methanol (1.327 g) was dissolved in 20 mL of a 1/1 mixed solvent of CHCl₃/DMF, followed by addition of triethylamine (2.24 mL). The reaction mixture was cooled to 0°C and tritylchloride (3.13 g) which had been dissolved in CHCl₃ (10 mL) was slowly added dropwise thereto. After completion of the dropwise addition, the reaction mixture was warmed to room temperature, followed by further stirring for 2 hours. The reaction mixture was concentrated under reduced pressure, followed by addition of EtOAc and water and being left to stand still at -10°C overnight. Thereafter, the precipitated solid was collected by filtration and washed with water and cold EtOAc to collect [6-(tritylamino)pyridin-3-yl]methanol as a white solid by filtration. Further, the filtrate was extracted with EtOAc by a liquid separation operation. The organic layer was dried over Na₂SO₄ and then filtered, and the solvent was evaporated under reduced pressure. This residue was purified by silica gel column chromatography (CHCl₃/MeOH = from 100/0 to 96/4) to obtain [6-(tritylamino)pyridin-3-yl]methanol as a white solid. A total amount of 6-(tritylamino)pyridin-3-yl]methanol obtained by collection by filtration and silica gel column chromatography as a white solid was 3.744 g.

### Preparation Example 18

To a mixture of 2-(trifluoromethyl)nicotinamide (424 mg), triethylamine (0.933 mL), and dichloromethane (8.5 mL) was added trifluoroacetic anhydride (0.394 mL) at 0°C, followed by stirring at room temperature for 4 hours. The reaction mixture was added to a saturated aqueous sodium hydrogen carbonate solution and a solution of CHCl₃, followed by performing a liquid separation operation, and the organic layer was dried over MgSO₄ and then concentrated under reduced pressure. The residue was purified by silica gel column chromatography (EtOA_{C}/CHCl₃ = from 0/100 to 20/80) to obtain 2-(trifluoromethyl)nicotinonitrile (357 mg).

In the same manner as in Preparation Example 18, the compound of Preparation Example 18-1 shown in the Tables as described later was prepared.

### Preparation Example 19

To ethyl 6-chloro-4-{[2-ethyl-3-(hydroxymethyl)phenyl]amino}nitcotinate (193 mg) were added ethanol (6 mL) and 0.35 mL of a 5 M aqueous NaOH solution, followed by heating under reflux for 4 hours. After cooling to room temperature, ethanol was concentrated under reduced pressure, followed by addition of 10 mL of water and adjustment to pH = 3 using 1 M hydrochloric acid under ice-cooling. The precipitated white solid was collected by filtration and dried under reduced pressure to obtain ethyl 6-chloro-4-{[2-ethyl-3-(hydroxymethyl)phenyl]amino}nicotinic acid (163 mg).

In the same manner as in Preparation Example 19, the compounds of Preparation Examples 19-1 to 19-20 shown in the Tables as described later were prepared.

### Preparation Example 20

Under a nitrogen gas atmosphere, to a mixed liquid of 1-benzyl-3-methyl-4-piperidinone (15 g) and (2,4-dimethoxybenzyl)amine (11.1 mL) in dichloroethane (225 mL) was added sodium triacetoxyborohydride (31.3 g) at 4°C. The reaction liquid was stirred at the same temperature for 30 minutes, and then stirred at room temperature for 2 hours. To the reaction liquid was added 30 mL of a 1M aqueous NaOH solution. The reaction liquid was extracted with CHCl₃, followed by washing with water. The organic layer was dried over MgSO₄ and then filtered, and the filtrate was concentrated under reduced pressure. The residue was purified by amino-silica gel column chromatography (EtOAc/hexane = from 1/3 to 1/2) to obtain (3RS,4SR)-1-benzyl-N-(2,4-dimethoxybenzyl)-3-methylpiperidin-4-amine (14 g) as a colorless oily substance.

In the same manner as in Preparation Example 20, the compound of Preparation Example 20-1 shown in the Tables as described later was prepared.

### Preparation Example 21

In the same manner as in Example 9, the compounds of Preparation Examples 21 and 21-1 shown in the Tables as described later were prepared.

### Preparation Example 22

A mixture of 6-chloro-4- [2-(methylsulfanyl)phenyl]amino}nicotinic acid (343 mg), N-[3-(dimethylamino) propyl]-N'-ethylcarbodiimide hydrochloride (335 mg), 1-hydroxybenzotriazole (236 mg), 4-dimethylaminopyridine (213 mg), ammonium chloride (124 mg), and DMF (3.4 mL) was stirred at room temperature for 24 hours, and then water (60 mL) was added thereto, followed by stirring at room temperature for 1 hour. The solid was collected by filtration and dried under reduced pressure to obtain 6-chloro-4-{[2-(methylsulfanyl)phenyl]amino}nicotinamide (310 mg).

In the same manner as in Preparation Example 22, the compounds of Preparation Examples 22-1 to 22-21 shown in the Tables as described later were prepared.

### Preparation Example 23

To a mixture of 2-(trifluoromethyl)nicotinonitrile (355 mg) and acetic acid (18 mL) was added 10% palladium/carbon (66.9 mg), followed by stirring at room temperature for 5 hours at a normal pressure under a hydrogen atmosphere. After completion of the reaction, the reaction liquid was filtered through celite and the filtrate was concentrated under reduced pressure. The resulting crude product was purified by basic silica gel column chromatography (MeOH/CHCl₃ = from 0/100 to 8/92) to obtain 1-[2-(trifluoromethyl)pyridin-3-yl]methanamine (337 mg).

In the same manner as in Preparation Example 23, the compounds of Preparation Examples 23-1 and 23-4 to 23-11 shown in the Tables as described later were prepared.

### Preparation Example 24

N-[(3RS,4SR)-1-Benzyl-3-methylpiperidin-4-yl]-N-(2,4-dimethoxybenzyl)-2,2,2-trifluoroacetamide (15.1 g) and trifluoroacetic acid (62 mL) were mixed at 4°C, followed by stirring at 50°C for 24 hours. The reaction liquid was concentrated under reduced pressure, and the residue was dissolved in CHCl₃ and neutralized with a saturated aqueous sodium hydrogen carbonate solution. The precipitate was removed by filtration, and the filtrate was extracted with CHCl₃, followed by washing with water. The organic layer was dried over MgSO₄ and then filtered, and the filtrate was concentrated under reduced pressure to obtain N-[(3RS,4SR)-1-benzyl-3-methylpiperidin-4-yl]-2,2,2-trifluoroacetamide (10.0 g) as a white solid.

In the same manner as in Preparation Example 24, the compound of Preparation Example 24-1 shown in the Tables as described later was prepared.

### Preparation Example 25

In the same manner as in Example 11 as described later, the compounds of Preparation Examples 25, 25-1 to 25-11, 25-13, 25-15 to 37, 25-39 to 25-56, 25-58 to 25-63 and 25-65 to 25-88 shown in the Tables as described later were prepared.

### Preparation Example 26

tert-Butyl 4-({5-carbamoyl-4-[(2,3,6-trifluorobenzyl)amino]pyridin-2-yl})benzoate (437 mg) was dissolved in trifluoroacetic acid (2 mL), followed by stirring at room temperature for 3 hours. Thereafter, trifluoroacetic acid was evaporated under reduced pressure and the solid was precipitated. Water (20 ml) and ethanol (6 ml) were added thereto, followed by further addition of a 1 M aqueous NaOH solution (about 4 ml) and first adjustment to pH=9.5. About 2 ml of 1 M hydrochloric acid was added thereto to adjust the pH to 5. After stirring at room temperature for 1 hour, the precipitate was collected by filtration. After washing with water and drying under reduced pressure at 65°C for 3 hours, 4-({5-carbamoyl-4-[(2,3,6-trifluorobenzyl)amino]pyridin-2-yl}amino)benzoic acid (385 mg) was obtained as an off-white powder.

In the same manner as in Preparation Example 26, the compound of Preparation Example 26-1 shown in the Tables as described later was prepared.

### Preparation Example 27

5-(Methoxymethyl)-N-tritylpyridin-2-amine (1522.8 mg) was dissolved in CHCl₃ (30 mL), followed by addition of para-toluenesulfonic acid monohydrate (2067 mg) at room temperature, and then the reaction mixture was stirred overnight. A standard buffer solution at pH = 6.86 and a saturated aqueous Na₂CO₃ solution were added thereto in this order, followed by stirring for about 1 hour and extraction three times with CHCl₃. This organic layer was combined, dried over Na₂SO₄, and filtered, and the filtrate was concentrated under reduced pressure. The resulting residue was purified by silica gel column chromatography (CHCl₃/MeOH = from 100/0 to 92/8) to obtain 5-(methoxymethyl)pyridin-2-amine (444.5 mg) as a pale yellow solid.

In the same manner as in Preparation Example 27, the compound of Preparation Example 27-1 shown in the Tables as described later was prepared.

### Preparation Example 28

In the same manner as in Example 12, the compounds of Preparation Example 28 and Preparation Examples 28-1, 28-2, 28-4 to 28-17, 28-19 to 28-59, 28-61, 28-63 to 28-72 and 28-75 to 28-97 shown in the Tables as described later were prepared.

### Preparation Example 29

To a solution of 4-[(tert-butoxycarbonyl)amino]cyclohex-1-en-1-yltrifluoromethanesulfonate (400 mg) in dimethoxyethane (10 mL) were added (4-cyanophenyl)boric acid (208 mg), lithium chloride (123 mg), a 2 M aqueous sodium carbonate solution (1.45 mL), and tetrakis(triphenylphosphine)palladium (0) (67 mg), followed by stirring at 90°C for 2 hours. After cooling to room temperature, the reaction liquid was extracted with CHCl₃, followed by washing with water. The organic layer was dried over MgSO₄ and then filtered, and the filtrate was concentrated under reduced pressure. The residue was purified by silica gel column chromatography (EtOAc:hexane = from 85:15 to 75:25) to obtain tert-butyl [4-(4-cyanophenyl)cyclohex-3-en-1-yl]carbamate (143 mg) as a white solid.

### Preparation Example 30

In the same manner as in Preparation Example 14, the compounds of Preparation Example 30 and Preparation Examples 30-1 to 30-3 shown in the Tables as described later were prepared.

### Preparation Example 31

In the same manner as in Preparation Example 19, the compound of Preparation Example 31 shown in the Tables as described later was prepared.

### Preparation Example 32

In the same manner as in Preparation Example 19, the compounds of Preparation Example 32 and Preparation Examples 32-1 to 32-9 shown in the Tables as described later were prepared.

### Preparation Example 33

To a suspension of 5-iodopyridin-2-amine (800 mg) in dioxane (6 mL) were added copper (I) iodide (6.9 mg), trans-cyclohexane-1,2-diamine (44 µL), morpholin-3-one (441 mg), and powdery tripotassium phosphate (1.54 g). The resulting reaction mixture was heated and stirred at 110°C for 23 hours. After cooling to room temperature, the reaction mixture was filtered through celite, and the solid was washed with CHCl₃. The liquid was concentrated under reduced pressure and the resulting residue was purified by silica gel column chromatography (CHCl₃/MeOH = from 100/0 to 88/12) to obtain 4-(6-aminopyridin-3-yl)morpholin-3-one (548.3 mg) as a pale yellow solid.

In the same manner as in Preparation Example 33, the compounds of Preparation Examples 33-1 to 33-3 shown in the Tables as described later were prepared.

### Preparation Example 34

tert-Butyl[(3-bromo-1-benzothiophene-7-yl)methyl]carbamate (400 mg) was mixed with dimethylacetamide (8 mL), followed by zinc cyanide (686 mg), zinc (229 mg), tris(dibenzylideneacetone)dipalladium (0) (214 mg), and 1,1'-bis(diphenylphosphino)ferrocene (324 mg). The reaction mixture was stirred at 90°C for 8 hours. To the reaction mixture was added CHCl₃, followed by stirring, and then the insoluble materials were removed by filtration. The filtrate was concentrated under reduced pressure and then the residue was purified by silica gel column chromatography (EtOAc:hexane = from 1:6 to 1:2) to obtain tert-butyl[(3-cyano-1-benzothiophene-7-yl)methyl]carbamate (130 mg) as a white solid.

### Preparation Example 35

In a microwave reaction device, tert-butyl (3S,4R)-4-[(5-carbamoyl-2-chloropyridin-4-yl)amino]-3-fluoropiperidine-1-carboxylate (200 mg), 4-(pyrimidin-2-yloxy)aniline (181 mg), dicyclohexyl(2',4',6'-triisopropylbiphenyl-2-yl)phosphine (26 mg), sodium tert-butoxide (103 mg), and tris(dibenzylideneacetone)dipalladium (0) (49 mg) were mixed with tert-butanol (4 mL), followed by reaction at 130°C for 1 hour under microwave irradiation. To the reaction mixture was added a mixed solution of CHCl₃/MeOH (4:1), followed by stirring for 10 minutes, and then the insoluble materials were filtered through celite. The filtrate was concentrated under reduced pressure and then the residue was purified by silica gel column chromatography (CHCl₃:MeOH = from 100:0 to 85:15) to obtain tert-butyl (3S,4R)-4-[(5-carbamoyl-2-{[4-(pyrimidin-2-yloxy)phenyl]amino}pyridin-4-yl)amino]-3-fluoropiperidine-1-carboxylate (51 mg) as a white solid and tert-butyl (3S,4R)-4-({5-carbamoyl-2-[(4-hydroxyphenyl)amino]pyridin-4-yl}amino)-3-fluoropiperidine-1-carboxylate (85 mg) as a white solid.

### Preparation Example 36

Under a nitrogen gas atmosphere, (1-methylpiperidin-4-yl)methanol (1.1 g) was mixed with THF (7 mL) at 4°C, further followed by addition of potassium tert-butoxide (0.95 g). The mixture was stirred at 4°C for 1 hour and 1-fluoro-4-nitrobenzene (0.75 mL) was added thereto. The reaction mixture was stirred at room temperature for 2 hours, then extracted with CHCl₃, followed by washing with water. The organic layer was dried over MgSO₄ and then filtered. The filtrate was concentrated under reduced pressure to obtain 1-methyl-4-[(4-nitrophenoxy)methyl]piperidine (1.7 g) as a yellow amorphous substance.

### Preparation Example 37

2-Aminonicotinonitrile (250 mg) and a 40% aqueous chloroacetaldehyde solution (0.49 mL) were mixed with EtOH (5 mL) and allowed to undergo a reaction at 120°C for 1 hour in a microwave reaction device. The reaction mixture was extracted with CHCl₃ and washed with a saturated aqueous sodium hydrogen carbonate solution. The organic layer was dried over MgSO₄ and then concentrated under reduced pressure. The residue was washed with diisopropyl ether and then filtered to obtain imidazo[1,2-a]pyridine-8-carbonitrile (284 mg) as a white solid.

### Preparation Example 39

In the same manner as in Preparation Example 7, the compound of Preparation Example 39 shown in the Tables as described later was prepared.

### Preparation Example 40

In the same manner as in Preparation Example 20, the compounds of Preparation Example 40 and Preparation Examples 40-1 to 40-3 shown in the Tables as described later were prepared.

### Preparation Example 41

In the same manner as in Preparation Example 21, the compounds of Preparation Example 41 and Preparation Examples 41-1 to 41-3 shown in the Tables as described later were prepared.

### Preparation Example 42

In the same manner as in Preparation Example 23, the compounds of Preparation Example 42 and Preparation Example 42-1 shown in the Tables as described later were prepared.

### Preparation Example 43

In the same manner as in Preparation Example 25, the compounds of Preparation Example 43 and Preparation Examples 43-1 to 43-2 shown in the Tables as described later were prepared.

### Preparation Example 44

In the same manner as in Preparation Example 25, the compounds of Preparation Example 44 and Preparation Example 44-1 shown in the Tables as described later were prepared.

### Preparation Example 45

In the same manner as in Preparation Example 28, the compounds of Preparation Example 45 and Preparation Examples 45-1 to 45-3 shown in the Tables as described later were prepared.

### Preparation Example 46

In the same manner as in Preparation Example 25, the compound of Preparation Example 46 shown in the Tables as described later was prepared.

### Preparation Example 47

In the same manner as in Preparation Example 20, the compound of Preparation Example 47 shown in the Tables as described later was prepared.

### Preparation Example 48

In the same manner as in Preparation Example 14, the compound of Preparation Example 48 shown in the Tables as described later was prepared.

### Preparation Example 49

In the same manner as in Preparation Example 14, the compound of Preparation Example 49 shown in the Tables as described later was prepared.

For the Preparation Example Compounds, the structures are shown in Tables 2 to 27 and Tables 85 to 96 and the physicochemical data are shown in Tables 28 to 34 and Tables 97 to 99.

**[Table 2]**

| Pr | Structure | Pr | Structure |
|---|---|---|---|
| 1 | 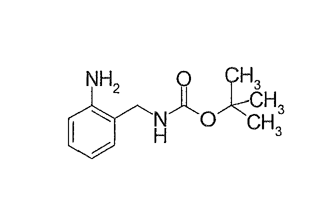 | 1-1 | 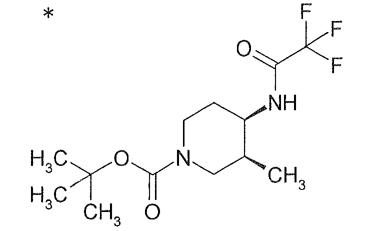 |
| 2 | 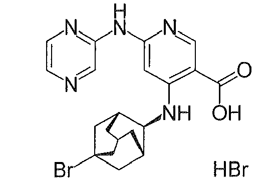 | 3 | 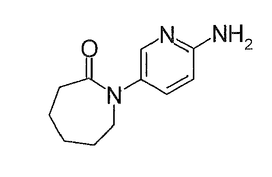 |
| 4 | 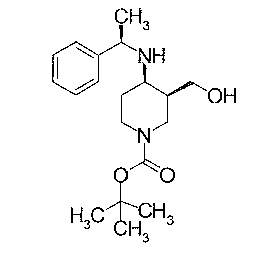 | 5 | 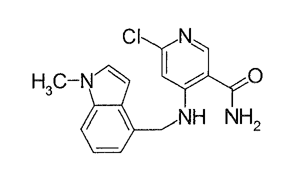 |
| 5-1 | 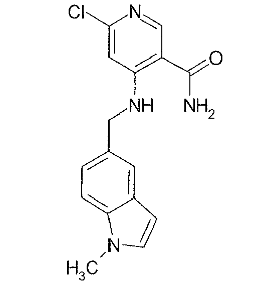 | 6 | 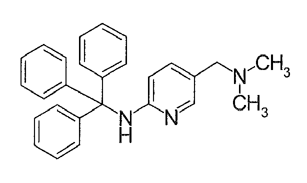 |
| 7 | 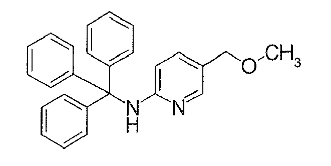 | 39 | 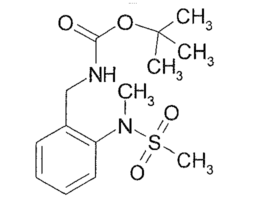 |

**[Table 3]**

| Pr | Structure | Pr | Structure |
|---|---|---|---|
| 8 | 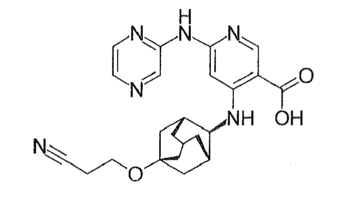 | 9 | 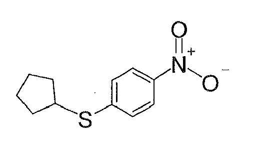 |
| 10 | 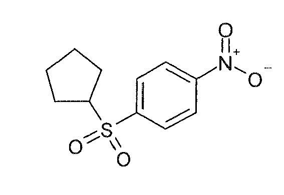 | 11 | 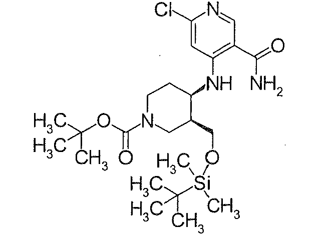 |
| 12 | 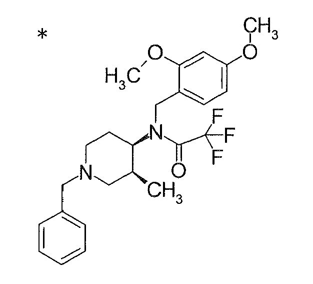 | 13 | 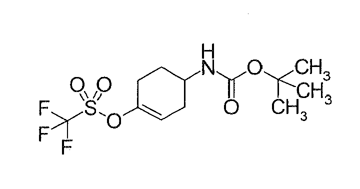 |
| 14 | 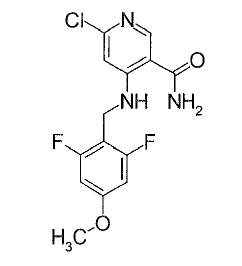 | 14-1 | 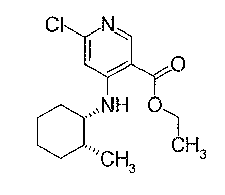 |
| 14-2 | 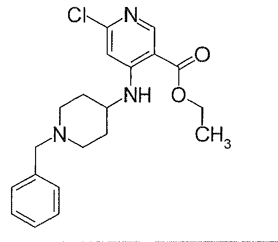 | 14-3 | 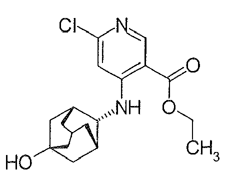 |

**[Table 4]**

| Pr | Structure | Pr | Structure |
|---|---|---|---|
| 14-4 | 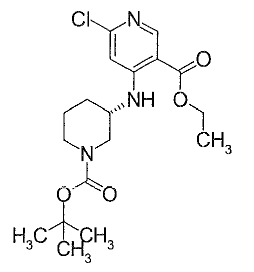 | 14-5 | 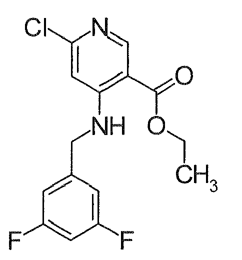 |
| 14-6 | 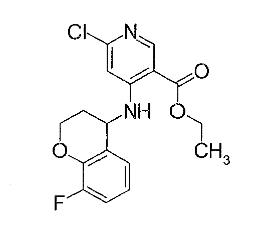 | 14-7 | 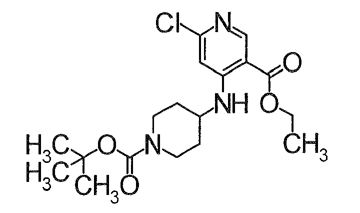 |
| 14-8 | 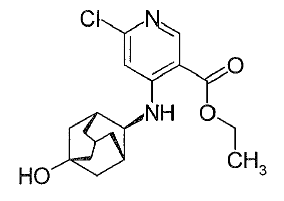 | 14-9 | 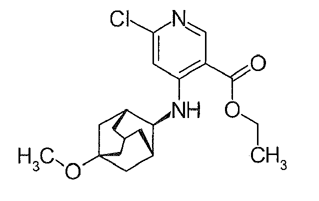 |
| 14-10 | 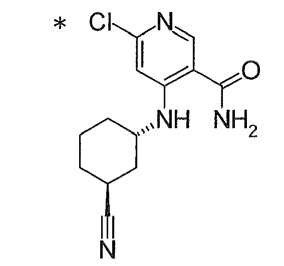 | 14-11 | 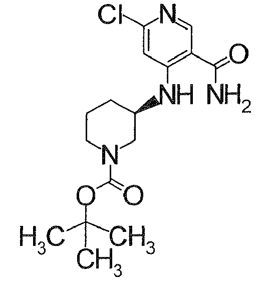 |
| 14-12 | 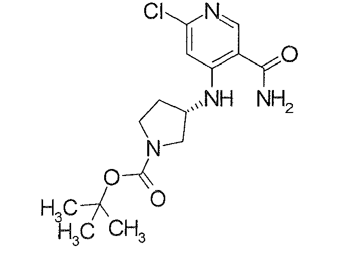 | 14-13 | 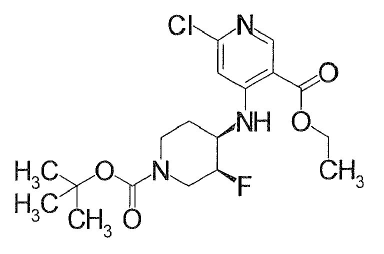 |

**[Table 5]**

| Pr | Strucure | Pr | Structure |
|---|---|---|---|
| 14-14 | 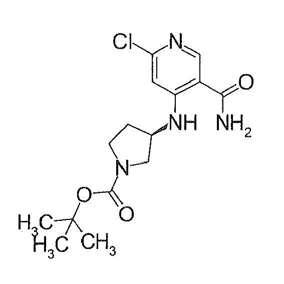 | 14-15 | 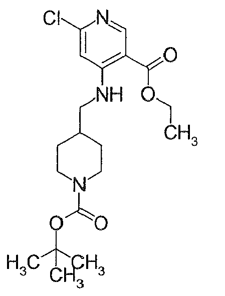 |
| 14-16 | 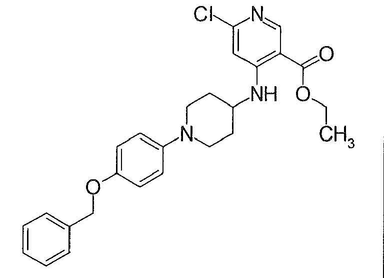 | 14-17 | 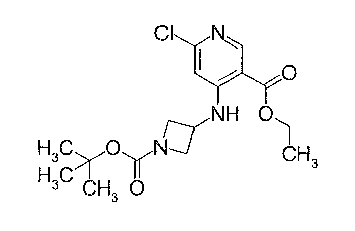 |
| 14-18 | 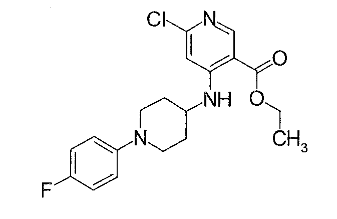 | 14-19 | 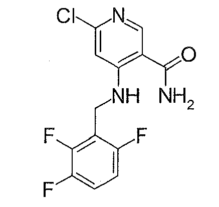 |
| 14-20 | 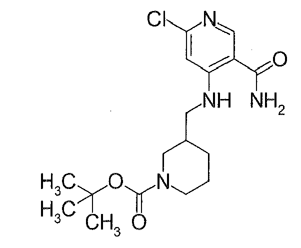 | 14-21 | 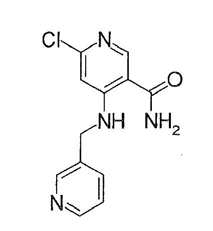 |
| 14-22 | 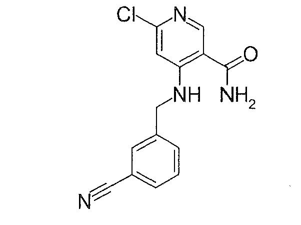 | 14-23 | 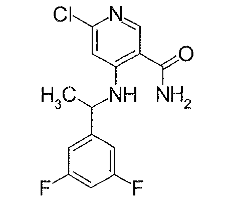 |

**[Table 6]**

| Pr | Structure | Pr | Structure |
|---|---|---|---|
| 14-24 | 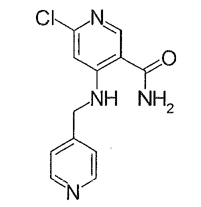 | 14-25 | 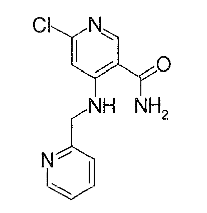 |
| 14-26 | 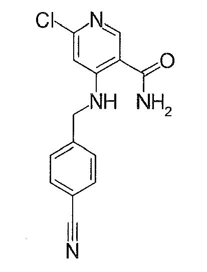 | 14-27 | 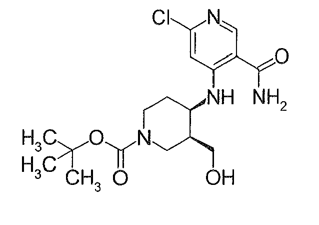 |
| 14-28 | 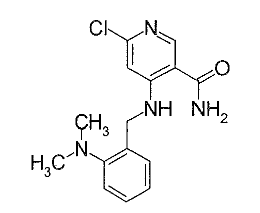 | 14-29 | 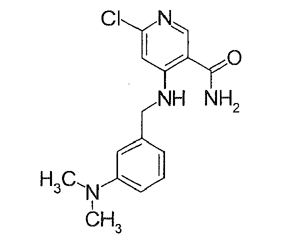 |
| 14-30 | 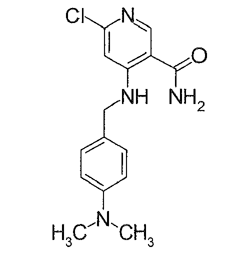 | 14-31 | 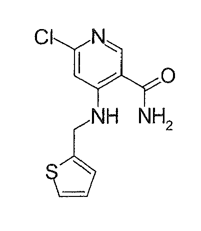 |
| 14-32 | 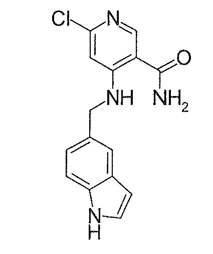 | 14-33 | 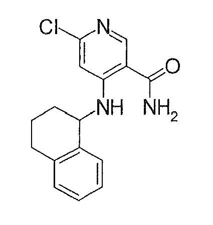 |

**[Table 7]**

| Pr | Structure | Pr | Structure |
|---|---|---|---|
| 14-34 | 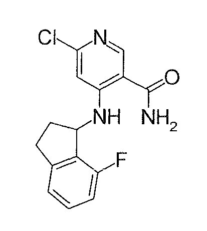 | 14-35 | 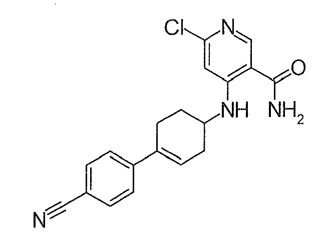 |
| 14-36 | 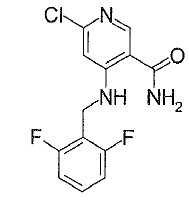 | 14-39 | 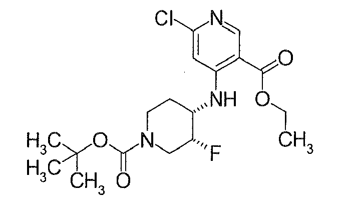 |
| 14-38 | 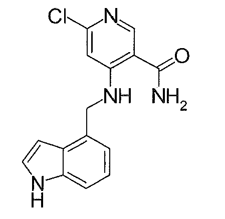 | 14-41 | 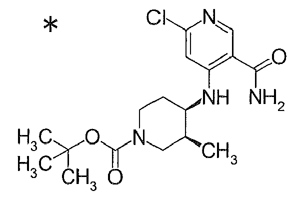 |
| 14-40 | 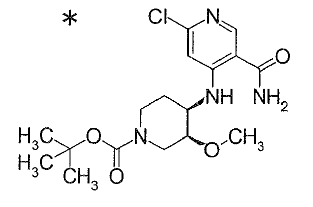 | 14-43 | 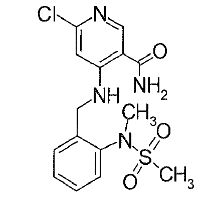 |
| 14-42 | 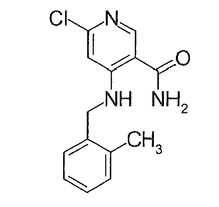 | | |

**[Table 8]**

| Pr | Structure | Pr | Structure |
|---|---|---|---|
| 14-44 | 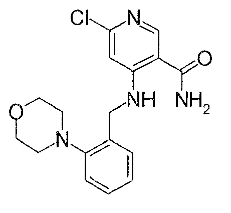 | 14-45 | 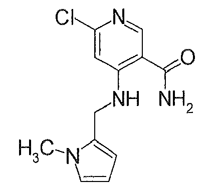 |
| 14-46 | 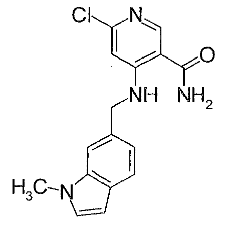 | 14-47 | 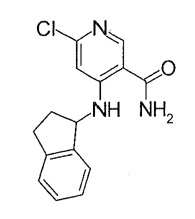 |
| 14-48 | 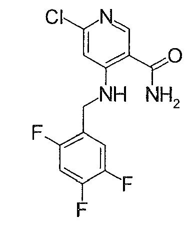 | 14-49 | 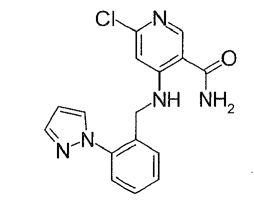 |
| 14-50 | 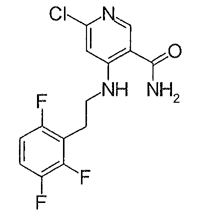 | 14-51 | 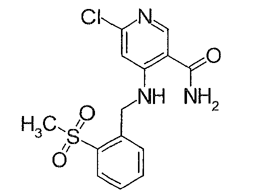 |
| 14-52 | 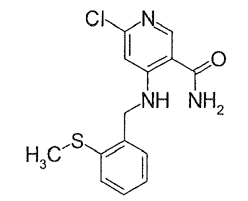 | 14-53 | 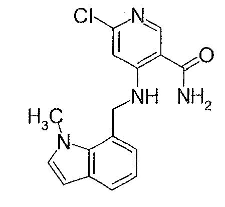 |

**[Table 9]**

| Pr | Structure | Pr | Structure |
|---|---|---|---|
| 14-54 | 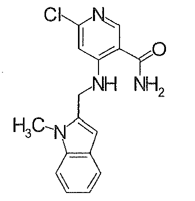 | 14-55 | 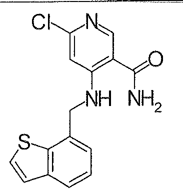 |
| 14-56 | 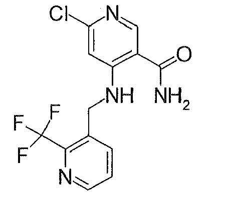 | 14-57 | 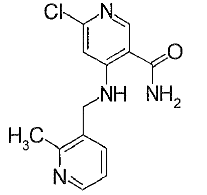 |
| 15 | 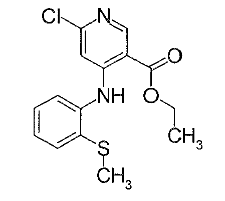 | 15-1 | 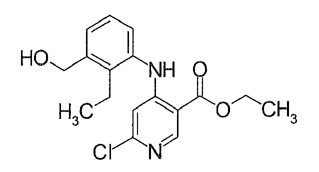 |
| 16 | 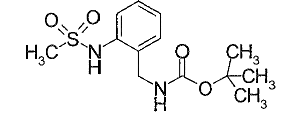 | 16-1 | 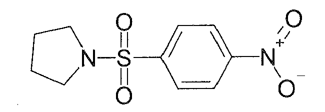 |
| 17 | 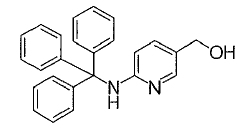 | 18 | 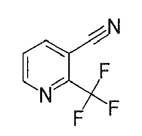 |
| 19 | 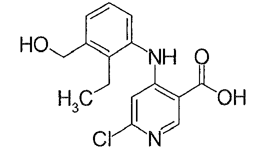 | 19-1 | 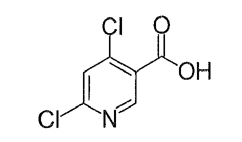 |
| 19-2 | 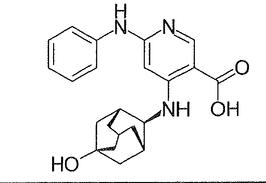 | 19-3 | 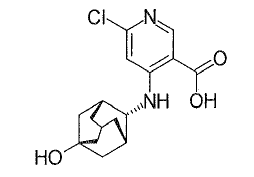 |

**[Table 10]**

| Pr | Structure | Pr | Structure |
|---|---|---|---|
| 19-4 | 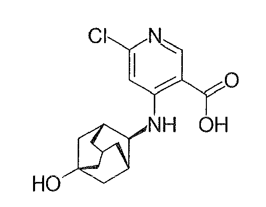 | 19-5 | 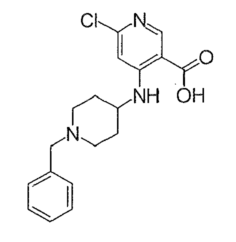 |
| 19-6 | 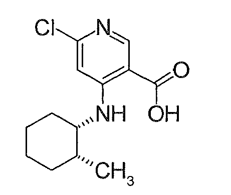 | 19-7 | 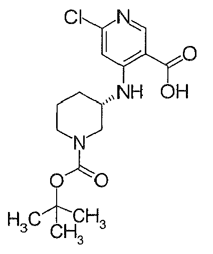 |
| 19-8 | 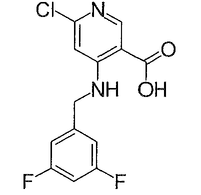 | 19-9 | 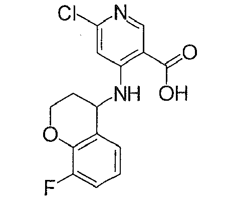 |
| 19-10 | 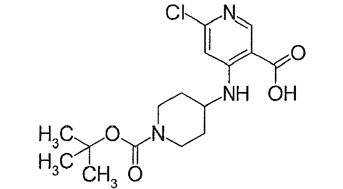 | 19-11 | 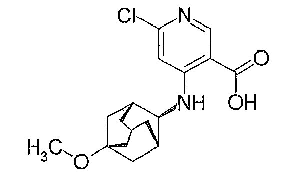 |
| 19-12 | 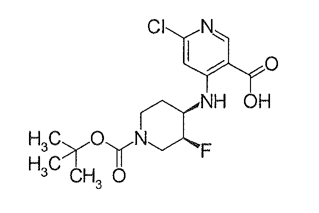 | 19-13 | 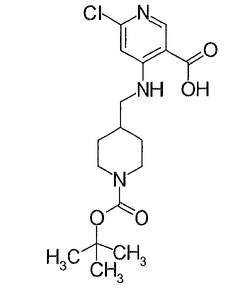 |

**[Table 11]**

| Pr | Structure | Pr | Structure |
|---|---|---|---|
| 19-14 | 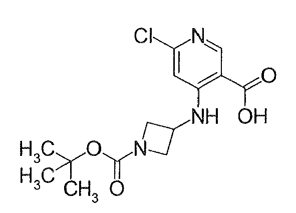 | 19-15 | 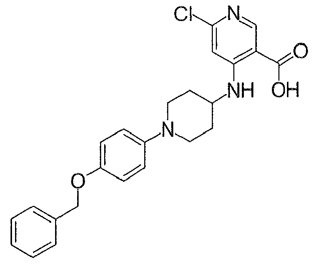 |
| 19-16 | 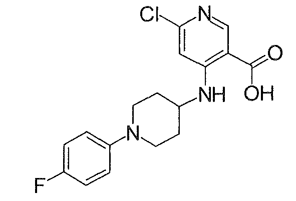 | 19-17 | 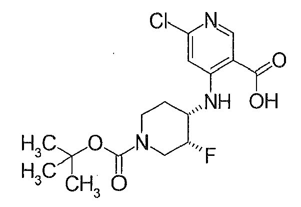 |
| 19-18 | 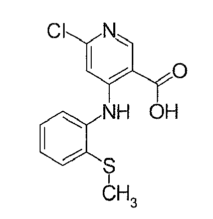 | 20 | 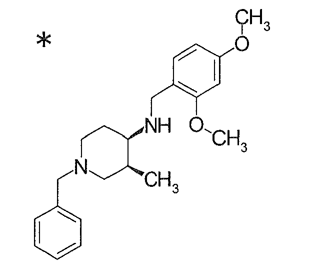 |
| 40 | 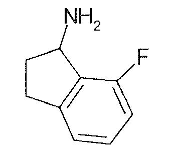 | 21 | 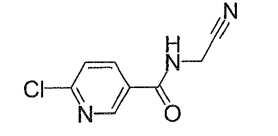 |
| 21-1 | 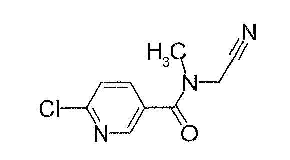 | 41 | 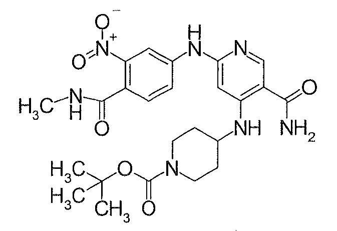 |

**[Table 12]**

| Pr | Structure | Pr | Structure |
|---|---|---|---|
| 41-1 | 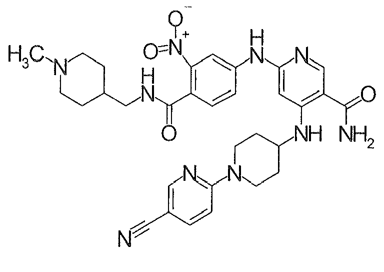 | 41-2 | 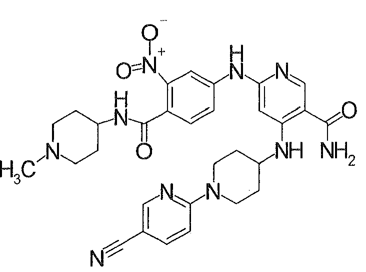 |
| 41-3 | 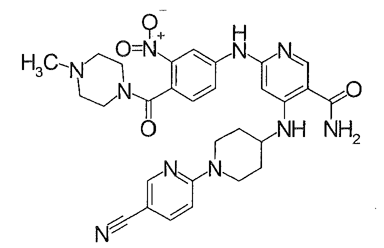 | 22 | 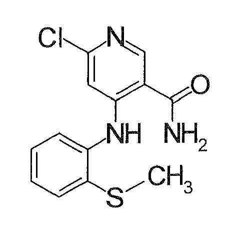 |
| 22-1 | 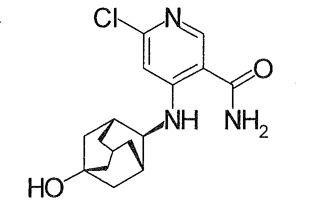 | 22-2 | 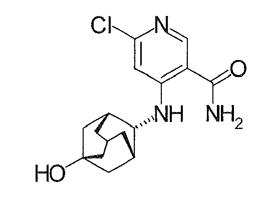 |
| 22-3 | 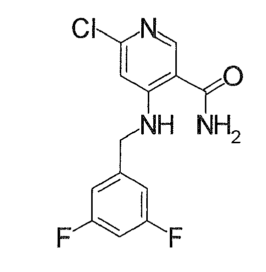 | 22-4 | 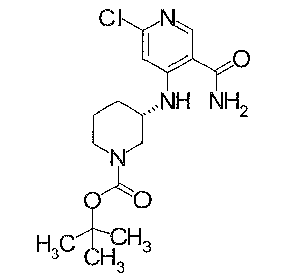 |
| 22-5 | 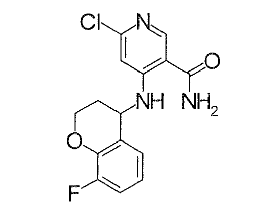 | 22-6 | 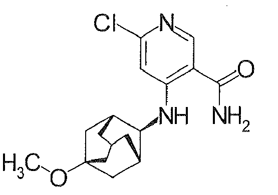 |

**[Table 13]**

| Pr | Structure | Pr | Structure |
|---|---|---|---|
| 22-7 | 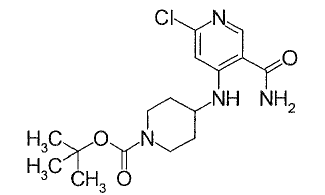 | 22-8 | 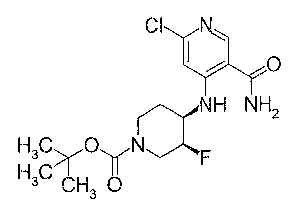 |
| 22-9 | 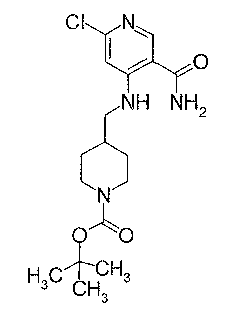 | 22-10 | 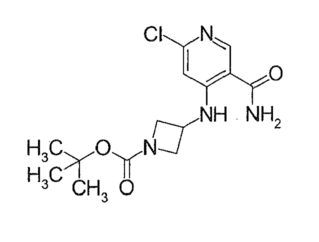 |
| 22-11 | 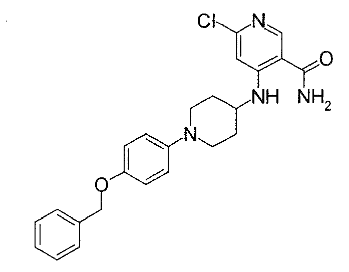 | 22-12 | 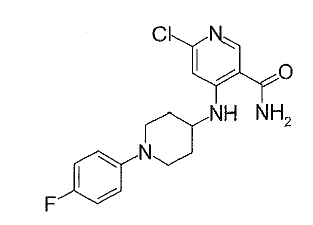 |
| 22-13 | 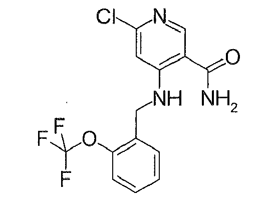 | 22-14 | 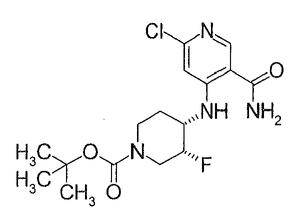 |
| 22-15 | 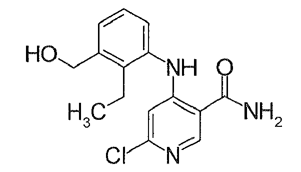 | 22-16 | 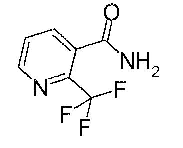 |

**[Table 14]**

| Pr | Structure | Pr | Structure |
|---|---|---|---|
| 22-17 | 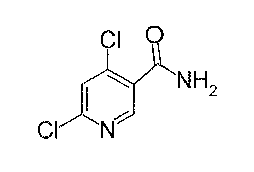 | 22-18 | 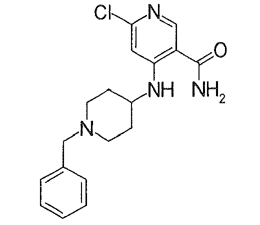 |
| 22-19 | 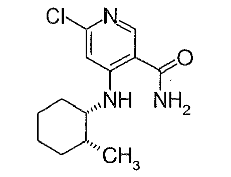 | 23 | 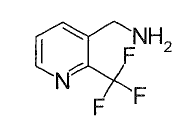 |
| 23-1 | 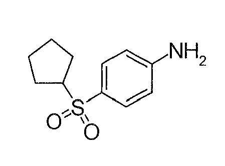 | 42 | 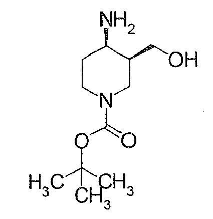 |
| 42-1 | 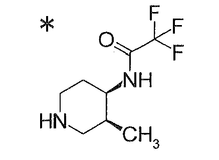 | 23-4 | 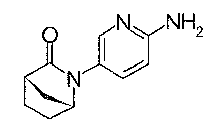 |
| 23-5 | 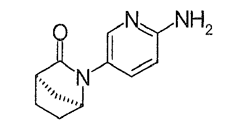 | 23-6 | 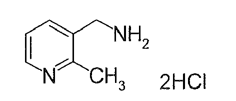 |
| 23-7 | 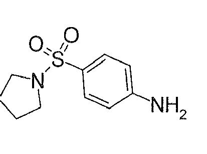 | 24 | 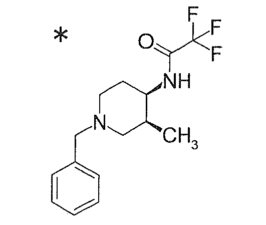 |

**[Table 15]**

| Pr | Structure | Pr | Structure |
|---|---|---|---|
| 25 | 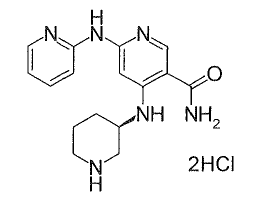 | 25-1 | 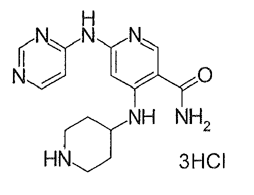 |
| 25-2 | 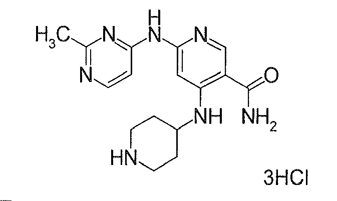 | 25-3 | 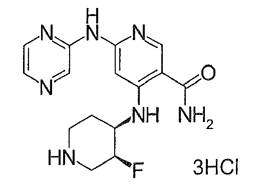 |
| 25-4 | 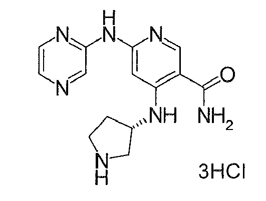 | 25-5 | 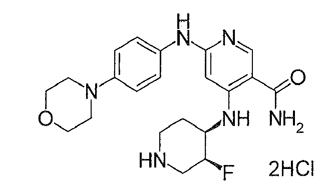 |
| 25-6 | 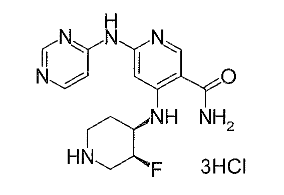 | 25-7 | 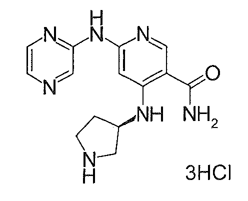 |
| 25-8 | 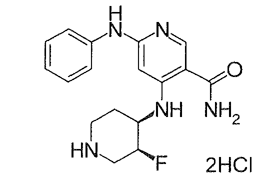 | 25-9 | 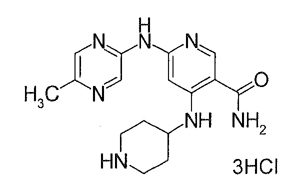 |
| 25-10 | 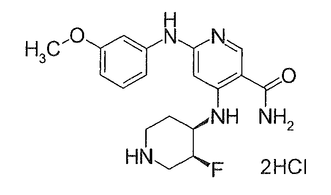 | 25-11 | 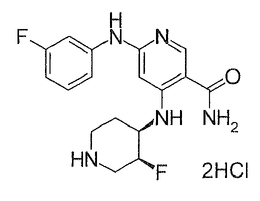 |

**[Table 16]**

| Pr | Structure | Pr | Structure |
|---|---|---|---|
| 43 | 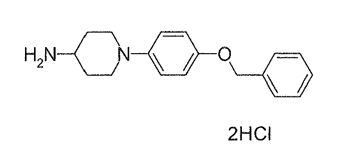 | 25-13 | 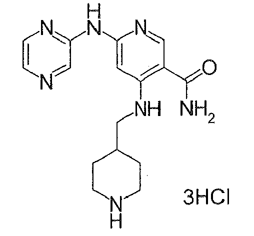 |
| 43-1 | 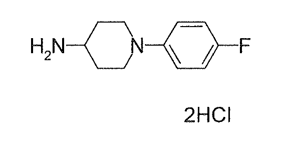 | 25-15 | 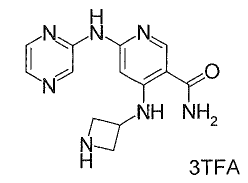 |
| 25-16 | 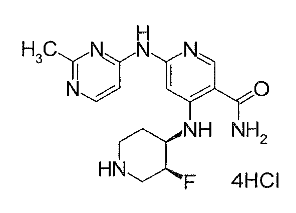 | 25-17 | 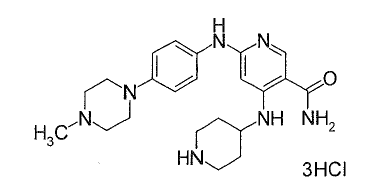 |
| 25-18 | 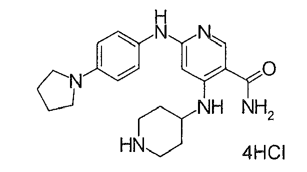 | 25-19 | 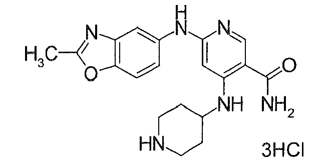 |
| 25-20 | 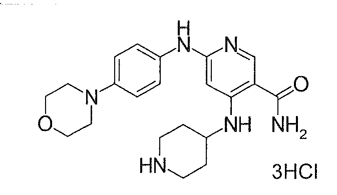 | 25-21 | 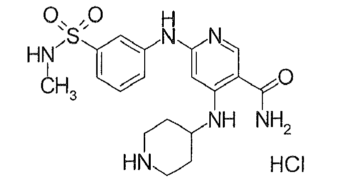 |
| 25-22 | 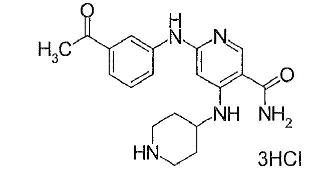 | 25-23 | 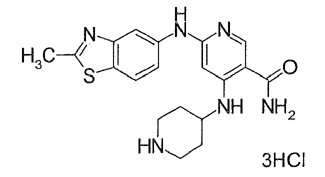 |

**[Table 17]**

| Pr | Structure | Pr | Structure |
|---|---|---|---|
| 25-24 | 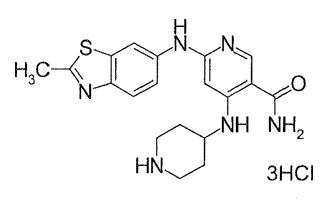 | 25-25 | 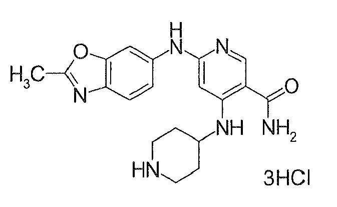 |
| 25-26 | 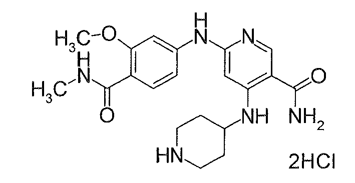 | 25-27 | 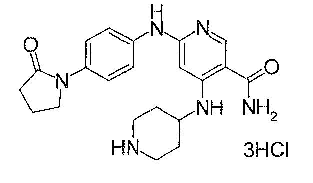 |
| 25-28 | 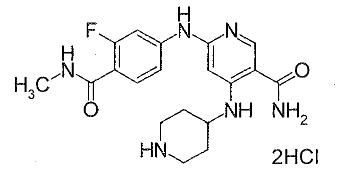 | 25-29 | 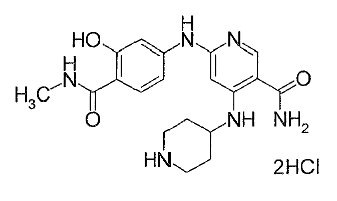 |
| 25-30 | 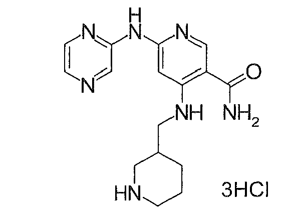 | 25-31 | 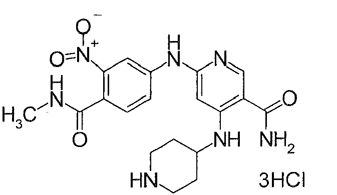 |
| 25-32 | 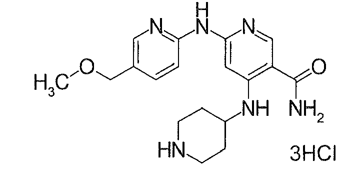 | 25-33 | 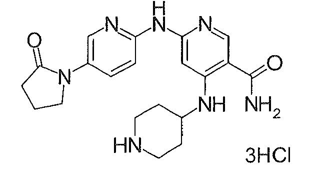 |
| 25-34 | 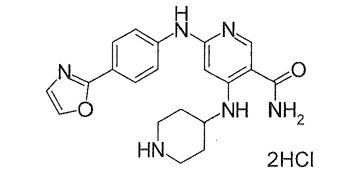 | 25-35 | 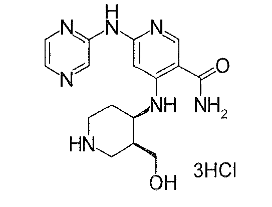 |

**[Table 18]**

| Pr | Structure | Pr | Structure |
|---|---|---|---|
| 25-36 | 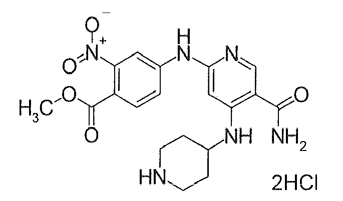 | 25-37 | 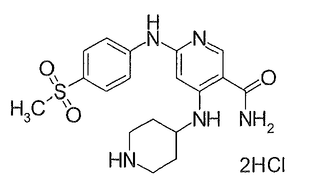 |
| 43-2 | 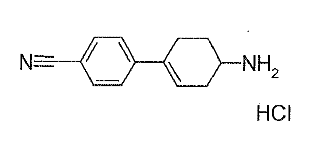 | 25-39 | 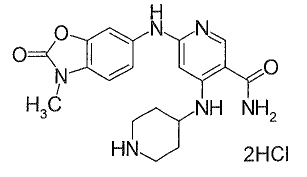 |
| 25-40 | 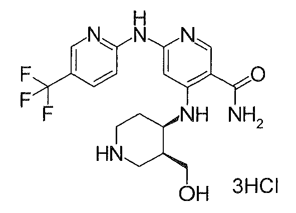 | 25-41 | 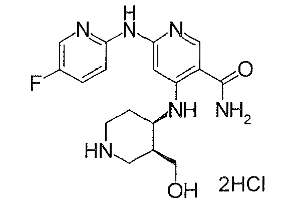 |
| 25-42 | 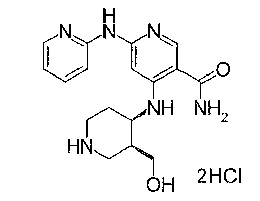 | 25-43 | 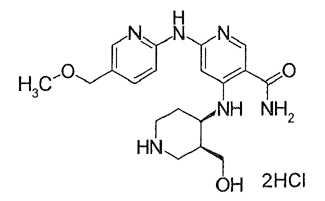 |
| 25-44 | 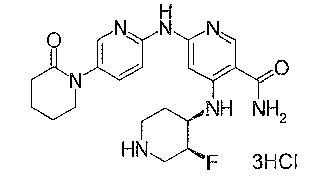 | 25-45 | 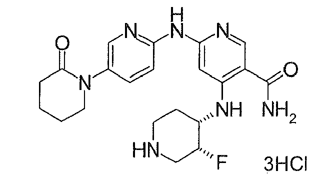 |
| 25-46 | 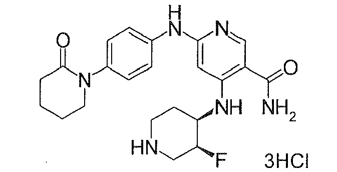 | 25-47 | 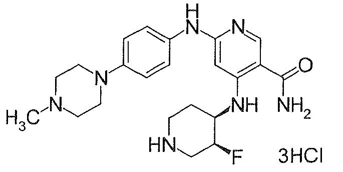 |

**[Table 19]**

| Pr | Structure | Pr | Structure |
|---|---|---|---|
| 25-48 | 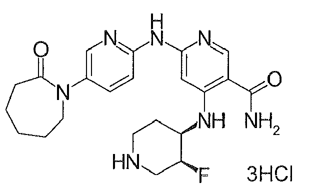 | 25-49 | 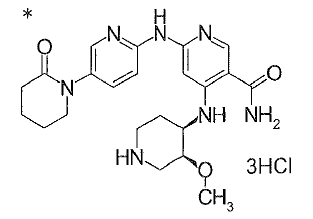 |
| 25-50 | 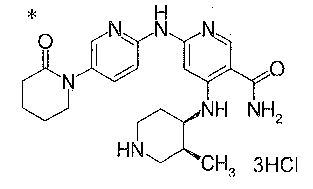 | 25-51 | 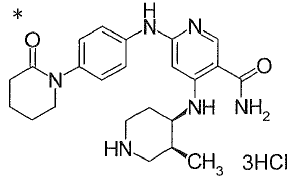 |
| 25-52 | 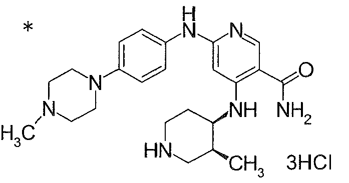 | 25-53 | 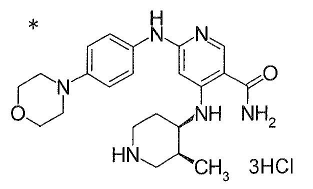 |
| 25-54 | 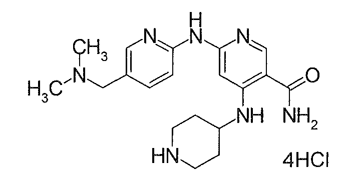 | 25-55 | 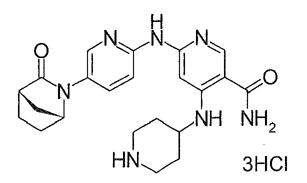 |
| 25-56 | 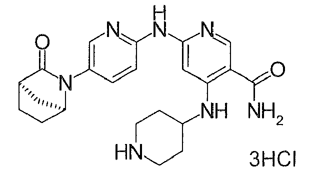 | 44 | 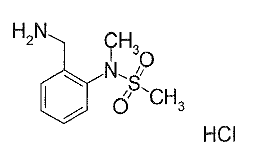 |
| 25-58 | 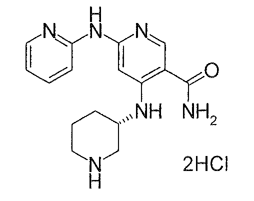 | 26 | 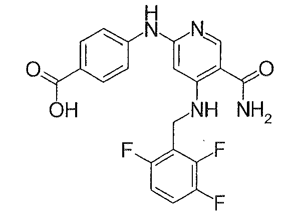 |

**[Table 20]**

| Pr | Structure | Pr | Structure |
|---|---|---|---|
| 26-1 | 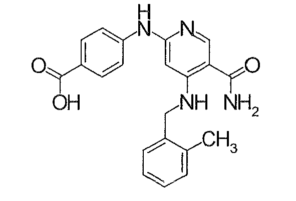 | 27 | 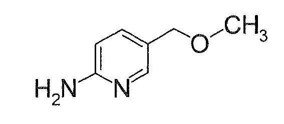 |
| 27-1 | 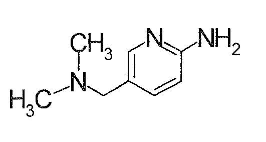 | 28 | 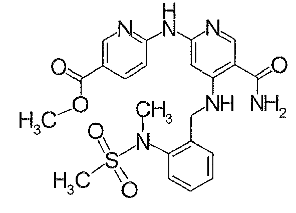 |
| 28-1 | 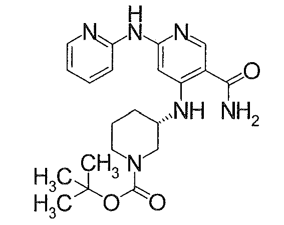 | 28-2 | 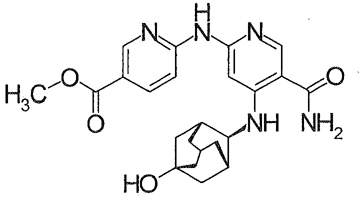 |
| 45 | 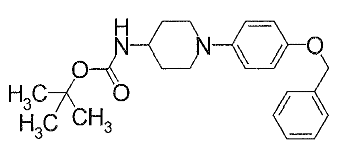 | 28-4 | 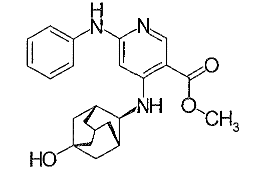 |
| 28-5 | 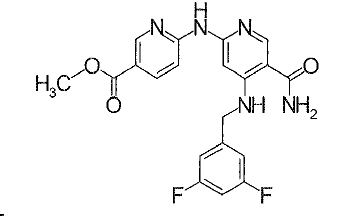 | 28-6 | 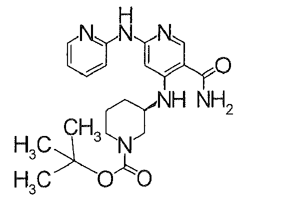 |
| 28-7 | 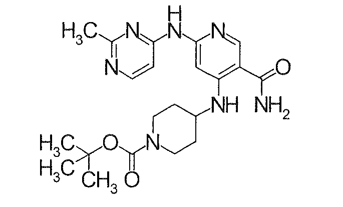 | 28-8 | 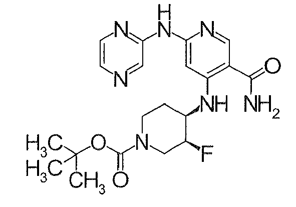 |

**[Table 21]**

| Pr | Structure | Pr | Structure |
|---|---|---|---|
| 28-9 | 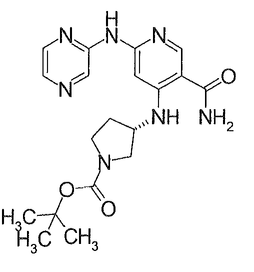 | 28-10 | 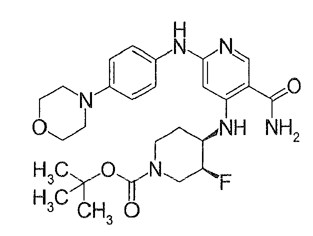 |
| 28-11 | 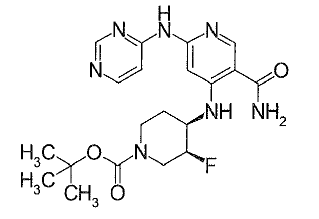 | 28-12 | 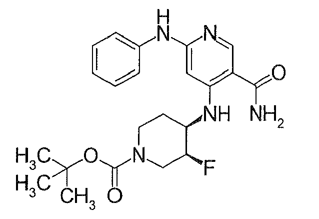 |
| 28-13 | 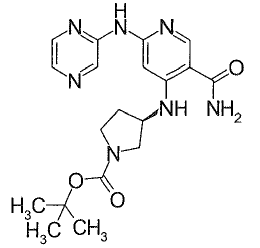 | 28-14 | 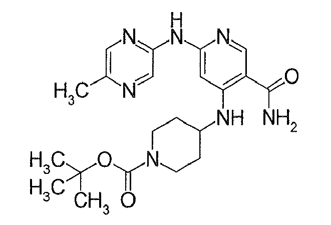 |
| 28-15 | 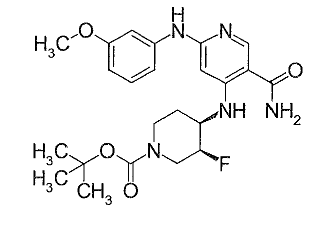 | 28-16 | 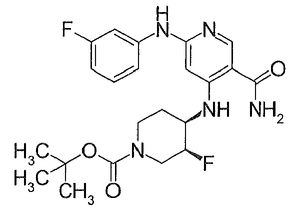 |
| 28-17 | 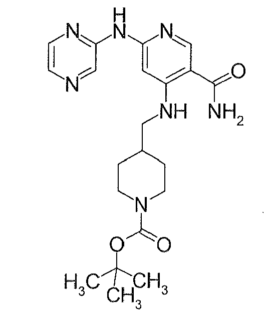 | 45-1 | 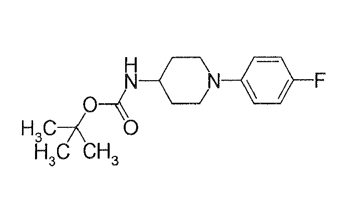 |

**[Table 22]**

| Pr | Structure | Pr | Structure |
|---|---|---|---|
| 28-19 | 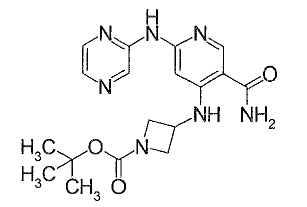 | 28-20 | 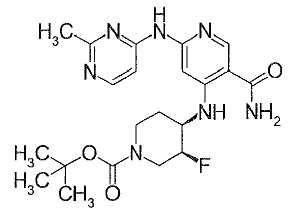 |
| 28-21 | 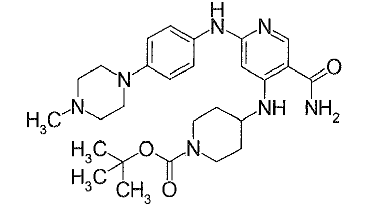 | 28-22 | 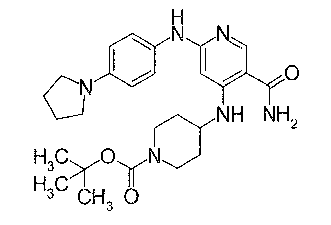 |
| 28-23 | 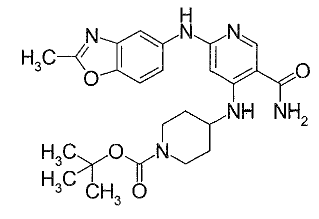 | 28-24 | 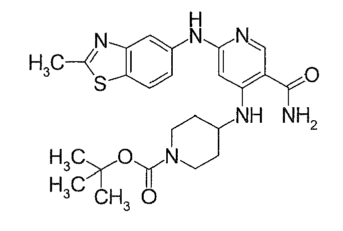 |
| 28-25 | 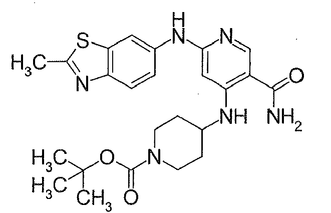 | 28-26 | 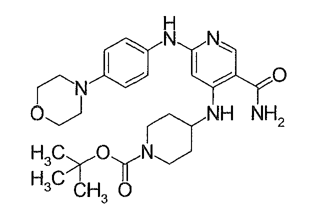 |
| 28-27 | 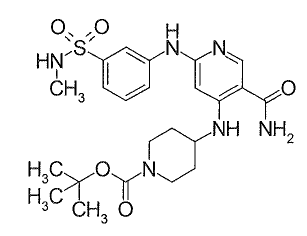 | 28-28 | 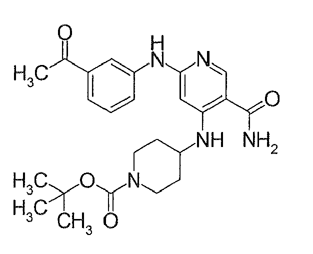 |

**[Table 23]**

| Pr | Structure | Pr | Structure |
|---|---|---|---|
| 28-29 | 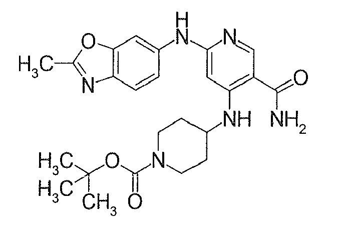 | 28-30 | 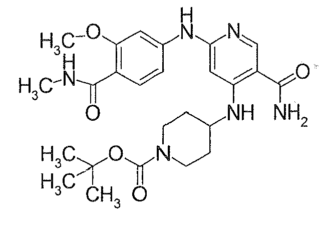 |
| 28-31 | 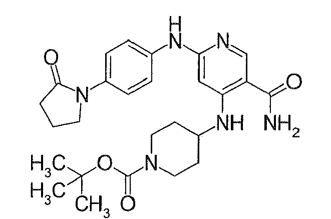 | 28-32 | 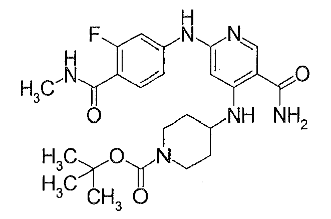 |
| 28-33 | 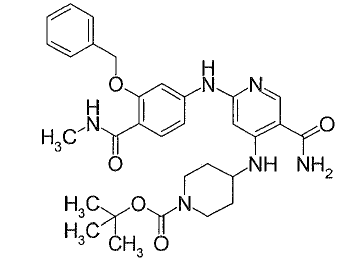 | 28-34 | 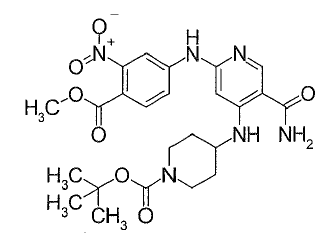 |
| 28-35 | 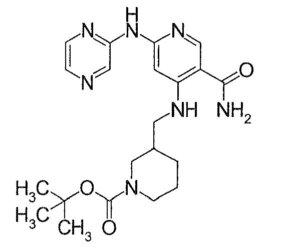 | 28-36 | 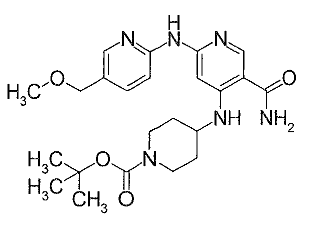 |
| 28-37 | 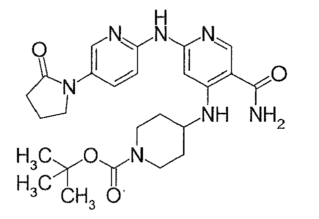 | 28-38 | 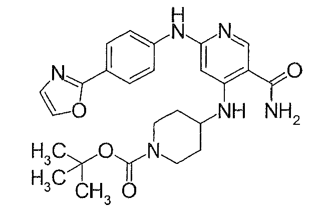 |

**[Table 24]**

| Pr | Structure | Pr | Structure |
|---|---|---|---|
| 28-39 | 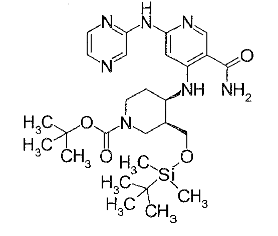 | 28-40 | 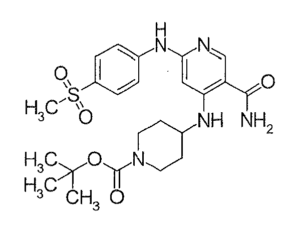 |
| 28-41 | 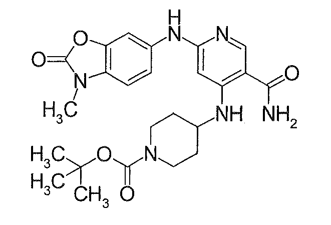 | 28-42 | 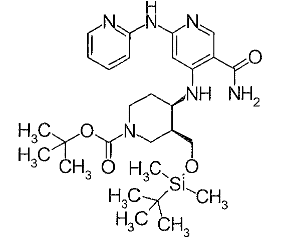 |
| 28-43 | 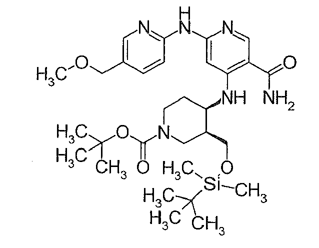 | 28-44 | 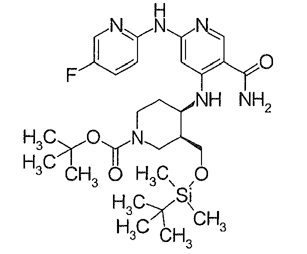 |
| 28-45 | 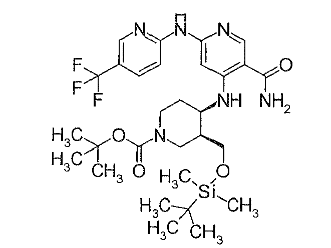 | 28-46 | 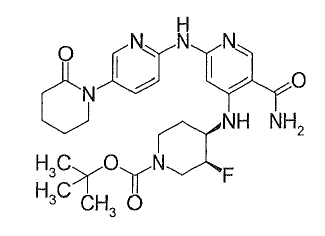 |
| 28-47 | 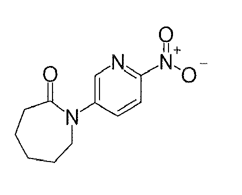 | 28-48 | 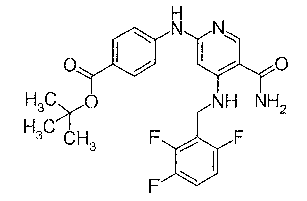 |

**[Table 25]**

| Pr | Structure | Pr | Structure |
|---|---|---|---|
| 28-49 | 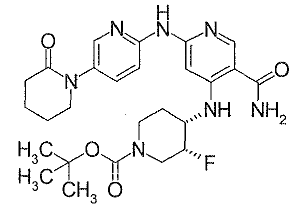 | 28-50 | 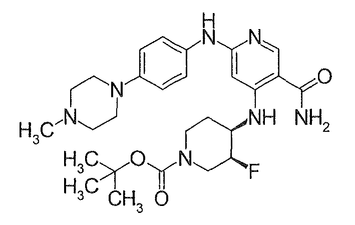 |
| 28-51 | 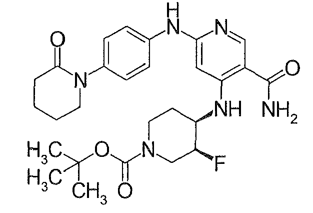 | 28-52 | 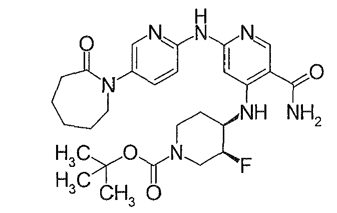 |
| 28-53 | 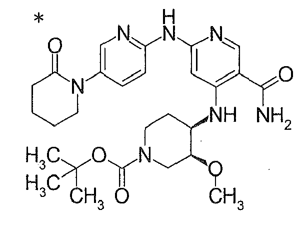 | 28-54 | 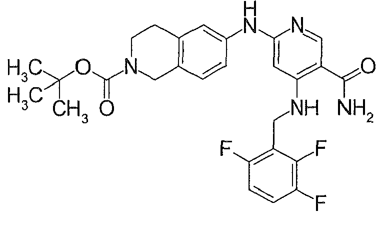 |
| 28-55 | 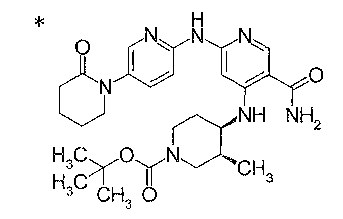 | 28-56 | 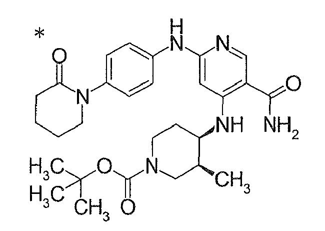 |
| 28-57 | 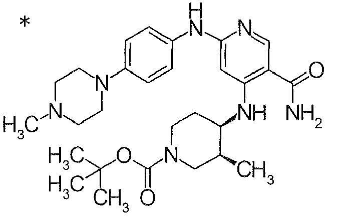 | 28-58 | 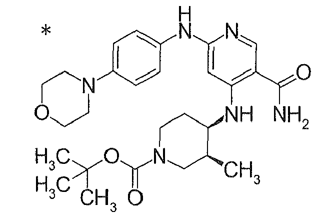 |

**[Table 26]**

| Pr | Structure | Pr | Structure |
|---|---|---|---|
| 28-59 | 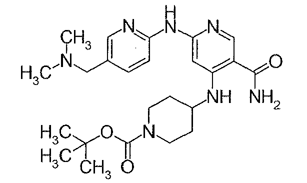 | 45-2 | 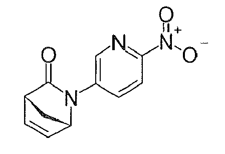 |
| 28-61 | 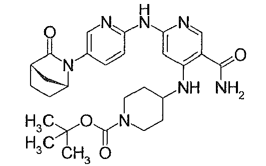 | 45-3 | 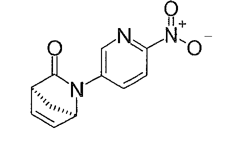 |
| 28-63 | 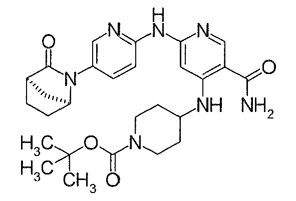 | 28-64 | 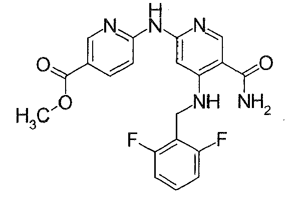 |
| 28-65 | 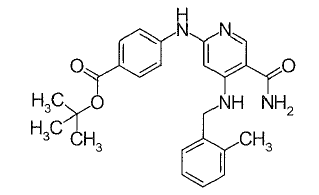 | 29 | 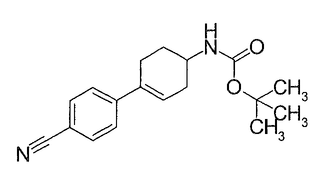 |
| 30 | 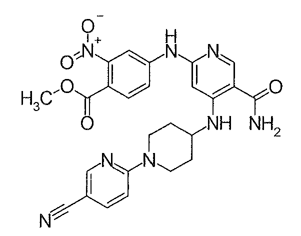 | 31 | 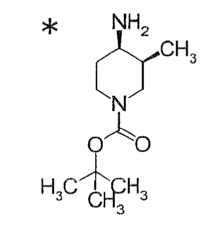 |
| 32 | 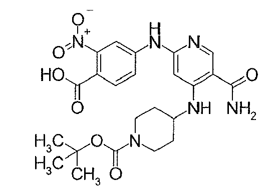 | 32-1 | 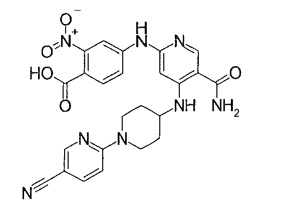 |

**[Table 27]**

| Pr | Structure | Pr | Structure |
|---|---|---|---|
| 32-2 | 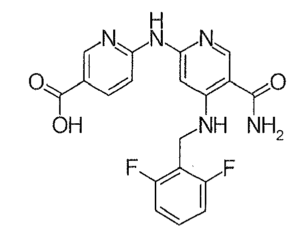 | 32-3 | 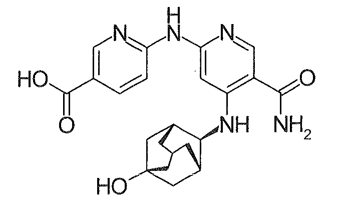 |
| 32-4 | 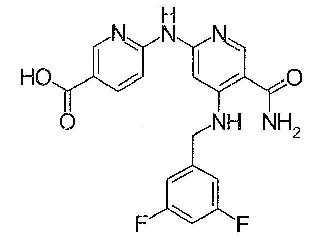 | 32-5 | 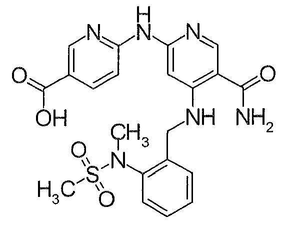 |
| 33 | 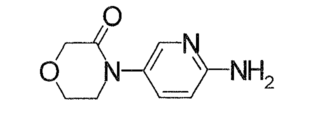 | 33-1 | 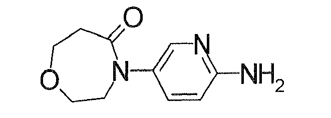 |
| 33-2 | 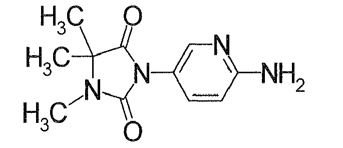 | 33-3 | 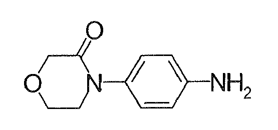 |

**[Table 28]**

| Pr | Data |
|---|---|
| 1 | ESI+ : 245 [M+Na]+ |
| 1-1 | ESI- : 309 [M-H]- |
| 2 | ESI+ : 444,446 [M+H]+ |
| 3 | ESI+ : 206 [M+H]+ |
| 4 | ESI+ : 335 [M+H]+ |
| 5 | ESI+ : 337 [M+Na]+ |
| 5-1 | ESI+ : 315 [M+H]+ |
| 6 | ESI+ : 394 [M+H]+ |
| 7 | ESI+ : 381 [M+H]+ |
| 39 | ESI+ : 337 [M+Na]+ |
| 8 | ESI+ : 435 [M+H]+ |
| 9 | ESI+ : 246 [M+Na]+ |
| 10 | ESI+ : 278 [M+Na]+ |
| 11 | ESI+ : 521 [M+Na]+ |
| 12 | ESI+ : 451 [M+H]+ |
| 13 | ESI+ : 368 [M+Na]+ |
| 14 | ESI+ : 350 [M+Na]+ |
| 14-1 | ESI+ : 297 [M+H]+ |
| 14-2 | ESI+ : 374 [M+H]+ |
| 14-3 | ESI+ : 351 [M+H]+ |
| 14-4 | ESI+ : 384 [M+H]+ |
| 14-5 | ESI+ : 327 [M+H]+ |
| 14-6 | ESI+ : 351 [M+H]+ |
| 14-7 | ESI+ : 384 [M+H]+ |
| 14-8 | ESI+ : 351 [M+H]+ |
| 14-9 | ESI+ : 365 [M+H]+ |
| 14-10 | ESI+ : 279 [M+H]+ |
| 14-11 | ESI+ : 377 [M+Na]+ |
| 14-12 | ESI+ : 363 [M+Na]+ |
| 14-13 | ESI+ : 402 [M+H]+ |
| 14-14 | ESI+ : 363 [M+Na]+ |
| 14-15 | ESI+ : 398 [M+H]+ |
| 14-16 | ESI+ : 466 [M+H]+ |
| 14-17 | ESI+ : 356 [M+H]+ |
| 14-18 | ESI+ : 378 [M+H]+ |
| 14-19 | ESI+ : 316 [M+H]+ |
| 14-20 | ESI+ : 391 [M+Na]+ |
| 14-21 | ESI+ : 285 [M+Na]+ |
| 14-22 | ESI+ : 309 [M+Na]+ |
| 14-23 | ESI+ : 312[M+H]+ |
| 14-24 | ESI- : 261 [M-H]- |
| 14-25 | ESI+ : 285 [M+Na]+ |
| 14-26 | ESI- : 285 [M-H]- |
| 14-27 | ESI+ : 407 [M+Na]+ |
| 14-28 | ESI+ : 305 [M+H]+ |
| 14-29 | ESI- : 303 [M-H]- |
| 14-30 | ESI- : 303 [M-H]- |

**[Table 29]**

| Pr | Data |
|---|---|
| 14-31 | ESI+ : 268 [M+H]+ |
| 14-32 | ESI+ : 323 [M+Na]+ |
| 14-33 | ESI+ : 302 [M+H]+ |
| 14-34 | ESI+ : 306 [M+H]+ |
| 14-35 | ESI+ : 353 [M+H]+ |
| 14-36 | ESI- : 296 [M-H]- |
| 14-38 | ESI+ : 301 [M+H]+ |
| 14-39 | ESI+ : 424 [M+Na]+ |
| 14-40 | ESI+ : 407 [M+Na]+ |
| 14-41 | ESI+ : 391 [M+Na]+ |
| 14-42 | ESI+ : 276 [M+H]+ |
| 14-43 | ESI+ : 369 [M+H]+, ESI+ : 391 [M+Na]+ |
| 14-44 | ESI- : 345 [M-H]- |
| 14-45 | ESI+ : 265 [M+H]+ |
| 14-46 | ESI- : 313 [M-H]- |
| 14-47 | ESI+ : 288 [M+H]+ |
| 14-48 | ESI+ : 316 [M+H]+ |
| 14-49 | ESI+ : 350 [M+Na]+ |
| 14-50 | ESI+ : 330 [M+H]+ |
| 14-51 | ESI+ : 362 [M+Na]+ |
| 14-52 | ESI+ : 330 [M+Na]+ |
| 14-53 | ESI+ : 315[M+H]+ |
| 14-54 | ESI+ : 337 [M+Na]+ |
| 14-55 | ESI+ : 340 [M+Na]+ |
| 14-56 | ESI- : 329 [M-H]- |
| 14-57 | ESI+ : 299 [M+Na]+ |
| 15 | ESI+ : 323 [M+H]+ |
| 15-1 | NMR-DMSO-d₆: 1.03 (3H, t, J = 7.2 Hz), 1.36 (3H, t, J = 7.2 Hz), 2.57 (2H, q, J = 7.2 Hz), 4.38 (2H, q, J = 7.2 Hz), 4.59 (2H, d, J = 5.2 Hz), 5.22 (1H, t, J = 5.2 Hz), 6.38 (1H, s), 7.21-7.25 (1H, m), 7.30-7.45 (1H, m), 7.40-7.43 (1H, m), 8.66 (1H, s), 9.62 (1H, s) ESI+ : 335 [M+H}+ |
| 16 | ESI+ : 323 [M+H]+ |
| 16-1 | ESI+ : 279 [M+Na]+ |
| 17 | ESI+ : 367 [M+H]+ |
| 18 | NMR-CDCl₃ : 7.68 (1H, dd, J = 7.9, 4.9 Hz), 8.21 (1H, ddd, J = 7.9,1.4, 0.5 Hz), 8.92 (1H, dd, J = 4.8,1.4 Hz) |
| 19 | ESI+ : 329 [M+Na]+ |
| 19-1 | ESI- : 190 [M-H]- |
| 19-2 | ESI+ : 380 [M+H]+ |
| 19-3 | ESI- : 321 [M-H]- |
| 19-4 | ESI+ : 323 [M+H]+ |
| 19-5 | ESI+ : 346 [M+H]+ |
| 19-6 | ESI+ : 269 [M+H]+ |
| 19-7 | ESI+ : 378 [M+Na]+ |

**[Table 30]**

| Pr | Data |
|---|---|
| 19-8 | ESI- : 297 [M-H]- |
| 19-9 | ESI+ : 323 [M+H]+ |
| 19-10 | ESI+ : 356 [M+H]+ |
| 19-11 | ESI+ : 337 [M+H]+ |
| 19-12 | ESI+ : 396 [M+Na]+ |
| 19-13 | ESI+: 392 [M+Na]+ |
| 19-14 | ESI+ : 350 [M+Na]+ |
| 19-15 | ESI- : 436 [M-H]- |
| 19-16 | ESI+ : 350 [M+H]+ |
| 19-17 | ESI+ : 396 [M+Na]+ |
| 19-18 | ESI- : 293 [M-H]- |
| 20 | ESI+ : 355 [M+H]+ |
| 40 | NMR-DMSO-d₆ : 1.68-1.75 (1H, m), 1.84 (2H, br), 2.24-2.33 (1H, m), 2.70-2.79 (1H, m), 2.97-3.06 (1H, m), 4.47-4.51 (1H, m), 6.89-6.95 (1H, m), 7.03 (1H, d, J = 7.6 Hz), 7.17-7.23 (1H, m) |
| 21 | ESI+ : 218 [M+Na]+ |
| 21-1 | ESI+ : 232 [M+Na]+ |
| 41 | ESI+ : 536 [M+Na]+ |
| 41-1 | ESI+ : 613 [M+H]+ |
| 41-2 | ESI+ : 599 [M+H]+ |
| 41-3 | ESI+ : 607 [M+Na]+ |
| 22 | ESI+ : 316 [M+Na]+ |
| 22-1 | ESI+ : 322 [M+H]+ |
| 22-2 | ESI+ : 344 [M+Na]+ |
| 22-3 | ESI- : 296 [M-H]- |
| 22-4 | ESI+ : 377 [M+Na]+ |
| 22-5 | ESI+ : 322 [M+H]+ |
| 22-6 | ESI+ : 336 [M+H]+ |
| 22-7 | ESI+ : 377 [M+Na]+ |
| 22-8 | ESI+ : 395 [M+Na]+ |
| 22-9 | ESI+ : 391 [M+Na]+ |
| 22-10 | ESI+ : 349 [M+Na]+ |
| 22-11 | ESI+ : 437 [M+H]+ |
| 22-12 | ESI- : 347 [M-H]- |
| 22-13 | NMR-DMSO-d6 : 9.19 (1H, t, J = 6.0 Hz), 8.43 (1H, s), 8.13 (1H, bs), 7.54 (1H, bs), 7.47-7.37 (4H, m), 6.62 (1H, s), 4.55 (2H, d, J = 6.0 Hz). ESI+ : 346 [M+H]+ |
| 22-14 | ESI+ : 395 [M+Na]+ |
| 22-15 | ESI+ : 328 [M+Na]+ |
| 22-16 | ESI+ : 213 [M+Na]+ |
| 22-17 | ESI- : 189 [M-H]- |
| 22-18 | ESI+ : 345 [M+H]+ |
| 22-19 | ESI+ : 268 [M+H]+ |
| 23 | NMR-DMSO-d₆ : 3.90 (2H, br s), 7.72 (1H, dd, J = 7.9, 4.7 Hz), 8.26 (1H, dd, J = 7.9, 0.7 Hz), 8.57 (1H, dd, J = 4.7, 0.7 Hz) ESI+ : 177 [M+H]+ |
| 23-1 | ESI+ : 248 [M+Na]+ |
| 42 | ESI+ : 231 [M+H]+ |

**[Table 31]**

| Pr | Data |
|---|---|
| 42-1 | ESI+ : 211 [M+H]+ |
| 23-4 | ESI+ : 204 [M+H]+ |
| 23-5 | ESI+ : 204 [M+H]+ |
| 23-6 | ESI+ : 123 [M+H]+ |
| 23-7 | ESI+ : 227 [M+H]+ |
| 24 | ESI+ : 301 [M+H]+ |
| 25 | ESI+ : 313 [M+H]+ |
| 25-1 | ESI+ : 314 [M+H]+ |
| 25-2 | ESI+ : 328 [M+H]+ |
| 25-3 | ESI+ : 332 [M+H]+ |
| 25-4 | ESI+ : 300 [M+H]+ |
| 25-5 | ESI+ : 415 [M+H]+ |
| 25-6 | ESI+ : 332 [M+H]+ |
| 25-7 | ESI+ : 300 [M+H]+ |
| 25-8 | ESI+ : 330 [M+H]+ |
| 25-9 | ESI+ : 328 [M+H]+ |
| 25-10 | ESI+ : 360 [M+H]+ |
| 25-11 | ESI+ : 348 [M+H]+ |
| 43 | ESI+ : 283 [M+H]+ |
| 25-13 | ESI+ : 328 [M+H]+ |
| 43-1 | ESI+ : 195 [M+H]+ |
| 25-15 | ESI+ : 286 [M+H]+ |
| 25-16 | ESI+ : 346 [M+H]+ |
| 25-17 | ESI+ : 410 [M+H]+ |
| 25-18 | ESI+ : 381 [M+H]+ |
| 25-19 | ESI+ : 367 [M+H]+ |
| 25-20 | ESI+ : 397 [M+H]+ |
| 25-21 | ESI+ : 405 [M+H]+ |
| 25-22 | ESI+ : 354 [M+H]+ |
| 25-23 | ESI+ : 383 [M+H]+ |
| 25-24 | ESI+ : 383 [M+H]+ |
| 25-25 | ESI+ : 367 [M+H]+ |
| 25-26 | ESI+ : 399 [M+H]+ |
| 25-27 | ESI+ : 395 [M+H]+ |
| 25-28 | ESI+ : 387 [M+H]+ |
| 25-29 | ESI+ : 385 [M+H]+ |
| 25-30 | ESI+ : 328 [M+H]+ |
| 25-31 | ESI+ : 414 [M+H]+ |
| 25-32 | ESI+ : 357 [M+H]+ |
| 25-33 | ESI+ : 396 [M+H]+ |
| 25-34 | ESI+ : 379 [M+H]+ |
| 25-35 | ESI+ : 344 [M+H]+ |
| 25-36 | ESI+ : 415 [M+H]+ |
| 25-37 | ESI+ : 390 [M+H]+ |
| 43-2 | ESI+ : 199 [M+H]+ |
| 25-39 | ESI+ : 383 [M+H]+ |
| 25-40 | ESI+ : 411 [M+H]+ |

**[Table 32]**

| Pr | Data |
|---|---|
| 25-41 | ESI+ : 361 [M+H]+ |
| 25-42 | NMR-DMSO-d₆: 1.97-2.03 (2H, m), 2.36-2.42 (1H, m), 2.75-2.85 (1H, m), 2.87-2.97 (1H, m), 3.18-3.33 (2H, m), 3.42-3.47 (2H, m), 3.97-4.02 (1H, m), 6.61 (1H, s), 7.17-7.22 (2H, m), 7.82 (1H, br), 7.90-7.94 (1H, m), 8.35-8.38 (1H, m), 8.44 (1H, br), 8.49 (1H, s), 8.85-8.91 (1H, m), 9.15-9.23 (1H, m), 9.83 (1H, d, J = 7.6 Hz), 11.83 (1H, s), 14.56 (1H, br) |
| 25-43 | ESI+ : 387 [M+H]+ |
| 25-44 | ESI+ : 428 [M+H]+ |
| 25-45 | ESI+ : 428 [M+H]+ |
| 25-46 | ESI+ : 427 [M+H]+ |
| 25-47 | ESI+ : 428 [M+H]+ |
| 25-48 | ESI+ : 442 [M+H]+ |
| 25-49 | ESI+ : 440 [M+H]+ |
| 25-50 | ESI+ : 424 [M+H]+ |
| 25-51 | ESI+ : 423 [M+H]+ |
| 25-52 | ESI+ : 424 [M+H]+ |
| 25-53 | ESI+ : 411 [M+H]+ |
| 25-54 | ESI+ : 370 [M+H]+ |
| 25-55 | ESI+ : 422 [M+H]+ |
| 25-56 | ESI+ : 422 [M+H]+ |
| 44 | ESI+ : 215 [M+H]+ |
| 25-58 | ESI+ : 313 [M+H]+ |
| 26 | ESI+ : 417 [M+H]+ |
| 26-1 | ESI+ : 377 [M+H]+ |
| 27 | ESI+ : 139 [M+H]+ |
| 27-1 | ESI+ : 152 [M+H]+ |
| 28 | ESI+ : 485 [M+H]+ |
| 28-1 | ESI+ : 413 [M+H]+ |
| 28-2 | ESI+ : 438 [M+H]+ |
| 45 | ESI+ : 383 [M+H]+ |
| 28-4 | ESI+ : 394 [M+H]+ |
| 28-5 | NMR-DMSO-d₆: 3.83 (3H, s), 4.47 (2H, d, J = 6.1 Hz), 7.07-7.30 (5H, m), 7.62 (1H, d, J = 8.8 Hz), 7.90 (1H, br), 8.08 (1H, dd, J = 2.4, 8.8 Hz), 8.45 (1H, s), 8.72-8.76 (1H, m), 9.17 (1H, t, J = 6.2 Hz), 10.09 (1H, s) ESI+ : 414 [M+H]+ |
| 28-6 | ESI+ : 413 [M+H]+ |
| 28-7 | ESI+ : 428 [M+H]+ |
| 28-8 | ESI+ : 432 [M+H]+ |
| 28-9 | ESI+ : 400 [M+H]+ |
| 28-10 | ESI+ : 515 [M+H]+ |
| 28-11 | ESI+ : 432 [M+H]+ |
| 28-12 | ESI+ : 430 [M+H]+ |
| 28-13 | ESI+ : 400 [M+H]+ |
| 28-14 | ESI+ : 428 [M+H]+ |
| 28-15 | ESI+ : 460 [M+H]+ |
| 28-16 | ESI+ : 448 [M+H]+ |
| 28-17 | ESI+ : 428 [M+H]+ |

**[Table 33]**

| Pr | Data |
|---|---|
| 45-1 | ESI+ 295 [M+H]+ |
| 28-19 | ESI+ : 386 [M+H]+ |
| 28-20 | ESI+ : 446 [M+H]+ |
| 28-21 | ESI+ : 510 [M+H]+ |
| 28-22 | ESI+ : 481 [M+H]+ |
| 28-23 | ESI+ : 467 [M+H]+ |
| 28-24 | ESI+ : 483 [M+H]+ |
| 28-25 | ESI+: 483 [M+H]+ |
| 28-26 | ESI+ : 497 [M+H]+ |
| 28-27 | ESI+ : 505 [M+H]+ |
| 28-28 | ESI+ : 454 [M+H]+ |
| 28-29 | ESI+ : 467 [M+H]+ |
| 28-30 | ESI+ : 521 [M+Na]+ |
| 28-31 | ESI+ : 495 [M+H]+ |
| 28-32 | ESI+ : 509 [M+Na]+ |
| 28-33 | ESI- : 573 [M-H]- |
| 28-34 | ESI+ : 537 [M+Na]+ |
| 28-35 | ESI+ : 428 [M+H]+ |
| 28-36 | ESI+: 457 [M+H]+ |
| 28-37 | ESI+ 496 [M+H]+ |
| 28-38 | ESI+ : 479 [M+H]+ |
| 28-39 | ESI+ : 558 [M+H]+ |
| 28-40 | ESI+ : 512 [M+Na]+ |
| 28-41 | ESI+ : 505 [M+Na]+ |
| 28-42 | ESI+ : 557 [M+H]+ |
| 28-43 | ESI+ : 601 [M+H]+ |
| 28-44 | ESI+ : 575 [M+H]+ |
| 28-45 | ESI+ : 625 [M+H]+ |
| 28-46 | ESI+ : 528 [M+H]+ |
| 28-47 | ESI+ : 258 [M+Na]+ |
| 28-48 | ESI+ : 473 [M+H]+ |
| 28-49 | ESI+ : 550 [M+Na]+ |
| 28-50 | ESI+ : 528 [M+H]+ |
| 28-51 | ESI+ : 527 [M+H]+ |
| 28-52 | ESI+ : 564 [M+Na]+ |
| 28-53 | ESI+ : 540 [M+H]+ |
| 28-54 | NMR-DMSO-d₆: 1.43 (9H, s), 2.73 (2H, t, J = 6 Hz), 3.54 (2H, t, J = 6 Hz), 4.40-4.46 (4H, m), 6.02 (1H, s), 6.99-7.55 (6H, m), 7.75 (1H, brs), 8.38 (1H, s), 8.92 (1H, s), 9.04 (1H, t, J = 6 Hz) ESI+ : 528 [M+H]+ |
| 28-55 | ESI+ : 524 [M+H]+ |
| 28-56 | ESI+ : 523 [M+H]+ |
| 28-57 | ESI+ : 524 [M+H]+ |
| 28-58 | ESI+ : 511 [M+H]+ |
| 28-59 | ESI+ : 470 [M+H]+ |
| 45-2 | ESI+ : 254 [M+Na]+ |
| 28-61 | ESI+ : 522 [M+H]+ |

**[Table 34]**

| Pr | Data |
|---|---|
| 45-3 | ESI+ : 254 [M+Na]+ |
| 28-63 | ESI+ : 544 [M+Na]+ |
| 28-64 | ESI+ : 414 [M+H]+ |
| 28-65 | ESI+ : 433 [M+H]+ |
| 29 | ESI+ : 321 [M+Na]+ |
| 30 | ESI+ : 539 [M+Na]+ |
| 31 | ESI+ : 237 [M+Na]+ |
| 32 | NMR-DMSO-d₆: 1.26-1.36 (2H, m), 1.41 (9H, s),1.91-2.00 (2H, m), 2.93-3.08 (2H, m), 3.42-3.52 (1H, m), 3.78-3.88 (2H, m), 6.10 (1H, s), 7.19 (1H, br), 7.67 (1H, dd, J = 2.0, 8.8 Hz), 7.81 (1H, d, J = 8.8 Hz), 7.85 (1H, br), 8.46 (2H, d, J = 2.4 Hz), 8.75 (1H, d, J = 7.6 Hz), 9.81 (1H, s), 13.31 (1H, br) |
| 32-1 | ESI- : 501 [M-H]- |
| 32-2 | ESI+ : 400 [M+H]+ |
| 32-3 | NMR-DMSO-d₆: 1.43-1..47 (2H, m), 1.65-1.82 (8H, m), 2.02-2.07 (1H, m), 2.12-2.17 (2H, m), 3.55-3.60 (1H, m), 4.53-4.58 (1H, br), 7.16 (1H, br), 7.64 (1H, br), 7.98 (1H, br), 8.,14 (1H, m), 8.45 (1H, s), 8.75 (1H, s), 9.25 (1H, br), 10.03 (1H, br), 12.9 (1H, br), 14.2 (1H, br) ESI- : 422 [M-H]- |
| 32-4 | ESI- : 398 [M-H]- |
| 32-5 | ESI+ : 471 [M+H]+ |
| 33 | ESI+ : 194 [M+H]+ |
| 33-1 | ESI+ : 208 [M+H]+ |
| 33-2 | ESI+ : 235 [M+H]+ |
| 33-3 | ESI+ : 215 [M+Na]+ |

### Example 1

To a solution of 4-{[1-(5-cyanopyridin-2-yl)piperidin-4-yl]amino}-6-({4-[(1-methylpiperidin-4-yl)carbamoyl]-3-nitrophenyl}amino)nicotinamide (78 mg) in 2.6 mL of ethanol were added reduced iron (22 mg), ammonium chloride (7 mg), and water (0.78 mL), followed by heating and refluxing for 3 hours. The reaction liquid was cooled, and then EtOAc and a saturated aqueous sodium hydrogen carbonate solution were added thereto. The mixed liquid was filtered through celite and the filtrate was extracted with CHCl₃, followed by washing with water. The organic layer was dried over MgSO₄ and then filtered, and the filtrate was concentrated under reduced pressure. The residue was purified by amino-silica gel column chromatography (CHCl₃/MeOH = from 100/0 to 90/10) to obtain 6-({3-amino-4-[(1-methylpiperidin-4-yl)carbamoyl]phenyl}amino)-4-{[1-(5-cyanopyridin-2-yl)piperidin-4-yl]amino}nicotinamide (37 mg) as a white solid.

In the same manner as in Example 1, the compounds of Examples 1-1 to 1-4 shown in the Tables as described later were prepared.

### Example 2

To a suspension of 4-{[(1R,2s,3S,5s,7s)-5-hydroxyadamantan-2-yl]amino}-6-(pyrazin-2-ylamino)nicotinamide (50 mg) in 1 mL of dichloromethane was added bis(2-methoxyethyl)aminosulfotrifluoride (29 µL) at 4°C, followed by stirring at room temperature for 2.5 hours. To the reaction liquid was added a saturated aqueous sodium hydrogen carbonate solution, followed by stirring at room temperature. The mixed liquid was extracted with CHCl₃, followed by washing with water. The organic layer was dried over MgSO₄ and then filtered, and the filtrate was concentrated under reduced pressure. The residue was purified by amino-silica gel column chromatography (CHCl₃/MeOH = from 100/0 to 95/5) to obtain 4-{[(1R,2s,3S,5s,7s)-5-fluoroadamantan-2-yl]amino}-6-(pyrazin-2-ylamino)nicotinamide (40 mg) as a white solid.

### Example 3

To a mixed liquid of 4-(piperidin-4-ylamino)-6-(pyrazin-2-ylamino)nicotinamide trihydrochloride (25 mg) and potassium carbonate (41 mg) in 1 mL of DMF was added 4-(bromomethyl)benzonitrile (13 mg). The reaction liquid was stirred at room temperature for 2 hours. The reaction liquid was quenched with water, then extracted with a 9:1 mixed liquid of CHCl₃ and MeOH, followed by washing with water. The organic layer was dried over MgSO₄ and then filtered, and the filtrate was concentrated under reduced pressure. The residue was purified by silica gel column chromatography (CHCl₃/MeOH = from 100/0 to 95/5) to obtain 4-{[1-(4-cyanobenzyl)piperidin-4-yl]amino}-6-(pyrazin-2-ylamino)nicotinamide (15 mg) as a pale yellow solid.

### Example 4

To a solution of 4-{[(1R,2s,3S,5s,7s)-5-hydroxyadamantan-2-yl]amino}-6-[(4-hydroxyphenyl)amino]nicotinamide (30 mg) in DMF (0.3 mL) were added bromoacetonitrile (5.6 µL) and potassium carbonate (12.6 mg). The reaction liquid was stirred at room temperature for 2 hours. The reaction liquid was quenched with water, then extracted with a 9:1 mixed liquid of CHCl₃ and MeOH, followed by washing with water. The organic layer was dried over MgSO₄ and then filtered, and the filtrate was concentrated under reduced pressure. The residue was purified by silica gel column chromatography (CHCl₃/MeOH = from 100/0 to 90/10) to obtain 6-{[4-(cyanomethoxy)phenyl]amino}-4-{ [(1R,2s,3S,5 s,7s)-5-hydroxyadamantan-2-yl]amino}nicotinamide (10 mg) as a white solid.

In the same manner as in Example 4, the compound of Example 4-1 shown in the Tables as described later was prepared.

### Example 5

In the same manner as in Example 12, the compounds of Example 5 and Examples 5-1 to 5-107 shown in the Tables as described later were prepared.

### Example 6

To a solution of 4-(piperidin-4-ylamino)-6-(pyrazin-2-ylamino)nicotinamide trihydrochloride (30 mg) and triethylamine (50 µL) in 1 mL of DMF was added 4-cyanophenyl 4-isocynate (11 mg), followed by stirring at room temperature for 1 hour. The reaction liquid was quenched with water, then extracted with a 9:1 mixed liquid of CHCl₃ and MeOH, followed by washing with water. The organic layer was dried over MgSO₄ and then filtered, and the filtrate was concentrated under reduced pressure. The residue was purified by silica gel column chromatography (CHCl₃/MeOH = from 100/0 to 90/10) to obtain 4-({1-[(4-cyanophenyl)carbamoyl]piperidin-4-yl}amino)-6-(pyrazin-2-ylamino)nicotinamide (32 mg) as a white solid.

In the same manner as in Example 6, the compounds of Example 6-1 and Example 6-2 shown in the Tables as described later were prepared.

### Example 7

To a mixed liquid of 4-(piperidin-4-ylamino)-6-(pyrazin-2-ylamino)nicotinamide trihydrochloride (30 mg) in 1 mL of DMF were added triethylamine (50 µL) and 4-cyanobenzenesulfonyl chloride (16 mg), followed by stirring at room temperature for 1 hour. The reaction liquid was quenched with water, then extracted with a 9:1 mixed liquid of CHCl₃ and MeOH, followed by washing with water. The organic layer was dried over MgSO₄ and then filtered, and the filtrate was concentrated under reduced pressure. The residue was purified by silica gel column chromatography (CHCl₃/MeOH = from 100/0 to 90/10) to obtain 4-({1-[(4-cyanophenyl)sulfonyl]piperidin-4-yl}amino)-6-(pyrazin-2-ylamino)nicotinamide (15 mg) as a white solid.

### Example 8

6-[(5-Carbamoyl-4-{[(1R,2s,3 S,5s,7s)-5-hydroxyadamantan-2-yl]amino}pyridin-2-yl)amino]nicotinic acid (40 mg) was suspended in DMF (0.84 mL), and CDI (24.5 mg) was added thereto, followed by stirring at room temperature for 1 hour. The solution in DMF was cooled to 0°C, and dimethylamine (a 2 M solution in MeOH, 104 µL) was added thereto, followed by warming to room temperature and stirring for an additional 2 hours. Water was added thereto to stop the reaction, followed by extraction with a 9/1 mixed solvent of CHCl₃/MeOH. The resulting organic layer was washed with brine and dried over MgSO₄ that had been added thereto. After filtration, the organic solvent was evaporated under reduced pressure and the resulting residue was purified by amino-silica gel column chromatography (CHCl₃/MeOH = from 100/0 to 90/10) to obtain 6-[(5-carbamoyl-4-{[(1R,2s,3S,5s,7s)-5-hydroxyadamantan-2-yl]amino}pyridin-2-yl)amino]-N,N-dimethyl nicotinamide (29.8 mg) as a pale yellow solid.

In the same manner as in Example 8, the compounds of Example 8-1 and Examples 8-3 to 8-49 shown in the Tables as described later were prepared.

### Example 9

6-aniline-4-{[(1R,2s,3S,5s,7s)-5-hydroxyadamantan-2-yl]amino}nicotinic acid (23 mg) was suspended in dimethylformamide (0.46 ml), and CDI (20 mg) was added thereto, followed by stirring at room temperature for 1 hour. Then, 28% aqueous ammonia (18 µL) was added thereto, followed by stirring at room temperature for 30 minutes.
To the reaction solution was added water (5 ml), and the resulting solid was collected by filtration and washed with H₂O to obtain 6-anilino-4-{[(1R,2s,3S,5s,7s)-5-hydroxyadamantan-2-yl]amino}nicotinamide (12 mg).

In the same manner as in Example 9, the compound of Example 9-1 shown in the Tables as described later was prepared.

### Example 10

To a solution of 6-{[4-(benzyloxy)phenyl]amino}-4-{[(1R,2s,3S,Ss,7s)-5-hydroxyadamantan-2-yl]amino}nicotinamide (40 mg) in 1.5 mL of ethanol was added 15 mg of 10% palladium on carbon (wetted with 50% water), followed by stirring at room temperature for 5 hours under a hydrogen gas atmosphere. After the reaction, the solution was filtered through celite and then the filtrate was concentrated under reduced pressure to obtain 4-{[(1R,2s,3S,5s,7s)-5-hydroxyadamantan-2-yl]amino}-6-[(4-hydroxyphenyl)amino]nicotinamide (6 mg) as a pale brown solid.

In the same manner as in Example 10, the compounds of Examples 10-2 and 10-3 shown in the Tables as described later were prepared.

### Example 11

tert-Butyl 6-({5-carbamoyl-4-[(2,3,6-trifluorobenzyl)amino]pyridin-2-yl}amino)-3,4-dihydroisoquinoline-2(1H)-carboxylate (93 mg) was dissolved in EtOAc (5 mL), and a 4 M hydrogen chloride-EtOAc solution (0.22 mL) was added thereto under ice-cooling, followed by stirring at room temperature for 10 hours. EtOAc was concentrated under reduced pressure to obtain 6-(1,2,3,4-tetrahydroisoquinoline-6-ylamino)-4-[(2,3,6-trifluorobenzyl)amino]nicotinamide dihydrochloride (85 mg) as a pale brown solid.

In the same manner as in Example 11, the compounds of Examples 11-1 to 11-2 shown in the Tables as described later were prepared.

### Example 12

6-Chloro-4-[(2,6-difluoro-4-methoxybenzyl)amino]nicotinamide (70.0 mg), 1-(6-aminopyridin-3-yl)piperidin-2-one (81.7 mg), dicyclohexyl(2',4',6'-triisopropylbiphenyl-2-yl) phosphine (5.1 mg), sodium tert-butoxide (30.8 mg), and tris(dibenzylideneacetone)dipalladium (0) (9.8 mg) were suspended in tert-butyl alcohol (1.1 mL), followed by stirring at 130°C for 30 minutes under microwave irradiation.
The suspension was ice-cooled to room temperature, then diluted with a mixed solvent (4:1) of CHCl₃ and MeOH, and filtered through celite. The filtrate was concentrated under reduced pressure and the residue was purified by column chromatography (CHCl₃/MeOH = from 0/100 to 5/95) using basic silica gel to obtain 4-[(2,6-difluoro-4-methoxybenzyl)amino]-6-{[5-(2-oxopiperidin-1-yl)pyridin-2-yl]amino}nicotinamide (31.0 mg).

In the same manner as in Example 12, the compounds of Examples 12-1 to 12-151 shown in the Tables as described later were prepared.

### Example 13

4-[(4,7-Difluoro-2,3-dihydro-1H-inden-1-yl)amino]-6-{[5-(7-oxo-1,4-diazepan-1-yl)piperidin-2-yl]amino}nicotinamide trihydrochloride (60 mg) was added to DMF (1 mL), and further potassium carbonate (69 mg) and methyl iodide (7 µL) were added thereto. The reaction mixture was extracted with CHCl₃, followed by washing with water. The organic layer was dried over MgSO₄ and then filtered. The filtrate was concentrated under reduced pressure and then the residue was purified by silica gel column chromatography (CHCl₃:MeOH = from 100:0 to 95:5) to obtain 4-[(4,7-difluoro-2,3-dihydro-1 H-inden-1-yl)amino]-6-{ [5-(4-methyl-7-oxo-1,4-diazepan-1-yl)pyridin-2-yl]amino}nicotinamide (10 mg) as a white solid.

### Example 14

4-{[(3S,4R)-1-(5-Bromopyrimidin-2-yl)-3-fluoropiperidin-4-yl]amino}-6-(pyridin-2-ylamino)nicotinamide (70 mg), zinc cyanide (51 mg), and tetrakis(triphenylphosphine) palladium (0) (17 mg) were added to a mixture of DMF (2.1 mL) and 1,3-dimethyl-2-imidazolidinone (2.1 mL). The reaction mixture was stirred at 150°C for 1 hour under microwave irradiation. The reaction mixture was purified by silica gel column chromatography (CHCl₃:MeOH = from 100:0 to 90:10) to obtain 4-{[(3S,4R)-1-(5-cyanopyrimidin-2-yl)-3-fluoropiperidin-4-yl]amino}-6-(pyridin-2-ylamino)nicotinamide (10 mg) as a white solid.

### Example 15

In the same manner as in Example 7 described above, the compound of Example 15 shown in the Tables as described later was prepared.

### Example 16

In the same manner as in Example 8 described above, the compound of Example 16 was prepared.

### Example 17

In the same manner as in Example 10 described above, the compound of Example 17 was prepared.
References: Tetrahedron Letters, 2007, 48, 1637-1639

For the Example Compounds, the structures are shown in Tables 35 to 59 and Tables 100 to 110 and the physicochemical data are shown in Tables 60 to 84 and Tables 111 to 121.

**[Table 35]**

| Ex | Structure | Ex | Structure |
|---|---|---|---|
| 1 | 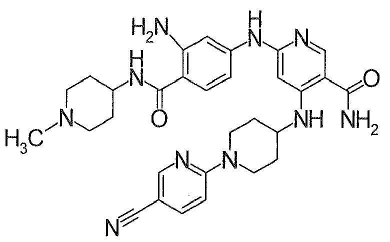 | 1-1 | 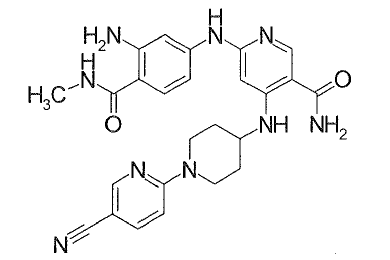 |
| 1-2 | 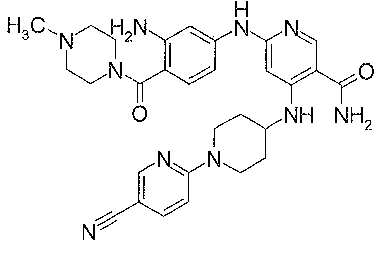 | 1-3 | 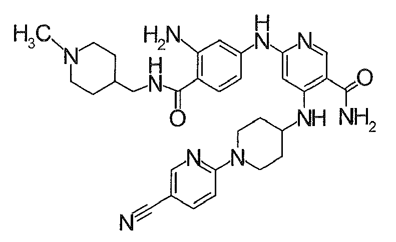 |
| 2 | 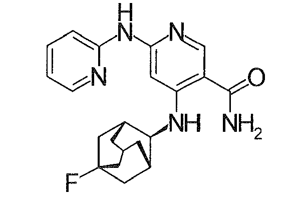 | 3 | 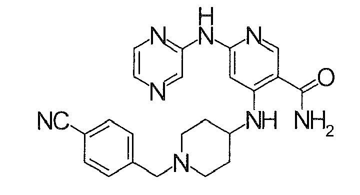 |
| 4 | 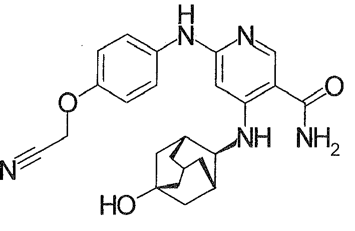 | 4-1 | 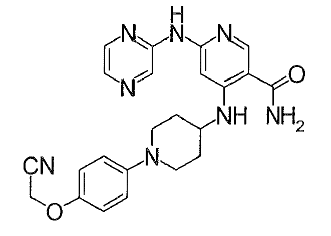 |
| 5 | 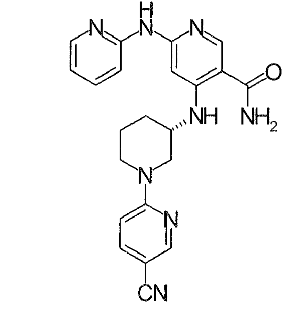 | 5-1 | 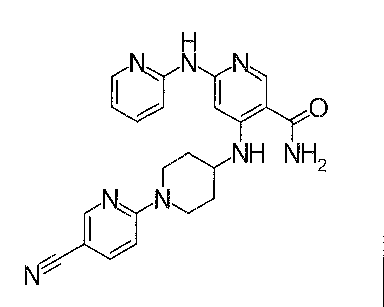 |

**[Table 36]**

| Ex | Structure | Ex | Structure |
|---|---|---|---|
| 5-2 | 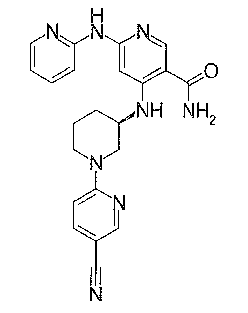 | 5-3 | 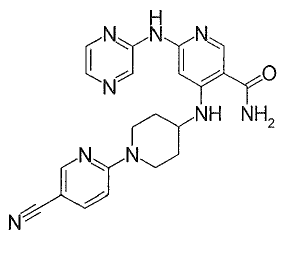 |
| 5-4 | 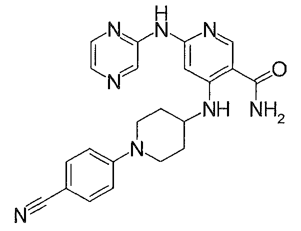 | 5-5 | 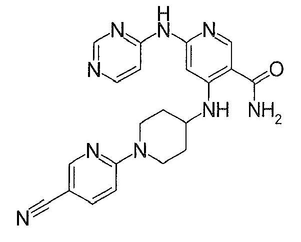 |
| 5-6 | 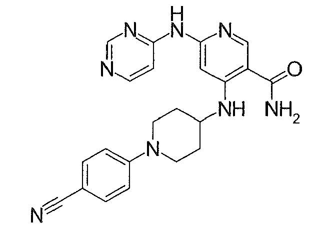 | 5-7 | 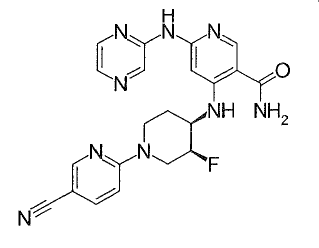 |
| 5-8 | 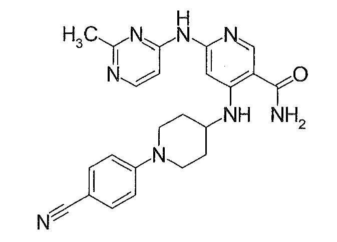 | 5-9 | 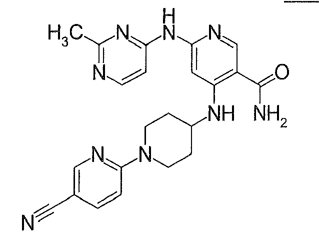 |

**[Table 37]**

| Ex | Structure | Ex | Structure |
|---|---|---|---|
| 5-10 | 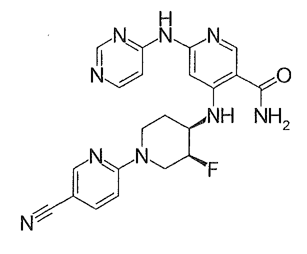 | 5-11 | 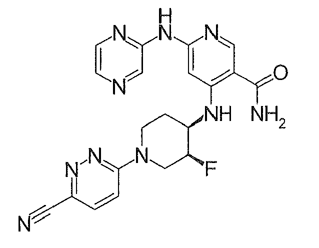 |
| 5-12 | 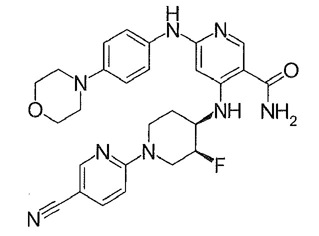 | 5-13 | 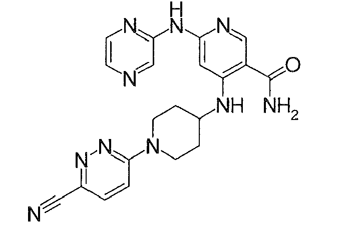 |
| 5-14 | 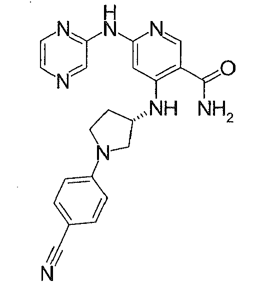 | 5-15 | 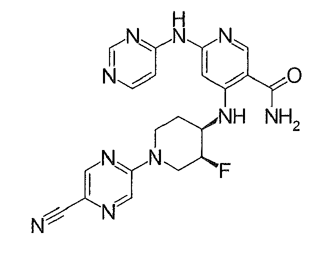 |
| 5-16 | 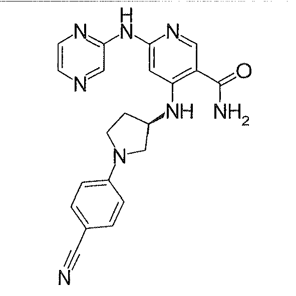 | 5-17 | 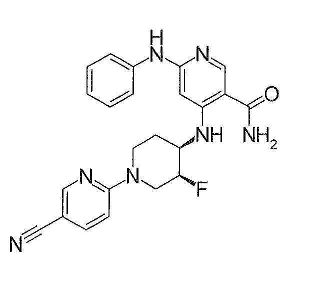 |

**[Table 38]**

| Ex | Structure | Ex | Structure |
|---|---|---|---|
| 5-18 | 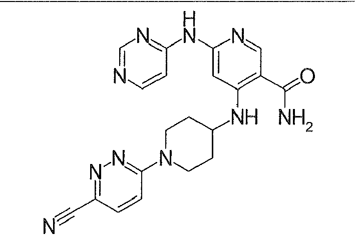 | 5-19 | 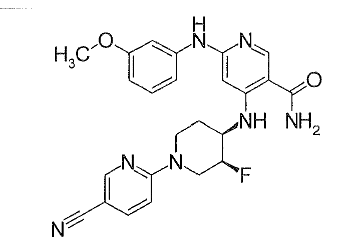 |
| 5-20 | 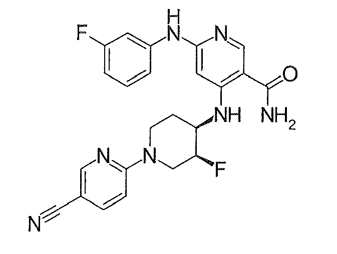 | 5-21 | 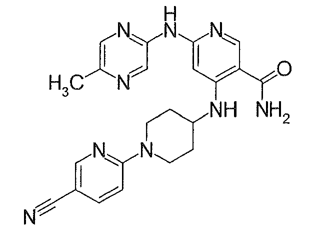 |
| 5-22 | 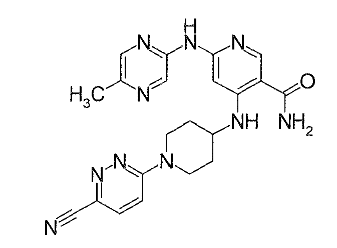 | 5-23 | 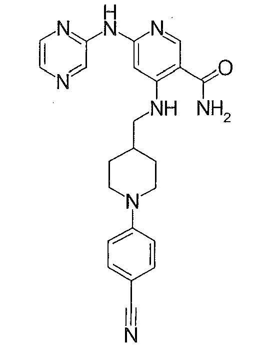 |
| 5-24 | 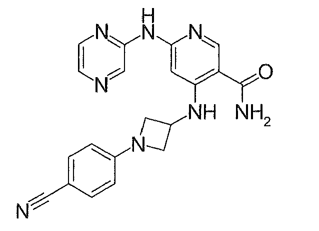 | 5-25 | 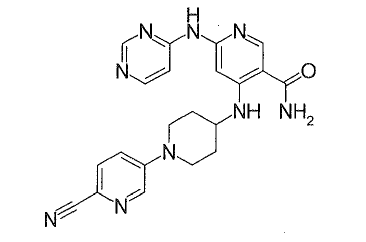 |

**[Table 39]**

| Ex | Structure | Ex | Structure |
|---|---|---|---|
| 5-26 | 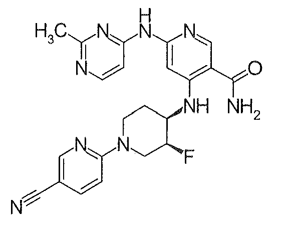 | 5-27 | 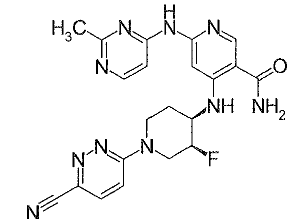 |
| 5-28 | 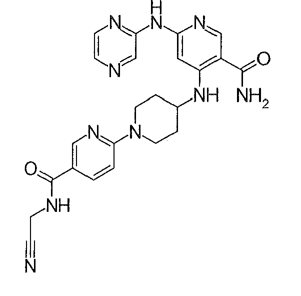 | 5-29 | 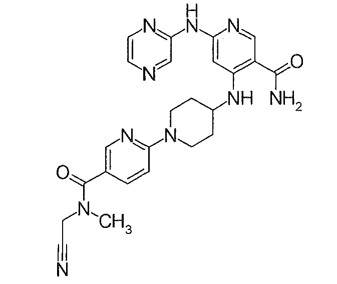 |
| 5-30 | 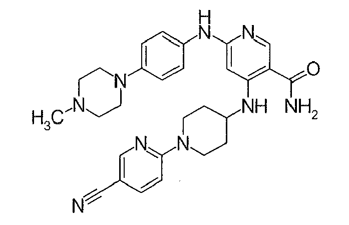 | 5-31 | 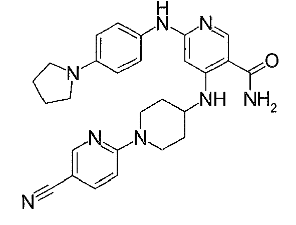 |
| 5-32 | 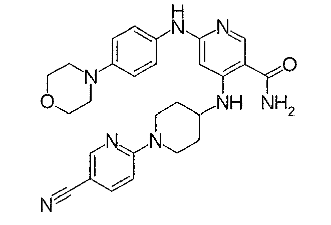 | 5-33 | 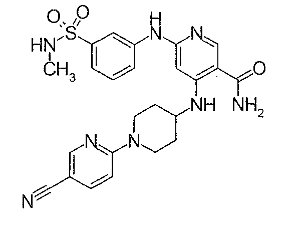 |
| 5-34 | 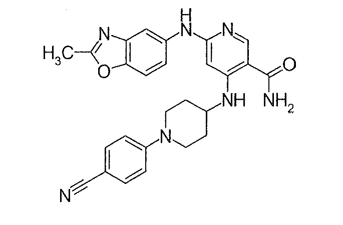 | 5-35 | 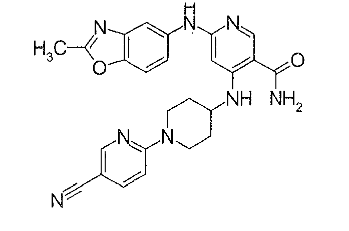 |

**[Table 40]**

| Ex | Structure | Ex | Structure |
|---|---|---|---|
| 5-36 | 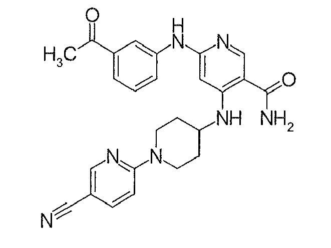 | 5-37 | 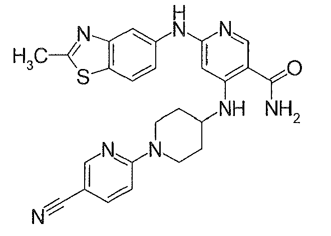 |
| 5-38 | 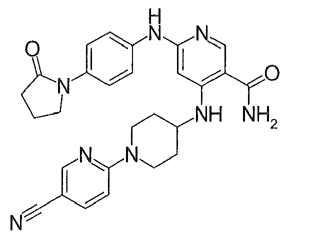 | 5-39 | 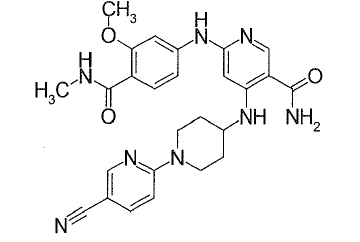 |
| 5-40 | 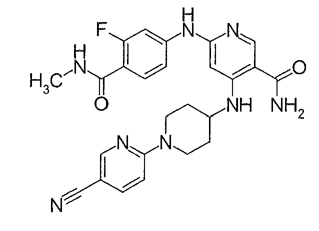 | 5-41 | 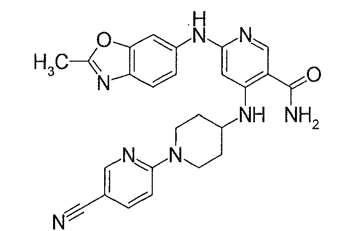 |
| 5-42 | 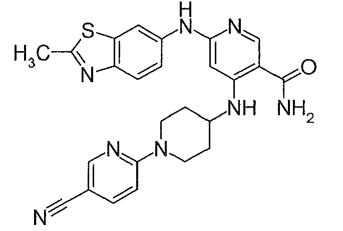 | 5-43 | 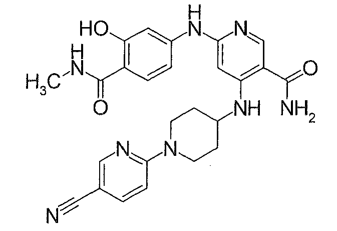 |
| 5-44 | 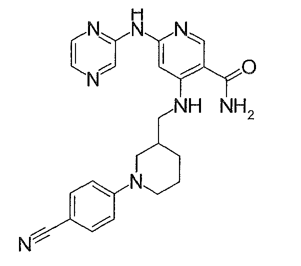 | 5-45 | 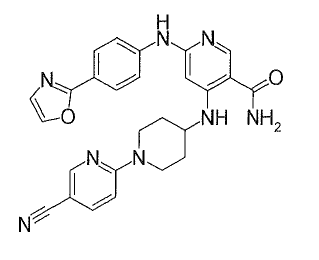 |

**[Table 41]**

| Ex | Structure | Ex | Structure |
|---|---|---|---|
| 5-46 | 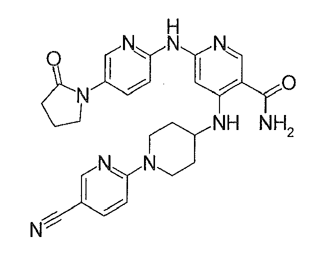 | 5-47 | 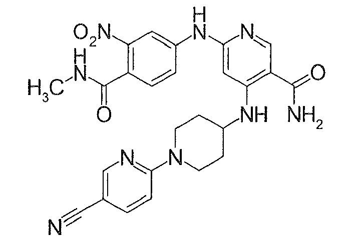 |
| 5-48 | 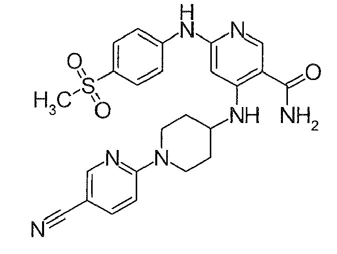 | 5-49 | 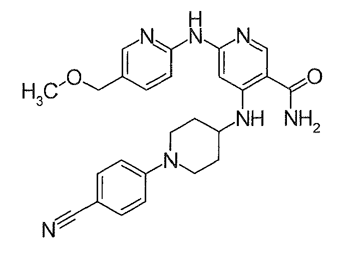 |
| 5-50 | 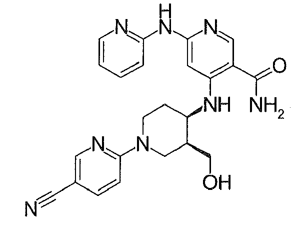 | 5-51 | 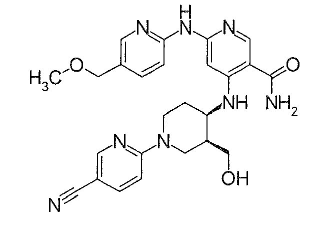 |
| 5-52 | 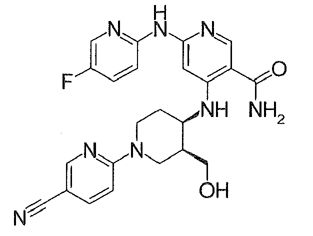 | 5-53 | 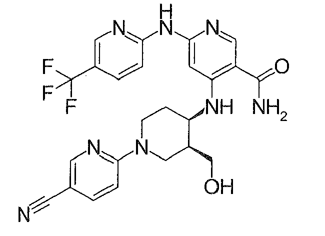 |
| 5-54 | 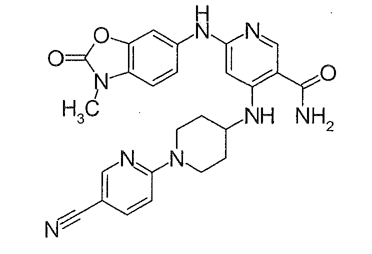 | 5-55 | 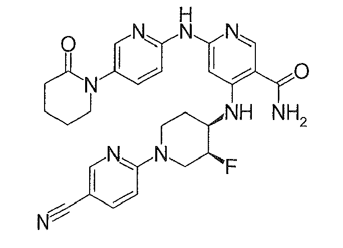 |

**[Table 42]**

| Ex | Structure | Ex | Structure |
|---|---|---|---|
| 5-56 | 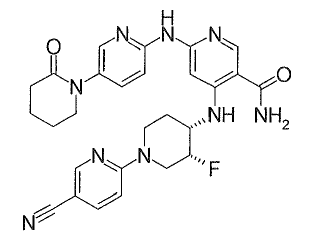 | 5-57 | 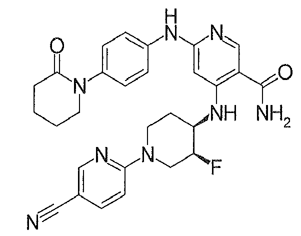 |
| 5-58 | 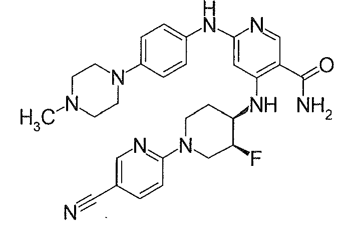 | 5-59 | 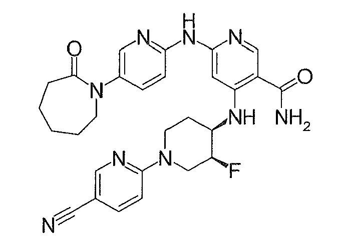 |
| 5-60 | 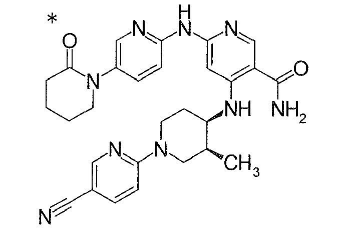 | 5-61 | 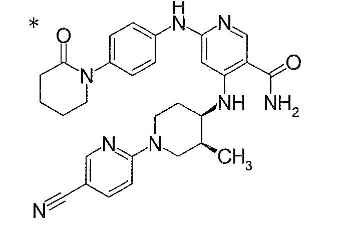 |
| 5-62 | 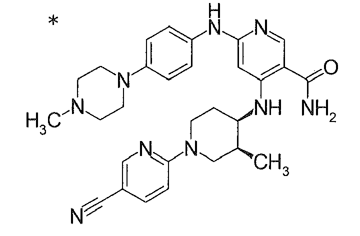 | 5-63 | 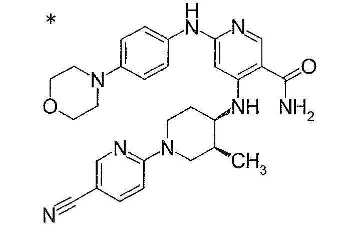 |
| 5-64 | 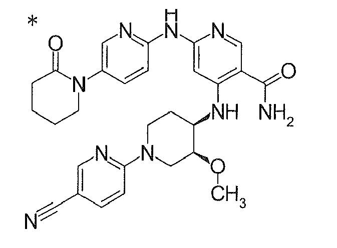 | 5-65 | 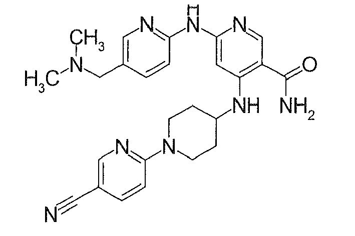 |

**[Table 43]**

| Ex | Structure | Ex | Structure |
|---|---|---|---|
| 5-66 | 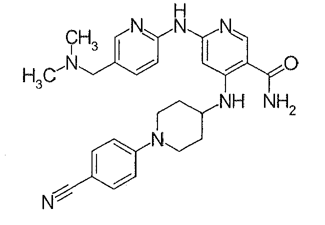 | 5-67 | 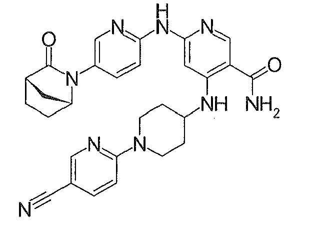 |
| 5-68 | 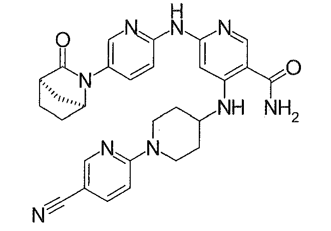 | 5-69 | 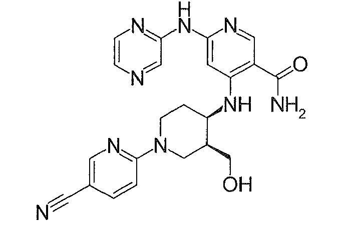 |
| 5-70 | 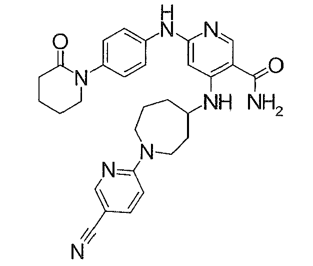 | 6 | 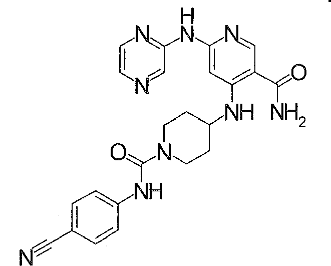 |
| 8 | 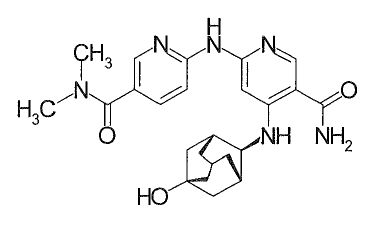 | 8-1 | 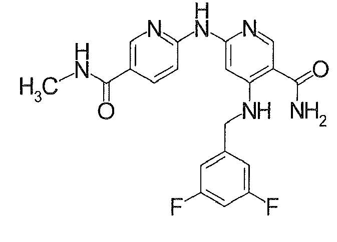 |
| 16 | 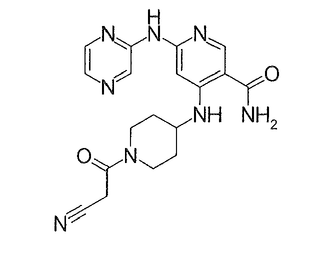 | 8-3 | 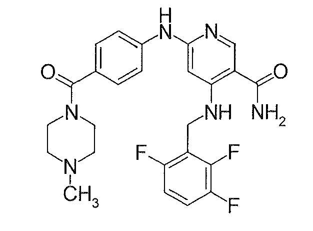 |

**[Table 44]**

| Ex | Structure | Ex | Structure |
|---|---|---|---|
| 8-4 | 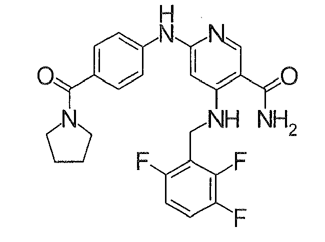 | 8-5 | 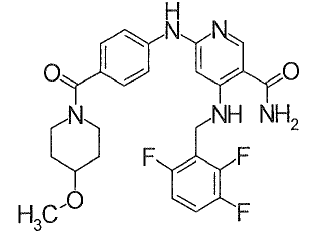 |
| 8-6 | 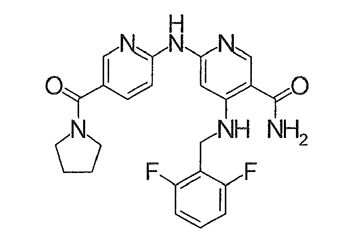 | 8-7 | 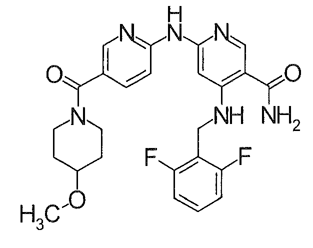 |
| 8-8 | 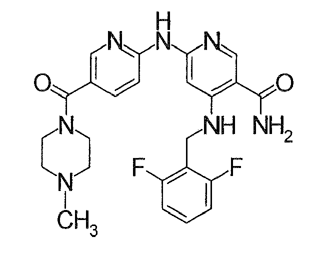 | 8-9 | 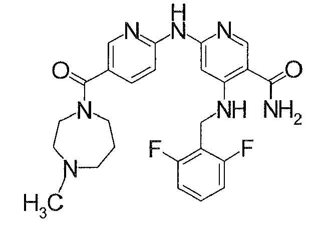 |
| 8-10 | 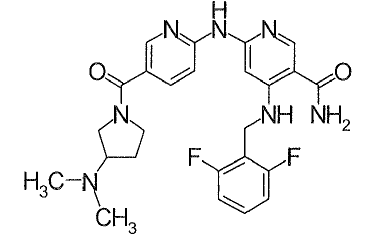 | 8-11 | 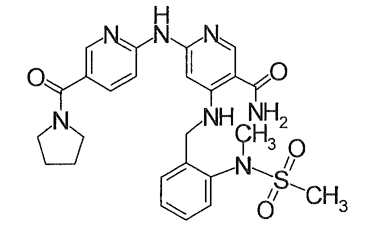 |
| 8-12 | 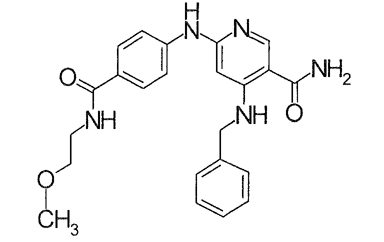 | 8-13 | 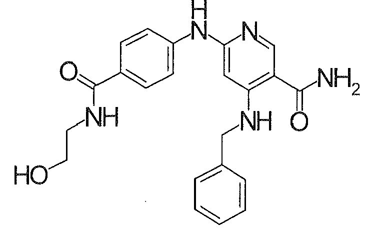 |

**[Table 45]**

| Ex | Structure | Ex | Structure |
|---|---|---|---|
| 8-14 | 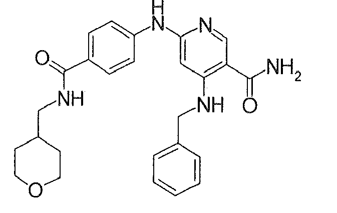 | 8-15 | 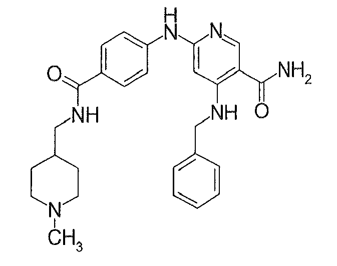 |
| 8-16 | 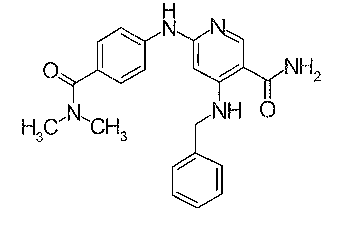 | 8-17 | 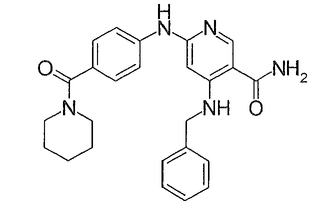 |
| 8-18 | 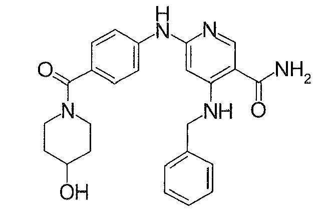 | 8-19 | 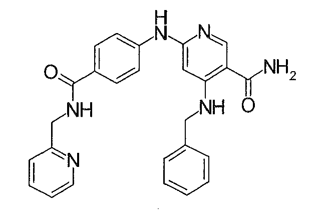 |
| 8-20 | 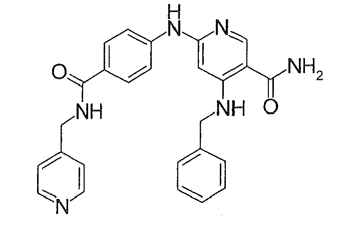 | 8-21 | 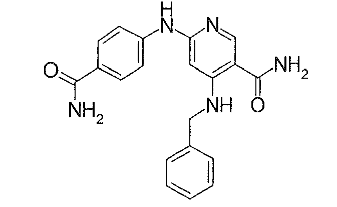 |
| 8-22 | 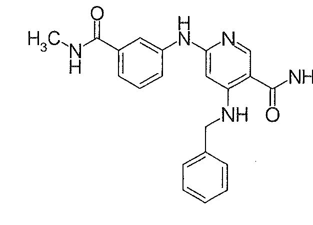 | 8-23 | 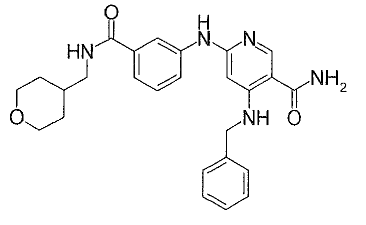 |

**[Table 46]**

| Ex | Structure | Ex | Structure |
|---|---|---|---|
| 8-24 | 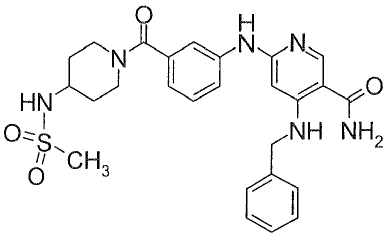 | 8-25 | 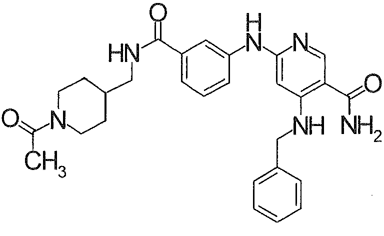 |
| 8-26 | 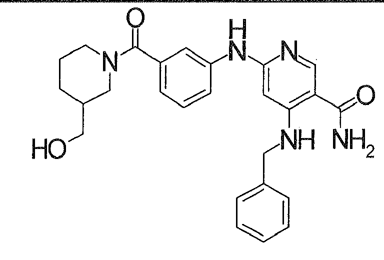 | 8-27 | 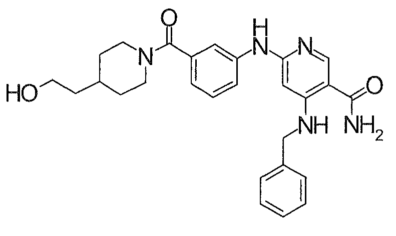 |
| 8-28 | 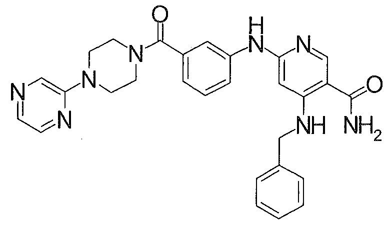 | 8-29 | 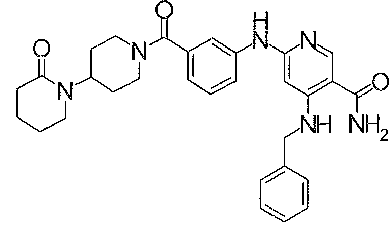 |
| 8-30 | 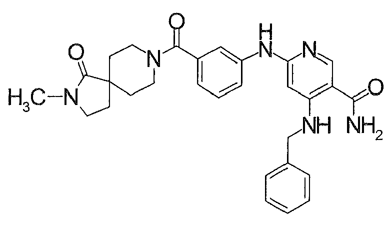 | 8-31 | 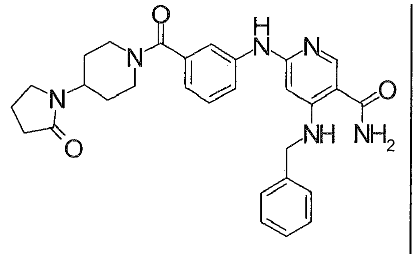 |
| 8-32 | 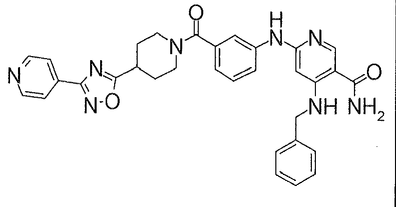 | 8-33 | 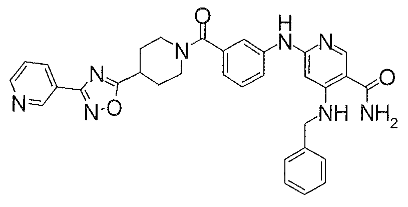 |

**[Table 47]**

| Ex | Structure | Ex | Structure |
|---|---|---|---|
| 8-34 | 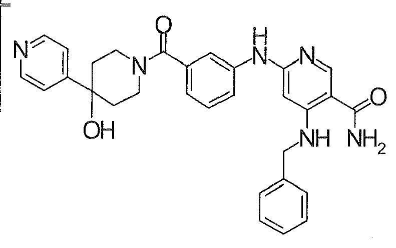 | 8-35 | 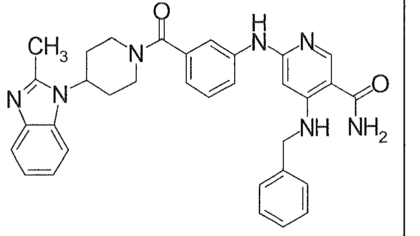 |
| 8-36 | 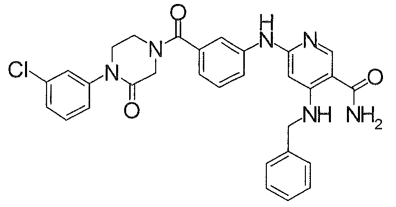 | 8-37 | 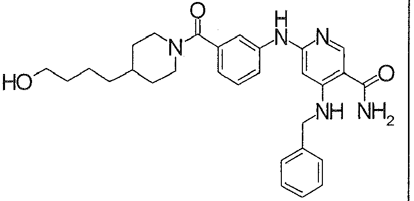 |
| 8-38 | 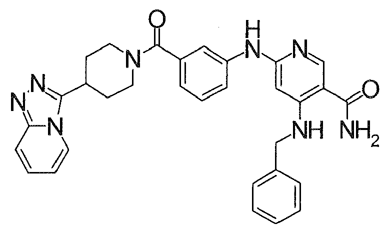 | 8-39 | 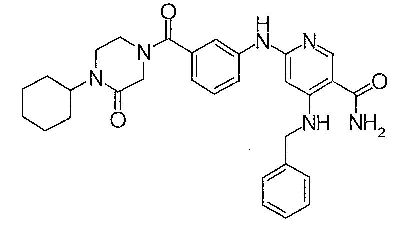 |
| 9 | 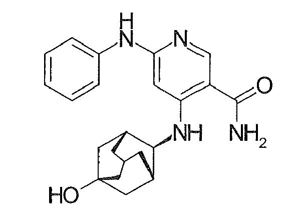 | 9-1 | 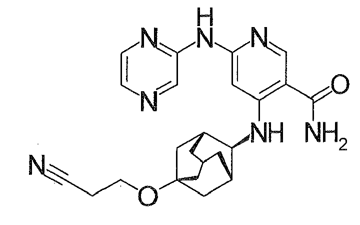 |
| 10 | 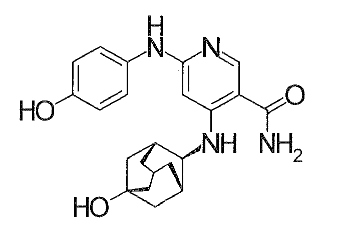 | 17 | 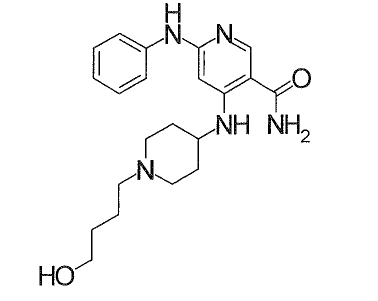 |

**[Table 48]**

| Ex | Structure | Ex | Structure |
|---|---|---|---|
| 10-2 | 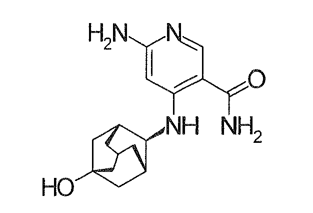 | 10-3 | 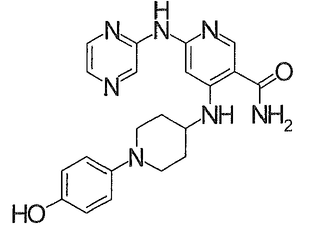 |
| 11 | 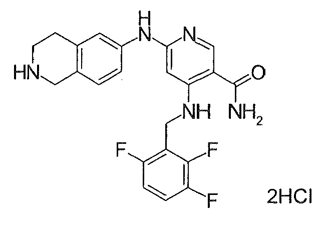 | 11-1 | 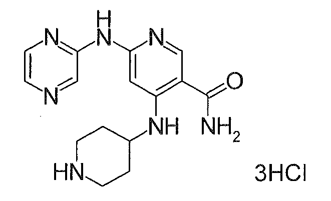 |
| 11-2 | 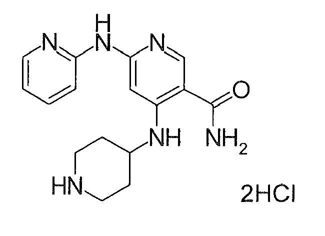 | 12 | 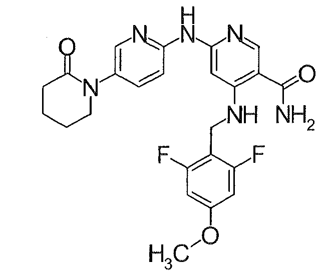 |
| 12-1 | 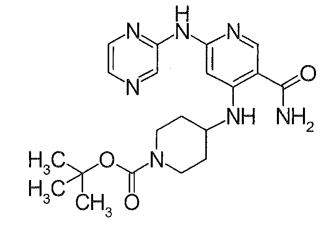 | 12-2 | 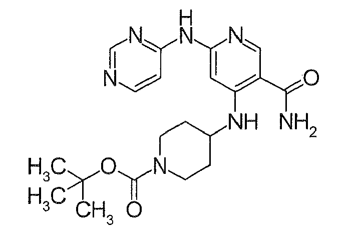 |
| 12-3 | 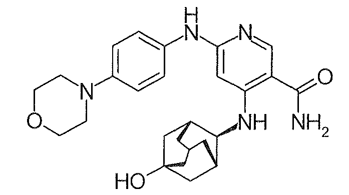 | 12-4 | 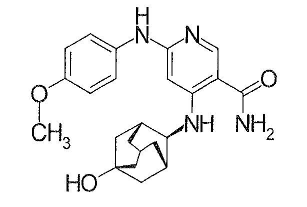 |

**[Table 49]**

| Ex | Structure | Ex | Structure |
|---|---|---|---|
| 12-5 | 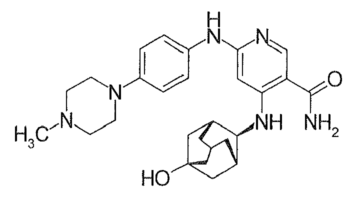 | 12-6 | 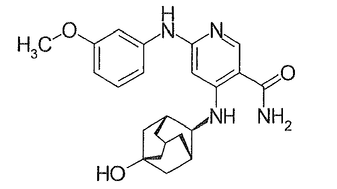 |
| 12-7 | 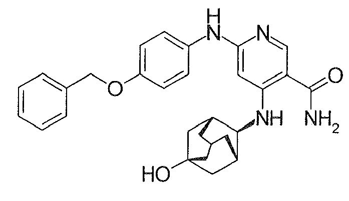 | 12-8 | 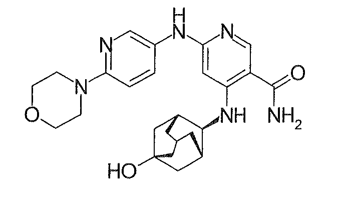 |
| 12-9 | 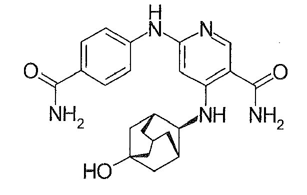 | 12-10 | 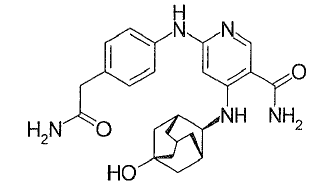 |
| 12-11 | 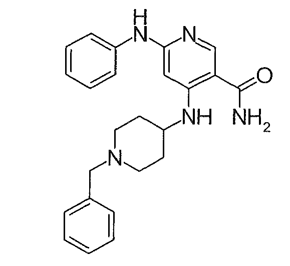 | 12-12 | 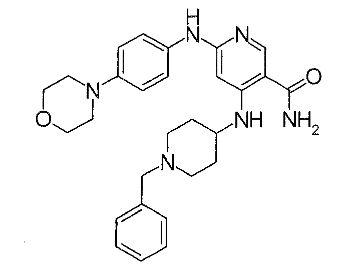 |
| 12-13 | 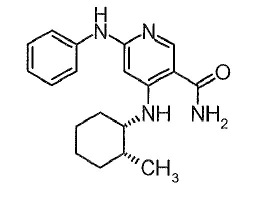 | 12-14 | 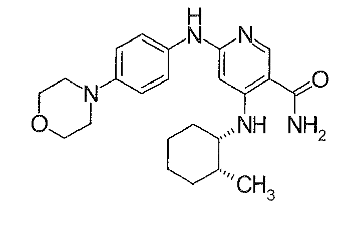 |

**[Table 50]**

| Ex | Structure | Ex | Structure |
|---|---|---|---|
| 12-15 | 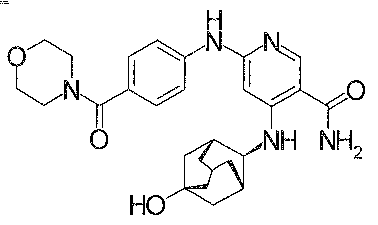 | 12-16 | 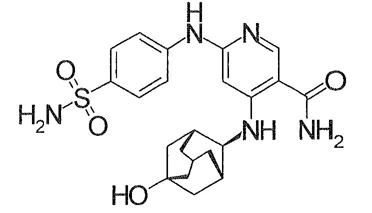 |
| 12-17 | 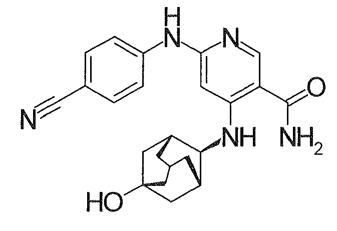 | 12-18 | 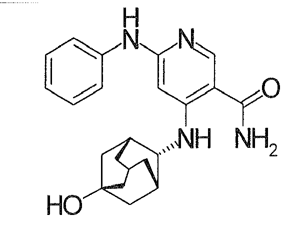 |
| 12-19 | 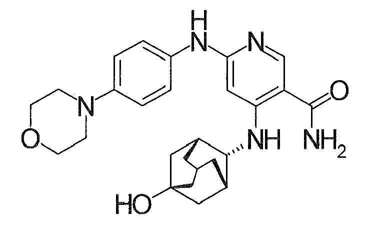 | 12-20 | 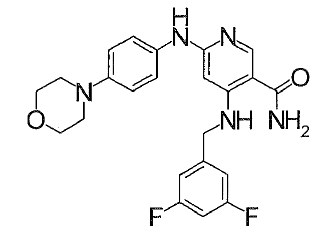 |
| 12-21 | 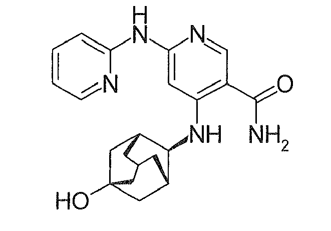 | 12-22 | 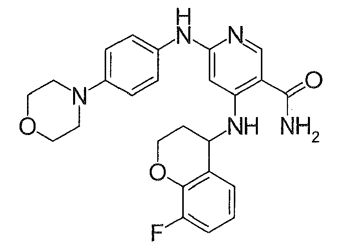 |
| 12-23 | 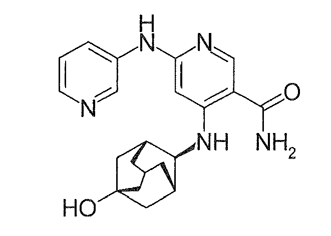 | 12-24 | 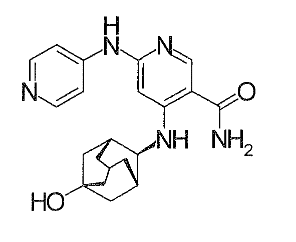 |

**[Table 51]**

| Ex | Structure | Ex | Structure |
|---|---|---|---|
| 12-25 | 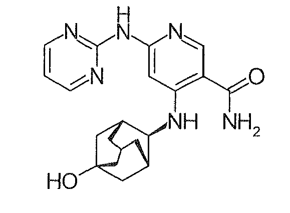 | 12-26 | 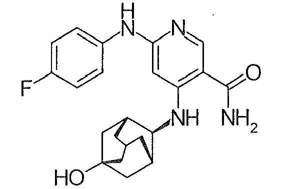 |
| 12-27 | 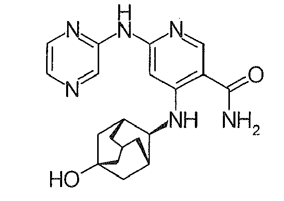 | 12-28 | 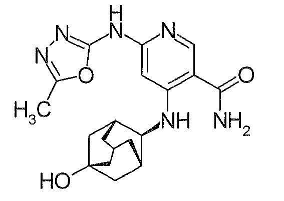 |
| 12-29 | 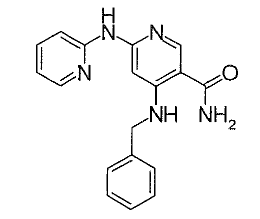 | 12-30 | 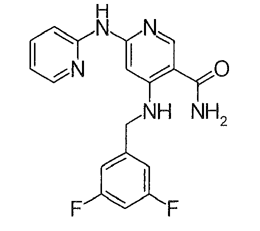 |
| 12-31 | 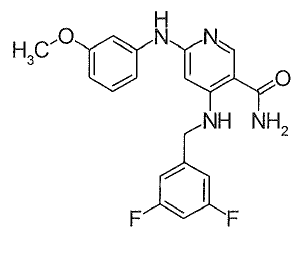 | 12-32 | 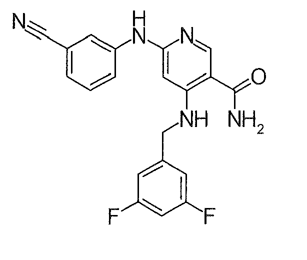 |
| 12-33 | 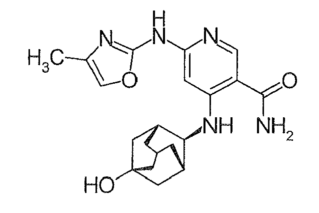 | 12-34 | 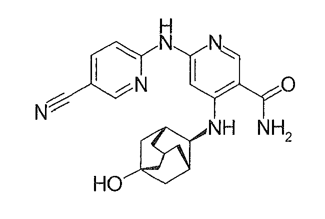 |

**[Table 52]**

| Ex | Structure | Ex | Structure |
|---|---|---|---|
| 12-35 | 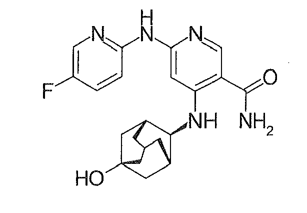 | 12-36 | 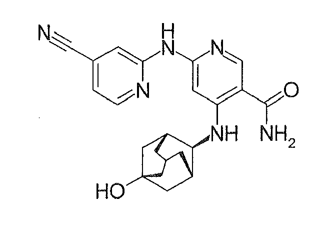 |
| 12-37 | 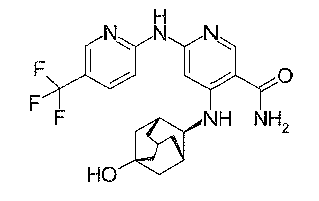 | 12-38 | 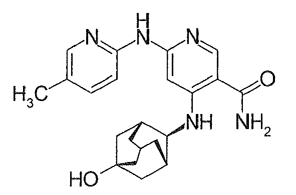 |
| 12-39 | 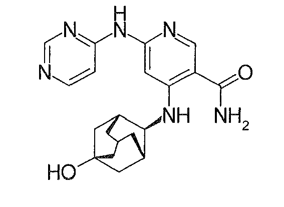 | 12-40 | 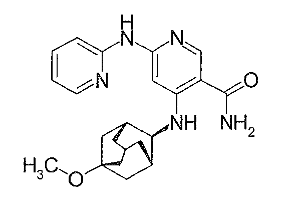 |
| 12-41 | 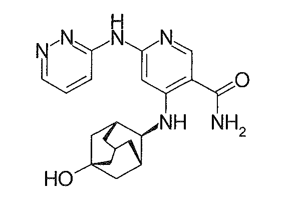 | 12-42 | 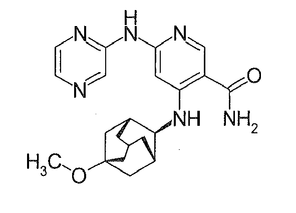 |
| 12-43 | 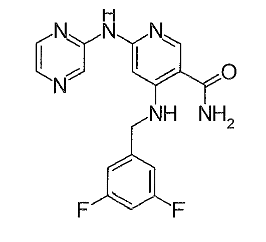 | 12-44 | 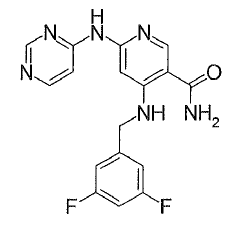 |

**[Table 53]**

| Ex | Structure | Ex | Structure |
|---|---|---|---|
| 12-45 | 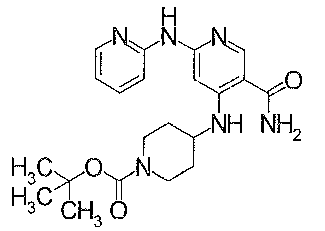 | 12-46 | 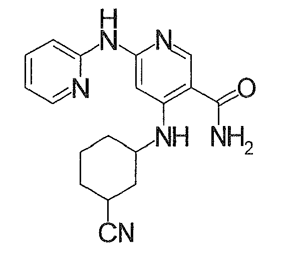 |
| 12-47 | 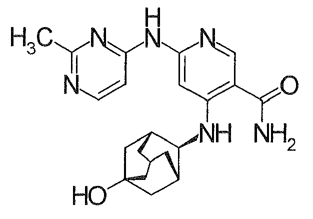 | 12-48 | 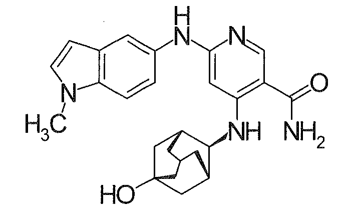 |
| 12-49 | 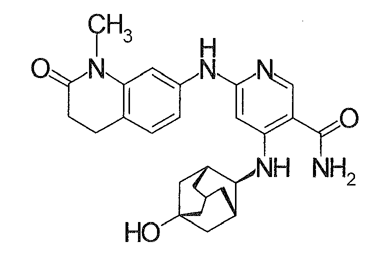 | 12-50 | 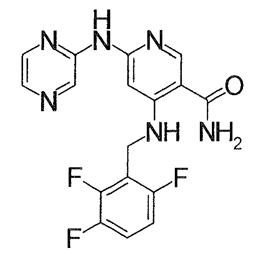 |
| 12-51 | 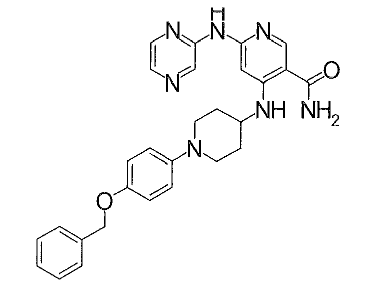 | 12-52 | 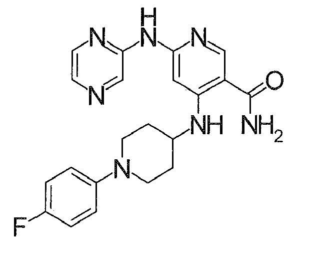 |
| 12-53 | 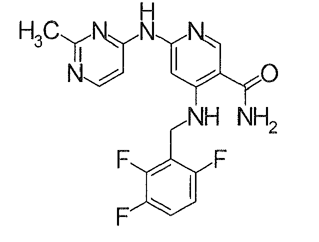 | 12-54 | 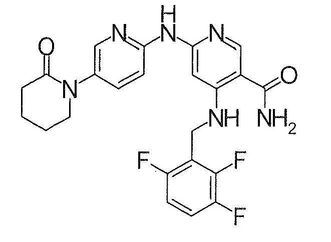 |

**[Table 54]**

| Ex | Structure | Ex | Structure |
|---|---|---|---|
| 12-55 | 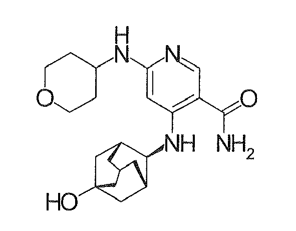 | 12-56 | 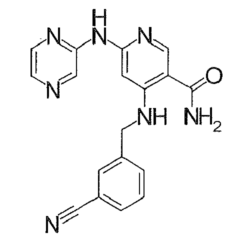 |
| 12-57 | 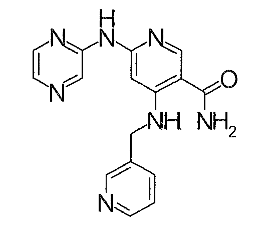 | 12-58 | 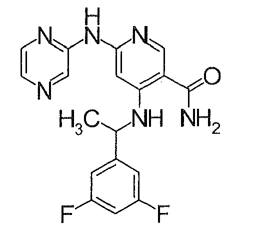 |
| 12-59 | 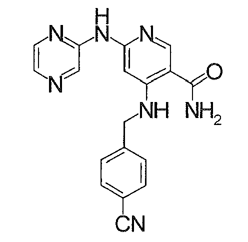 | 12-60 | 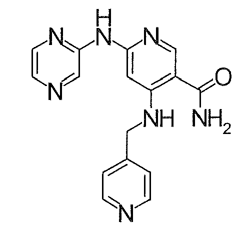 |
| 12-61 | 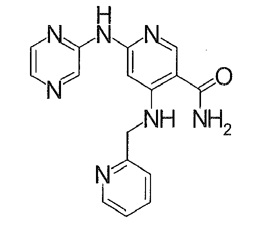 | 12-62 | 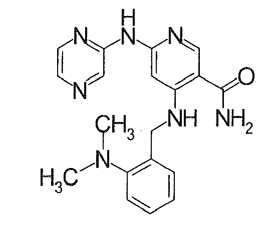 |
| 12-63 | 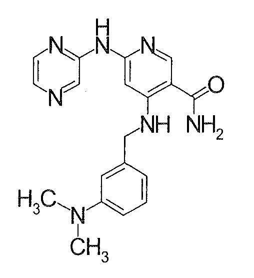 | 12-64 | 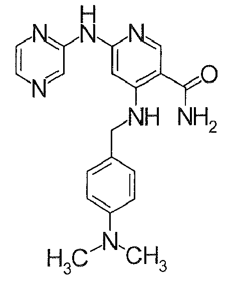 |

**[Table 55]**

| Ex | Structure | Ex | Structure |
|---|---|---|---|
| 12-65 | 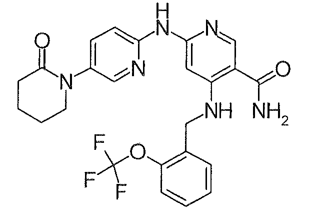 | 12-66 | 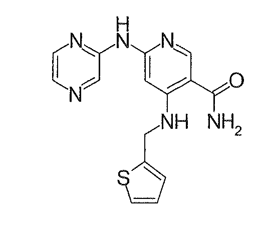 |
| 12-67 | 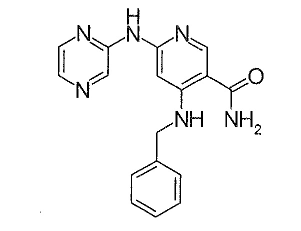 | 12-68 | 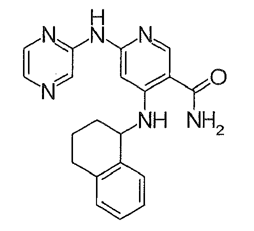 |
| 12-69 | 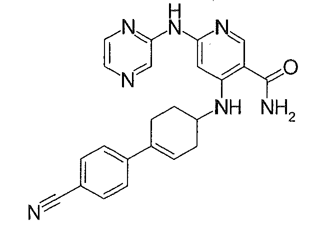 | 12-70 | 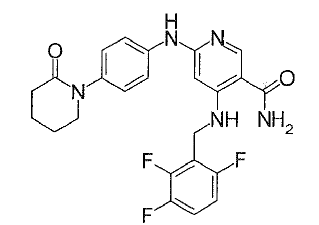 |
| 12-71 | 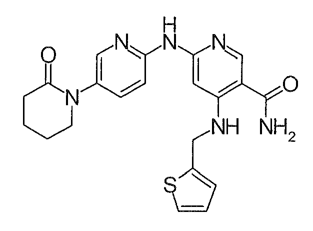 | 12-72 | 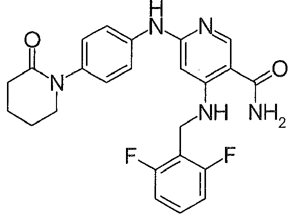 |
| 12-73 | 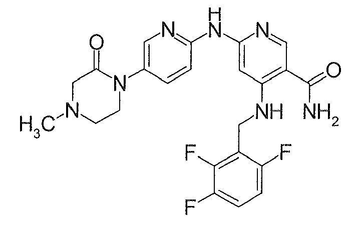 | 12-74 | 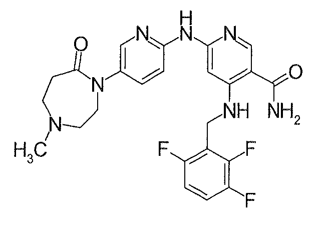 |

**[Table 56]**

| Ex | Structure | Ex | Structure |
|---|---|---|---|
| 12-75 | 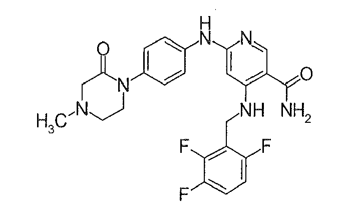 | 12-76 | 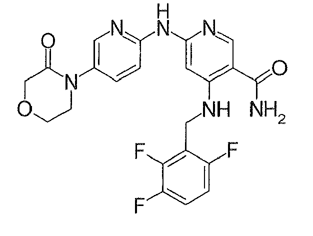 |
| 12-77 | 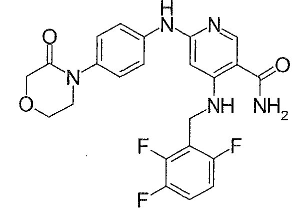 | 12-78 | 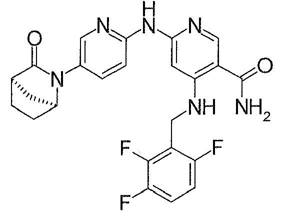 |
| 12-79 | 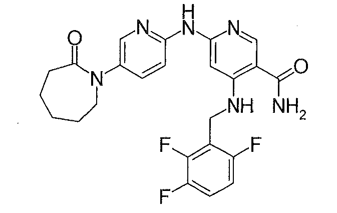 | 12-80 | 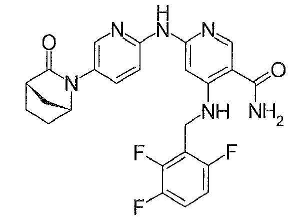 |
| 12-81 | 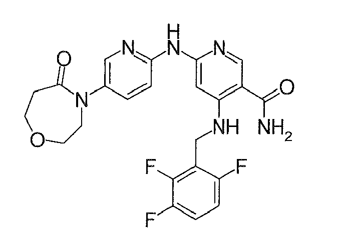 | 12-82 | 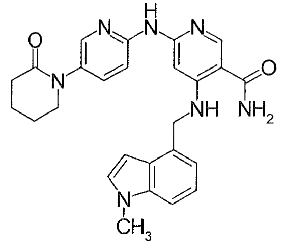 |
| 12-83 | 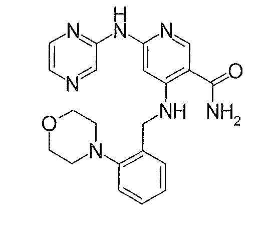 | 12-84 | 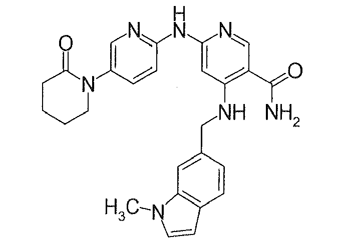 |

**[Table 57]**

| Ex | Structure | Ex | Structure |
|---|---|---|---|
| 12-85 | 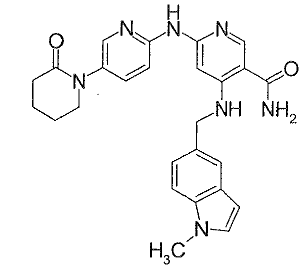 | 12-86 | 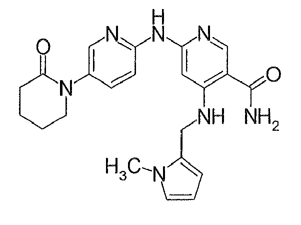 |
| 12-87 | 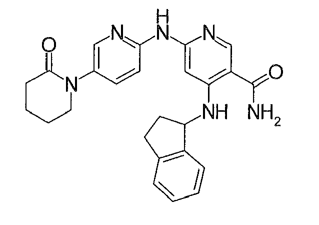 | 12-88 | 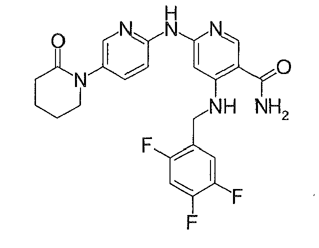 |
| 12-89 | 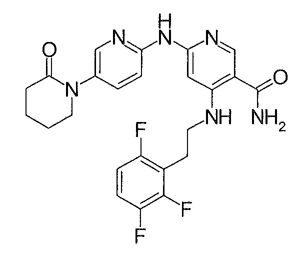 | 12-90 | 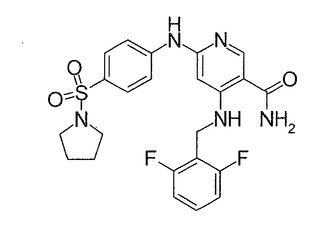 |
| 12-91 | 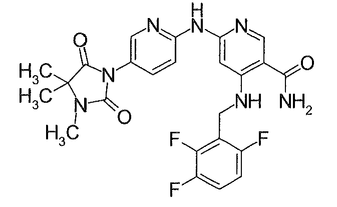 | 12-92 | 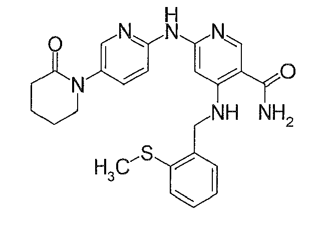 |
| 12-93 | 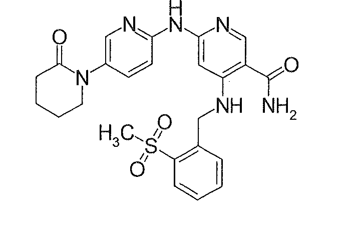 | 12-94 | 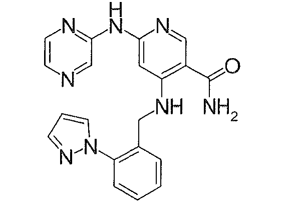 |

**[Table 58]**

| Ex | Structure | Ex | Structure |
|---|---|---|---|
| 12-95 | 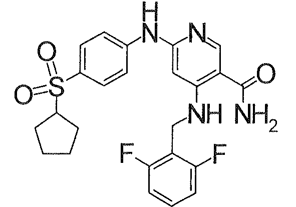 | 12-96 | 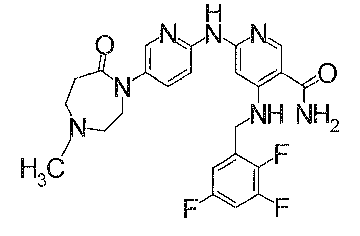 |
| 12-97 | 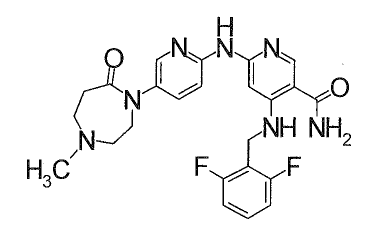 | 12-98 | 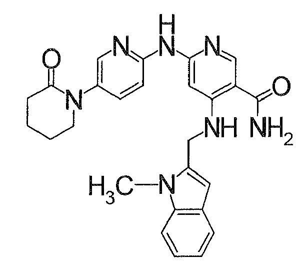 |
| 12-99 | 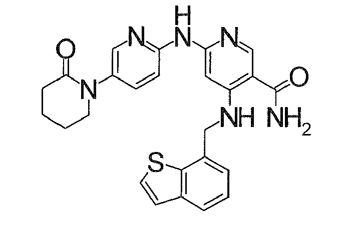 | 12-100 | 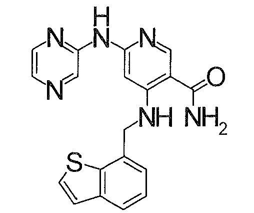 |
| 12-101 | 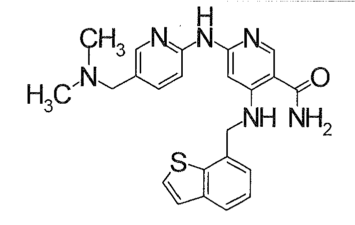 | 12-102 | 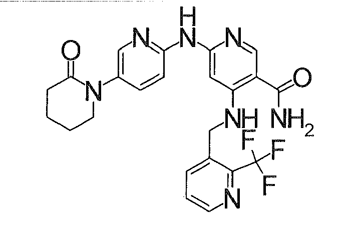 |
| 12-103 | 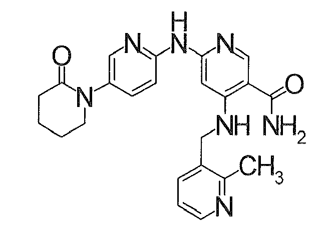 | 12-104 | 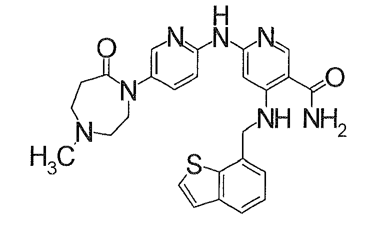 |

**[Table 59]**

| Ex | Structure | Ex | Structure |
|---|---|---|---|
| 12-105 | 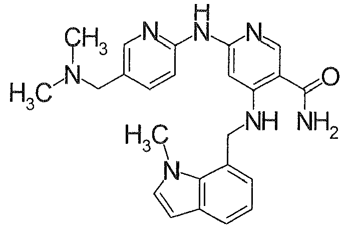 | 12-106 | 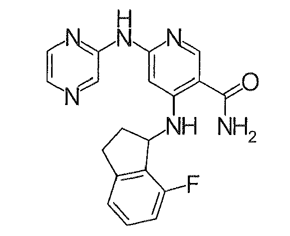 |
| 12-107 | 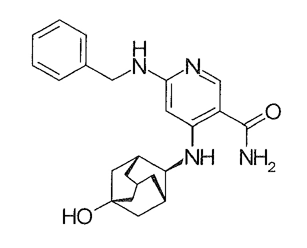 | 7 | 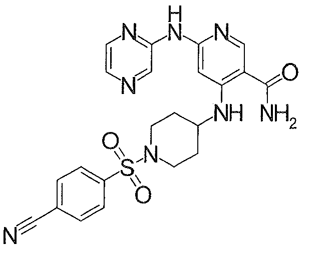 |

**[Table 60]**

| **Ex** | **DATA** |
|---|---|
| **1** | NMR-DMSO-d₆ : 1.03 (3H, d, J = 6.0 Hz), 1.36-1.47 (2H, m), 1.57-1.67 (2H, m), 1.76-1.83 (2H, m), 2.02-2.09 (2H, m), 2.24-2.38 (3H, m), 3.26-3.29 (2H, m), 3.56-3.63 (2H, m), 3.71-3.79 (1H, m), 4.24-4.30 (2H, m), 6.10 (1H, s), 6.48 (2H, brs), 6.70 (1H, dd, J = 2.0, 8.8 Hz), 6.99 (1H, d, J = 9.2 Hz), 7.06 (1H, br), 7.08 (1H, d, J = 2.4 Hz), 7.42 (1H, d, J = 8.8 Hz), 7.73-7.80 (2H, m), 7.85 (1H, dd, J = 2.4, 9.2 Hz), 8.38 (1H, s), 8.49 (1H, d), 8.68-8.71 (1H, m), 8.91 (1H, brs) ESI+ : 569 [M+H]+ |
| **1-1** | NMR-DMSO-d₆: 1.37-1.46 (2H, m), 2.02-2.09 (2H, m), 2.70 (3H, d, J = 4.4 Hz), 3.26-3.30 (2H, m), 3.56-3.63 (1H, m), 4.24-4.31 (2H, m), 6.09 (1H, s), 6.51 (2H, m), 6.70-6.74 (1H, m), 6.99 (1H, d, J = 9.1 Hz), 7.03 (1H, d, J = 2.1 Hz), 7.05 (1H, br), 7.36 (1H, d, J = 8.8 Hz), 7.77 (1H, br), 7.85 (1H, dd, J = 2.3, 9.1 Hz), 7.88-7.92 (1H, m), 8.38 (1H, s), 8.49 (1H, d, J = 2.1 Hz), 8.69 (1H, d, J = 7.1 Hz), 8.89 (1H, brs) ESI+ : 508 [M+Na]+ |
| **1-2** | NMR-DMSO-d₆ : 1.03 (3H, d, J = 6.0 Hz), 1.36-1.46 (2H, m), 2.02-2.09 (2H, m), 2.19-2.42 (4H, m), 3.25-3.30 (2H, m), 3.45-3.63 (5H, m), 4.24-4.31 (2H, m), 5.26 (2H, brs), 6.08 (1H, s), 6.82-6.85 (1H, m), 6.89-6.92 (1H, m), 6.99 (1H, d, J = 8.8 Hz), 7.02 (1H, d, J = 2.0 Hz), 7.05 (1H, br), 7.77 (1H, br), 7.85 (1H, dd, J = 2.4, 9.2 Hz), 8.36 (1H, s), 8.49 (1H, d, J = 2.4 Hz), 8.69 (1H, d, J = 7.2 Hz), 8.88 (1H, brs) ESI+: 555 [M+H]+ |
| **1-3** | NMR-DMSO-d₆ : 1.03 (3H, d, J = 6.1 Hz), 1.18-1.29 (2H, m), 1.37-1.47 (2H, m), 1.53-1.61 (1H, m), 1.66-1.73 (2H, m), 2.02-2.22 (4H, m), 2.30-2.37 (2H, m), 2.91-2.99 (2H, m), 3.06-3.11 (2H, m), 3.57-3.62 (1H, m), 4.24-4.30 (2H, m), 6.10 (1H, s), 6.48 (2H, brs), 6.71 (1H, dd, J = 2.1, 8.8 Hz), 6.99 (1H, d, J = 9.2 Hz), 7.04 (1H, br), 7.06 (1H, d, J = 2.1 Hz), 7.40 (1H, d, J = 8.8 Hz), 7.76 (1H, br), 7.85 (1H, dd, J = 2.4, 9.2 Hz), 7.95-8.00 (1H, m), 8.38 (1H, s), 8.49 (1H, d, J = 2.4 Hz), 8.69 (1H, d, J = 7.3 Hz), 8.89 (1H, brs) ESI+ : 583 [M+H]+ |
| **2** | NMR-DMSO-d₆ : 1.46-1.52 (2H, m), 1.71-1.77 (2H, m),1.88-1.98 (4H, m), 2.01-2.07 (2H, m), 2.17-2.21 (1H, m), 2.26-2.30 (2H, m), 3.58-3.62 (1H, m), 6.83-6.84 (1H, m), 7.07 (1H, br), 7.18 (1H, s), 7.62-7.64 (2H, m), 7.82 (1H, br), 8.19-8.21 (1H, m), 8.42 (1H, s), 9.13 (1H, d, J = 7.4 Hz), 9.47 (1H; s) ESI+ : 382 [M+H]+ |
| **3** | NMR-DMSO-d₆: 1.44-1.53 (2H, m), 1.95-2.02 (2H, m), 2.20-2.28 (2H, m), 2.69-2.76 (3H, m), 3.60 (2H, s), 7.04 (1H, s), 7.11 (1H, br), 7.54 (2H, d, J = 8.2 Hz), 7.80 (2H, d, J = 8.2 Hz), 7.86 (1H, br), 8.06 (1H, d, J = 2.6 Hz), 8.20-8.21 (1H, m), 8.44 (1H, s), 8.77 (1H, d, J = 7.1 Hz), 9.03 (1H, d, J = 1.4 Hz), 9.81 (1H, s) ESI+ : 429 [M+H]+ |
| **4** | NMR-DMSO-d₆ : 1.38-1.44 (2H, m), 1.63-1.77 (8H, m), 2.01-2.11 (3H, m), 3.43-3.46 (1H, m), 4.50 (1H, s), 5.09 (2H, s), 5.85 (1H, s), 6.98 (2H, d, J = 9.2 Hz), 7.01 (1H, br), 7.56 (2H, d, J = 9.2 Hz), 7.71 (1H, br), 8.34 (1H, s), 8.81 (1H, s), 9.06 (1H, d, J = 7.4 Hz) ESI+ : 434 [M+H]+ |
| **4-1** | NMR-DMSO-d₆: 1.54-1.64 (2H, m), 2.08-2.14 (2H, m), 2.87-2.94 (2H, m), 3.46-3.54 (3H, m), 5.07 (2H, s), 6.55 (1H, s), 6.95-7.02 (3H, m), 7.14 (1H, s), 7.86 (1H, br), 8.05-8.06 (1H, m), 8.23-8.24 (1H, m), 8.46 (1H, s), 8.62 (1H, br), 8.81-8.83 (1H, m), 9.02 (1H, s), 9.85 (1H, brs) ESI+ : 445 [M+H]+ |

**[Table 61]**

| **Ex** | **DATA** |
|---|---|
| **5** | NMR-DMSO-d₆: 1.56-1.81 (4H, m), 2.03-2.10 (1H, m), 3.44-3.53 (2H, m), 3.91-3.98 (1H, m), 4.19-4.24 (1H, m), 6.82-6.85 (1H, m), 6.92 (1H, d, J = 9.2 Hz), 7.06 (1H, br), 7.23 (1H, s), 7.59-7.66 (2H, m), 7.76 (1H, dd, J = 2.4, 9.2 Hz), 7.78 (1H, br), 8.05-8.07 (1H, m), 8.34 (1H, d, J = 2.0 Hz), 8.41 (1H, s), 8.85 (1H, d, J = 7.7 Hz), 9.50 (1H, s) ESI+ : 415 [M+H]+ |
| **5-1** | NMR-DMSO-d₆: 1.38-1.48 (2H, m), 2.07-2.14 (2H, m), 3.27-3.30 (2H, m), 3.60-3.68 (1H, m), 4.27-4.33 (2H, m), 6.84-6.88 (1H, m), 7.00 (1H, d, J = 9.0 Hz), 7.07 (1H, br), 7.26 (1H, s), 7.61-7.68 (2H, m), 7.80 (1H, br), 7.85 (1H, dd, J = 2.3, 9.0 Hz), 8.21-8.23 (1H, m), 8.41 (1H, s), 8.49-8.50 (1H, m), 8.74 (1H, d, J = 7.1 Hz), 9.49 (1H, s) ESI+ : 415 [M+H]+ |
| **5-2** | NMR-DMSO-d₆: 1.56-1.80 (4H, m), 2.03-2.09 (1H, m), 3.44-3.53 (2H, m), 3.91-3.98 (1H, m), 4.18-4.24 (1H, m), 6.82-6.85 (1H, m), 6.92 (1H, d, J = 9.0 Hz), 7.04 (1H, br), 7.23 (1H, s), 7.61-7.64 (2H, m), 7.76 (1H, dd, J = 2.4, 9.0 Hz), 7.80 (1H, br), 8.05-8.07 (1H, m), 8.34-8.35 (1H, m), 8.41 (1H, s), 8.84 (1H, d, J = 7.8 Hz), 9.49 (1H, s) ESI+ : 415 [M+H]+ |
| **5-3** | NMR-DMSO-d₆ : 1.40-1.49 (2H, m), 2.06-2.12 (2H, m), 3.28-3.35 (2H, m), 3.62-3.68 (1H, m), 4.26-4.32 (2H, m), 7.00 (1H, d, J = 9.1 Hz), 7.11 (1H, br), 7.14 (1H, s), 7.85 (1H, dd, J = 2.4, 9.1 Hz), 7.87 (1H, br), 8.07 (1H, d, J = 2.7 Hz), 8.23-8.24 (1H, m), 8.46 (1H, s), 8.49 (1H, m), 8.81 (1H, d, J = 7.2 Hz), 9.03 (1H, d, J = 1.5 Hz), 9.86 (1H, s) ESI+ : 416 [M+H]+ |
| **5-4** | NMR-DMSO-d₆: 1.45-1.55 (2H, m), 2.06-2.13 (2H, m), 3.13-3.21 (2H, m), 3.56-3.63 (1H, m), 3.82-3.89 (2H, m), 7.07 (2H, d, J = 9.1 Hz), 7.11 (1H, br), 7.15 (1H, s), 7.58 (2H, d, J = 9.1 Hz), 7.85 (1H, br), 8.07 (1H, d, J = 2.6 Hz), 8.23-8.24 (1H, dd, J =1.4, 2.6 Hz), 8.46 (1H, s), 8.81 (1H, d, J = 7.1 Hz), 9.01 (1H, d, J =1.4 Hz), 9.86 (1H, s) ESI+ : 415 [M+H]+ |
| **5-5** | NMR-DMSO-d₆: 1.45-1.54 (2H, m), 2.05-2.12 (2H, m), 3.24-3.29 (2H, m), 3.67-3.76 (1H, m), 4.28-4.35 (2H, m), 6.94 (1H, br), 7.01 (1H, d, J = 9.1 Hz), 7.53 (1H, br), 7.86 (1H, dd, J = 2.4, 9.1 Hz), 8.11 (1H, br), 8.46 (1H, s), 8.50 (1H, d, J = 2.4 Hz), 8.51-8.54 (1H, m), 8.83 (1H, s), 9.10 (1H, br), 10.77 (1H, br) ESI+ : 416 [M+H]+ |
| **5-6** | NMR-DMSO-d₆: 1.45-1.55 (2H, m), 2.06-2.13 (2H, m), 3.13-3.21 (2H, m), 3.55-3.63 (1H, m), 3.83-3.89 (2H, m), 7.07 (2H, d, J = 9.0 Hz), 7.17 (1H, s), 7.19 (1H, br), 7.58 (2H, d, J = 9.0 Hz), 7.73-7.76 (1H, m), 7.89 (1H, br), 8.40 (1H, d, J = 6.0 Hz), 8.46 (1H, s), 8.71 (1H, s), 8.79 (1H, d, J = 7.1 Hz), 10.00 (1H, s) ESI+ : 415 [M+H]+ |
| **5-7** | NMR-DMSO-d₆: 1.57-1.68 (1H, m), 1.99-2.07 (1H, m), 3.13-3.22 (1H, m), 3.403.49 (1H, m), 3.81^3.95 (1H, m), 4.53-4.61 (1H, m), 4.84-5.10 (2H, m), 7.05 (1H, d, J = 9.2 Hz), 7.12 (1H, s), 7.20 (1H, br), 7.87 (1H, dd, J = 2.4, 9.2 Hz), 7.92 (1H, br), 8.11 (1H, d, J = 2.8 Hz), 8.26 (1H, dd, J = 1.6,2.8 Hz), 8.45 (1H, s), 8.49 (1H, d, J = 1.6 Hz), 8.99 (1H, s), 9.02-9.06 (1H, m), 9.93 (1H, brs) ESI+ : 434 [M+H]+ |

**[Table 62]**

| **Ex** | **DATA** |
|---|---|
| **5-8** | NMR-DMSO-d₆ : 1.44-1.56 (2H, m), 2.08-2.18 (2H, m), 2.49 (3H, s), 3.10-3.20 (2H, m), 3.54-3.65 (1H, m), 3.85-3.94 (2H, m), 7.07 (2H, d, J = 9.1 Hz), 7.16 (1H, br), 7.34 (1H, d, J = 5.8 Hz), 7.46 (1H, s), 7.58 (2H, d, J = 9.0 Hz), 7.86 (1H, br), 8.29 (1H, d, J = 5.9 Hz), 8.44 (1H, s), 8.80 (1H, d, J = 6.8 Hz), 9.94 (1H, s) ESI+ : 429 [M+H]+ |
| **5-9** | NMR-DMSO-d₆: 1.38-1.50 (2H, m), 2.09-2.18 (2H, m), 2.50 (3H, s), 3.24-3.44 (2H, m), 3.60-3.70 (1H, m), 4.30-4.40 (2H, m), 7.00 (1H, d, J = 9.0 Hz), 7.16 (1H, br), 7.34 (1H, d, J = 5.8 Hz), 7.46 (1H, s), 7.82-7.92 (2H, m), 8.29 (1H, d, J = 5.9 Hz), 8.44 (1H, s), 8.49 (1H:, d, J = 2.3 Hz), 8.79 (1H d, J = 6.9 Hz), 9.94 (1H, s) ESI+ : 430 [M+H]+ |
| **5-10** | NMR-DMSO-d₆: 1.56-1.70 (1H, m), 2.00-2.16 (1H, m), 3.13-3.21 (1H, m), 3.40-3.47 (1H, m), 3.79-3.92 (1H, m), 4.54-4.61 (1H, m), 4.84-5.10 (2H, m), 7.05 (1H, d, J = 9.0 Hz), 7.19 (1H, s), 7.22 (1H, br), 7.73-7.75 (1H, m), 7.87 (1H, dd, J = 2.4, 9.0 Hz), 7.91 (1H, br), 8.73 (1H, s), 8.48 (1H, s), 8.50 (1H, d, J = 2.2 Hz), 8.72 (1H, s), 8.99 (1H, d, J = 8.1 Hz),10.00 (1H, s) ESI+ : 434 [M+H]+ |
| **5-11** | NMR-DMSO-d₆: 1.61-1.73 (1H, m), 2.05-2.11 (1H, m), 3.36-3.59 (2H, m), 3.84-3.98 (1H, m), 4.65-4.73 (1H, m), 4.91-5.14 (2H, m), 7.17 (1H, s), 7.19 (1H, br), 7.49 (1H, d, J = 9.8 Hz), 7.86 (1H, br), 7.90 (1H, d, J = 9.8 Hz), 8.09 (1H, d, J = 2.6 Hz), 8.25-8.27 (1H, m), 8.48 (1H, s), 9.00-9.03 (2H, m), 9.87 (1H, s) ESI+ : 435 [M+H]+ |
| **5-12** | NMR-DMSO-d₆: 1.54-1.64 (1H, m), 1.95-2.00 (1H, m), 3.00-3.04 (4H, m), 3.08-3.15 (1H, m), 3.35-3.43 (1H, m), 3.70-3.77 (4H, m), 3.79-3.85 (1H, m), 4.51-4.58 (1H, m), 4.79-5.04 (2H, m), 5.96 (1H, s), 6.88 (2H, d, J = 8.9 Hz), 7.00 (1H, br), 7.04 (1H, d, J = 9.2 Hz), 7.43 (2H, d, J = 8.9 Hz), 7.71 (1H, br), 7.86 (1H, dd, J = 2.4, 9.2 Hz), 8.34 (1H, s), 8.49 (1H, d, J = 2.1 Hz), 8.67-8.74 (1H, m), 8.86-8.92 (1H, m) ESI+ : 517 [M+H]+ |
| **5-13** | NMR-DMSO-d₆ : 1.45-1.55 (2H, m), 2.10-2.17 (2H, m), 3.35-3.45 (2H, m), 3.65-3.73 (1H, m), 4.36-4.43 (2H, m), 7.12 (1H, br), 7.15 (1H, s), 7.42 (1H, d, J = 9.8 Hz), 7.85 (1H, br), 7.87 (1H, d, J = 9.8 Hz), 8.08 (1H, d, J = 2.6 Hz), 8.23-8.25 (1H, m), 8.46 (1H, s), 8.82 (1H, d, J = 7.0 Hz), 9.04 (1H, s), 9.86 (1H, s) ESI+: 417 [M+H]+ |
| **5-14** | NMR-DMSO-d₆: 2.02-2.10 (1H, m), 2.38-2.45 (1H, m), 3.24-3.29 (1H, m), 3.45-3.49 (2H, m), 3.78-3.83 (1H, m), 4.18-4.22 (1H, m), 6.67 (2H, d, J = 8.8 Hz), 7.12 (1H, s), 7.15 (1H, br), 7.55 (2H, d, J = 8.8 Hz), 7.87 (1H, br), 8.08 (1H, d, J = 2.7 Hz), 8.23-8.24 (1H, m), 8.47 (1H, s), 8.95 (1H, d, J = 6.3 Hz), 9.04 (1H, d, J = 1.4 Hz), 9.90 (1H, s) ESI+ : 423 [M+Na]+ |
| **5-15** | NMR-DMSO-d₆: 1.59-1.73 (1H, m), 2.05-2.11 (1H, m), 3.22-3.27 (1H, m), 3.42-3.57 (1H, m), 3.82-3.98 (1H, m), 4.62-4.68 (1H, m), 4.91-5.15 (2H, m), 7.20 (1H, s), 7.24 (1H, s), 7.74 (1H, d, J = 6.0 Hz), 7.93 (1H, br), 8.42 (1H, d, J = 6.0 Hz), 8.49 (1H, s), 8.55-8.56 (1H, m), 8.58 (1H, d, J = 1.2 Hz), 8.72-8.73 (1H, m), 8.99 (1H, d, J = 8.0 Hz), 10.00 (1H, s) ESI+ : 435 [M+H]+ |

**[Table 63]**

| **Ex** | **DATA** |
|---|---|
| **5-16** | NMR-DMSO-d₆ : 2.04-2.11 (1H, m), 2.40-2.46 (1H, m), 3.24-3.29 (1H, m), 3.44-3.49 (2H, m), 3.78-3.83 (1H, m), 4.18-4.23 (1H, m), 6.67 (2H, d, J = 8.9 Hz), 7.12 (1H, s), 7.15 (1H, br), 7.55 (2H, d, J = 8.9 Hz), 7.88 (1H, br), 8.08 (1H, d, J = 2.6 Hz), 8.23-8.24 (1H, m), 8.47 (1H, s), 8.95 (1H, d, J = 6.4 Hz), 9.04 (1H, d, J = 1.5 Hz), 9.90 (1H, s) ESI+ : 401 [M+H]+ |
| **5-17** | NMR-DMSO-d₆: 1.54-1.66 (1H, m), 1.96-2.02 (1H, m), 3.08-3.17 (1H, m), 3.36-3.43 (1H, m), 3.75-3.88 (1H, m), 4.52-4.59 (1H, m), 4.82-5.05 (2H, m), 6.07 (1H, m), 6.86-6.91 (1H, m), 7.05 (1H, d, J = 9.1 Hz), 7.07 (1H, br), 7.22-7.27 (2H, m), 7.62 (2H, d, J = 7.8 Hz), 7.75 (1H, br), 7.86 (1H, dd, J = 2.3, 9.1 Hz), 8.39 (1H, s), 8.49 (1H, d, J = 2.3 Hz), 8.88 (1H, d, J = 8.3 Hz), 8.94 (1H, s) ESI+ : 432 [M+H]+ |
| **5-18** | NMR-DMSO-d₆ : 1.45-1.55 (2H, m), 2.10-2.17 (2H, m), 3.67-3.44 (2H, m), 3.65-3.72 (1H, m), 4.37-4.44 (2H, m), 7.17 (1H, s), 7.21 (1H, br), 7.42 (1H, d, J = 9.7 Hz), 7.75-7.78 (1H, m), 7.87 (1H, d, J = 9.7 Hz), 7.90 (1H, br), 8.41 (1H, d, J = 6.0 Hz), 8.46 (1H, s), 8.72 (1H, s), 8.80 (1H, d, J = 7.1 Hz), 10.00 (1H, s) ESI+ : 417 [M+H]+ |
| **5-19** | NMR-DMSO-d₆ : 1.54-1.63 (1H, m), 1.96-2.02 (1H, m), 2.28-2.36 (1H, m), 3.08-3.16 (1H, m), 3.73 (3H, s), 3.76-3.88 (1H, m), 4.51-4.60 (1H, m), 4.82-5.05 (2H, m), 6.08 (1H, s), 6.46-6.48 (1H, m), 7.00-7.06 (1H, m), 7.12-7.15 (3H, m), 7.37-7.39 (1H, m), 7.77 (1H, br), 7.86 (1H, dd, J = 2.4, 9.2 Hz), 8.40 (1H, s), 8.49 (1H, d, J = 2.4 Hz), 8.88 (1H, d, J = 8.2 Hz), 8.94 (1H, s) ESI+ : 462 [M+H]+ |
| **5-20** | NMR-DMSO-d₆ : 1.55-1.65 (1H, m), 1.96-2.03 (1H, m), 2.30-2.37 (1H, m), 3.09-3.17 (1H, m), 3.76-3.90 (1H, m), 4.54-4.60 (1H, m), 4.82-5.05 (2H, m), 6.08 (1H, s), 6.64-6.68 (1H, m), 7.05 (1H, d, J = 9.1 Hz), 7.12 (1H, br), 7.23-7.26 (2H, m), 7.79-7.88 (3H, m), 8.43 (1H, s), 8.49 (1H, d, J = 2.3 Hz), 8.90 (1H, d, J = 8.3 Hz), 9.21 (1H, s) ESI+ : 450 [M+H]+ |
| **5-21** | NMR-DMSO-d₆ : 1.38-1.48 (2H, m), 2.04-2.13 (2H, m), 2.39 (3H, s), 3.26-3.40 (2H, m), 3.58-3.68 (1H, m), 4.26-4.34 (2H, m), 7.00 (1H, d, J = 9.1 Hz), 7.07 (1H, br), 7.69 (1H, br), 7.85 (1H, dd, J = 0.6, 8.4 Hz), 8.13 (1H, s), 8.18-8.27 (1H, m), 8.44 (1H, s), 8.495 (1H, d, J = 2.2 Hz), 8.79 ESI+ : 430 [M+H]+ |
| **5-22** | NMR-DMSO-d₆ : 1.44-1.55 (2H, m), 2.09-2.18 (2H, m), 2.39 (3H, s), 3.36-3.44 (2H, m), 3.62-3.72 (1H, m), 4.36-4.45 (2H, m), 7.05-7.20 (2H, m), 7.42 (1H, d, J = 9.8 Hz), 7.70-7.90 (2H, m), 8.12-8.15 (1H, m), 8.44 (1H, s), 8.80 (1H, d, J = 7.0 Hz), 8.94 (1H, d, J = 1.4 Hz), 9.70 (1H, s) ESI+ : 431 [M+H]+ |
| **5-23** | NMR-DMSO-d₆: 1.23-1.34 (2H, m), 1.76-1.83 (2H, m), 1.87-1.95 (1H, m), 2.83-2.91 (2H, m), 3.06-3.11 (2H, m), 3.95-4.02 (2H, m), 7.01 (2H, d, J = 9.0 Hz), 7.03-7.05 (1H, m), 7.12 (1H, br), 7.55 (2H, d, J = 9.0 Hz), 7.84 (1H, br), 8.06 (1H, d, J = 2.7 Hz), 8.19-8.21 (1H, m), 8.43 (1H, s), 8.83-8.87 (1H, m), 9.01 (1H, s), 9.85 (1H, brs) ESI+ : 429 [M+H]+ |

**[Table 64]**

| **Ex** | **DATA** |
|---|---|
| **5-24** | NMR-DMSO-d₆: 3.79-3.83 (2H, m), 4.38-4.44 (3H, m), 6.55 (2H, d, J = 8.8 Hz), 6.99 (1H, brs), 7.25 (1H, br), 7.57 (2H, d, J = 8.8 Hz), 7.92 (1H, br), 8.09 (1H, d, J = 2.6 Hz), 8.27-8.28 (1H, m), 8.50 (1H, s), 8.96 (1H, s), 9.09-9.12 (1H, m), 9.99 (1H, brs) ESI+ : 387 [M+H]+ |
| **5-25** | NMR-DMSO-d₆: 1.47-1.57 (2H, m), 2.08-2.15 (2H, m), 3.18-3.26 (2H, m), 3.57-3.65 (1H, m), 3.91-3.98 (2H, m), 7.18 (1H, s), 7.21 (1H, br), 7.42 (1H, dd, J = 3.0, 8.9 Hz), 7.73-7.76 (2H, m), 7.90 (1H, br), 8.40-8.42 (1H, m), 8.45-8.48 (2H, m), 8.72 (1H, s), 8.79 (1H, d, J = 7.0 Hz), 10.01 (1H, brs) ESI+ : 416 [M+H]+ |
| **5-26** | NMR-DMSO-d₆: 1.58-1.69 (1H, m), 2.05-2.12 (1H, m), 3.12-3.20 (1H, m), 3.32 (3H, s), 3.40-3.51 (1H, m), 3.81-3.95 (1H, m), 4.58-4.64 (1H, m), 4.85-4.94 (1H, m), 4.97-5.12 (1H, m), 7.06 (1H, d, J = 9.0 Hz), 7.19 (1H, br), 7.34 (1H, d, J = 5.9 Hz), 7.47 (1H, s), 7.87 (1H, dd, J = 2.3, 9.0 Hz), 7.90 (1H, br), 8.30 (1H, d, J = 5.9 Hz), 8.47 (1H, s), 8.49 (1H, d, J = 2.3 Hz), 9.01 (1H, d, J = 7.9 Hz), 9.95 (1H, brs) ESI+ : 448 [M+H]+ |
| **5-27** | NMR-DMSO-d₆: 1.63-1.73 (1H, m), 2.09-2.16 (1H, m), 3.32 (3H, s), 3.40-3.58 (2H, m), 3.86-4.00 (1H, m), 4.68-4.76 (1H, m), 4.94-5.17 (2H, m), 7.21 (1H, br), 7.35 (1H, d, J = 5.9 Hz), 7.56-7.52 (2H, m), 7.89 (1H, br), 7.90 (1H, d, J = 9.7 Hz), 8.30 (1H, d, J = 5.9 Hz), 8.47 (1H, s), 9.03 (1H, d, J = 7.8 Hz), 9.96 (1H, brs) ESI+ : 449 [M+H]+ |
| **5-28** | NMR-DMSO-d₆ : 1.40-1.50 (2H, m), 2.06-2.12 (2H, m), 3.25-3.28 (2H, m), 3.59-3.67 (1H, m), 4.25-4.31 (4H, m), 6.95 (1H, d, J = 9.0 Hz), 7.13 (1H, br), 7.15 (1H, s), 7.85 (1H, br), 7.96 (1H, dd, J = 2.4, 9.0 Hz), 8.07 (1H, d, J = 2.7 Hz), 8.23-8.25 (1H, m), 8.46 (1H, s), 8.62 (1H, d, J = 2.4 Hz), 8.81-8.84 (1H, m), 8.92-8.96 (1H, m), 9.03 (1H, d, J =1.4 Hz), 9.86 (1H, brs) ESI+ : 473 [M+H]+ |
| **5-29** | NMR-D₂O: 1.40-1.50 (2H, m), 2.06-2.12 (2H, m), 3.10 (3H, s), 3.24-3.29 (2H, m), 3.58-3.66 (1H, m), 4.21-4.28 (2H, m), 4.49 (2H, s), 6.94 (1H, d, J = 8.9 Hz), 7.12 (1H, br), 7.15 (1H, s), 7.68 (1H, dd, J = 2.4, 8.9 Hz), 7.85 (1H, br), 8.07 (1H, d, J = 2.7 Hz), 8.23-8.25 (1H, m), 8.30-8.32 (1H, m), 8.46 (1H, s), 8.82 (1H, d, J = 7.2 Hz), 9.03 (1H, d, J =1.4 Hz), 9.86 (1H, brs) ESI+ : 487 [M+H]+ |
| **5-30** | NMR-DMSO-d₆: 1.02-1.05 (4H, m), 1.35-1.45 (2H, m), 2.00-2.07 (2H, m), 2.23 (3H, s), 3.03-3.07 (4H, m), 3.26-3.30 (2H, m), 3.53-3.60 (1H, m), 4.21-4.28 (2H, m), 5.92 (1H, s), 6.86 (2H, d, J = 9.0 Hz), 6.94 (1H, br), 6.99 (1H, d, J = 9.2 Hz), 7.41 (2H, d, J = 9.0 Hz), 7.68 (1H, br), 7.84 (1H, dd, J = 2.4, 9.2 Hz), 8.33 (1H, s), 8.49 (1H, d, J = 2.0 Hz), 8.65-8.69 (2H, m) ESI+ : 512 [M+H]+ |
| **5-31** | NMR-DMSO-d₆: 1.34-1.44 (2H, m), 1.92-1.96 (4H, m), 1.98-2.05 (2H, m), 3.16-3.20 (4H, m), 3.27-3.30 (2H, m), 3.51-3.57 (1H, m), 4.19-4.25 (2H, m), 5.86 (1H, s), 6.49 (2H, d, J = 8.9 Hz), 6.89 (1H, br), 6.98 (1H, d, J = 9.1 Hz), 7.30 (2H, d, J = 8.9 Hz), 7.62 (1H, br), 7.84 (1H, dd, J = 2.3, 9.1 Hz), 8.30 (1H, s), 8.46 (1H, s), 8.48 (1H, d, J = 2.1 Hz), .69 (1H, d, J = 7.2 Hz) ESI+ : 483 [M+H]+ |

**[Table 65]**

| **Ex** | **DATA** |
|---|---|
| **5-32** | NMR-DMSO-d₆ : 1.36-1.45 (2H, m), 2.00-2.07 (2H, m), 2.99-3.04 (4H, m), 3.26-3.28 (2H, m), 3.53-3.60 (1H, m), 3.70-3.75 (4H, m), 4.21-4.28 (2H, m), 5.93 (1H, s), 6.87 (2H, d, J = 9.0 Hz), 6.94 (1H, br), 6.99 (1H, d, J = 9.2 Hz), 7.44 (2H, d, J = 9.0 Hz), 7.67 (1H, br), 7.84 (1H, dd, J = 2.4, 9.2 Hz), 8.33 (1H, s), 8.49 (1H, d, J = 2.3 Hz), 8.66-8.70 (2H, m) ESI+ : 499 [M+H]+ |
| **5-33** | NMR-DMSO-d₆: 1.38-1.48 (2H, m), 2.03-2.09 (2H, m), 2.45 (3H, d, J = 5.0 Hz), 3.25-3.29 (2H, m), 3.59-3.67 (1H, m), 4.25-4.32 (2H, m), 6.10 (1H, s), 7.00 (1H, d, J = 9.2 Hz), 7.13 (1H, br), 7.24-7.50 (3H, m), 7.75-7.82 (2H, m), 7.85 (1H, dd, J = 2.4, 9.2 Hz), 8.23 (1H, br), 8.42 (1H, s), 8.49 (1H, d, J = 2.2 Hz), .76 (1H, br), 9.36 (1H, br) ESI+ : 507 [M+H]+ |
| **5-34** | NMR-DMSO-d₆: 1.42-1.54 (2H, m), 2.00-2.10 (2H, m), 2.58 (3H, s), 3.10-3.20 (2H, m), 3.50-3.60 (1H, m), 3.78-3.88 (2H, m), 6.01 (1H, s), 7.00-7.15 (3H, m), 7.38 (1H, dd, J = 2.1, 8.8 Hz), 7.50-7.60 (3H, m), 7.80 (1H, br), 8.06-8.12 (1H, m), 8.38 (1H, s), 8.74 (1H, br), 9.08 (1H, br) ESI+ : 468 [M+H]+ |
| **5-35** | NMR-DMSO-d₆: 1.36-1.48 (2H, m), 2.02-2.10 (2H, m), 2.58 (3H, s), 3.25-3.36 (2H, m), 3.56-3.66 (1H, m), 4.20-4.30 (2H, m), 6.02 (1H, s), 6.97-7.18 (2H, m), 7.39 (1H, dd, J = 2.1, 8.8 Hz), 7.52 (1H, d, J = 8.8 Hz), 7.72-7.89 (2H, m), 8.10 (1H, br), 8.39 (1H, s), 8.49 (1H, d, J = 2.1 Hz), 8.74 (1H, br), 9.08 (1H, br) ESI+ : 469 [M+H]+ |
| **5-36** | NMR-DMSO-d₆: 1.38-1.47 (2H, m), 2.03-2.10 (2H, m), 2.56 (3H, s), 3.25-3.32 (2H, m), 3.58-3.64 (1H, m), 4.25-4.32 (2H, m), 6.07 (1H, s), 7.00 (1H, d, J = 9.1 Hz), 7.09 (1H, br), 7.37-7.42 (1H, m), 7.48-7.51 (1H, m), 7.79 (1H, br), 7.85 (1H, dd, J = 2.4, 9.1 Hz), 7.87-7.90 (1H, m), 8.26 (1H, s), 8.42 (1H, s), 8.49 (1H, d, J = 2.2 Hz), 8.72 (1H, d, J = 7.2 Hz), 9.19 (1H, brs) ESI+ : 456 [M+H]+ |
| **5-37** | NMR-DMSO-d₆ : 1.42-1.54 (2H, m), 1.98-2.08 (2H, m), 2.80 (3H, s), 3.20-3.40 (2H, m), 3.66-3.78 (1H, m), 4.20-4.30 (2H, m), 6.15 (1H, s), 7.00 (1H, d, J = 9.2 Hz), 7.38-7.60 (2H, m), 7.86 (1H, dd, J = 2.4, 9.1 Hz), 7.92-8.30 (3H, m), 8.50 (1H, s), 9.32 (1H, br), 9.84 (1H, br), 12.63 (1H, br) ESI+ : 485 [M+H]+ |
| **5-38** | NMR-DMSO-d₆: 1.37-1.46 (2H, m), 2.01-2.09 (4H, m), 2.42-2.47 (2H, m), 3.26-3.31 (2H, m), 3.56-3.63 (1H, m), 3.77-3.82 (2H, m), 4.24-4.30 (2H, m), 6.01 (1H, s), 6.99 (1H, d, J = 9.2 Hz), 7.02 (1H, br), 7.51 (2H, d, J = 9.1 Hz), 7.60 (2H, d, J = 9.1 Hz), 7.71 (1H, br), 7.85 (1H, dd, J = 2.4, 9.2 Hz), 8.37 (1H, s), 8.49 (1H, d, J = 2.4 Hz), 8.69 (1H, d, J = 7.2 Hz), 8.94 (1H, brs) ESI+ : 497 [M+H]+ |
| **5-39** | NMR-DMSO-d₆ : 1.37-1.47 (2H, m), 2.03-2.10 (2H, m), 2.79 (3H, d, J = 4.6 Hz), 3.26-3.30 (2H, m), 3.58-3.65 (1H, m), 3.88 (3H, s), 4.25-4.31 (2H, m), 6.12 (1H, s), 7.00 (1H, d, J = 9.1 Hz), 7.10 (1H, br), 7.26 (1H, dd, J = 1.9, 8.6 Hz), 7.59 (1H, d, J = 1.9 Hz), 7.77 (1H, d, J = 8.6 Hz), 7.80 (1H, br), 7.85 (1H, dd, J = 2.4, 9.1 Hz), 7.94-7.99 (1H, m), 8.43 (1H, s), 8.49 (1H, d, J = 2.1 Hz), 8.72 (1H, d, J = 7.2 Hz), 9.27 (1H, s) ESI+ : 523 [M+Na]+ |

**[Table 66]**

| **Ex** | **DATA** |
|---|---|
| **5-40** | NMR-DMSO-d₆: 1.39-1.49 (2H, m), 2.02-2.09 (2H, m), 2.77 (3H, d, J = 4.5 Hz), 3.25-3.29 (2H, m), 3.60-3.66 (1H, m), 4.25-4.32 (2H, m), 6.13 (1H, s), 7.00 (1H, d, J = 9.1 Hz), 7.20 (1H, br), 7.25-7.28 (1H, m), 7.58-7.64 (1H, m), 7.84 (1H, d, J = 2.4 Hz), 7.86 (1H, d, J = 2.4 Hz), 7.86-7.91 (2H, m), 8.43 (1H, s), 8.49 (1H, d, J = 2.3 Hz), 8.79 (1H, br), 9.52 (1H, br) ESI+ : 511 [M+Na]+ |
| **5-41** | NMR-DMSO-d₆: 1.36-1.48 (2H, m), 2.01-2.10 (2H, m), 2.56 (3H, s), 3.25-3.36 (2H, m), 3.56-3.66 (1H, m), 4.24-4.32 (2H, m), 6.05 (1H, s), 6.96-7.16 (2H, m), 7.31 (1H, dd, J = 2.0, 8.6 Hz), 7.49 (1H, d, J = 8.6 Hz), 7.70-7.88 (2H, m), 8.28 (1H, d, J = 1.9 Hz), 8.42 (1H, s), 8.49 (1H, d, J = 2.2 Hz), 8.70 (1H, d, J = 7.2 Hz), 9.18 (1H, s) ESI+ : 469 [M+H]+ |
| **5-42** | NMR-DMSO-d₆: 1.36-1.48 (2H, m), 2.02-2.10 (2H, m), 2.74 (3H, s), 3.24-3.36 (2H, m), 3.56-3.66 (1H, m), 4.24-4.33 (2H, m), 6.07 (1H, s), 6.97-7.16 (2H, m), 7.48 (1H, dd, J = 2.2, 8.8 Hz), 7.66-7.78 (2H, m), 7.85 (1H, dd, J = 2.4, 9.1 Hz), 8.42 (1H, s), 8.49 (1H, d, J = 2.3 Hz), 8.54 (1H, d, J = 2.1 Hz), 8.71 (1H, d, J = 7.3 Hz), 9.19 (1H, s) ESI- : 483 [M-H]- |
| **5-43** | NMR-DMSO-d₆: 1.37-1.47 (2H, m), 2.02-2.09 (2H, m), 2.78 (3H, d, J = 4.4 Hz), 3.25-3.30 (2H, m), 3.58-3.63 (1H, m), 4.25-4.32 (2H, m), 6.10 (1H, s), 6.95-6.99 (1H, m), 7.00 (1H, d, J = 9.2 Hz), 7.10 (1H, br), 7.39-7.45 (1H, m), 7.63 (1H, d, J = 8.9 Hz), 7.80 (1H, br), 7.85 (1H, dd, J = 2.4, 9.2 Hz), 8.42 (1H, s), 8.47-8.50 (2H, m), 8.71 (1H, d, J = 7.2 Hz), 9.20 (1H, brs), 12.95 (1H, brs) ESI+ : 487 [M+H]+ |
| **5-44** | NMR-DMSO-d₆: 1.23-1.34 (2H, m),1.76-1.82 (2H, m), 1.86-1.94 (1H, m), 2.83-2.91 (2H, m), 3.06-3.11 (2H, m), 3.95-4.02 (2H, m), 7.00-7.05 (3H, m), 7.13 (1H, br), 7.54 (2H, d, J = 9.1 Hz), 7.85 (1H, br), 8.06 (1H, d, J = 2.7 Hz), 8.19-8.21 (1H, m), 8.44 (1H, s), 8.83-8.88 (1H, m), 9.00 (1H, s), 9.86 (1H, brs) ESI+ : 429 [M+H]+ |
| **5-45** | NMR-DMSO-d₆: 1.36-1.47 (2H, m), 2.03-2.10 (2H, m), 3.26-3.30 (2H, m), 3.59-3.65 (1H, m), 4.24-4.32 (2H, m), 6.12 (1H, s), 7.00 (1H, d, J = 9.1 Hz), 7.11 (1H, br), 7.30 (1H, m), 7.80-7.88 (6H, m), 8.12 (1H, m), 8.43 (1H, s), 8.49 (1H, d, J = 2.1 Hz), 8.72 (1H, d, J = 7.3 Hz), 9.33 (1H, brs) ESI- : 479 [M-H]- |
| **5-46** | NMR-DMSO-d₆: 1.37-1.49 (2H, m), 2.03-2.14 (4H, m), 2.44-2.56 (2H, m), 3.27-3.38 (2H, m), 3.58-3.68 (1H, m), 3.82 (2H, t, J = 7.0 Hz), 4.24-4.34 (2H, m), 6.96-7.18 (3H, m), 7.68-7.88 (3H, m), 7.96 (1H, dd, J = 2.8, 9.1 Hz), 8.41 (1H, s), 8.46 (1H, d, J = 2.7 Hz), 8.49 (1H, d, J = 2.3 Hz), 8.75 (1H, d, J = 7.1 Hz), 9.52 (1H, s) ESI+ : 498 [M+H]+ |
| **5-47** | NMR-DMSO-d₆: 1.39-1.48 (2H, m), 2.03-2.09 (2H, m), 2.73 (3H, d, J = 4.6 Hz), 3.25-3.30 (2H, m), 3.59-3.67 (1H, m), 4.26-4.32 (2H, m), 6.11 (1H, s), 7.00 (1H, d, J = 9.1 Hz), 7.16 (1H, br), 7.48 (1H, d, J = 8.5 Hz), 7.72 (1H, dd, J = 2.2, 8.5 Hz), 7.82 (1H, br), 7.85 (1H, dd, J = 2.2, 9.1 Hz), 8.41-8.44 (1H, m), 8.47 (1H, s), 8.49 (1H, d, J = 2.4 Hz), 8.58 (1H, d, J = 2.2 Hz), 8.77 (1H, d, J = 7.2 Hz), 9.65 (1H, brs) ESI+ : 538 [M+Na]+ |

**[Table 67]**

| Ex | DATA |
|---|---|
| 5-48 | NMR-DMSO-d₆ : 1.37-1.49 (2H, m), 2.02-2.10 (2H, m), 3.12 (3H, s), 3.26-3.38 (2H, m), 3.58-3.69 (1H, m), 4.25-4.33 (2H, m), 6.15 (1H, s), 7.00 (1H, d, J = 9.1 Hz), 7.10-7.25 (1H, m), 7.74-7.92 (6H, m), 8.44 (1H, s), 8.50 (1H, d, J = 2.2 Hz), 8.75 (1H, d, J = 7.4 Hz), 9.55 (1H, s) ESI+ : 492 [M+H]+ |
| 5-49 | NMR-DMSO-d₆ : 1.42-1.58 (2H, m), 2.04-2.14 (2H, m), 3.12-3.21 (2H, m), 3.27 (3H, s), 3.55-3.67 (1H, m), 3.82-3.92 (2H, m), 4.35 (2H, s), 7.08 (2H, d, J = 9.1 Hz), 7.14-7.34 (2H, m), 7.54-7.70 (4H, m), 7.72-8.04 (1H, m), 8.20 (1H, s), 8.41 (1H, s), 8.80 (1H, br), 9.58 (1H, br) ESI+ : 458 [M+H]+ |
| 5-50 | NMR-DMSO-d₆ : 1.74-1.82 (2H, m), 2.12-2.20 (1H, m), 3.34-3.38 (1H, m), 3.40-3.47 (2H, m), 3.59-3.66 (1H, m), 3.82-3.91 (2H, m), 4.00-4.06 (1H, m), 4.67 (1H, t, J = 5.2 Hz), 6.84-6.87 (1H, m), 6.97 (1H, d, J = 9.2 Hz), 7.08 (1H, br), 7.24 (1H, s), 7.61-7.67 (2H, m), 7.83 (1H, br), 7.85 (1H, dd, J = 2.4, 8.8 Hz), 8.0-8.23 (1H, m), 8.43 (1H, s), 8.49 (1H, d, J = 2.4 Hz), 9.16 (1H, d, J = 7.6 Hz), 9.50 (1H, s) ESI+ : 445 [M+H]+ |
| 5-51 | NMR-DMSO-d₆ : 1.74-1.82 (2H, m), 2.12-2.20 (1H, m), 3.27 (3H, s), 3.34-3.37 (1H, m), 3.41-3.48 (2H, m), 3.59-3.67 (1H, m), 3.81-3.92 (2H, m), 4.00-4.06 (1H, m), 4.33 (2H, s), 4.65-4.68 (1H, m), 6.97 (1H, d, J = 9.2 Hz), 7.09 (1H, br), 7.26 (1H, s), 7.58-7.66 (2H, m), 7.82 (1H, br), 7.85 (1H, dd, J = 2.4, 9.2 Hz), 8.17-8.18 (1H, m), 8.43 (1H, s), 8.49 (1H, d, J = 2.4 Hz), 9.16 (1H, d, J = 8.0 Hz), 9.57 (1H, s) ESI+ : 489 [M+H]+ |
| 5-52 | NMR-DMSO-d₆: 1.73-1.81 (2H, m), 2.11-2.18 (1H, m), 3.33-3.37 (2H, m), 3.40-3.47 (2H, m), 3.82-3.90 (2H, m), 4.00-4.06 (1H, m), 4.65-4.68 (1H, m), 6.97 (1H, d, J = 9.2 Hz), 7.06 (1H, s), 7.10 (1H, br), 7.60-7.65 (1H, m), 7.79 (1H, dd, J = 4.0, 9.2 Hz), 7.82 (1H, br), 7.85 (1H, dd, J = 2.4, 9.2 Hz), 8.20 (1H, d, J = 3.2 Hz), 8.42 (1H, s), 8.49 (1H, d, J = 2.4 Hz), 9.16 (1H, d, J = 7.6 Hz), 9.59 (1H, s) ESI+ : 463 [M+H]+ |
| 5-53 | NMR-DMSO-d₆ : 1.76-1.82 (2H, m), 2.12-2.19 (1H, m), 3.34-3.38 (1H, m), 3.41-3.47 (2H, m), 3.60-3.67 (1H, m), 3.82-3.93 (2H, m), 4.01-4.08 (1H, m), 4.66-4.69 (1H, m), 6.97 (1H, d, J = 9.2 Hz), 7.18 (1H, br), 7.23 (1H, s), 7.84-7.92 (3H, m), 7.99 (1H, dd, J = 2.4, 9.2 Hz), 8.48 (1H, s), 8.49 (1H, d, J = 2.4 Hz), 8.57-8.59 (1H, m), 9.21 (1H, d, J = 7.6 Hz), 10.07 (1H, s) ESI+ : 513 [M+H]+ |
| 5-54 | NMR-DMSO-d₆: 1.36-1.48 (2H, m), 2.00-2.10 (2H, m), 3.25-3.38 (5H, m), 3.54-3.65 (1H, m), 4.22-4.32 (2H, m), 5.99 (1H, s), 6.95-7.10 (2H, m), 7.12 (1H, d, J = 8.5 Hz), 7.25 (1H, dd, J = 1.9, 8.5 Hz), 7.64-7.82 (1H, m), 7.85 (1H, dd, J = 2.4, 9.1 Hz), 7.93 (1H, d, J = 1.9 Hz), 8.39 (1H, s), 8.49 (1H, d, J = 2.3 Hz), 8.70 (1H, d, J = 7.3 Hz), 9.04 (1H, s) ESI+ : 485 [M+H]+ |
| 5-55 | NMR-DMSO-d₆: 1.56-1.67 (1H, m), 1.81-1.90 (4H, m), 2.00-2.07 (1H, m), 2.37-2.41 (2H, m), 3.13-3.21 (1H, m), 3.36-3.51 (1H, m), 3.57-3.61 (2H, m), 3.77-3.91 (1H, m), 4.54-4.61 (1H, m), 4.84-4.92 (1H, m), 4.95-5.09 (1H, m), 7.05 (1H, d, J = 8.8 Hz), 7.11 (1H, br), 7.28 (1H, s), 7.57 (1H, dd, J = 2.4, 8.8 Hz), 7.65 (1H, d, J = 8.8 Hz), 7.83 (1H, br), 7.86 (1H, dd, J = 2.4, 8.8 Hz), 8.16 (1H, d, J = 2.4 Hz), 8.43 (1H, s), 8.49 (1H, d, J = 2.4 Hz), 8.94 (1H, d, J = 8.0 Hz), 9.59 (1H, brs) ESI+ : 530 [M+H]+ |

**[Table 68]**

| Ex | DATA |
|---|---|
| 5-56 | NMR-DMSO-d₆: 1.56-1.67 (1H, m), 1.80-1.90 (4H, m), 2.01-2.07 (1H, m), 2.36-2.41 (2H, m), 3.13-3.21 (1H, m), 3.41-3.51 (1H, m), 3.57-3.62 (2H, m), 3.77-3.91 (1H, m), 4.55-4.62 (1H, m), 4.84-4.93 (1H, m), 4.95-5.09 (1H, m), 7.05 (1H, d, J = 9.1 Hz), 7.11 (1H, br), 7.29 (1H, s), 7.57 (1H, dd, J = 2.4, 8.8 Hz), 7.65 (1H, d, J = 8.8 Hz), 7.82 (1H, br), 7.87 (1H, dd, J = 2.4, 9.1 Hz), 8.16 (1H, d, J = 2.4 Hz), 8.44 (1H, s), 8.49 (1H, d, J = 2.1 Hz), 8.94 (1H, d, J = 8.0 Hz), 9.60 (1H, brs) ESI+ : 530 [M+H]+ |
| 5-57 | NMR-DMSO-d₆: 1.54-1.65 (1H, m), 1.79-1.88 (4H, m), 1.96-2.03 (1H, m), 2.32-2.38 (2H, m), 3.08-3.17 (1H, m), 3.37-3.43 (1H, m), 3.53-3.58 (2H, m), 3.76-3.90 (1H, m), 4.53-4.60 (1H, m), 4.82-4.90 (1H, m), 4.91-5.06 (1H, m), 6.06 (1H, s), 7.05 (1H, d, J = 9.1 Hz), 7.07 (1H, br), 7.12 (2H, d, J = 8.8 Hz), 7.60 (2H, d, J = 8.8 Hz), .76 (1H, br), 7.87 (1H, dd, J = 2.4, 9.1 Hz), 8.40 (1H, s), 8.49 (1H, d, J = 2.0 Hz), 8.88 (1H, d, J = 8.3 Hz), 9.01 (1H, brs) ESI+ : 529 [M+H]+ |
| 5-58 | NMR-DMSO-d₆: 1.02-1.05 (2H, m), 1.16-1.21 (4H, m),1.54-1.64 (1H, m), 1.94-2.01 (1H, m), 3.03-3.15 (7H, m), 3.72-3.86 (1H, m), 4.52-4.59 (1H, m), 4.81-5.04 (2H, m), 5.99 (1H, s), 6.92 (2H, d, J = 9.2 Hz), 6.99 (1H, br), 7.05 (1H, d, J = 9.2 Hz), 7.48 (2H, d, J = 9.2 Hz), 7.73 (1H, br), 7.87 (1H, dd, J = 2.4, 9.2 Hz), 8.35 (1H, s), 8.49 (1H, d, J = 2.0 Hz), 8.86-8.90 (1H, m), 9.69 (1H, br) ESI+ : 530 [M+H]+ |
| 5-59 | NMR-DMSO-d₆: 1.56-1.78 (7H, m), 2.01-2.07 (1H, m), 2.58-2.62 (2H, m), 3.13-3.22 (1H, m), 3.36-3.51 (1H, m), 3.68-3.72 (2H, m), 3.78-3.91 (1H, m), 4.54-4.61 (1H, m), 4.83-4.93 (1H, m), 4.95-5.09 (1H, m), 7.05 (1H, d, J = 8.8 Hz), 7.11 (1H, br), 7.24 (1H, s), 7.50 (1H, dd, J = 2.4, 8.8 Hz), 7.67 (1H, d, J = 8.8 Hz), 7.81 (1H, br), 7.86 (1H, dd, J = 6.4, 8.8 Hz), 8.09 (1H, d, J = 2.4 Hz), 8.43 (1H, s), 8.49-8.50 (1H, m), 8.94 (1H, d, J = 8.1 Hz), 9.57 (1H, s) ESI+ : 544 [M+H]+ |
| 5-60 | NMR-DMSO-d₆: 0.88 (3H, d, J = 6.8 Hz), 1.73-1.90 (6H, m), 2.20-2.27 (1H, m), 2.37-2.41 (2H, m), 3.57-3.66 (4H, m), 3.70-3.77 (2H, m), 3.82-3.90 (1H, m), 7.01 (1H, d, J = 9.2 Hz), 7.08 (1H, br), 7.24 (1H, s), 7.56 (1H, dd, J = 2.4, 8.8 Hz), 7.65 (1H, d, J = 8.8 Hz), 7.83 (1H, dd, J = 2.4, 8.8 Hz), 7.86 (1H, br), 8.13-8.15 (1H, m), 8.43 (1H, s), 8.47-8.48 (1H, m), 9.06 (1H, d, J = 7.6 Hz), 9.59 (1H, brs) ESI+ : 526 [M+H]+ |
| 5-61 | NMR-DMSO-d₆: 0.88 (3H, d, J = 6.8 Hz), 1.71-1.88 (7H, m), 2.16-2.22 (1H, m), 2.33-2.39 (2H, m), 3.54-3.57 (2H, m), 3.58-3.84 (4H, m), 6.03 (1H, s), 7.00 (1H, d, J = 9.2 Hz), 7.07 (1H, br), 7.11 (2H, d, J = 8.8 Hz), 7.59 (2H, d, J = 8.8 Hz), 7.79 (1H, br), 7.83 (1H, dd, J = 2.4, 9.2 Hz), 8.40 (1H, s), 8.47-8.48 (1H, m), 8.98-9.02 (2H, m) ESI+ : 525 [M+H]+ |
| 5-62 | NMR-DMSO-d₆: 0.87 (3H, d, J = 6.8 Hz), 1.69-1.76 (2H, m), 2.13-2.20 (1H, m), 2.21 (3H, s), 2.42-2.46 (4H, m), 3.02-3.06 (4H, m), 3.58-3.68 (3H, m), 3.71-3.81 (2H, m), 5.91 (1H, s), 6.86 (2H, d, J = 9.2 Hz), 6.95 (1H, br), 6.99 (1H, d, J = 9.6 Hz), 7.39 (2H, d, J = 9.2 Hz), 7.68 (1H, br), 7.83 (1H, dd, J = 2.4, 9.2 Hz), 8.34 (1H, s), 8.46-8.48 (1H, m), 8.65 (1H, s), 8.99 (1H, d, J = 8.0 Hz) ESI+ : 526 [M+H]+ |

**[Table 69]**

| Ex | DATA |
|---|---|
| 5-63 | NMR-DMSO-d₆: 0.87 (3H, d, J = 6.9 Hz), 1.69-1.75 (2H, m), 2.13-2.19 (1H, m), 2.99-3.04 (4H, m), 3.61-3.68 (3H, m), 3.71-3.79 (6H, m), 5.92 (1H, s), 6.87 (2H, d, J = 9.1 Hz), 6.95 (1H, br), 6.99 (1H, d, J = 9.2 Hz), 7.42 (2H, s, J = 9.1 Hz), 7.74 (1H, br), 7.83 (1H, dd, J = 2.4, 9.1 Hz), 8.35 (1H, s), 8.47 (1H, d, J = 2.4 Hz), 8.67 (1H, s), 8.99 (1H, d, J = 7.8 Hz) ESI+ : 513 [M+H]+ |
| 5-64 | NMR-DMSO-d₆ 1.53-1.64 (1H, m), 1.81-1.92 (4H, m), 2.37-2.40 (2H, m), 3.12-3.27 (2H, m), 3.30 (3H, s), 3.57-3.62 (4H, m), 3.71-3.77 (1H, m), 4.39-4.45 (1H, m), 4.73-4.80 (1H, m), 7.02 (1H, d, J = 8.8 Hz), 7.06 (1H, br), 7.25 (1H, s), 7.56 (1H, dd, J = 2.8, 9.2 Hz), 7.66 (1H, d, J = 8.8 Hz), 7.77 (1H, br), 7.82 (1H, dd, J = 2.8, 9.2 Hz), 8.15 (1H, d, J = 2.4 Hz), 8.40 (1H, s), 8.47 (1H, d, J = 2.4 Hz), 8.91 (1H, d, J = 7.6 Hz), 9.55 (1H, s) ESI+ : 542 [M+H]+ |
| 5-65 | NMR-DMSO-d₆: 1.36-1.50 (2H, m), 2.02-2.18 (8H, m), 3.26-3.38 (4H, m), 3.58-3.70 (1H, m), 4.24-4.36 (2H, m), 6.94-7.18 (2H, m), 7.28 (1H, s), 7.55 (1H, dd, J = 2.3, 8.6 Hz), 7.58-7.90 (3H, m), 8.10 (1H, d, J = 1.9 Hz), 8.41 (1H, s), 8.48-8.52 (1H, m), 8.75 (1H, d, J = 7.0 Hz), 9.50 (1H, s) ESI+ : 472 [M+H]+ |
| 5-66 | NMR-DMSO-d₆: 1.42-1.55 (2H, m), 2.02-2.16 (8H, m), 3.12-3.22 (2H, m), 3.30 (2H, s), 3.53-3.64 (1H, m), 3.81-3.90 (2H, m), 6.95-7.16 (3H, m), 7.28 (1H, s), 7.52-7.63 (4H, m), 7.79 (1H, br), 8.10 (1H, d, J = 1.8 Hz), 8.41 (1H, s), 8.75 (1H, d, J = 7.0 Hz), 9.50 (1H, s) ESI+ : 471 [M+H]+ |
| 5-67 | NMR-DMSO-d₆: 1.38-1.48 (2H, m), 1.51-1.57 (2H, m),1.67-1.74 (1H, m), 1.86-1.98 (3H, m), 2.06-2.13 (2H, m), 2.78-2.81 (1H, m), 3.29-3.37 (2H, m), 3.58-3.67 (1H, m), 4.25-4.32 (2H, m), 4.55-4.58 (1H, m), 7.00 (1H, d, J = 9.2 Hz), 7.05 (1H, br), 7.10 (1H, s), 7.73 (1H, d, J = 9.2 Hz), 7.78 (1H, br), 7.81-7.87 (2H, m), 8.39-8.40 (2H, m), 8.49 (1H, d, J = 2.0 Hz), 8.75 (1H, d, J = 7.2 Hz), 9.48 (1H, s) ESI+ : 524 [M+H]+ |
| 5-68 | NMR-DMSO-d₆: 1.38-1.48 (2H, m), 1.51-1.57 (2H, m), 1.67-1.74 (1H, m),1.86-1.98 (3H, m), 2.05-2.13 (2H, m), 2.79-2.81 (1H, m), 3.30-3.36 (1H, m), 3.59-3.67 (2H, m), 4.25-4.31 (2H, m), 4.55-4.58 (1H, m), 7.00 (1H, d, J = 8.8 Hz), 7.05 (1H, br), 7.10 (1H, s), 7.73 (1H, d, J = 8.8 Hz), 7.79 (1H, br), 7.81-7.87 (2H, m), 8.40-8.41 (2H, m), 8.49-8.41 (1H, m), 8.75 (1H, d, J = 6.8 Hz), 9.48 (1H, brs) ESI+ : 524 (M+H)+ |
| 5-69 | NMR-DMSO-d₆: 1.74-1.84 (2H, m), 2.11-2.19 (1H, m), 3.34-3.46 (3H, m), 3.56-3.63 (1H, m), 3.84-3.93 (2H, m), 4.02-4.08 (1H, m), 4.65-4.68 (1H, m), 6.96 (1H, d, J = 9.2 Hz), 7.13 (1H, s), 7.17 (1H, br), 7.85 (1H, dd, J = 2.4, 9.1 Hz), 7.90 (1H, br), 8.07 (1H, d, J = 5.5 Hz), 8.22-8.24 (1H, m), 8.48 (1H, s), 8.49 (1H, d, J = 2.4 Hz), 9.02 (1H, d, J = 1.4 Hz), 9.23 (1H, d, J = 7.7 Hz), 9.86 (1H, brs) ESI+ : 446 [M+H]+ |
| 5-70 | NMR-CDCl₃ : 8.86 (1H, d, J = 7.2 Hz), 8.39 (1H, d, J = 2.0 Hz), 8.33 (1H, s), 7.59 (1H, dd, J = 8.8, 2.4 Hz), 7.34-7.21 (4H, m), 6.50 (1H, d, J = 8.4 Hz), 5.89 (1H, s), 5.78 (2H, bs), 3.88-3.39 (8H, m), 2.60-2.57 (2H, m), 2.21-2.14 (1H, m), 2.07-1.50 (9H, m) ESI+ : 525 [M+H]+ |

**[Table 70]**

| **Ex** | **DATA** |
|---|---|
| 6 | NMR-DMSO-d₆: 1.38-1.47 (2H, m), 2.02-2.09 (2H, m), 3.13-3.20 (2H, m), 3.53-3.60 (1H, m), 3.98-4.05 (2H, m), 7.12 (1H, s), 7.14 (1H, br), 7.67 (4H, m), 7.85 (1H, br), 8.07 (1H, d, J = 2.6 Hz), 8.23-8.24 (1H, m), 8.46 (1H, s), 8.82 (1H, d, J = 7.1 Hz), 9.03-9.04 (2H, m), 9.85 (1H, s) ESI+ : 458 [M+H]+ |
| 8 | NMR-DMSO-d₆: 1.40-1.45 (2H, m), 1.64-1.81 (8H, m), 2.02-2.04 (1H, m), 2.12-2.14 (2H, m), 2.99 (6H, s), 3.51-3.54 (1H, m), 4.51 (1H, s), 7.08 (1H, br), 7.13 (1H, s), 7.64 (1H, s), 7.83 (1H, br), 8.27 (1H, t, J = 1.6 Hz), 8.42 (1H, s), 9.13 (1H, d, J = 7.3 Hz), 9.75 (1H, s), 12.04 (1H, br) ESI+ : 451 [M+H]+ |
| 8-1 | NMR-DMSO-d₆: 2.77 (3H, d, J = 4.5 Hz), 4.45 (2H, d, J = 6.1 Hz), 6.86-7.32 (4H, m), 7.62-7.68 (2H, m), 7.87 (1H, br), 8.01 (1H, dd, J = 2.4, 8.8 Hz), 8.30-8.36 (1H, m), 8.44 (1H, s), 8.66 (1H, d, J = 2.2 Hz), 9.14 (1H, t, J = 6.1 Hz), 9.86 (1H, s) ESI+ : 413 [M+H]+ |
| 16 | NMR-DMSO-d₆: 1.27-1.37 (1H, m), 1.45-1.54 (1H, m), 1.98-2.05 (2H, m), 2.96-3.05 (1H, m), 3.19-3.27 (1H, m), 3.54-3.68 (2H, m), 4.06-4.17 (3H, m), 7.09 (1H, s), 7.15 (1H, br), 7.87 (1H, br), 8.07 (1H, d, J = 2.7 Hz), 8.22-8.24 (1H, m), 8.46 (1H, s), 8.80 (1H, d, J = 7.2 Hz), 9.03 (1H, d, J = 1.4 Hz), 9.84 (1H, s) ESI+ : 381 [M+H]+ |
| 8-3 | NMR-DMSO-d₆ : 9.25 (1H, s), 9.06 (1H, t, J = 6.0 Hz), 8.40 (1H, s), 7.82 (1H, bs), 7.63 (2H, d, J = 8.8 Hz), 7.51 (1H, ddd, J = 19.2, 9.6, 5.2 Hz), 7.30 (2H, d, J = 8.8 Hz), 7.23-7.17 (1H, m), 7.12 (1H, bs), 6.10 (1H, s), 4.47 (2H, d, J = 6.0 Hz), 3.60-3.40 (4H, m), 2.38-2.25 (4H, m), 2.19 (3H, s). ESI+ : 499 [M+H]+ |
| 8-4 | NMR-CDCl₃ : 9.08 (1H, t, J = 6.0 Hz), 8.30 (1H, s), 7.57 (2H, d, J = 8.4 Hz), 7.30 (2H, d, J = 8.4 Hz), 7.14-7.05 (2H, m), 6.89-6.83 (1H, m), 6.18 (1H, s), 5.70 (2H, bs), 4.44 (2H, d, J = 6.0 Hz), 3.66 (2H, t, J = 6.4 Hz), 3.52 (2H, t, J = 6.4 Hz), 2.00-1.89 (4H, m). ESI+ : 470 [M+H]+ |
| 8-5 | NMR-CDCl₃: 9.46 (1H, bs), 8.52 (1H, s), 7.46 (2H, d, J = 8.4 Hz), 7.30 (2H, d, J = 8.4 Hz), 7.16-7.08 (2H, m), 6.89-6.85 (2H, m), 6.12 (1H, s), 4.44 (2H, d, J = 6.4 Hz), 3.52-3.49 (4H, m), 3.38 (3H, s), 3.38-3.37 (1H, m), 2.02-1.81 (4H, m). ESI+ : 514 [M+H]+ |
| 8-6 | NMR-CDCl₃ : 9.10 (1H, t, J = 5.2 Hz), 8.55-8.48 (1H, m), 8.29 (1H, bs), 7.88 (1H, dd, J = 8.8, 2.0 Hz), 7.55-7.48 (1H, m), 7.32-7.25 (4H, m), 7.03-7.88 (3H, m), 4.45 (2H, d, J = 5.2 Hz), 3.67-3.64 (2H, m), 3.53-3.50 (2H, m), 2.01-1.91 (4H, m). ESI+ : 453 [M+H]+ |
| 8-7 | NMR-CDCl₃: 8.95 (1H, t, J = 6.0 Hz), 8.37 (1H, s), 8.29 (1H, s), 7.69 (1H, dd, J = 8.4, 2.4 Hz), 7.44 (1H, d, J = 8.4 Hz), 7.29-7.21 (2H, m), 7.13 (1H, bs), 6.94-6.87 (2H, m), 5.76 (2H, bs), 4.52 (2H, d, J = 6.0 Hz), 4.10-3.70 (2H, m), 3.58-3.25 (6H, m), 2.0-1.81 (2H, m), 1.78-1.50 (2H, m). ESI+ : 497 [M+H]+ |
| 8-8 | NMR-CDCl₃ : 8.95 (1H, t, J = 6.0 Hz), 8.38 (1H, s), 8.29 (1H, s), 7.70 (1H, dd, J = 8.8, 2.4 Hz), 7.44 (1H, d, J = 8.8 Hz), 7.31-7.21 (2H, m), 7.14 (1H, bs), 6.94-6.87 (2H, m), 5.76 (2H, bs), 4.52 (2H, d, J = 6.0 Hz), 3.90-3.45 (4H, m), 2.54-2.38 (4H, m), 2.34 (3H, s). ESI+ : 482 [M+H]+ |

**[Table 71]**

| Ex | DATA |
|---|---|
| 8-9 | NMR-DMSO-d₆: 9.85 (1H, s), 9.14 (1H, t, J = 6.0 Hz), 8.42 (1H, s), 8.30 (1H, d, J = 2.0 Hz), 7.84 (1H, bs), 7.69 (1H, d, J = 8.4 Hz), 7.48-7.37 (3H, m), 7.15-7.09 (2H, m), 4.49 (2H, d, J = 6.0 Hz), 3.72-3.44 (4H, m), 2.66-2.55 (4H, m), 2.28 (3H, bs), 1.91-1.73 (2H, m). ESI+ : 496 [M+H]+ |
| 8-10 | NMR-CDCl₃: 8.92 (1H, bs), 8.54 (1H, bs), 8.25 (1H, s), 7.82 (1H, dd, J = 8.8, 2.4 Hz), 7.54 (1H, bs), 7.45-7.42 (1H, m), 7.28-7.21 (1H, m), 7.15 (1H, s), 6.94-6.87 (2H, m), 5.64 (2H, bs), 4.54 (2H, d, J = 6.0 Hz), 3.96-3.57 (3H, m), 3.48-3.42 (1H, m), 2.83-2.65 (1H, m), 2.32 (3H, s), 2.25 (3H, s), 2.22-2.07 (1H, m), 1.92-1.82 (1H, m). ESI+ : 496 [M+H]+ |
| 8-11 | NMR-DMSO-d₆: 9.79 (1H, s), 9.11 (1H, t, J = 6.0 Hz), 8.44 (1H, s), 8.34 (1H, dd, J = 2.4, 0.4 Hz), 7.87 (1H, bs), 7.81 (1H, dd, J = 8.8, 2.4 Hz), 7.61-7.56 (2H, m), 7.42-7.33 (3H, m), 7.17 (1H, bs), 7.12 (1H, s), 4.61 (1H, bs), 4.49 (1H, bs), 3.51-3.44 (4H, m), 3.22 (3H, s), 3.10 (3H, s), 1.92-1.78 (4H, m). ESI+ : 524 [M+H]+ |
| 8-12 | ESI+ : 420 [M+H]+ |
| 8-13 | ESI+ : 406 [M+H]+ |
| 8-14 | ESI+ : 460 [M+H]+ |
| 8-15 | ESI+ : 473 [M+H]+ |
| 8-16 | ESI+ : 390 [M+H]+ |
| 8-17 | ESI+ : 430 [M+H]+ |
| 8-18 | ESI+ : 446 [M+H]+ |
| 8-19 | ESI+ : 453 [M+H]+ |
| 8-20 | ESI+ : 453 [M+H]+ |
| 8-21 | ESI+ : 362 [M+H]+ |
| 8-22 | ESI+ : 376 [M+H]+ |
| 8-23 | ESI+ : 460 [M+H]+ |
| 8-24 | ESI+ : 523 [M+H]+ |
| 8-25 | ESI+ : 501 [M+H]+ |
| 8-26 | ESI+ : 460 [M+H]+ |
| 8-27 | ESI+ : 474 [M+H]+ |
| 8-28 | ESI+ : 509 [M+H]+ |
| 8-29 | ESI+ : 527 [M+H]+ |
| 8-30 | ESI+ : 513 [M+H]+ |
| 8-31 | ESI+ : 513 [M+H]+ |
| 8-32 | ESI+ : 575 [M+H]+ |
| 8-33 | ESI+ : 575 [M+H]+ |
| 8-34 | ESI+ : 523 [M+H]+ |
| 8-35 | ESI+ : 560 [M+H]+ |
| 8-36 | ESI+ : 555 [M+H]+ |
| 8-37 | ESI+ : 502 [M+H]+ |
| 8-38 | ESI+ : 547 [M+H]+ |
| 8-39 | ESI+ : 527 [M+H]+ |

**[Table 72]**

| Ex | DATA |
|---|---|
| 9 | NMR-DMSO-d₆ 1.38-1.45 (2H, m), 1.62-1.79 (8H, m), 2.00-2.12 (3H, m), 3.43-3.48 (1H, m), 4.51 (1H, s), 5.92 (1H, s), 6.84-6.89 (1H, m), 6.92-7.17 (1H, m), 7.20-7.24 (2H, m), 7.59-7.61 (2H, m), 7.65-7.85 (1H, m), 8.37 (1H, s), 8.88 (1H, s), 9.06 (1H, d, J = 7.6 Hz) ESI+ : 379 [M+H]+ |
| 9-1 | NMR-DMSO-d₆ : 1.45-1.51 (2H, m), 1.72-1.91 (8H, m), 2.09-2.14 (1H, m), 2.18-2.22 (2H, m), 2.69 (2H, t, J = 5.9 Hz), 3.54-3.58 (1H, m), 3.60 (2H, t, J = 5.9 Hz), 7.04 (1H, s), 7.13 (1H, br), 7.86 (1H, br), 8.06 (1H, d, J = 2.7 Hz), 8.21-8.22 (1H, m), 8.46 (1H, s), 9.02 (1H, d, J = 1.5 Hz), 9.21 (1H, d, J = 7.5 Hz), 9.81 (1H, s) ESI+ : 434 [M+H]+ |
| 10 | NMR-DMSO-d₆ : 1.37-1.43 (2H, m),1.62-1.75 (8H, m), 2.00-2.07 (3H, m), 3.38-3.41 (1H, m), 4.49 (1H, s), 5.76 (1H, s), 6.66 (2H, d, J = 8.8 Hz), 6.91 (1H, br), 7.28 (2H, d, J = 8.8 Hz), 7.67 (1H, br), 8.30 (1H, s), 8.50 (1H, s), 8.99 (1H, brs), 9.03 (1H, d, J = 7.9 Hz) ESI+ : 395 [M+H]+ |
| 17 | NMR-DMSO-d₆ : 1.37-1.49 (6H, m), 1.90-1.98 (2H, m), 2.06-2.17 (2H, m), 2.25-2.33 (2H, m), 2.71-2.78 (2H, m), 3.22-3.29 (1H, m), 3.37-3.41 (2H, m), 4.57 (1H, br), 5.96 (1H, s), 6.84-6.89 (1H, m), 7.01 (1H, br), 7.21-7.25 (2H, m), 7.59-7.63 (2H, m), 7.74 (1H, br), 8.35 (1H, s), 8.61-8.64 (1H, m), 8.90 (1H, s) ESI+ : 384 [M+H]+ |
| 10-2 | NMR-DMSO-d₆ 1.36-1.42 (2H, m), 1.62-1.75 (8H, m), 2.00-2.05 (3H, m), 3.39-3.43 (1H, m), 4.48 (1H, s), 5.53 (1H, s), 5.86 (2H, s), 6.78 (1H, br), 7.55 (1H, br), 8.17 (1H, s), 9.02 (1H, d, J = 7.8 Hz) ESI+ : 303 [M+H]+ |
| 10-3 | NMR-DMSO-d₆ : 1.54-1.63 (2H, m), 2.06-2.12 (2H, m), 2.78-2.85 (2H, m), 3.28-3.38 (2H, m), 3.40-3.47 (1H, m), 6.65 (2H, d, J = 8.9 Hz), 6.83 (2H, d, J = 8.9 Hz), 7.08 (1H, br), 7.12 (1H, s), 7.84 (1H, br), 8.06 (1H, d, J = 2.7 Hz), 8.22-8.24 (1H, m), 8.45 (1H, s), 8.80-8.83 (2H, m), 9.02 (1H, d, J = 1.4 Hz), 9.85 (1H, brs) ESI+ : 406 [M+H]+ |
| 11 | NMR-DMSO-d₆ : 3.02 (2H, t, J = 6 Hz), 3.34-3.41 (2H, m), 4.25-4.41 (2H, m), 4.57 (2H, d, J = 5.6 Hz), 6.04 (1H, s), 7.13-7.31 (4H, m), 7.48-7.58 (1H, m), 7.66 (1H, brs), 8.25 (1H, brs), 8.34 (1H, s), 9.43-9.52 (2H, m), 9.84-10.1 (2H, m), 13.0 (1H, brs) ESI+ : 428 [M+H]+ |
| 11-1 | NMR-DMSO-d₆ : 1.71-1.81 (2H, m), 2.14-2.21 (2H, m), 3.02-3.12 (2H, m), 3.29-3.35 (2H, m), 3.73-3.79 (1H, m), 6.89 (1H, s), 7.83 (1H, m), 8.35-8.47 (4H, m), 8.59 (1H, s), 8.93-9.07 (2H, m), 9.45 (1H, d, J = 6.8 Hz), 12.09 (1H, br) ESI+ : 314 [M+H]+ |
| 11-2 | NMR-DMSO-d₆ : 1.70-1.81 (2H, m), 2.14-2.21 (2H, m), 3.00-3.10 (2H, m), 3.29-3.35 (2H, m), 3.68-3.76 (1H, m), 6.71 (1H, s), 7.17-7.23 (2H, m), 7.80 (1H, br), 7.90-7.95 (1H, m), 8.35-8.38 (1H, m), 8.40 (1H, br), 8.47 (1H, s), 8.96-9.10 (2H, m), 9.63 (1H, d, J = 7.2 Hz), 11.95 (1H, s), 14.50 (1H, br) ESI+ : 313 [M+H]+ |
| 12 | NMR-DMSO-d₆ 1.84-1.90 (4H, m), 2.40 (2H, t, J = 6.3 Hz), 3.59-3.62 (2H, m), 3.77 (3H, s), 4.35 (2H, d, J = 5.7 Hz), 6.78 (2H, d, J = 9.8 Hz), 7.06 (1H, br s), 7.36 (1H, s), 7.49 (1H, d, J = 8.9 Hz), 7.56 (1H, dd, J = 8.9, 2.6 Hz), 7.79 (1H, br s), 8.17 (1H, d, J = 2.6 Hz), 8.40 (1H, s), 8.99 (1H, t, J = 5.7 Hz), 9.63 (1H, s) ESI+: 483 [M+H]+ |

**[Table 73]**

| **Ex** | **DATA** |
|---|---|
| 12-1 | NMR-DMSO-d₆ : 1.28-1.39 (2H, m), 1.41 (9H, s), 1.95-2.01 (2H, m), 2.99-3.08 (2H, m), 3.45-3.52 (1H, m), 3.80-3.86 (2H, m), 7.11 (1H, s), 7.14 (1H, br), 7.85 (1H, br), 8.07 (1H, d, J = 2.7 Hz), 8.23-8.24 (1H, m), 8.45 (1H, s), 8.77 (1H, d, J = 7.2 Hz), 9.06 (1H, d, J = 4.1 Hz), 9.84 (1H, s) ESI+ : 414 [M+H]+ |
| 12-2 | NMR-DMSO-d₆: 1.28-1.38 (2H, m), 1.41 (9H, s), 1.94-2.01 (2H, m), 2.98-3.11 (2H, m), 3.44-3.52 (1H, m), 3.80-3.88 (2H, m), 7.12 (1H, s), 7.19 (1H, br), 7.74 (1H, d, J = 5.2 Hz), 7.89 (1H, br), 8.39 (1H, d, J = 6.0 Hz), 8.45 (1H, s), 8.70 (1H, s), 8.76 (1H, d, J = 7.2 Hz), 9.98 (1H, s) ESI+ : 414 [M+H]+ |
| 12-3 | NMR-DMSO-d₆ 1.39-1.43 (2H, m), 1.62-1.77 (8H, m), 2.00-2.08 (3H, m), 2.99-3.03 (4H, m), 3.40-3.44 (1H, m), 3.71-3.75 (4H, m), 4.50 (1H, s), 5.81 (1H, s), 6.86 (2H, d, J = 9.1 Hz), 6.94 (1H, br), 7.42 (2H, d, J = 9.1 Hz), 7.69 (1H, br), 8.32 (1H, s), 8.62 (1H, s), 9.04 (1H, d, J = 7.6 Hz) ESI+ : 464 [M+H]+ |
| 12-4 | NMR-DMSO-d₆ : 1.38-1.44 (2H, m), 1.62-1.76 (8H, m), 2.00-2.09 (3H, m), 3.41-3.44 (1H, m), 3.71 (3H, s), 4.50 (1H, s), 5.80 (1H, s), 6.84 (2H, d, J = 9.1 Hz), 6.94 (1H, br), 7.46 (2H, d, J = 9.1 Hz), 7.68 (1H, br), 8.32 (1H, s), 8.66 (1H, s), 9.04 (1H, d, J = 7.6 Hz) ESI+ : 409 [M+H]+ |
| 12-5 | NMR-DMSO-d₆: 1.37-1.43 (2H, m), 1.62-1.76 (8H, m), 2.00-2.08 (3H, m), 2.21 (3H, s), 2.43-2.46 (4H, m), 3.02-3.06 (4H, m), 3.40-3.44 (1H, m), 4.50 (1H, s), 5.80 (1H, s), 6.85 (2H, d, J = 9.1 Hz), 6.90 (1H, br), 7.39 (2H, d, J = 9.1 Hz), 7.69 (1H, br), 8.32 (1H, s), 8.60 (1H, s), 9.03 (1H, d, J = 7.5 Hz) ESI+ : 477 [M+H]+ |
| 12-6 | NMR-DMSO-d₆ : 1.39-1.45 (2H, m), 1.63-1.77 (8H, m), 2.01-2.10 (3H, m), 3.43-3.47 (1H, m), 3.72 (3H, s), 4.52 (1H, s), 5.93 (1H, s), 6.44-6.46 (1H, m), 7.01 (1H, br), 7.10-7.13 (2H, m), 7.35-7.37 (1H, m), 7.77 (1H, br), 8.38 (1H, s), 8.88 (1H, brs), 9.07 (1H, d, J = 7.6 Hz) ESI+ : 409 [M+H]+ |
| 12-7 | NMR-DMSO-d₆: 1.38-1.44 (2H, m), 1.63-1.76 (8H, m), 2.01-2.09 (3H, m), 3.41-3.44 (1H, m), 4.51 (1H, s), 5.04 (2H, s), 5.81 (1H, s), 6.92 (2H, d, J = 9.0 Hz), 7.32-7.48 (8H, m), 7.70 (1H, br), 8.33 (1H, s), 8.69 (1H, s), 9.04 (1H, d, J = 7.5 Hz) ESI+ : 485 [M+H]+ |
| 12-8 | NMR-DMSO-d₆ : 1.38-1.44 (2H, m), 1.62-1.76 (8H, m), 2.01-2.09 (3H, m), 3.31-3.39 (4H, m), 3.42-3.45 (1H, m), 3.69-3.73 (4H, m), 4.50 (1H, s), 5.78 (1H, s), 6.79 (1H, d, J = 9.1 Hz), 6.96 (1H, br), 7.72 (1H, br), 7.84 (1H, dd, J = 2.6, 9.1 Hz), 8.28 (1H, d, J = 2.6 Hz), 8.31 (1H, s), 8.66 (1H, s), 9.06 (1H, d, J = 7.5 Hz) ESI+ : 465 [M+H]+ |
| 12-9 | NMR-DMSO-d₆: 1.39-1.45 (2H, m), 1.63-1.79 (8H, m), 2.02-2.11 (3H, m), 3.46-3.50 (1H, m), 4.52 (1H, s), 5.98 (1H, s), 7.08 (2H, br), 7.68 (2H, d, J = 8.8 Hz), 7.73 (2H, br), 7.77 (2H, d, J = 8.8 Hz), 8.42 (1H, s), 9.09 (1H, d, J = 7.6 Hz), 9.18 (1H, s) ESI+ : 422 [M+H]+ |

**[Table 74]**

| **Ex** | **DATA** |
|---|---|
| 12-10 | NMR-DMSO-d₆: 1.39-1.45 (2H, m), 1.63-1.77 (8H, m), 2.05-2.11 (3H, m), 3.27 (2H, s), 3.43-3.47 (1H, m), 4.51 (1H, s), 5.90 (1H, s), 6.82 (1H, brs), 6.99 (1H, br), 7.11 (2H, d, J = 8.5 Hz), 7.37 (1H, brs), 7.51 (2H, d, J = 8.5 Hz), 7.73 (1H, br), 8.36 (1H, brs), 8.83 (1H, brs), 9.06 (1H, d, J = 7.6 Hz) ESI+ : 436 [M+H]+ |
| 12-11 | NMR-DMSO-d₆: 1.41-1.49 (2H, m), 1.91-1.98 (2H, m), 2.13-2.20 (2H, m), 2.68-2.75 (2H, m), 3.50 (2H, s), 3.57-3.63 (1H, m), 6.00 (1H, s), 6.84-6.88 (1H, m), 7.02 (1H, br), 7.21-7.27 (3H, m), 7.30-7.35 (4H, m), 7.58-7.61 (2H, m), 7.74 (1H, br), 8.36 (1H, s), 8.63 (1H, d, J = 7.0 Hz), 8.88 (1H, brs) ESI+ : 402 [M+H]+ |
| 12-12 | NMR-DMSO-d₆: 1.39-1.48 (2H, m), 1.89-1.95 (2H, m), 2.12-2.19 (2H, m), 2.67-2.73 (2H, m), 2.99-3.02 (4H, m), 3.20-3.25 (1H, m), 3.49 (2H, s), 3.72-3.75 (4H, m), 5.84 (1H, s), 6.86 (2H, d, J = 9.1 Hz), 6.95 (1H, br), 7.23-7.27 (1H, m), 7.29-7.34 (4H, m), 7.42 (2H, d), 7.68 (1H, br), 8.31 (1H, s), 8.60-8.63 (2H, m) ESI+ : 487 [M+H]+ |
| 12-13 | NMR-DMSO-d₆: 0.87 (3H, d, J = 7.0 Hz), 1.30-1.61 (7H, m), 1.67-1.73 (1H, m), 1.87-1.94 (1H, m), 3.44-3.49 (1H, m), 5.96 (1H, s), 6.84-6.88 (1H, m), 6.99 (1H, br), 7.21-7.25 (2H, m), 7.57-7.60 (2H, m), 7.74 (1H, br), 8.39 (1H, s), 8.86 (1H, s), 8.88 (1H, d, J = 8.2 Hz) ESI+ : 325 [M+H]+ |
| 12-14 | NMR-DMSO-d₆: 0.86 (3H, d, J = 6.9 Hz), 1.29-1.61 (7H, m), 1.66-1.72 (1H, m), 1.85-1.92 (1H, m), 3.00-3.02 (4H, m), 3.42-3.46 (1H, m), 3.71-3.74 (4H, m), 5.84 (1H, s), 6.86 (2H, d, J = 9.1 Hz), 6.92 (1H, br), 7.40 (2H, d, J = 9.1 Hz), 7.67 (1H, br), 8.31 (1H, s), 8.59 (1H, s), 8.87 (1H, d, J = 8.2 Hz) ESI+ : 410 [M+H]+ |
| 12-15 | NMR-DMSO-d₆: 1.40-1.45 (2H, m), 1.63-1.78 (8H, m), 2.02-2.11 (3H, m), 3.46-3.62 (9H, m), 4.53 (1H, s), 5.96 (1H, s), 7.06 (1H, br), 7.32 (2H, d, J = 8.5 Hz), 7.70 (2H, d, J = 8.5 Hz), 7.79 (1H, br), 8.39 (1H, s), 9.09 (1H, d, J = 7.7 Hz), 9.15 (1H, s) ESI+ : 492 [M+H]+ |
| 12-16 | NMR-DMSO-d₆ : 1.40-1.45 (2H, m), 1.64-1.79 (8H, m), 2.02-2.11 (3H, m), 3.47-3.50 (1H, m), 4.53 (1H, s), 6.00 (1H, s), 7.12 (3H, brs), 7.67 (2H, d, J = 8.9 Hz), 7.79 (2H, d, J = 8.9 Hz), 7.83 (1H, br), 8.42 (1H, s), 9.12 (1H, d, J = 7.6 Hz), 9.32 (1H, s) ESI+ : 458 [M+H]+ |
| 12-17 | NMR-DMSO-d₆: 1.40-1.45 (2H, m),1.63-1.78 (8H, m), 2.02-2.11 (3H, m), 3.47-3.50 (1H, m), 4.53 (1H, s), 6.01 (1H, s), 6.83 (1H, br), 7.50 (1H, br), 7.65 (2H, d, J = 8.8 Hz), 7.84 (2H, d, J = 8.8 Hz), 8.42 (1H, s), 9.14 (1H, d, J = 7.6 Hz), 9.48 (1H, s) ESI+ : 404 [M+H]+ |
| 12-18 | NMR-DMSO-d₆: 1.43-1.48 (2H, m), 1.59-1.77 (8H, m), 2.05-2.08 (1H, m), 2.14-2.17 (2H, m), 3.57-3.63 (1H, m), 4.51 (1H, s), 5.93 (1H, s), 6.84-6.88 (1H, m), 7.00 (1H, br), 7.20-7.25 (2H, m), 7.60 (2H, d, J = 7.6 Hz), 7.76 (1H, br), 8.37 (1H, s), 8.88 (1H, s), 9.06 (1H, d, J = 7.3 Hz) ESI+ : 379 [M+H]+ |

**[Table 75]**

| Ex | DATA |
|---|---|
| 12-19 | NMR-DMSO-d₆ 1.42-1.47 (2H, m), 1.58-1.77 (8H, m), 2.04-2.07 (1H, m), 2.12-2.15 (2H, m), 3.00-3.02 (4H, m), 3.29-3.31 (1H, m), 3.72-3.74 (4H, m), 4.49 (1H, s), 5.82 (1H, s), 6.86 (2H, d, J = 9.0 Hz), 6.93 (1H, br), 7.42 (2H, d, J = 9.0 Hz), 7.68 (1H, br), 8.32 (1H, s), 8.63 (1H, s), 9.03 (1H, d, J = 7.3 Hz) ESI+ : 464 [M+H]+ |
| 12-20 | NMR-DMSO-d₆: 2.99-3.02 (4H, m), 3.71-3.75 (4H, m), 4.39 (2H, d, J = 6.0 Hz), 5.65 (1H, s), 6.80 (2H, d, J = 9.0 Hz), 6.97-7.00 (2H, m), 7.07 (1H, br), 7.11-7.17 (1H, m), 7.21 (2H, d, J = 9.0 Hz), 7.74 (1H, br), 8.34 (1H, s), 8.60 (1H, s), 9.02-9.06 (1H, m) ESI+ : 440 [M+H]+ |
| 12-21 | NMR-DMSO-d₆: 1.39-1.45 (2H, m), 1.63-1.81 (8H, m), 2.02-2.05 (1H, m), 2.11-2.15 (2H, m), 3.49-3.53 (1H, m), 4.51 (1H, s), 6.85 (1H, ddd, J = 1.3, 5.0,11.7 Hz), 7.05 (1H, br), 7.13 (1H, s), 7.59-7.68 (2H, m), 7.80 (1H, br), 8.17-8.19 (1H, m), 8.40 (1H, s), 9.11 (1H, d, J = 7.5 Hz), 9.45 (1H, s) ESI+ : 380 [M+H]+ |
| 12-22 | NMR-DMSO-d₆: 2.03-2.18 (2H, m), 3.00-3.03 (4H, m), 3.71-3.75 (4H, m), 4.15-4.21 (1H, m), 4.34-4.39 (1H, m), 4.67-4.72 (1H, m), 6.05 (1H, s), 6.85-6.92 (4H, m), 7.06-7.18 (2H, m), 7.42 (2H, d, J = 9.1 Hz), 7.74 (1H, br), 8.38 (1H, s), 8.76 (1H, s), 8.96 (1H, d, J = 7.3 Hz) ESI+: 464 [M+H]+ |
| 12-23 | NMR-DMSO-d₆ : 1.40-1.45 (2H, m), 1.63-1.79 (8H, m), 2.02-2.11 (3H, m), 3.46-3.50 (1H, m), 4.52 (1H, s), 5.94 (1H, s), 7.07 (1H, br), 7.25 (1H, dd, J = 4.6, 8.3 Hz), 7.81 (1H, br), 8.07 (1H, dd, J =1.4, 4.6 Hz), 8.12-8.16 (1H, m), 8.39 (1H, s), 8.74 (1H, d, J = 2.5 Hz), 9.08 (1H, s), 9.11 (1H, d, J = 7.5 Hz) ESI+ : 380 [M+H]+ |
| 12-24 | NMR-DMSO-d₆ 1.39-1.46 (2H, m), 1.64-1.79 (8H, m), 2.02-2.11 (3H, m), 3.46-3.51 (1H, m), 4.53 (1H, s), 6.02 (1H, s), 7.14 (1H, br), 7.61 (2H, d, J = 6.3 Hz), 7.87 (1H, br), 8.26 (2H, d, J = 6.3 Hz), 8.44 (1H, s), 9.13 (1H, d, J = 7.5 Hz), 9.36 (1H, s) ESI+ : 380 [M+H]+ |
| 12-25 | NMR-DMSO-d₆: 1.40-1.46 (2H, m), 1.64-1.83 (8H, m), 2.02-2.06 (1H, m), 2.14-2.18 (2H, m), 3.56-3.60 (1H, m), 4.51 (1H, s), 6.95-6.97 (1H, m), 7.10 (1H, br), 7.68 (1H, s), 7.87 (1H, br), 8.42 (1H, s), 8.53 (2H, d, J = 4.8 Hz), 9.23 (1H, d, J = 7.4 Hz), 9.54 (1H, s) ESI+ : 381 [M+H]+ |
| 12-26 | NMR-DMSO-d₆ 1.38-1.45 (2H, m), 1.62-1.78 (8H, m), 2.00-2.11 (3H, m), 3.43-3.48 (1H, m), 4.51 (1H, s), 5.86 (1H, s), 6.94-7.14 (3H, m), 7.57-7.87 (3H, m), 8.35 (1H, s), 8.90 (1H, s), 9.07 (1H, d, J = 7.6 Hz) ESI+ : 397 [M+H]+ |
| 12-27 | NMR-DMSO-d₆ : 1.40-1.46 (2H, m), 1.64-1.81 (8H, m), 2.02-2.06 (1H, m), 2.11-2.15 (2H, m), 3.49-3.53 (1H, m), 4.52 (1H, s), 6.99 (1H, s), 7.13 (1H, br), 7.86 (1H, br), 8.06 (1H, d, J = 2.6 Hz), 8.19 (1H, dd, J = 1.5,2.6 Hz), 8.45 (1H, s), 9.05 (1H, d, J = 1.5 Hz), 9.19 (1H, d, J = 7.4 Hz), 9.83 (1H, s) ESI+ : 381 [M+H]+ |
| 12-28 | NMR-DMSO-d₆: 1.40-1.47 (2H, m), 1.62-1.81 (8H, m), 2.02-2.06 (1H, m), 2.09-2.13 (2H, m), 2.38 (3H, s), 3.54-3.58 (1H, m), 4.52 (1H, s), 7.09 (1H, s), 7.21 (1H, br), 7.91 (1H, br), 8.36 (1H, s), 9.35 (1H, d, J = 7.5 Hz), 10.86 (1H, br) ESI+ : 385 [M+H]+ |

**[Table 76]**

| Ex | DATA |
|---|---|
| 12-29 | NMR-DMSO-d₆ : 4.39 (2H, d, J = 5.8 Hz), 6.83 (1H, ddd, J = 1.0, 5.0,7.0 Hz), 7.11 (1H, br), 7.23 (1H, s), 7.25-7.27 (1H, m), 7.33-7.41 (4H, m), 7.51 (1H, d, J = 8.5 Hz), 7.58-7.62 (1H, m), 7.82 (1H, br), 8.17-8.19 (1H, m), 8.40 (1H, s), 9.07-9.11 (1H, m), 9.49 (1H, s) ESI+ : 320 [M+H]+ |
| 12-30 | NMR-DMSO-d₆ : 4.44 (2H, d, J = 6.0 Hz), 6.83-6.86 (1H, m), 7.07-7.18 (5H, m), 7.48 (1H, d, J = 8.3 Hz), 7.58-7.62 (1H, m), 7.84 (1H, br), 8.13-8.15 (1H, m), 8.41 (1H, s), 9.12-9.15 (1H, m), 9.52 (1H, s) ESI+ : 356 [M+H]+ |
| 12-31 | NMR-DMSO-d₆: 3.70 (3H, s), 4.43 (2H, d, J = 6.1 Hz), 5.80 (1H, s), 6.44-6.47 (1H, m), 6.96-7.03 (4H, m), 7.06-7.16 (2H, m), 7.22-7.24 (1H, m), 7.83 (1H, br), 8.40 (1H, s), 8.87 (1H, s), 9.02-9.06 (1H, m) ESI+ : 385 [M+H]+ |
| 12-32 | NMR-DMSO-d₆ : 4.46 (2H, d, J = 6.0 Hz), 5.81 (1H, s), 7.02 (2H, d, J = 6.6 Hz), 7.10-7.16 (1H, m), 7.22 (1H, br), 7.28 (1H, d, J = 7.6 Hz), 7.38-7.43 (1H, m), 7.68 (1H, d, J = 8.2 Hz), 7.85 (1H, br), 8.28 (1H, s), 8.46 (1H, s), 9.05-9.09 (1H, m), 9.28 (1H, s) ESI+ : 380 [M+H]+ |
| 12-33 | NMR-DMSO-d₆ : 1.37-1.46 (2H, m), 1.62-1.82 (9H, m), 2.04 (3H, s), 2.15-2.19 (2H, m), 3.52-3.56 (1H, m), 4.52 (1H, s), 7.23 (1H, br), 7.38 (1H, s), 7.49 (1H, s), 7.84 (1H, br), 8.39 (1H, s), 9.21 (1H, d, J = 7.2 Hz), 10.44 (1H, br) ESI+ : 384 [M+H]+ |
| 12-34 | NMR-DMSO-d₆ 1.40-1.46 (2H, m), 1.64-1.82 (8H, m), 2.02-2.05 (1H, m), 2.10-2.13 (2H, m), 3.50-3.54 (1H, m), 4.51 (1H, s), 7.00 (1H, s), 7.17 (1H, br), 7.89 (1H, br), 7.93 (1H, d, J = 9.0 Hz), 8.03 (1H, dd, J = 2.4, 9.0 Hz), 8.45 (1H, s), 8.62 (1H, d, J = 3.4 Hz), 9.18 (1H, d, J = 7.6 Hz), 10.14 (1H, s) ESI+ : 405 [M+H]+ |
| 12-35 | NMR-DMSO-d₆ : 1.39-1.45 (2H, m), 1.64-1.81 (8H, m), 2.02-2.05 (1H, m), 2.10-2.14 (2H, m), 3.48-3.52 (1H, m), 4.50 (1H, s), 6.90 (1H, s), 7.06 (1H, br), 7.59-7.65 (1H, m), 7.78 (1H, br), 7.83 (1H, dd, J = 3.9, 9.3 Hz), 8.15 (1H, d, J = 3.2 Hz), 8.40 (1H, s), 9.11 (1H, d, J = 7.5 Hz), 9.55 (1H, s) ESI+ : 398 [M+H]+ |
| 12-36 | NMR-DMSO-d₆ : 1.40-1.45 (2H, m), 1.63-1.81 (8H, m), 2.01-2.05 (1H, m), 2.10-2.13 (2H, m), 3.47-3.52 (1H, m), 4.52 (1H, s), 6.81 (1H, s), 7.14 (1H, br), 7.24 (1H, dd, J = 1.4,5.1 Hz), 7.85 (1H, br), 8.31 (1H, d, J = 5.3 Hz), 8.41 (1H, d, J = 5.1 Hz), 8.47 (1H, s), 9.15 (1H, d, J = 7.5 Hz), 9.94 (1H, s) ESI+ : 405 [M+H]+ |
| 12-37 | NMR-DMSO-d₆ : 1.40-1.46 (2H, m), 1.64-1.83 (8H, m), 2.02-2.05 (1H, m), 2.11-2.14 (2H, m), 3.51-3.55 (1H, m), 4.51 (1H, s), 7.05 (1H, s), 7.13 (1H, br), 7.86 (1H, br), 7.92-8.00 (2H, m), 8.45 (1H, s), 8.51-8.53 (1H, m), 9.17 (1H, d, J = 7.5 Hz), 10.02 (1H, s) ESI+ : 448 [M+H]+ |
| 12-38 | NMR-DMSO-d₆ : 1.39-1.45 (2H, m), 1.62-1.81 (8H, m), 2.00-2.05 (1H, m), 2.12-2.15 (2H, m), 2.20 (3H, s), 3.49-3.52 (1H, m), 4.51 (1H, s), 7.02 (1H, br), 7.12 (1H, s), 7.45-7.48 (1H, m), 7.55 (1H, d, J = 8.5 Hz), 7.78 (1H, br), 8.01-8.02 (1H, m), 8.39 (1H, s), 9.10 (1H, d, J = 7.3 Hz), 9.35 (1H, s) ESI+ : 394 [M+H]+ |

**[Table 77]**

| Ex | DATA |
|---|---|
| 12-39 | NMR-DMSO-d₆: 1.40-1.46 (2H, m), 1.64-1.81 (8H, m), 2.02-2.05 (1H, m), 2.11-2.14 (2H, m), 3.49-3.53 (1H, m), 4.51 (1H, s), 6.98 (1H, s), 7.18 (1H, br), 7.80 (1H, d, J = 5.9 Hz), 7.90 (1H, br), 8.40 (1H, d, J = 5.9 Hz), 8.45 (1H, s), 8.67 (1H, s), 9.18 (1H, d, J = 7.6 Hz), 9.97 (1H, s) ESI+ : 381 [M+H]+ |
| 12-40 | NMR-DMSO-d₆ 1.43-1.49 (2H, m),1.71-1.86 (8H, m), 2.08-2.11 (1H, m), 2.19-2.22 (2H, m), 3.16 (3H, s), 3.52-3.56 (1H, m), 6.83-6.87 (1H, m), 7.05 (1H, br), 7.16 (1H, s), 7.60-7.66 (2H, m), 7.81 (1H, br), 8.18-8.21 (1H, m), 8.41 (1H, s), 9.13 (1H, d, J = 7.4 Hz), 9.46 (1H, s) ESI+ : 394 [M+H]+ |
| 12-41 | NMR-DMSO-d₆ : 1.40-1.46 (2H, m), 1.64-1.81 (7H, m), 1.86-1.88 (1H, m), 2.02-2.06 (1H, m), 2.11-2.14 (2H, m), 3.50-3.54 (1H, m), 4.53 (1H, s), 6.96 (1H, s), 7.12 (1H, br), 7.51 (1H, dd, J = 4.5, 9.0 Hz), 7.86 (1H, br), 8.12 (1H, dd, J =1.4, 9.0 Hz), 8.42 (1H, s), 8.74 (1H, dd, J = 1.4, 4.5 Hz), 9.17 (1H, d, J = 7.5 Hz), 9.90 (1H, s) ESI+ : 381 [M+H]+ |
| 12-42 | NMR-DMSO-d₆ : 1.43-1.49 (2H, m), 1.71-1.86 (8H, m), 2.08-2.12 (1H, m), 2.17-2.21 (2H, m), 3.15 (3H, s), 3.53-3.56 (1H, m), 7.03 (1H, s), 7.12 (1H, br), 7.86 (1H, br), 8.06 (1H, d, J = 2.7 Hz), 8.22 (1H, dd, J = 1.5,2.7 Hz), 8.46 (1H, s), 9.02 (1H, d, J = 1.5 Hz), 9.21 (1H, d, J = 7.4 Hz), 9.82 (1H, s) ESI+ : 395 [M+H]+ |
| 12-43 | NMR-DMSO-d₆ : 4.45 (2H, d, J = 6.1 Hz), 7.03 (1H, s), 7.07-7.13 (3H, m), 7.22 (1H, br), 7.89 (1H, br), 8.06 (1H, d, J = 2.6 Hz), 8.14 (1H, dd, J = 1.5,2.7 Hz), 8.45 (1H, s), 8.87 (1H, d, J = 1.4 Hz), 9.18 (1H, t, J = 6.2 Hz), 9.90 (1H, s) ESI+ : 357 [M+H]+ |
| 12-44 | NMR-DMSO-d₆ : 4.46 (2H, d, J = 6.0 Hz), 7.06 (1H, s), 7.08-7.14 (3H, m), 7.26 (1H, br), 7.59 (1H, d, J = 5.9 Hz), 7.92 (1H, br), 8.37 (1H, d, J = 5.9 Hz), 8.45 (1H, s), 8.63 (1H, s), 9.17 (1H, t, J = 6.0 Hz), 10.03 (1H, s) ESI+ : 357 [M+H]+ |
| 12-45 | NMR-DMSO-d₆ : 1.27-1.36 (2H, m), 1.41 (9H, s), 1.96-2.02 (2H, m), 2.98-3.09 (2H, m), 3.44-3.52 (1H, m), 3.80-3.86 (2H, m), 6.84-6.87 (1H, m), 7.07 (1H, br), 7.22 (1H, s), 7.62-7.66 (2H, m), 7.80 (1H, br), 8.20-8.22 (1H, m), 8.40 (1H, s), 8.70 (1H, d, J = 7.1 Hz), 9.47 (1H, s) ESI+ : 413 [M+H]+ |
| 12-46 | NMR-DMSO-d₆ : 1.19-1.77 (3H, m), 1.74-1.85 (1H, m), 1.96-2.04 (1H, m), 2.19-2.39 (1H, m), 3.21-3.26 (1H, m), 3.40-3.51 (2H, m), 4.54-4.57 (1H, m), 6.55 (1H, s), 6.81-6.88 (1H, m), 7.06 (1H, br), 7.44-7.52 (1H, m), 7.59-7.69 (1H, m), 7.79 (1H, br), 8.20-8.29 (1H, m), 8.40-8.41 (1H, m), 8.60-8.76 (1H, m), 9.44-9.55 (1H, m) ESI+ : 337 [M+H]+ |
| 12-47 | NMR-DMSO-d₆: 1.41-1.46 (2H, m), 1.65-1.82 (8H, m), 2.02-2.06 (1H, m), 2.14-2.18 (2H, m), 2.49 (3H, s), 3.51-3.57 (1H, m), 4.52 (1H, s), 7.14 (1H, br), 7.29-7.36 (2H, m), 7.87 (1H, br), 8.27 (1H, d, J = 5.8 Hz), 8.44 (1H, s), 9.22 (1H, d, J = 7.2 Hz), 9.89 (1H, s) ESI+ : 395 [M+H]+ |

**[Table 78]**

| Ex | DATA |
|---|---|
| 12-48 | NMR-DMSO-d₆ : 1.37-1.44 (2H, m), 1.62-1.77 (8H, m), 2.00-2.09 (3H, m), 3.36-3.43 (1H, m), 3.75 (3H, s), 4.47 (1H, s), 5.86 (1H, s), 6.31 (1H, dd, J = 0.7, 3.0 Hz), 6.73-7.97 (2H, m), 7.17 (1H, dd, J = 1.8, 8.7 Hz), 7.25 (1H, d, J = 3.0 Hz), 7.31 (1H, d, J = 8.8 Hz), 7.77 (1H, d, J = 1.8 Hz), 8.35 (1H, s), 8.60 (1H, s), 9.04 (1H, d, J = 7.6 Hz). ESI+ : 432 [M+H]+ |
| 12-49 | NMR-MSO-d₆ : 1.38-1.46 (2H, m), 1.62-1.78 (10H, m), 2.01-2.10 (4H, m), 2.75-2.80 (1H, m), 3.23 (3H, s), 3.43-3.48 (1H, m), 4.50 (1H, s), 5.92 (1H, s), 6.91-7.83 (2H, m), 7.06 (1H, d, J = 8.2 Hz), 7.24 (1H, dd, J = 8.2, 1.9 Hz), 7.42 (1H, d, J = 1.9 Hz), 8.38 (1H, s), 8.89 (1H, s), 9.08 (1H, d, J = 7.7 Hz). ESI+ : 462 [M+H]+ |
| 12-50 | NMR-DMSO-d₆: 4.56(2H, d, J = 6.2 Hz), 7.13-7.21 (2H, m), 7.34 (1H, s), 7.41-7.50 (1H, m), 7.86 (1H, br), 8.07 (1H, d, J = 2.7 Hz), 8.23 (1H, dd, J = 1.4, 2.7 Hz), 8.45 (1H, s), 8.75 (1H, d, J = 1.4 Hz), 9.26 (1H, t, J = 6.2 Hz), 9.98 (1H, s). ESI+ : 375 [M+H]+ |
| 12-51 | NMR-DMSO-d₆ : 1.54-1.63 (2H, m), 2.06-2.13 (2H, m), 2.82-2.90 (2H, m), 3.41-3.48 (3H, m), 5.03 (2H, s), 6.87-6.95 (4H, m), 7.12 (1H, br), 7.13 (1H, s), 7.29-7.45 (5H, m), 7.85 (1H, br), 8.06 (1H, d, J = 2.7 Hz), 8.23 (1H, dd, J =1.5, 2.7 Hz), 8.46 (1H, s), 8.82 (1H, d, J = 7.1 Hz), 9.02 (1H, d, J = 1.5 Hz), 9.85 (1H, brs) ESI+ : 496 [M+H]+ |
| 12-52 | NMR-DMSO-d₆: 1.53-1.63 (2H, m), 2.07-2.14 (2H, m), 2.89-2.96 (2H, m), 3.45-3.55 (3H, m), 6.98-7.07 (5H, m), 7.14 (1H, s), 7.85 (1H, br), 8.06 (1H, d, J = 2.7 Hz), 8.22-8.24 (1H, m), 8.46 (1H, s), 8.82 (1H, d, J = 7.1 Hz), 9.01 (1H, d, J = 1.3 Hz), 9.86 (1H, brs) ESI+ : 408 [M+H]+ |
| 12-53 | NMR-DMSO-d₆ : 2.50 (3H, s), 4.54 (2H, d, J = 6.1 Hz), 7.14-7.27 (2H, m), 7.34 (1H, s), 7.39 (1H, d, J = 5.9 Hz), 7.43-7.53 (1H, m), 7.90 (1H, br), 8.29 (1H, d, J = 5.8 Hz), 8.45 (1H, s), 9.18 (1H, t, J = 6.1 Hz), 10.0 (1H, s) ESI+ : 389 [M+H]+ |
| 12-54 | NMR-DMSO-d₆: 1.80-1<92 (4H, m), 2.40 (2H, t, J = 6.0 Hz), 3.60 (2H, t, J = 6.0 Hz), 4.54 (2H, d, J = 6.0 Hz), 7.00-7.28 (2H, m), 7.34-7.59 (4H, m), 7.83 (1H, brs), 8.15 (1H, d, J = 2.8 Hz), 8.41 (1H, s), 9.18-9.24 (1H, m), 9.68 (1H, brs) ESI+: 471 [M+H]+ |
| 12-55 | NMR-DMSO-d₆ : 1.35-1.43 (4H, m), 1.62-1.83 (11H, m), 2.00-2.05 (3H, m), 3.36-3.44 (2H, m), 3.81-3.93 (3H, m), 4.48 (1H, s), 5.51 (1H, s), 6.44 (1H, d, J = 7.5 Hz), 6.82 (1H, br), 7.50 (1H, br), 8.21 (1H, s), 8.95 (1H, d, J = 7.5 Hz) ESI+ : 387 [M+H]+ |
| 12-56 | NMR-DMSO-d₆: 4.48 (2H, d, J = 6.0 Hz), 7.03 (1H, s), 7.19 (1H, br), 7.54-7.59 (1H, m), 7.71-7.74 (2H, m), 7.85 (1H, s), 7.88 (1H, br), 8.05 (1H, d, J = 2.7 Hz), 8.17-8.19 (1H, m), 8.45 (1H, s), 8.87 (1H, d, J = 1.4 Hz), 9.19-9.23 (1H, m), 9.88 (1H, brs) ESI+ : 346 [M+H]+ |
| 12-57 | NMR-DMSO-d₆: 4.46 (2H, d, J = 5.9 Hz), 7.11 (1H, s), 7.17 (1H, br), 7.35-7.39 (1H, m), 7.76-7.80 (1H, m), 7.87 (1H, br), 8.05 (1H, s, J = 2.7 Hz), 8.18-8.19 (1H, m), 8.45 (1H, s), 8.45-8.47 (1H, m), 8.64-8.65 (1H, m), 8.87 (1H, d, J = 1.4 Hz), 9.16-9.19 (1H, m), 9.89 (1H, brs) ESI+ : 322 [M+H]+ |

**[Table 79]**

| Ex | DATA |
|---|---|
| 12-58 | NMR-DMSOd₆ : 1.50 (3H, d, J = 6.7 Hz), 4.54-4.61 (1H, m), 6.98 (1H, s), 7.05-7.14 (3H, m), 7.22 (1H, br), 7.90 (1H, br), 8.05 (1H, d, J = 2.7 Hz), 8.12-8.13 (1H, m), 8.45 (1H, s), 8.80 (1H, d, J = 1.5 Hz), 9.17 (1H, d, J = 5.9 Hz), 9.89 (1H, brs) ESI+ : 371 [M+H]+ |
| 12-59 | NMR-DMSO-d₆ : 4.53 (2H, d, J = 6.0 Hz), 7.00 (1H, s), 7.20 (1H, br), 7.56 (2H, d, J = 8.4 Hz), 7.83 (2H, d, J = 8.4 Hz), 7.89 (1H, br), 8.04 (1H, d, J = 2.7 Hz), 8.15 (1H, dd, J = 1.5, 2.7 Hz), 8.45 (1H, s), 8.85 (1H, s, J = 1.5 Hz), 9.21-9.25 (1H, m), 9.86 (1H, brs) ESI+ : 346 [M+H]+ |
| 12-60 | NMR-DMSO-d₆ : 4.48 (2H, d, J = 6.1 Hz), 6.98 (1H, s), 7.21 (1H, br), 7.36 (2H, d, J = 6.0 Hz), 7.90 (1H, br), 8.03 (1H, d, J = 2.7 Hz), 8.13 (1H, dd, J = 1.4, 2.7 Hz), 8.46 (1H, s), 8.51-8.53 (2H, m), 8.86 (1H, d, J = 1.4 Hz), 9.19-9.24 (1H, m), 9.86 (1H, brs) ESI+ : 322 [M+H]+ |
| 12-61 | NMR-DMSO-d₆: 4.51 (2H, d, J = 5.6 Hz), 7.01 (1H, s), 7.16 (1H, br), 7.27-7.30 (1H, m), 7.37 (1H, d, J = 8.0 Hz), 7.75-7.80 (1H, m), 7.86 (1H, br), 8.04 (1H, d, J = 2.7 Hz), 8.14-8.16 (1H, m), 8.45 (1H, s), 8.56-8.58 (1H, m), 8.92 (1H, d, J = 1.4 Hz), 9.28-9.32 (1H, m), 9.85 (1H, s) ESI+ : 322 [M+H]+ |
| 12-62 | NMR-DMSO-d₆ : 2.68 (6H, s), 4.44 (2H, d, J = 5.6 Hz), 6.98 (1H, s), 7.00-7.04 (1H, m), 7.13 (1H, br), 7.17-7.26 (2H, m), 7.86 (1H, s), 7.88 (1H, br), 8.03 (1H, d, J = 2.7 Hz), 8.13 (1H, dd, J = 1.5,2.7 Hz), 8.45 (1H, s), 8.91 (1H, d, J = 1.5 Hz), 9.09-9.13 (1H, m), 9.84 (1H, brs) ESI+ : 364 [M+H]+ |
| 12-63 | NMR-DMSO-d₆: 2.87 (6H, s), 4.31 (2H, d, J = 5.6 Hz), 6.62 (1H, dd, J = 2.6, 7.6 Hz), 6.67 (1H, d, J = 7.6 Hz), 6.74 (1H, s), 7.13 (1H, s), 7.13-7.18 (2H, m), 7.86 (1H, br), 8.06 (1H, d, J = 2.6 Hz), 8.20 (1H, dd, J = 1.5, 2.7 Hz), 8.44 (1H, s), 8.92 (1H, d, J = 1.5 Hz), 9.05-9.09 (1H, m), 9.87 (1H, s) ESI+ : 364 [M+H]+ |
| 12-64 | NMR-DMSO-d₆ : 2.86 (6H, s), 4.24 (2H, d, J = 5.4 Hz), 6.70 (2H, d, J = 8.7 Hz), 7.08 (1H, br), 7.15 (1H, s), 7.21 (2H, d, J = 8.7 Hz), 7.84 (1H, br), 8.05 (1H, d, J = 2.8 Hz), 8.23-8.25 (1H, m), 8.43 (1H, s), 8.92-8.97 (2H, m), 9.87 (1H, s) ESI+ : 364 [M+H]+ |
| 12-65 | NMR-DMSO-d₆: 9.60 (1H, s), 9.13 (1H, t, J = 6.0 Hz), 8.42 (1H, s), 8.03 (1H, d, J = 2.8 Hz), 7.83 (1H, bs), 7.59-7.52 (2H, m), 7.48-7.37 (4H, m), 7.14 (1H, bs), 7.06 (1H, s), 4.49 (2H, d, J = 6.0 Hz), 3.62-3.53 (2H, m), 2.39 (2H, t, J = 6.4 Hz), 1.92-1.78 (4H, m). ESI+ : 501 [M+H]+ |
| 12-66 | NMR-DMSO-d₆ : 4.60 (2H, d, J = 5.8 Hz), 6.99 (1H, dd, J = 3.5, 5.1 Hz), 7.14-7.16 (1H, m), 7.18 (1H, br), 7.25 (1H, s), 7.41 (1H, dd, J = 1.2, 5.1 Hz), 7.86 (1H, br), 8.06 (1H, d, J = 2.7 Hz), 8.23 (1H, dd, J = 1.5,2.7 Hz), 8.45 (1H, s), 8.90 (1H, d, J = 1.5 Hz), 9.10-9.14 (1H, m), 9.92 (1H, s) ESI+ : 327 [M+H]+ |
| 12-67 | NMR-DMSO-d₆: 4.40 (2H, d, J = 5.9 Hz), 7.11 (1H, s), 7.16 (1H, br), 7.23-7.28 (1H, m), 7.33-7.41 (4H, m), 7.86 (1H, br), 8.04 (1H, d, J = 2.7 Hz), 8.19 (1H, dd, J =1.5, 2.7 Hz), 8.44 (1H, s), 8.89 (1H, d, J = 1.5 Hz), 9.12-9.16 (1H, m), 9.87 (1H, s) ESI+ : 321 [M+H]+ |

**[Table 80]**

| Ex | DATA |
|---|---|
| 12-68 | NMR-DMSO-d₆ : 1.78-1.93 (3H, m), 1.98-2.05 (1H, m), 2.72-2.90 (2H, m), 4.67-4.71 (1H, m), 7.09 (1H, br), 7.14-7.28 (5H, m), 7.85 (1H, br), 8.06 (1H, d, J = 2.7 Hz), 8.21 (1H, dd, J = 1.5, 2.7 Hz), 8.47 (1H, s), 9.02-9.05 (2H, m), 9.87 (1H, s) ESI+ : 361 [M+H]+ |
| 12-69 | NMR-DMSO-d₆ : 1.74-1.83 (1H, m), 2.09-2.16 (1H, m), 2.20-2.29 (1H, m), 2.54-2.59 (2H, m), 2.67-2.76 (1H, m), 3.65-3.73 (1H, m), 6.34-6.41 (1H, m), 7.10 (1H, br), 7.16 (1H, s), 7.65-7.69 (2H, m), 7.79-7.82 (2H, m), 7.86 (1H, s), 8.06 (1H, d, J = 2.4 Hz), 8.23-8.25 (1H, m), 8.45 (1H, s), 8.88 (1H, d, J = 7.2 Hz), 9.01 (1H, d, J = 1.6 Hz), 9.85 (1H, s) ESI+ : 412 [M+H]+ |
| 12-70 | NMR-DMSO-d₆ : 1.78-1.90 (4H, m), 2.37 (2H, t, J = 6.3 Hz), 3.57 (2H, t, J = 5.6 Hz), 4.45 (2H, d, J = 6.0 Hz), 6.03 (1H, s), 6.94-7.16 (3H, m), 7.16-7.24 (1H, m), 7.45-7.55 (3H, m), 7.77 (1H, br), 8.38 (1H, s), 9.01-9.07 (2H, m) ESI+ : 470 [M+H]+ |
| 12-71 | NMR-DMSO-d₆ : 1.81-1.90 (4H, m), 2.37-2.41 (2H, m), 3.58-3.62 (2H, m), 4.59 (2H, d, J = 5.6 Hz), 6.99 (1H, dd, J = 3.2, 4.8 Hz), 7.10 (1H, br), 7.14-7.16 (1H, m), 7.25-7.27 (1H, m), 7.41 (1H, dd, J = 2.4, 4.8 Hz), 7.53-7.60 (2H, m), 7.82 (1H, br), 8.13 (1H, d, J = 2.4 Hz), 8.41 (1H, s), 9.05-9.09 (1H, m), 9.63 (1H, brs) ESI+ : 423 [M+H]+ |
| 12-72 | NMR-DMSO-d₆ : 8.87 (1H, t, J = 6.0 Hz), 8.19 (1H, s), 7.31-7.22 (5H, m), 6.93-6.86 (2H, m), 6.77 (1H, bs), 6.17 (1H, s), 5.54 (1H, bs), 4.40 (2H, d, J = 6.0 Hz), 3.67 (2H, t, J = 5.6 Hz), 2.60-2.57 (2H, m), 1.97-1.96 (4H, m). ESI+ : 452 [M+H]+ |
| 12-73 | NMR-DMSO-d₆ : 2.29 (3H, s), 2.74 (2H, t, J = 5.4 Hz), 3.12 (2H, s), 3.65 (2H, t), 4.54 (2H, d, J = 6.0 Hz), 7.06-7.22 (2H, m), 7.37 (1H, s), 7.42-7.50 (2H, m), 7.61 (1H, dd, J = 2.7, 8.9 Hz), 7.84 (1H, br), 8.19 (1H, d), 8.41 (1H, s), 9.21 (1H, t, J = 6.0 Hz), 9.72 (1H, s) ESI+ : 486 [M+H]+ |
| 12-74 | NMR-DMSO-d₆ : 2.29 (3H, s), 2.54-2.76 (6H, m), 3.75-3.81 (2H, m), 4.53 (2H, d, J = 6.0 Hz), 7.03-7.22 (2H, m), 7.37 (1H, s), 7.41-7.52 (3H, m), 7.83 (1H, brs), 8.08-8.09 (1H, m), 8.41 (1H, s), 9.19 (1H, t, J = 6.0 Hz), 9.68 (1H, brs) ESI+ : 500 [M+H]+ |
| 12-75 | NMR-DMSO-d₆ : 2.28 (3H, s), 2.71 (2H, t, J = 5.4 Hz), 3.08 (2H, s), 3.61 (2H, t, J = 5.4 Hz), 4.45 (2H, d, J = 6.0 Hz), 6.04 (1H, s), 6.92-7.25 (4H, m), 7.43-7.60 (3H, m), 7.78 (1H, br), 8.38 (1H, s), 9.00-9.12 (2H, m) ESI+ : 485 [M+H]+ |
| 12-76 | NMR-DMSO-d₆ : 3.70-3.77 (2H, m), 3.96-4.02 (2H, m), 4.22 (2H, s), 4.54 (2H, d, J = 6.1 Hz), 7.00-7.24 (2H, m), 7.37 (1H, s), 7.41-7.52 (2H, m), 7.68 (1H, dd, J = 2.7, 8.9 Hz), 7.84 (1H, br), 8.27 (1H, d, J = 2.6 Hz), 8.42 (1H, s), 9.21 (1H, t, J = 6.2 Hz), 9.73 (1H, s) ESI+: 473 [M+H]+ |
| 12-77 | NMR-DMSO-d₆ : 3.65-3.73 (2H, m), 3.93-4.00 (2H, m), 4.18 (2H, s), 4.46 (2H, d, J = 6.0 Hz), 6.04 (1H, s), 7.07 (1H, br), 7.16-7.28 (3H, m), 7.44-7.60 (3H, m), 7.78 (1H, br), 8.38 (1H, s), 9.01-9.12 (2H, m) ESI+ : 472 [M+H]+ |

**[Table 81]**

| Ex | DATA |
|---|---|
| 12-78 | NMR-DMSO-d₆: 1.53-1.58 (2H, m), 1.69-1.76 (1H, m), 1.89-1.98 (3H, m), 2.80-2.83 (1H, m), 4.51-4.58 (3H, m), 7.11 (1H, br), 7.13-7.20 (1H, m), 7.32 (1H, s), 7.42-7.51 (2H, m), 7.81 (1H, dd, J = 2.8, 9.2 Hz), 7.82 (lI-I, br), 8.40 (1H, s), 8.43 (1H, d, J = 2.8 Hz), 9.15- 9.19 (1H, m), 9.59 (1H, brs) ESI+ : 483 [M+H]+ |
| 12-79 | NMR-DMSO-d₆ : 1.69-1.79 (6H, m), 2.59-2.64 (2H, m), 3.69-3.75 (2H, m), 4.54 (2H, d, J = 6.0 Hz), 7.08-7.20 (2H, m), 7.37-7.52 (4H, m), 7.82 (1H, br), 8.08 (1H, d, J = 2.8 Hz), 8.41 (1H, s), 9.17-9.21 (1H, m), 9.66 (1H, s) ESI+ : 485 [M+H]+ |
| 12-80 | NMR-DMSO-d₆ : 1.52-1.58 (2H, m), 1.69-1.75 (1H, m), 1.89-1.98 (3H, run), 2.80-2.83 (1H, m), 4.51-4.57 (3H, m), 7.09 (1H, br), 7.13-7.20 (1H, m), 7.32 (1H, s), 7.43-7.50 (2H, m), 7.81 (1H, dd, J = 2.8, 9.2 Hz), 7.84 (1H, br), 8.40 (1H, s), 8.43 (1H, d, J = 2.8 Hz), 9.16-9.19 (1H, m), 9.59 (1H, s) ESI+ : 483 [M+H]+ |
| 12-81 | NMR-DMSO-d₆: 2.79-2.86 (2H, m), 3.76-3.89 (6H, m), 4.53 (2H, d, J = 6.1 Hz), 7.00-7.22 (2H, m), 7.36 (1H, s), 7.42-7.54 (3H, m), 7.83 (1H, br), 8.11 (1H, d, J = 2.6 Hz), 8.41 (1H, s), 9.19 (1H, t, J = 6.1 Hz), 9.69 (1H, s) ESI+ : 487 [M+H]+ |
| 12-82 | NMR-DMSO-d₆ 1.81-1.91 (4H, m), 2.37-2.41 (2H, m), 3.55-3.60 (2H, m), 3.80 (3H, s), 4.62 (2H, d, J = 5.6 Hz), 6.56 (1H, d, J = 2.4 Hz), 7.09-7.14 (3H, m), 7.20 (1H, s), 7.33-7.37 (2H, m), 7.50-5.58 (2H, m), 7.78 (1H, br), 8.00 (1H, d, J = 2.4 Hz), 8.39 (1H, s), 9.10-9.14 (1H, m), 9.54 (1H, s) ESI+ : 470 [M+H]+ |
| 12-83 | NMR-DMSO-d₆ : 9.88 (1H, s), 9.14 (1H, t, J = 5.6 Hz), 8.93 (1H, d, J = 1.2 Hz), 8.44 (1H, s), 8.15 (1H, dd, J = 2.4, 1.2 Hz), 8.05 (1H, d, J = 2.4 Hz), 7.86 (1H, bs), 7.34-7.08 (5H, m), 7.04 (1H, s), 4.46 (1H, d, J = 5.6 Hz), 3.78-3.76 (4H, m), 2.88-2.86 (4H, m). ESI+ : 406 [M+H]+ |
| 12-84 | NMR-DMSO-d₆ : 1.80-1.90 (4H, m), 2.37-2.41 (2H, m), 3.55-3.60 (2H, m), 3.75 (3H, s), 4.46 (2H, d, J = 5.6 Hz), 6.38 (1H, d, J = 2.8 Hz), 7.06-7.11 (2H, m), 7.25 (1H, s), 7.29 (1H, d, J = 3.2 Hz), 7.47 (1H, s), 7.50-7.58 (3H, m), 7.80 (1H, br), 8.10 (1H, d, J = 2.8 Hz), 8.40 (1H, s), 9.03-9.07 (1H, m), 9.57 (1H, s) ESI+ : 470 [M-H]- |
| 12-85 | NMR-DMSO-d₆ : 1.78-1.90 (4H, m), 2.37-2.42 (2H, m), 3.56-3.61 (2H, m), 3.76 (3H, s), 4.42 (2H, d, J = 5.2 Hz), 6.38-6.40 (1H, m), 7.07 (1H, m), 7.17-7.20 (1H, m), 7.22 (1H, s), 7.30 (1H, d, J = 2.8 Hz), 8.39-7.42 (1H, m), 7.51-7.58 (3H, m), 7.79 (1H, br), 8.12 (1H, d, J = 2.8 Hz), 8.39 (1H, s), 9.01-9.05 (1H, m), 9.56 (1H, s) ESI+ : 470 [M+H]+ |
| 12-86 | NMR-DMSO-d₆ : 1.80-1.90 (4H, m), 2.37-2.41 (2H, m), 3.32 (3H, s), 3.57-3.62 (2H, m), 4.30 (2H, d, J = 5.2 Hz), 5.90 (1H, dd, J = 2.4, 3.6 Hz), 6.11 (1H, dd, J = 1.6, 3.6 Hz), 6.68-6.70 (1H, m), 7.07 (1H, br), 7.24 (1H, s), 7.56 (1H, dd, J = 2.4, 8.8 Hz), 7.65 (1H, d, J = 8.8 Hz), 7.80 (1H, br), 8.17 (1H, d, J = 2.8 Hz), 8.40 (1H, s), 8.81-8.85 (1H, m), 9.63 (1H, brs) ESI+ : 420 [M-H]- |

**[Table 82]**

| Ex | DATA |
|---|---|
| 12-87 | NMR-DMSO-d₆: 1.79-1.90 (5H, m), 2.36-2.40 (2H, m), 2.63-2.71 (1H, m), 2.87-3.05 (2H, m), 3.57-3.61 (2H, m), 4.96-5.02 (1H, m), 7.06 (1H, br), 7.21-7.38 (5H, m), 7.57 (1H, dd, J = 2.8, 9.2 Hz), 7.67 (1H, d, J = 9.2 Hz), 7.81 (1H, br), 8.12 (1H, d, J = 2.8 Hz), 8.44 (1H, s), 9.00 (1H, d, J = 6.8 Hz), 9.64 (1H, s) ESI+: 443 [M+H]+ |
| 12-88 | NMR-MSO-d₆ : 1.81-1.90 (4H, m), 2.38-2.40 (2H, m), 3.57-3.61 (2H, m), 4.44 (2H, d, J = 6.0 Hz), 7.10 (1H, s), 7.14 (1H, br), 7.41-7.48 (1H, m), 7.54-7.64 (3H, m), 7.84 (1H, br), 8.08-8.10 (1H, m), 8.42 (1H, s), 9.07-9.11 (1H, m), 9.61 (1H, s) ESI+ : 471 [M+H]+ |
| 12-89 | NMR-DMSO-d₆ : 1.81-1.90 (4H, m), 2.37-2.42 (2H, m), 2.98-3.03 (2H, m), 3.41-3.46 (2H, m), 3.58-3.62 (2H, m), 7.05 (1H, br), 7.07-7.13 (1H, m), 7.19 (1H, s), 7.33-7.40 (1H, m), 7.54-7.61 (2H, m), 7.76 (1H, br), 8.13-8.14 (1H, m), 8.38 (1H, s), 8.76-8.80 (1H, m), 9.59 (1H, s) ESI+ : 485 [M-H]- |
| 12-90 | NMR-DMSO-d₆ : 9.56 (1H, s), 8.99 (1H, t, J = 6.0 Hz), 8.43 (1H, s), 7.86-7.83 (3H, m), 7.66 (2H, d, J = 8.8 Hz), 7.49-7.41 (1H, m), 7.19-7.13 (3H, m), 6.18 (1H, s), 4.42 (2H, d, J = 6.0 Hz), 3.13-3.09 (4H, m), 1.66-1.62 (4H, m). ESI+ : 488 [M+H]+ |
| 12-91 | NMR-DMSO-d₆ : 1.44 (6H, s), 2.89 (3H, s), 4.55 (2H, d, J = 6.2 Hz), 7.04-7.24 (2H, m), 7.38 (1H, s), 7.41-7.52 (1H, m), 7.54 (1H, d, J = 8.8 Hz), 7.65 (1H, dd, J = 2.6, 8.8 Hz), 7.85 (1H, br), 8.24 (1H, d, J = 2.6 Hz), 8.43 (1H, s), 9.21 (1H, t, J = 6.2 Hz), 9.85 (1H, s) ESI+ : 514 [M+H]+ |
| 12-92 | NMR-DMSO-d₆ : 1.80-1.90 (4H, m), 2.37-2.41 (2H, m), 3.17 (3H, d, J = 5.2 Hz), 3.56-3.60 (2H, m), 4.40 (2H, d, J = 5.6 Hz), 7.07 (1H, s), 7.09 (1H, br), 7.13-7.18 (1H, m), 7.27-7.36 (3H, m), 7.54 (1H, dd, J = 2.4, 8.8 Hz), 7.61 (1H, d, J = 8.8 Hz), 7.81 (1H, br), 8.08 (1H, d, J = 2.4 Hz), 8.41 (1H, s), 9.01-9.04 (1H, m), 9.58 (1H, s) ESI+ : 463 [M+H]+ |
| 12-93 | NMR-DMSO-d₆ : 1.80-1.90 (4H, m), 2.36-2.41 (2H, m), 3.28 (3H, s), 3.55-3.59 (2H, m), 4.86 (2H, d, J = 6.4 Hz), 7.01 (1H, s), 7.15 (1H, br), 7.51-7.61 (4H, m), 7.68-7.73 (1H, m), 7.86 (1H, br), 7.98 (1H, dd, J = 1.6,7.6 Hz), 8.03 (1H, d, J = 2.0 Hz), 8.44 (1H, s), 9.15-9.19 (1H, m), 9.59 (1H, s) ESI+ : 495 [M+H]+ |
| 12-94 | NMR-DMSO-d₆ : 9.84 (1H, s), 9.02 (1H, t, J = 6.0 Hz), 8.86 (1H, d, J = 1.2 Hz), 8.42 (1H, s), 8.16 (1H, dd, J = 2.8, 1.6 Hz), 8.11 (1H, dd, J = 1.6, 0.8 Hz), 8.04 (1H, d, J = 2.8 Hz), 7.84 (1H, bs), 7.79 (1H, dd, J = 2.0, 0.8 Hz), 7.52-7.50 (1H, m), 7.47-7.42 (3H, m), 7.15 (1H, bs), 6.95 (1H, s), 6.55 (1H, dd, J = 2.4, 2.0 Hz), 4.44 (2H, d, J = 6.0 Hz). ESI+ : 387 [M+H]+ |
| 12-95 | NMR-DMSO-d₆ : 9.11 (1H, bs), 8.33 (1H, s), 7.87-7.83 (2H, m), 7.52-7.48 (2H, m), 7.31-7.21 (2H, m), 6.96-6.89 (2H, m), 6.25 (1H, s), 5.70 (2H, bs), 4.46 (2H, d, J = 6.4 Hz), 3.53-3.45 (1H, m), 2.13-2.04 (2H, m), 1.95-1.82 (2H, m), 1.79-1.74 (2H, m), 1.67-1.50 (2H, m). ESI+ : 487 [M+H]+ |

**[Table 83]**

| Ex | DATA |
|---|---|
| 12-96 | NMR-DMSO-d₆ 2.29 (3H, s), 2.53-2.75 (6H, m), 3.72-3.80 (2H, m), 4.53 (2H, d, J = 6.1 Hz), 7.01-7.25 (3H, m), 7.40-7.50 (2H, m), 7.53-7.58 (1H, m), 7.86 (1H, br), 7.99 (1H, d, J = 2.6 Hz), 8.43 (1H, s), 9.13 (1H, t, J = 5.8 Hz), 9.62 (1H, s) ESI+ : 500 [M+H]+ |
| 12-97 | NMR-DMSO-d₆ : 2.29 (3H, s), 2.54-2.76 (6H, m), 3.76-3.82 (2H, m), 4.46 (2H, d, J = 6 Hz), 7.01-7.20 (3H, m), 7.37-7.53 (4H, m), 7.82 (1H, brs), 8.09-8.11 (1H, m), 8.40 (1H, s), 9.08 (1H, t, J = 6 Hz), 9.65 (1H, brs) ESI+ : 482 [M+H]+ |
| 12-98 | NMR-DMSO-d₆ : 1.82-1.90 (4H, m), 2.37-2.42 (2H, m), 3.32 (3H, s), 3.57-3.62 (2H, m), 4.60 (2H, d, J = 5.6 Hz), 6.50 (1H, s), 6.97-7.01 (1H, m), 7.09-7.14 (2H, m), 7.26 (1H, s), 7.43 (1H, d, J = 8.0 Hz), 7.48 (1H, d, J = 8.0 Hz), 7.55 (1H, dd, J = 2.4, 8.8 Hz), 7.65 (1H, d, J = 8.8 Hz), 7.84 (1H, br), 8.16 (1H, d, J = 2.4 Hz), 8.43 (1H, s), 9.08-9.12 (1H, m), 9.61 (1H, s) ESI+ : 470 [M+H]+ |
| 12-99 | NMR-DMSO-d₆ 1.80-1.90 (4H, m), 2.36-2.4 (2H, m), 3.54-3.58 (2H, m), 4.68 (2H, d, J = 6.0 Hz), 7.11 (1H, br), 7.16 (1H, s), 7.39-7.41 (2H, m), 7.48-7.52 (3H, m), 7.77-7.82 (2H, m), 7.84 (1H, br), 7.92-7.93 (1H, m), 8.42 (1H, s), 9.28-9.32 (1H, m), 9.55 (1H, s) ESI+ : 473 [M+H]+ |
| 12-100 | NMR-DMSO-d₆ : 4.70 (2H, d, J = 6.0 Hz), 7.14 (1H, s), 7.18 (1H, br), 7.39-7.41 (2H, m), 7.50 (1H, d, J = 5.6 Hz), 7.77-7.82 (2H, m), 7.88 (1H, br), 7.99-9.01 (2H, m), 8.46 (1H, s), 8.80 (1H, d, J = 1.2 Hz), 9.34-9.38 (1H, m), 9.84 (1H, s) ESI+ : 377 [M+H]+ |
| 12-101 | NMR-DMSO-d₆ : 1.04 (6H, d, J = 6.0 Hz), 3.26-3.28 (2H, m), 4.69 (2H, d, J = 5.6 Hz), 7.11 (1H, br), 7.24 (1H, s), 7.36-7.41 (3H, m), 7.45-7.51 (2H, m), 7.77-7.87 (4H, m), 8.41 (1H, s), 9.28-9.31 (1H, m), 9.46 (1H, s) ESI+ : 433 [M+H]+ |
| 12-102 | NMR-DMSO-d₆ : 1.81-1.88 (4H, m), 2.37 (2H, t, J = 6.2 Hz), 3.53-3.56 (2H, m), 4.69 (2H, d, J = 5.8 Hz), 6.96 (1H, s), 7.19 (1H, br s), 7.508-7.514 (2H, m), 7.68 (1H, dd, J = 8.0, 4.7 Hz), 7.89-7.93 (3H, m), 8.45 (1H, s), 8.61-8.62 (1H, m), 9.21 (1H, t, J = 6.2 Hz), 9.60 (1H, s) ESI+ : 486 [M+H]+ |
| 12-103 | NMR-DMSO-d₆ 1.83-1.89 (4H, m), 2.39 (2H, t, J = 6.1 Hz), 2.55 (3H, s), 3.58 (2H, t, J = 5.5 Hz), 4.42 (2H, d, J = 5.9 Hz), 6.55 (1H, s), 6.98 (1H, s), 7.18 (1H, dd, J = 7.6, 4.9 Hz), 7.52 (1H, dd, J = 8.9, 2.6 Hz), 7.58-7.64 (2H, m), 8.05 (1H, dd, J = 2.6, 0.4 Hz), 8.32 (1H, dd, J = 4.8, 1.7 Hz), 8.42 (1H, s), 8.60 (1H, s), 9.09 (1H, t, J = 6.0 Hz), 9.57 (1H, s) ESI+ : 432 [M+H]+ |
| 12-104 | NMR-DMSO-d₆ 2.30 (3H, s), 2.55-2.73 (6H, m), 3.70-3.76 (2H, m), 4.68 (2H, d, J = 6.0 Hz), 7.12 (1H, br), 7.16 (1H, s), 3.77-3.38 (4H, m), 7.51 (1H, d, J = 5.2 H), 7.76-7.82 (3H, m), 7.83 (1H, br), 8.42 (1H, s), 9.27-9.30 (1H, m), 9.54 (1H, s) ESI+ : 502 [M+H]+ |
| 12-105 | NMR-DMSO-d₆ : 2.13 (6H, s), 3.29-3.30 (2H, m), 4.03 (3H, s), 4.79 (2H, d, J = 5.2 Hz), 5.43 (1H, d, J = 2.8 Hz), 6.94-6.98 (1H, m), 7.04 (1H, br), 7.13 (1H, d, J = 6.4 Hz), 7.27 (1H, d, J = 2.8 Hz), 7.30 (1H, s), 7.47-7.50 (1H, m), 7.52-7.55 (1H, m), 7.61 (1H, d, J = 7.6 Hz), 7.81 (1H, br), 8.04 (1H, d, J = 2.0 Hz), 8.43 (1H, s), 8.93-8.97 (1H, m), 9.52 (1H, s) ESI- : 428 [M-H]- |

**[Table 84]**

| Ex | DATA |
|---|---|
| 12-106 | NMR-DMSO-d₆: 1.99-2.08 (1H, m), 2.54-2.63 (1H, m), 2.91-2.99 (1H, m), 3.06-3.15 (1H, m), 5.15-5.20 (1H, m), 7.01-7.06 (1H, m), 7.10 (1H, br), 7.19 (1H, d, J = 7.6 Hz), 7.26 (1H, s), 7.33-7.38 (1H, m), 7.86 (1H, br), 8.07 (1H, d, J = 2.6 Hz), 8.23 (1H, dd, J = 1.6, 2.6 Hz), 8.47 (1H, s), 8.99 (1H, d, J = 1.6 Hz), 9.02 (1H, d, J = 7.0 Hz), 9.92 (1H, s) ESI+ : 365 [M+H]⁺ |
| 12-107 | NMR-DMSO-d₆: 1.32-1.37 (2H, m), 1.60-1.73 (8H, m), 1.91-2.00 (3H, m), 3.34-3.39 (1H, m), 4.41 (2H, d, J = 6.1 Hz), 4.46 (1H, s), 5.46 (1H, s), 6.78 (1H, br), 7.00-7.04 (1H, m), 7.18-7.22 (1H, m), 7.28-7.32 (4H, m), 7.48 (1H, br), 8.21 (1H, s), 8.96 (1H, d, J = 7.6 Hz) ESI+ : 393 [M+H]+ |
| 7 | NMR-DMSO-d₆: 1.45-1.55 (2H, m), 2.03-2.09 (2H, m), 2.65-2.73 (2H, m), 3.29-3.30 (1H, m), 3.53-3.59 (2H, m), 6.94 (1H, s), 7.12 (1H, br), 7.84 (1H, br), 7.98 (2H, d, J = 9.2 Hz), 8.06 (1H, d, J = 2.6 Hz), 8.15-8.19 (3H, m), 8.43 (1H, s), 8.69 (1H, d, J = 7.0 Hz), 9.03 (1H, d, J =1.4 Hz), 9.75 (1H, s) ESI+ : 479 [M+H]+ |

**[Table 85]**

| Pr | Strucure | Pr | Strucure |
|---|---|---|---|
| 1-2 | 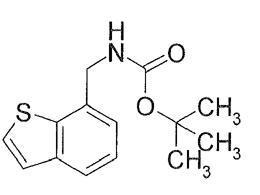 | 1-3 | 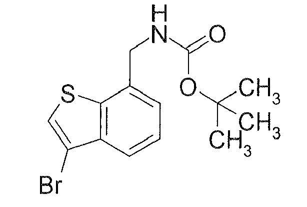 |
| 2-1 | 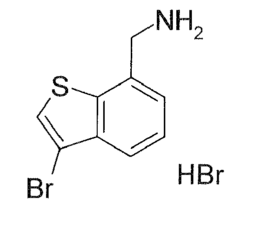 | 3-1 | 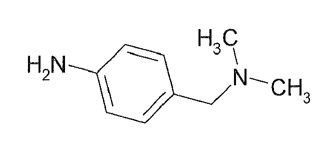 |
| 4-1 | 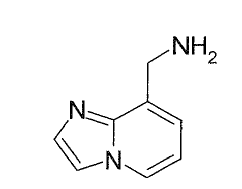 | 6-1 | 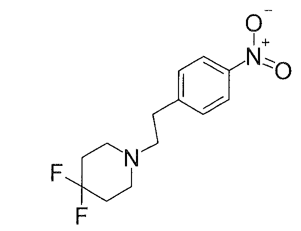 |
| 6-2 | 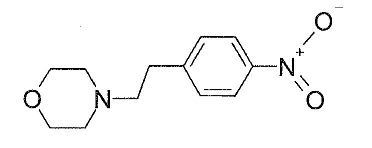 | 10-1 | 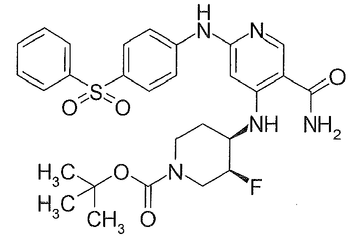 |
| 14-58 | 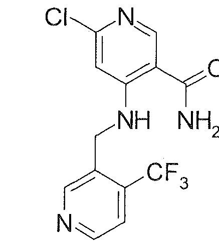 | 14-59 | 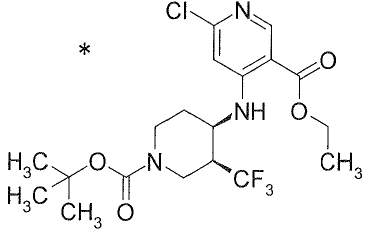 |

**[Table 86]**

| Pr | Strucure | Pr | Strucure |
|---|---|---|---|
| 14-62 | 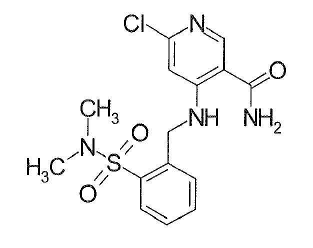 | 14-63 | 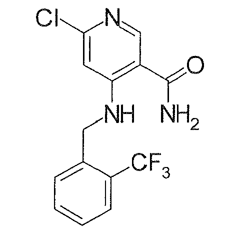 |
| 14-64 | 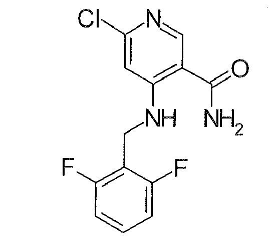 | 14-65 | 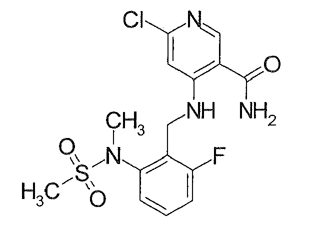 |
| 14-66 | 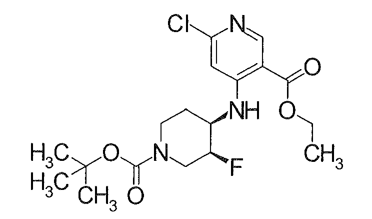 | 14-67 | 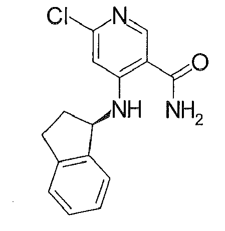 |
| 14-68 | 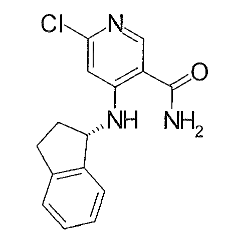 | 14-69 | 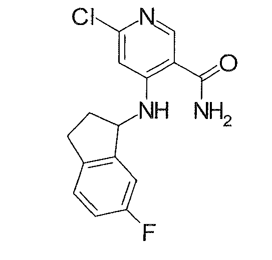 |

**[Table 87]**

| Pr | Strucure | Pr | Strucure |
|---|---|---|---|
| 14-70 | 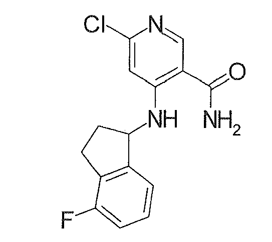 | 14-71 | 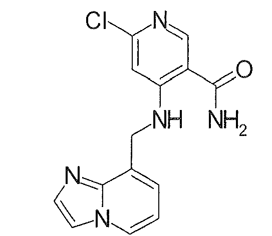 |
| 14-72 | 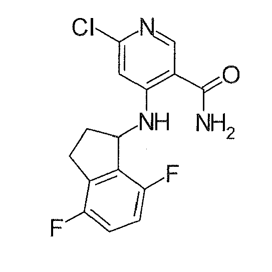 | 14-73 | 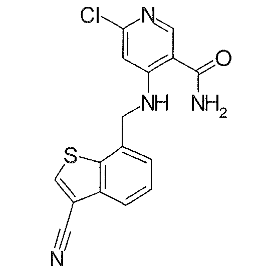 |
| 49 | 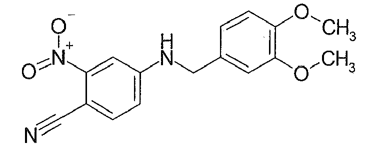 | 14-74 | 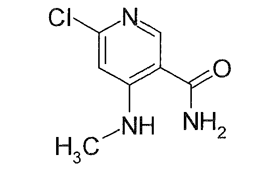 |
| 18-1 | 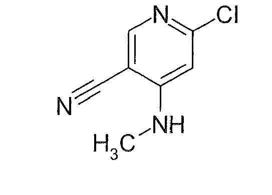 | 19-19 | 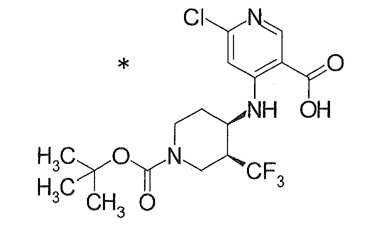 |
| 19-20 | 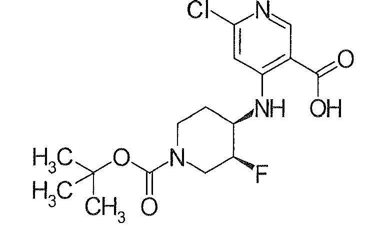 | 20-1 | 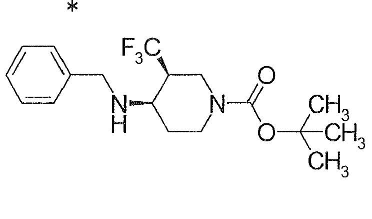 |

**[Table 88]**

| Pr | Strucure | Pr | Strucure |
|---|---|---|---|
| 40-1 | 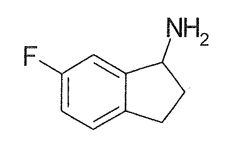 | 40-2 | 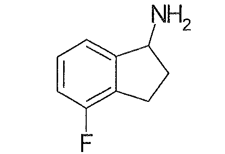 |
| 40-3 | 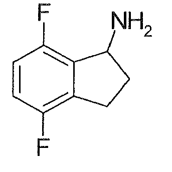 | 47 | 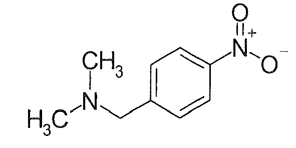 |
| 21-2 | 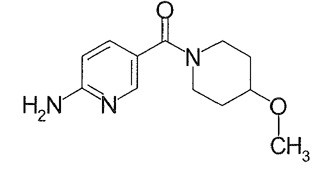 | 22-20 | 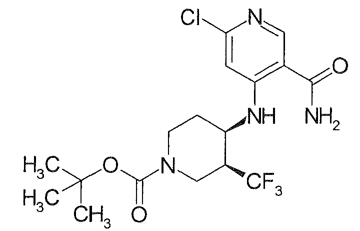 |
| 22-21 | 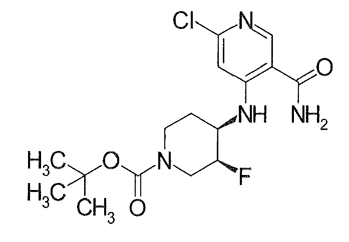 | 23-8 | 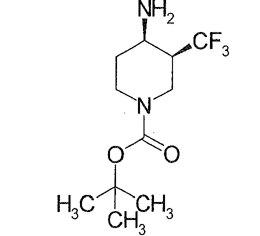 |
| 23-9 | 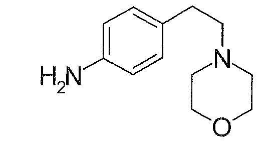 | 23-10 | 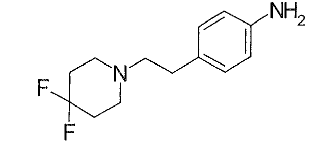 |
| 23-11 | 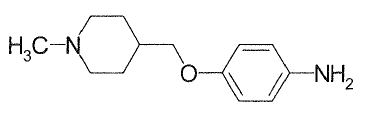 | 24-1 | 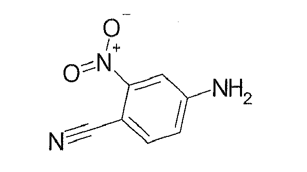 |

**[Table 89]**

| Pr | Strucure | Pr | Strucure |
|---|---|---|---|
| 25-59 | 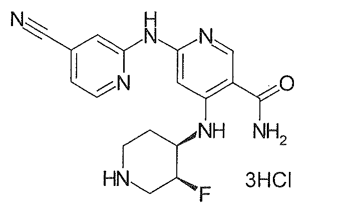 | 25-60 | 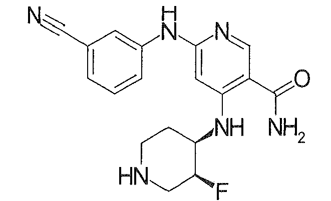 |
| 25-61 | 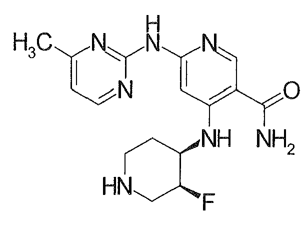 | 25-62 | 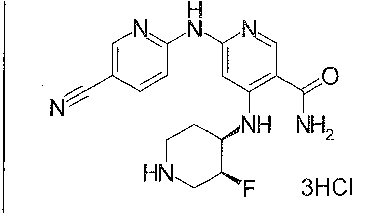 |
| 26-63 | 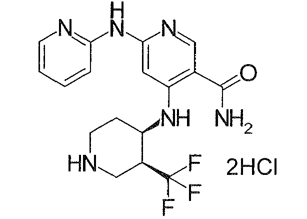 | 25-65 | 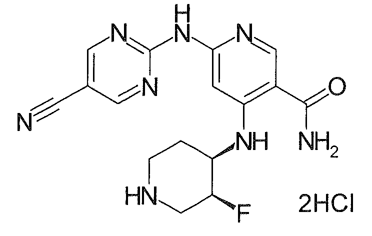 |
| 25-66 | 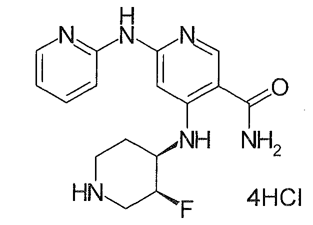 | 46 | 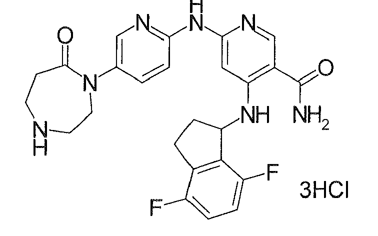 |
| 44-1 | 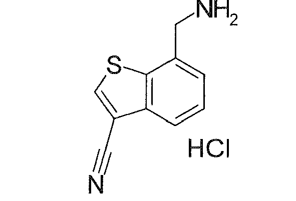 | | |

**[Table 90]**

| Pr | Strucure | Pr | Strucure |
|---|---|---|---|
| 25-67 | 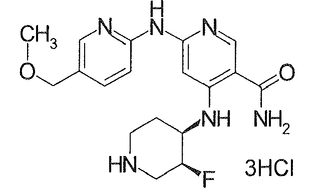 | 25-68 | 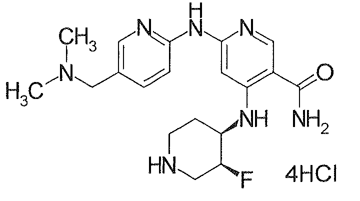 |
| 25-69 | 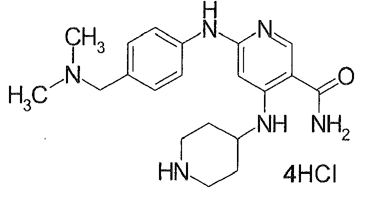 | 25-70 | 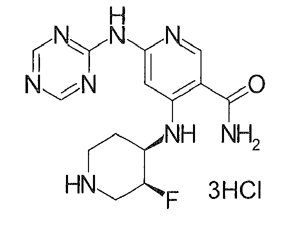 |
| 25-71 | 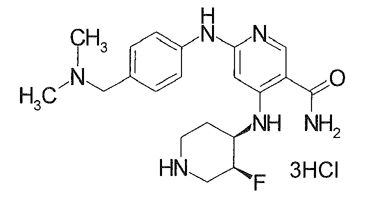 | 25-72 | 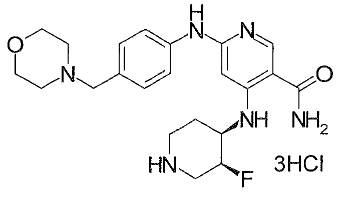 |
| 25-73 | 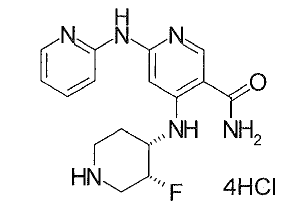 | 25-74 | 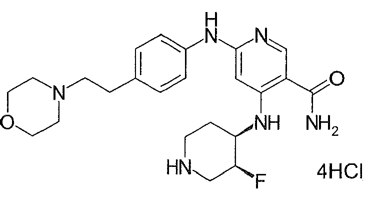 |
| 25-75 | 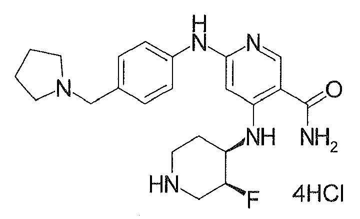 | 25-76 | 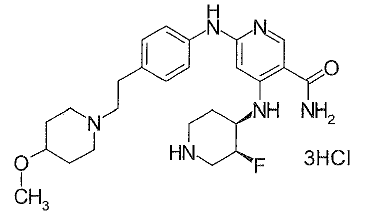 |
| 25-77 | 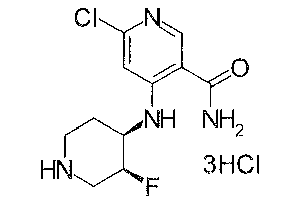 | 25-78 | 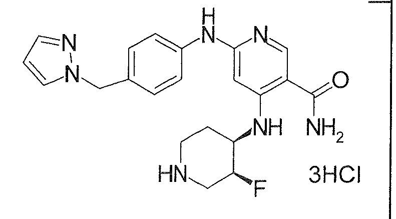 |

**[Table 91]**

| Pr | Strucure | Pr | Strucure |
|---|---|---|---|
| 25-79 | 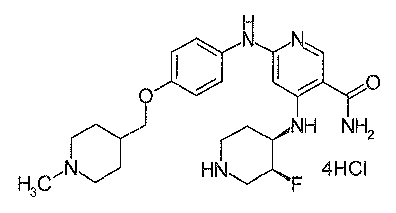 | 25-80 | 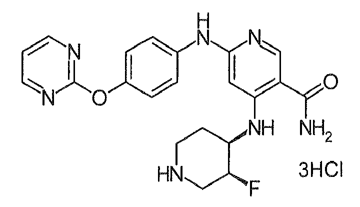 |
| 25-81 | 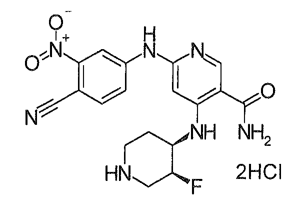 | 25-82 | 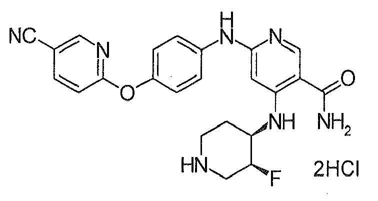 |
| 25-83 | 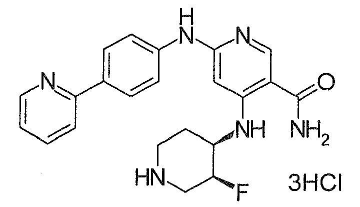 | 25-84 | 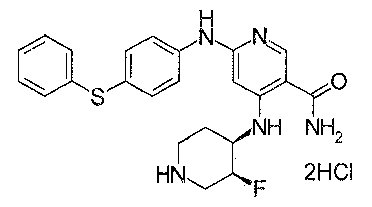 |
| 25-85 | 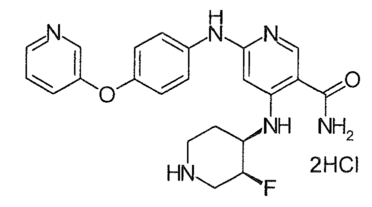 | 25-86 | 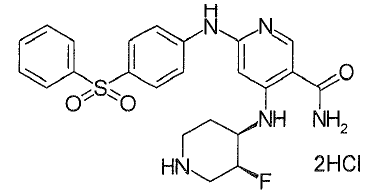 |
| 25-87 | 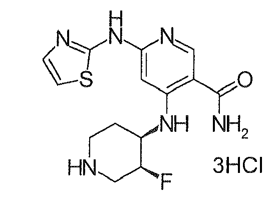 | 25-88 | 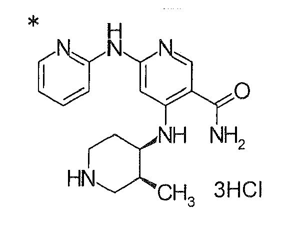 |
| 28-66 | 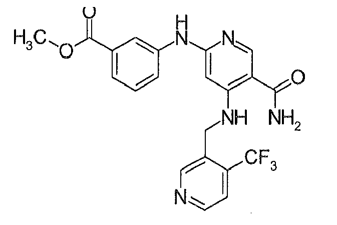 | 28-67 | 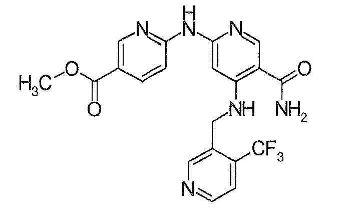 |

**[Table 92]**

| Pr | Strucure | Pr | Strucure |
|---|---|---|---|
| 28-68 | 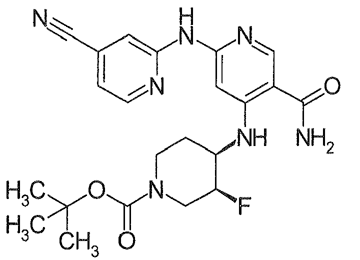 | 28-69 | 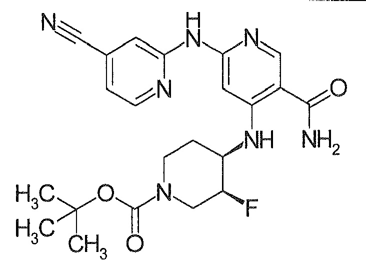 |
| 28-70 | 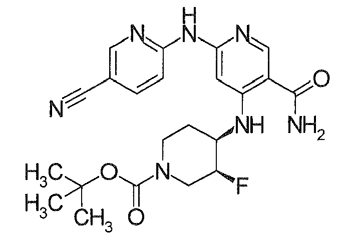 | 28-71 | 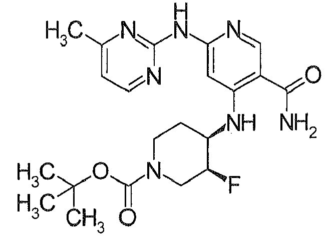 |
| 28-72 | 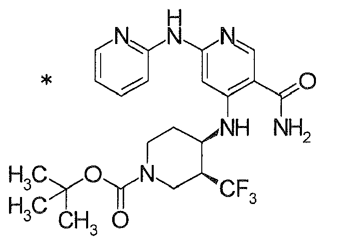 | 28-75 | 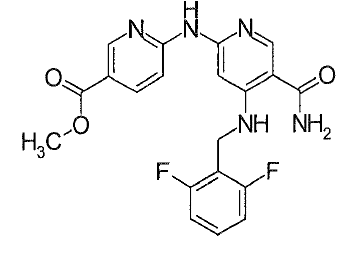 |
| 28-76 | 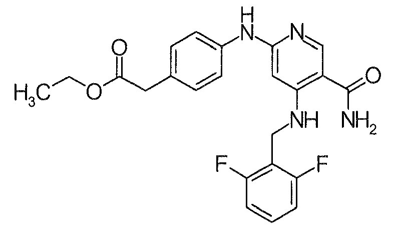 | 28-77 | 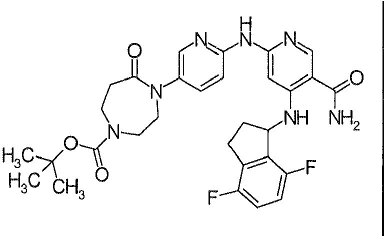 |

**[Table 93]**

| Pr | Strucure | Pr | Strucure |
|---|---|---|---|
| 28-78 | 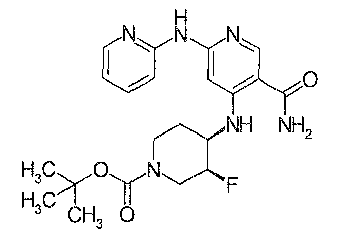 | 28-79 | 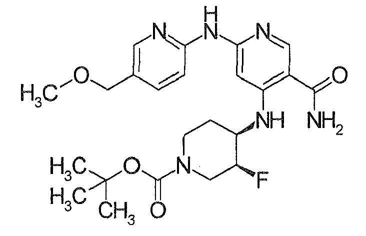 |
| 28-80 | 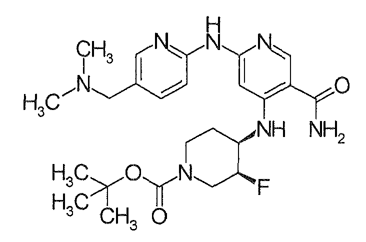 | 28-81 | 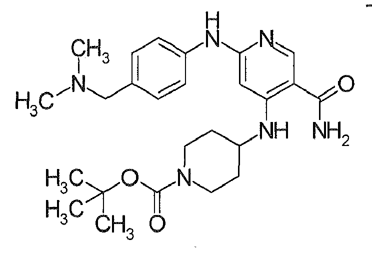 |
| 28-82 | 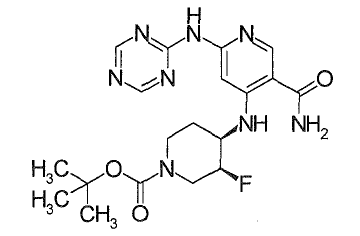 | 28-83 | 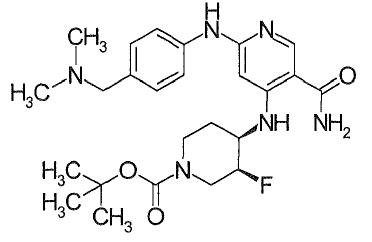 |
| 28-84 | 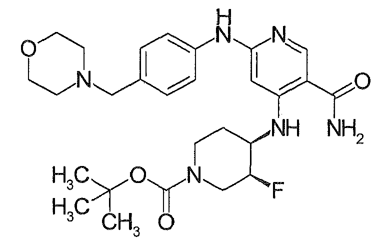 | 28-85 | 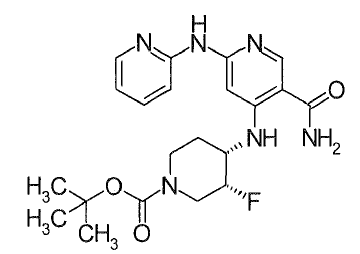 |
| 28-86 | 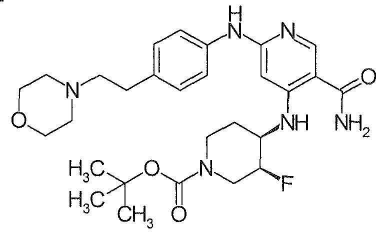 | 28-87 | 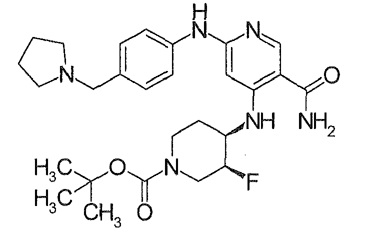 |

**[Table 94]**

| Pr | Strucure | Pr | Strucure |
|---|---|---|---|
| 28-88 | 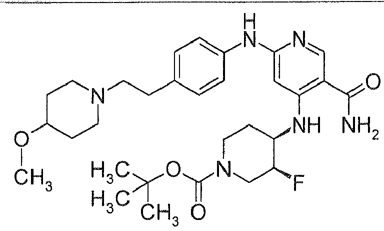 | 28-89 | 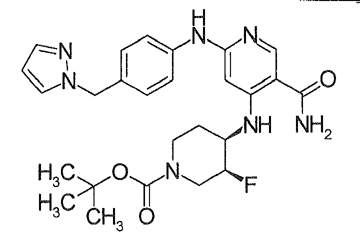 |
| 28-90 | 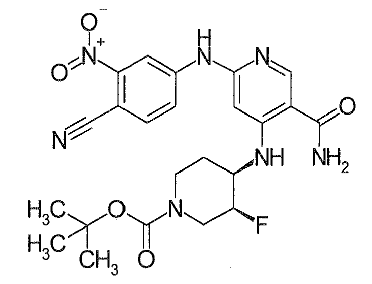 | 28-91 | 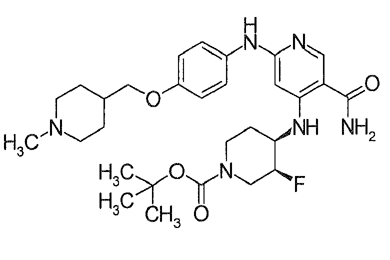 |
| 28-92 | 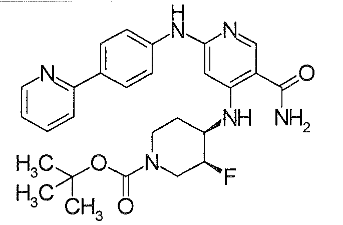 | 28-93 | 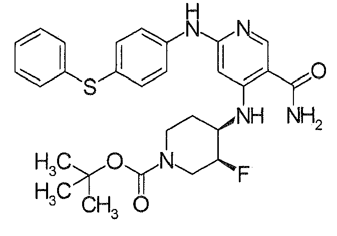 |
| 28-94 | 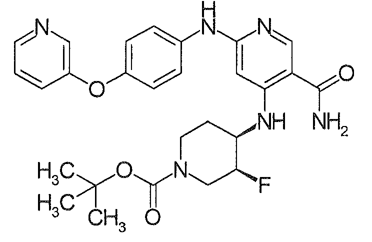 | 28-95 | 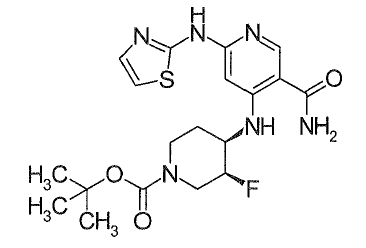 |
| 28-96 | 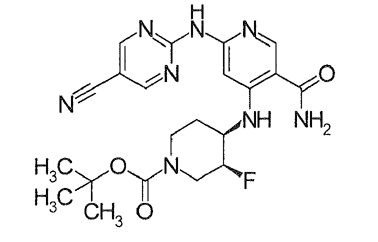 | 28-97 | 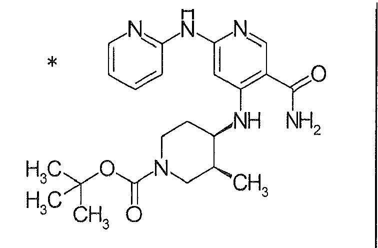 |

**[Table 95]**

| Pr | Strucure | Pr | Strucure |
|---|---|---|---|
| 30-1 | 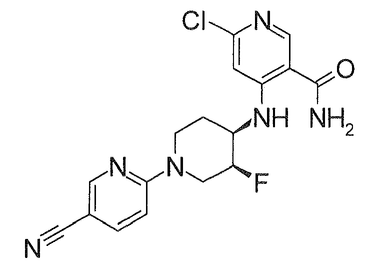 | 30-2 | 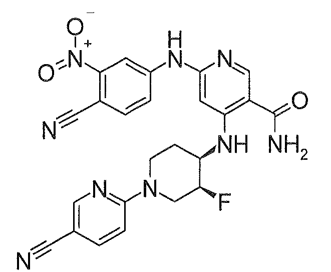 |
| 30-3 | 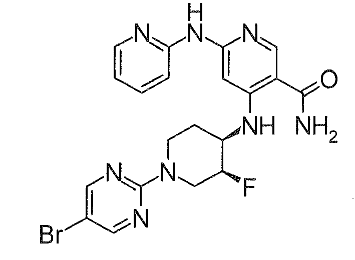 | 32-6 | 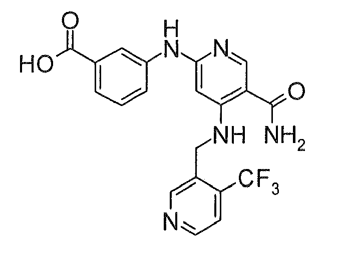 |
| 32-7 | 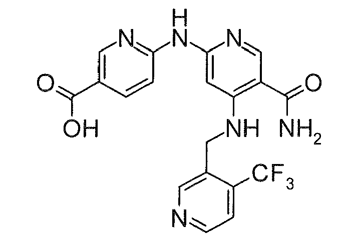 | 32-8 | 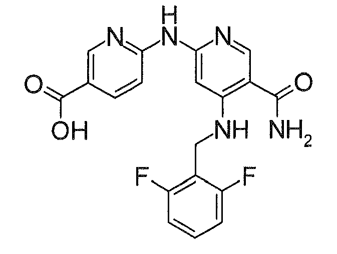 |
| 32-9 | 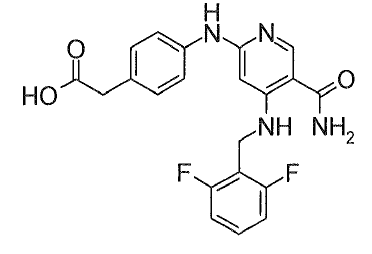 | 34 | 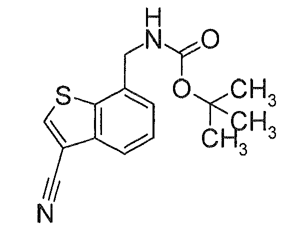 |
| 35 | 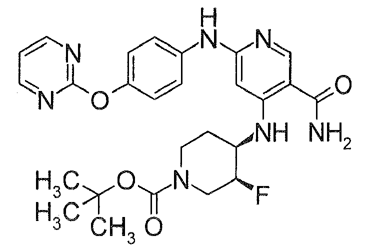 | 36 | 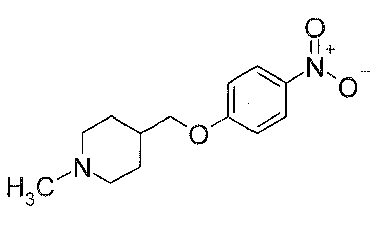 |

**[Table 96]**

| Pr | Strucure | Pr | Strucure |
|---|---|---|---|
| 37 | 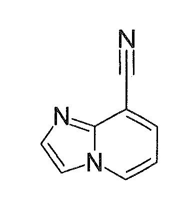 | 48 | 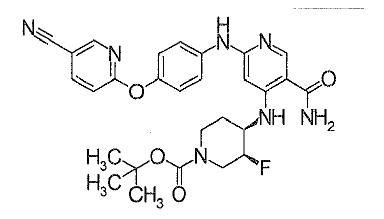 |

**[Table 97]**

| Pr | DATA |
|---|---|
| 1-2 | ESI+ : 286 [M+H]+ |
| 1-3 | ESI+ : 364, 366 [M+Na]+ |
| 2-1 | ESI+ : 242 [M+H]+ |
| 3-1 | ESI+ : 151 [M+H]+ |
| 4-1 | ESI+ : 148[M+H]+ |
| 6-1 | ESI+ : 271 [M+H]+ |
| 6-2 | ESI+ : 237 [M+H]+ |
| 10-1 | ESI+ : 592 [M+Na]+ |
| 14-58 | ESI+ : 353, 355 [M+Na]+ |
| 14-59 | ESI+ : 452 [M+H]+ |
| 14-62 | ESI+ : 391 [M+Na]+ |
| 14-63 | ESI+ : 330 [M+H]+ |
| 14-64 | ESI+ : 298 [M+H]+ |
| 14-65 | ESI+ : 387 [M+H]+ |
| 14-66 | ESI+ : 424 [M+Na]+ |
| 14-67 | ESI+ : 288 [M+H]+ |
| 14-68 | ESI+ : 288 [M+H]+ |
| 14-69 | ESI+ : 306 [M+H]+ |
| 14-70 | ESI+ : 306 [M+H]+ |
| 14-71 | ESI+ : 302 [M+H]+ |
| 14-72 | ESI+ : 324 [M+H]+ |
| 14-73 | ESI- : 341 [M-H]- |
| 49 | ESI- : 312 [M-H]- |
| 14-74 | ESI+ : 208 [M+Na]+ |
| 18-1 | ESI- : 166 [M-H]- |
| 19-19 | ESI+ : 424 [M+H]+ |
| 19-20 | ESI+ : 396 [M+Na]+ |
| 20-1 | ESI+ : 359 [M+H]+ |
| 40-1 | NMR-DMSO-d₆: 1.55-1.66 (1H, m), 2.31-2.39 (1H, m), 2.61-2.70 (1H, m), 2.77-2.84 (1H, m), 3.31 (2H, br), 4.16 (1H, t, J = 8.0 Hz), 6.91-6.96 (1H, m), 7.11-7.20 (2H, m) |
| 40-2 | NMR-DMSO-d₆: 1.59-1.67 (1H, m), 2.33-2.41 (1H, m), 2.64-2.72 (1H, m), 2.87-2.94 (1H, m), 3.32 (2H, brs), 4.22 (1H, t, J = 8.0 Hz), 6.93-6.98 (1H, m), 7.17-7.25 (2H, m) |
| 40-3 | ESI+ : 170 [M+H]+ |
| 47 | ESI+ : 181 [M+H]+ |
| 21-2 | ESI+ : 258 [M+Na]+ |
| 22-20 | ESI+ : 445 [M+Na]+ |
| 22-21 | ESI+ : 395 [M+Na]+ |
| 23-8 | ESI+ : 291 [M+Na]+ |
| 23-9 | ESI+ : 207 [M+H]+ |
| 23-10 | ESI+ : 241 [M+H]+ |
| 23-11 | ESI+ : 221 [M+H]+ |
| 24-1 | ESI+ : 186 [M+Na]+ |
| 25-59 | ESI+ : 356 [M+H]+, |
| 25-60 | ESI+ : 355 [M+H]+, |

**[Table 98]**

| Pr | DATA |
|---|---|
| 25-61 | ESI+ : 346 [M+H]+, |
| 25-62 | ESI+ : 356 [M+H]+, |
| 25-63 | |
| 25-65 | ESI+ : 357 [M+H]+ |
| 46 | ESI+: 494 [M+H]+ |
| 44-1 | ESI+ : 189 [M+H]+ |
| 25-66 | ESI+ : 331 [M+H]+ |
| 25-67 | NMR-DMSO-d₆: 1.81-1.92 (1H, m), 2.11-2.18 (1H, m), 3.05-3.15 (1H, m), 3.29-3.39 (4H, m), 3.96-4.11 (1H, m), 4.44 (2H, s), 5.12-5.26 (1H, m), 6.75 (1H, s), 7.22 (1H, d, J = 8.8 Hz), 7.82 (1H, br), 7.88 (1H, dd, J = 2.4, 8.8 Hz), 8.31 (1H, d, J = 2.0 Hz), 8.42 (1H, br), 8.51 (1H, s), 8.86-8.94 (1H, m), 9.59-9.67 (2H, m), 11.95 (1H, s), 14.55 (1H, br) |
| 25-68 | NMR-DMSO-d₆: 1.82-1.93 (1H, m), 2.11-2.18 (1H, m), 2.66-2.77 (7H, m), 3.05-3.15 (1H, m), 3.66-3.74 (2H, m), 3.98-4.12 (1H, m), 4.32 (2H, d, J = 5.2 Hz), 5.15-5.26 (1H, m), 6.83 (1H, s), 7.30 (1H, d, J = 8.8 Hz), 7.84 (1H, m), 8.10 (1H, dd, J = 2.4, 8.8 Hz), 8.46 (1H, m), 8.51 (1H, d, J = 2.0 Hz), 8.53 (1H, s), 8.85-8.94 (1H, m), 9.61-9.69 (2H, m), 11.02 (1H, m), 12.15 (1H, m) |
| 25-69 | ESI+ : 369 [M+H]+ |
| 25-70 | ESI+ : 333 [M+H]+ |
| 25-71 | ESI+ : 387 [M+H]+ |
| 25-72 | ESI+ : 429 [M+H]+ |
| 25-73 | ESI+ : 331 [MI+H]+ |
| 25-74 | ESI+ : 443 [M+H]+ |
| 25-75 | ESI+ : 413 [M+H]+ |
| 25-76 | ESI+ : 471 [M+H]+ |
| 25-77 | ESI+ : 273 [M+H]+ |
| 25-78 | ESI+ : 410 [M+H]+ |
| 25-79 | ESI+ : 457 [M+H]+ |
| 25-80 | ESI+ : 424 [M+H]+ |
| 25-81 | ESI+ : 400 [M+H]+ |
| 25-82 | ESI+ : 448 [M+H]+ |
| 25-83 | ESI+ : 407 [M+H]+ |
| 25-84 | ESI+ : 438 [M+H]+ |
| 25-85 | ESI+ : 423 [M+H]+ |
| 25-86 | ESI+ : 470 [M+H]+ |
| 25-87 | ESI+ : 337 [M+H]+ |
| 25-88 | ESI+ : 327 [M+H]+ |
| 28-66 | NMR-DMSO-d₆ : 3.84 (3H, s), 4.64 (2H, d, J = 5.7 Hz), 5.81 (1H, s), 7.18 (1H, br s), 7.35 (1H, t, J = 7.9 Hz), 7.46 (1H, d, J = 7.8 Hz), 7.79-7.87 (3H, m), 8.25 (1H, t, J =1.6 Hz), 8.46 (1H, s), 8.72 (1H, s), 8.80 (1H, d, J = 5.0 Hz), 9.11-9.15 (2H, m) |
| 28-67 | NMR-DMSO-d₆: 3.83 (3H, s), 4.72 (2H, d, J = 6.0 Hz), 7.22-7.28 (2H, m), 7.56 (1H, d, J = 8.9 Hz), 7.79 (1H, d, J = 5.1 Hz), 7.93 (1H, br s), 8.07 (1H, dd, J = 8.9, 2.4 Hz), 8.49 (1H, s), 8.65 (1H, dd, J = 2.4, 0.4 Hz), 8.77 (1H, d, J = 5.0 Hz), 8.80 (1H, s), 9.28 (1H, t, J = 6.2 Hz), 10.13 (1H, s) ESI+ : 447 [M+H]+ |
| 28-68 | ESI+ : 456 [M+H]+ |
| 28-69 | ESI+ : 455 [M+H]+ |

**[Table 99]**

| Pr | DATA |
|---|---|
| 28-70 | ESI+ : 456 [M+H]+, |
| 28-71 | ESI+ : 456 [M+H]+, |
| 28-72 | ESI+ : 481 [M+H]+ |
| 28-75 | ESI+ : 414 [M+H]+ |
| 28-76 | ESI+ : 441 [M+H]+ |
| 28-77 | ESI+ : 594 [M+H]+ |
| 28-78 | ESI+ : 431 [M+H]+ |
| 28-79 | ESI+ : 475 [M+H]+ |
| 28-80 | ESI+ : 488 [M+H]+ |
| 28-81 | ESI+ : 469 [M+H]+ |
| 28-82 | ESI+ : 455 [M+Na]+ |
| 28-83 | ESI+ : 487 [M+H]+ |
| 28-84 | ESI+ : 529 [M+H]+ |
| 28-85 | ESI+ : 431 [M+H]+ |
| 28-86 | ESI+ : 543 [M+H]+ |
| 28-87 | ESI+ : 513 [M+H]+ |
| 28-88 | ESI+ : 571 [M+H]+ |
| 28-89 | ESI+ : 532 [M+Na]+ |
| 28-90 | ESI+ : 522 [M+Na]+ |
| 28-91 | ESI+ : 557 [M+H]+ |
| 28-92 | ESI+ : 507 [M+H]+ |
| 28-93 | ESI+ : 560 [M+Na]+ |
| 28-94 | ESI+ : 545 [M+Na]+ |
| 28-95 | ESI+ : 437 [M+H]+ |
| 28-96 | ESI+ : 457 [M+H]+ |
| 28-97 | ESI+ : 427 [M+H]+ |
| 30-1 | ESI+ : 397 [M+Na]+ |
| 30-2 | NMR-DMSO-d₆: 1.59-1.70 (1H, m), 1.97-2.04 (1H, m), 2.28-2.36 (1H, m), 3.36-3.45 (1H, m), 3.81-3.94 (1H, m), 4.53-4.60 (1H, m), 4.85-5.08 (2H, m), 5.99 (1H, d, J = 2.8 Hz), 6.27 (1H, s), 7.06 (1H, d, J = 9.2 Hz), 7.26 (1H, br), 7.90 (1H, dd, J = 2.4, 9.2 Hz), 7.92-8.02 (2H, m), 8.49 (1H, d, J = 2.0 Hz), 8.54 (1H, s), 9.02-9.05 (2H, m), 10.24 (1H, s) |
| 30-3 | ESI+ : 487, 489 [M+H]+ |
| 32-6 | ESI- : 430 [M-H]- |
| 32-7 | ESI- : 431 [M-H]- |
| 32-8 | ESI+ : 400 [M+H]+ |
| 32-9 | ESI+ : 413 [M+H]+ |
| 34 | ESI+ : 311 [M+Na]+ |
| 35 | ESI+ : 524 [M+H]+ |
| 36 | ESI+ : 251 [M+H]+ |
| 37 | ESI+ : 166 [M+Na]+ |
| 48 | ESI+ : 548 [M+H]+ |

**[Table 100]**

| Ex | Structure | Ex | Structure |
|---|---|---|---|
| 1-4 | 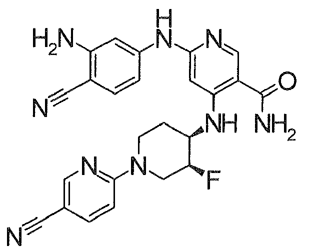 | 5-71 | 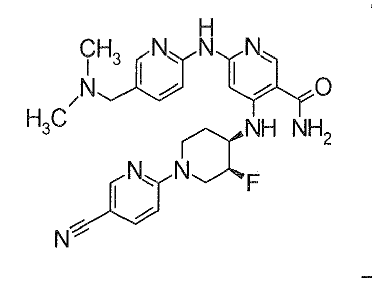 |
| 5-72 | 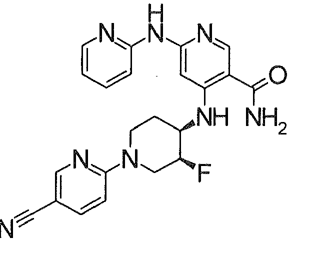 | 5-73 | 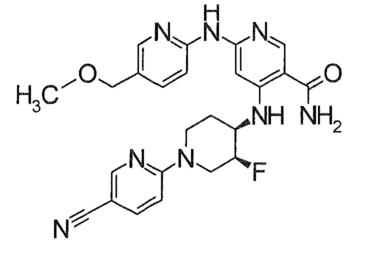 |
| 5-74 | 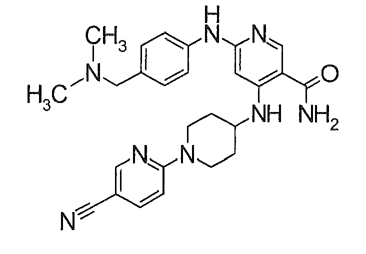 | 5-75 | 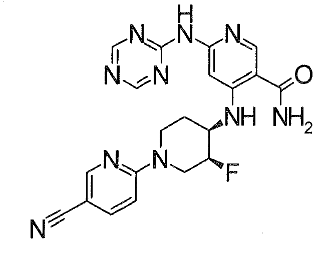 |
| 5-76 | 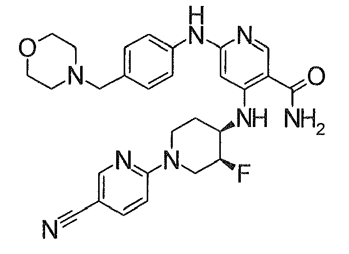 | 5-77 | 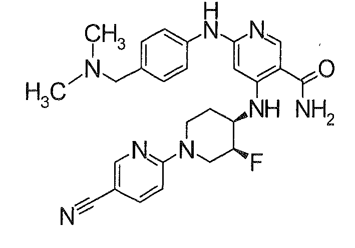 |
| 5-78 | 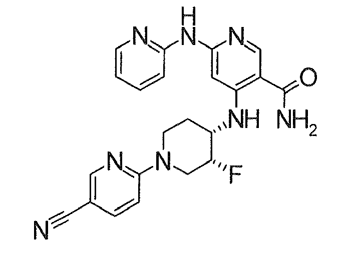 | 5-79 | 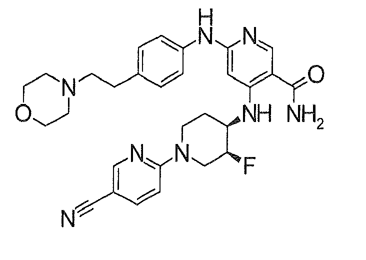 |

**[Table 101]**

| Ex | Structure | Ex | Structure |
|---|---|---|---|
| 5-80 | 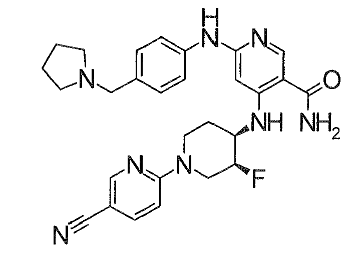 | 5-81 | 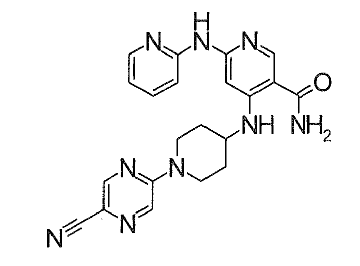 |
| 5-82 | 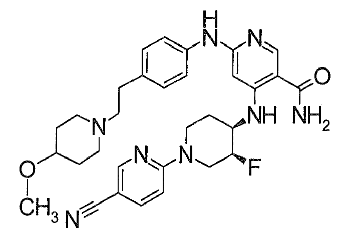 | 5-83 | 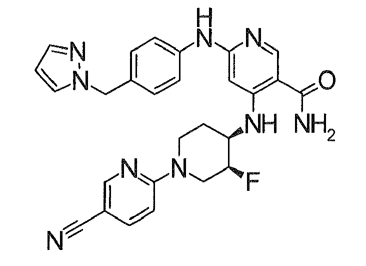 |
| 5-84 | 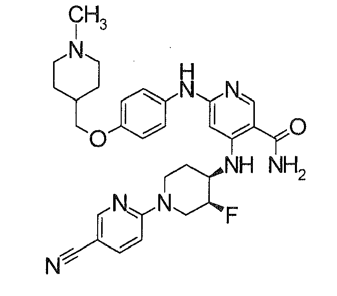 | 5-85 | 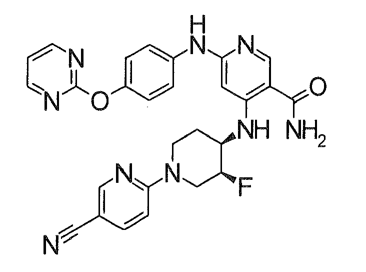 |
| 5-86 | 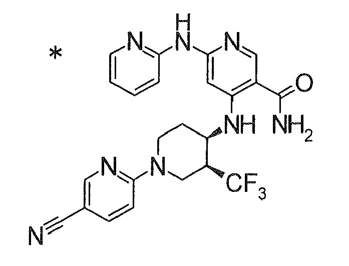 | 5-87 | 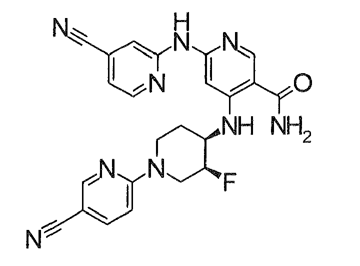 |

**[Table 102]**

| Ex | Structure | Ex | Structure |
|---|---|---|---|
| 5-88 | 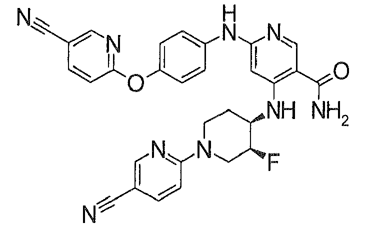 | 5-89 | 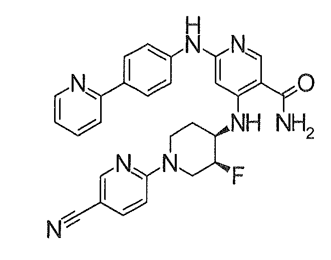 |
| 5-90 | 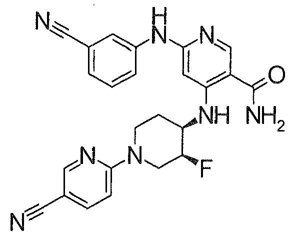 | 5-91 | 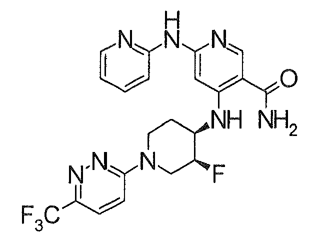 |
| 5-92 | 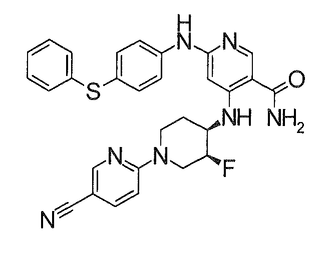 | 5-93 | 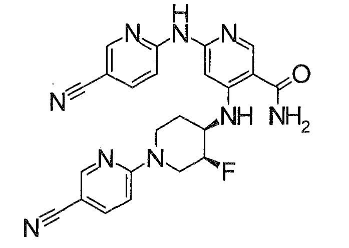 |
| 5-94 | 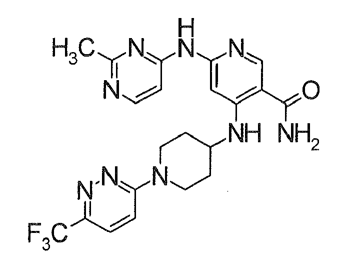 | 5-95 | 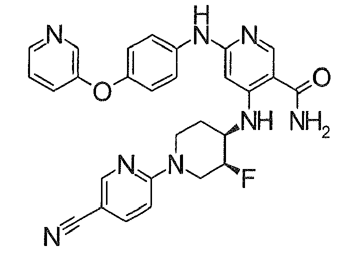 |
| 5-96 | 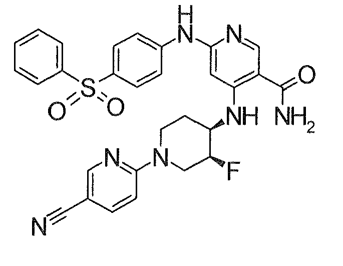 | 5-97 | 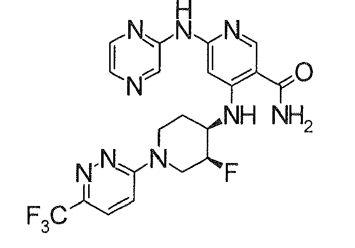 |

**[Table 103]**

| Ex | Structure | Ex | Structure |
|---|---|---|---|
| 5-98 | 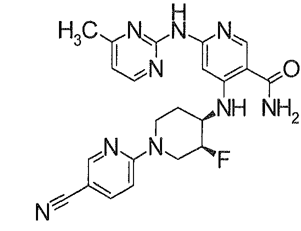 | 5-99 | 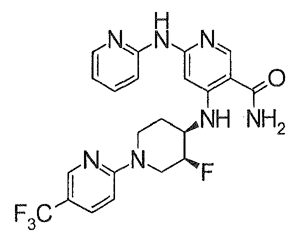 |
| 5-100 | 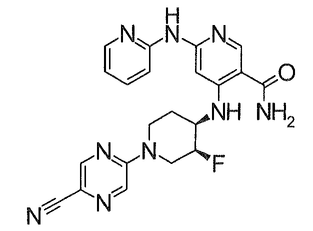 | 5-101 | 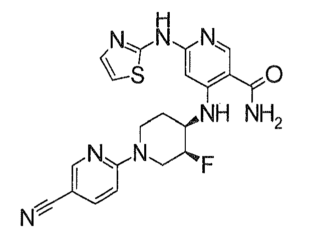 |
| 5-102 | 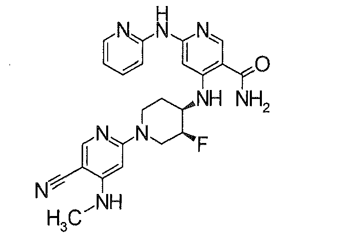 | 5-103 | 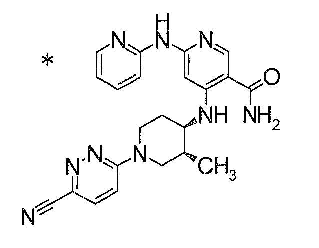 |
| 5-104 | 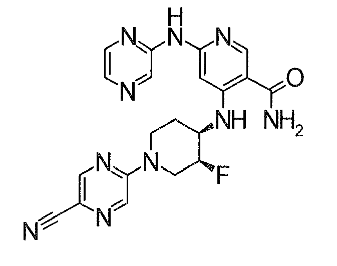 | 5-105 | 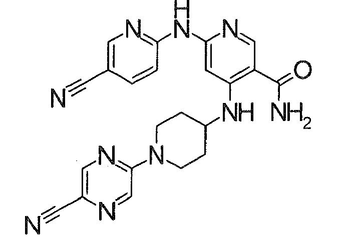 |
| 5-106 | 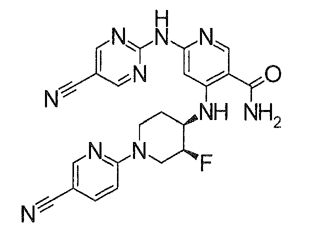 | 5-107 | 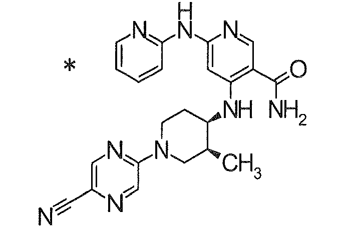 |

**[Table 104]**

| Ex | Structure | Ex | Structure |
|---|---|---|---|
| 6-1 | 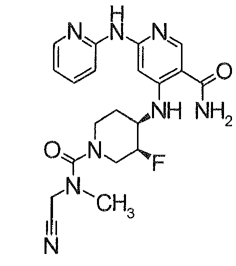 | 6-2 | 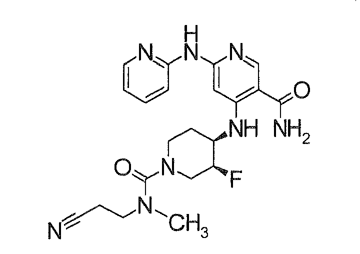 |
| 15 | 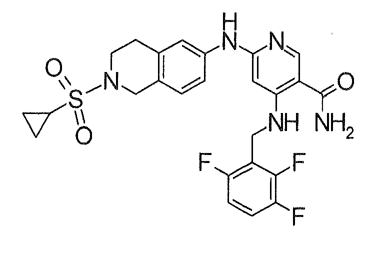 | 8-40 | 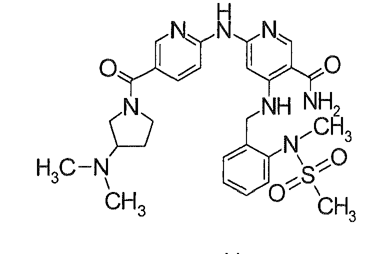 |
| 8-41 | 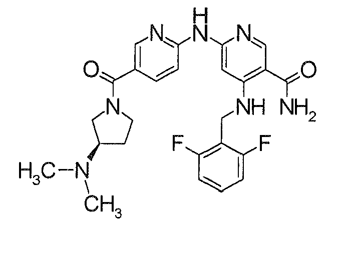 | 8-42 | 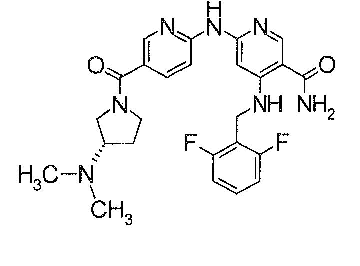 |
| 8-43 | 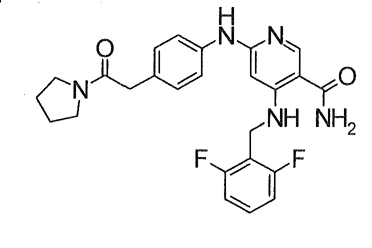 | 8-44 | 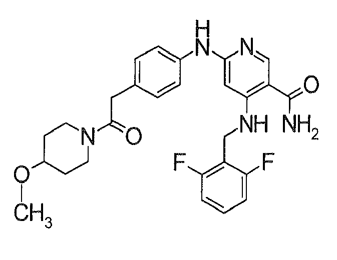 |

**[Table 105]**

| Ex | Structure | Ex | Structure |
|---|---|---|---|
| 8-45 | 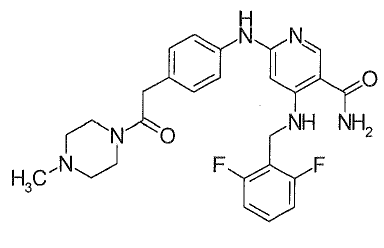 | 8-46 | 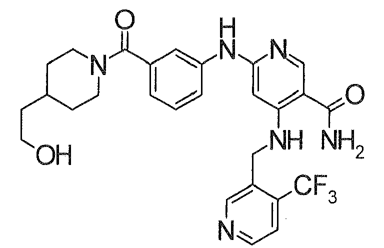 |
| 8-47 | 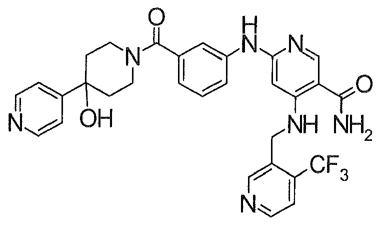 | 8-48 | 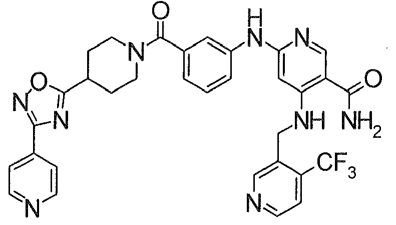 |
| 8-49 | 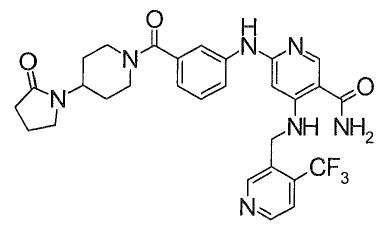 | 12-108 | 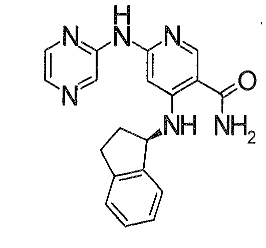 |
| 12-109 | 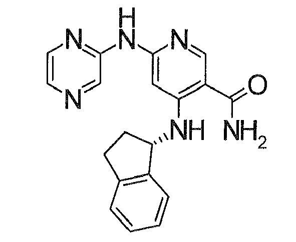 | 12-110 | 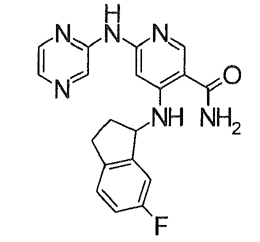 |
| 12-111 | 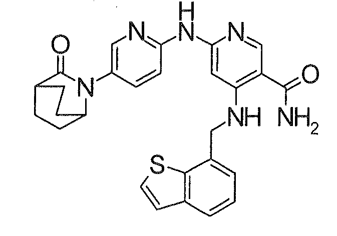 | 12-112 | 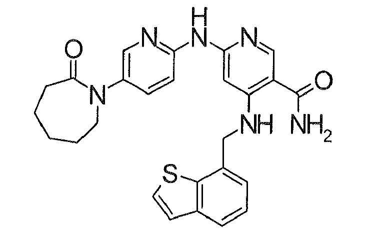 |

**[Table 106]**

| Ex | Structure | Ex | Structure |
|---|---|---|---|
| 12-113 | 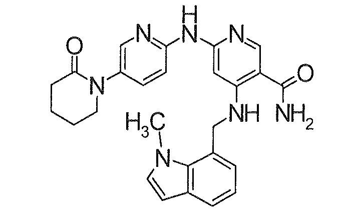 | 12-114 | 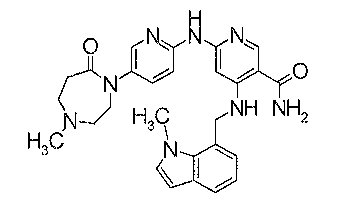 |
| 12-115 | 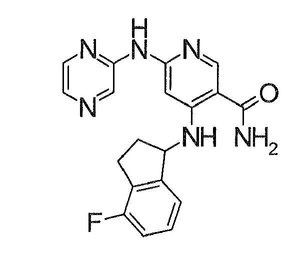 | 12-116 | 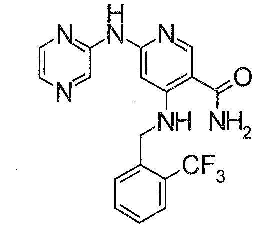 |
| 12-117 | 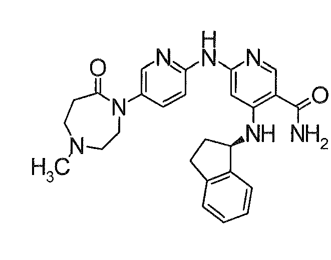 | 12-118 | 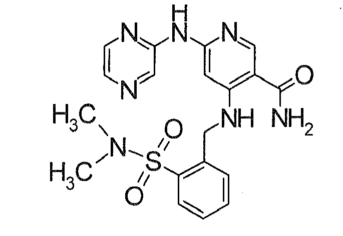 |
| 12-119 | 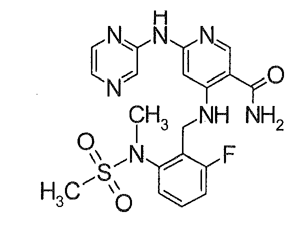 | 12-120 | 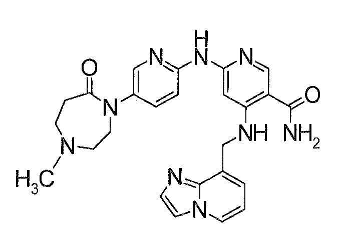 |
| 12-121 | 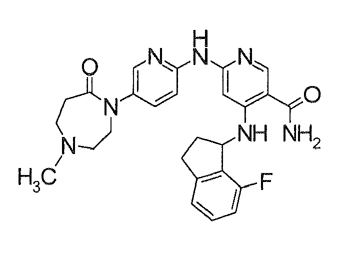 | 12-122 | 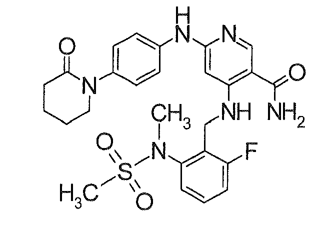 |

**[Table 107]**

| Ex | Structure | Ex | Structure |
|---|---|---|---|
| 12-123 | 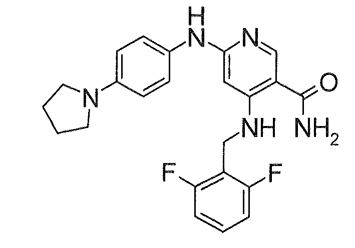 | 12-124 | 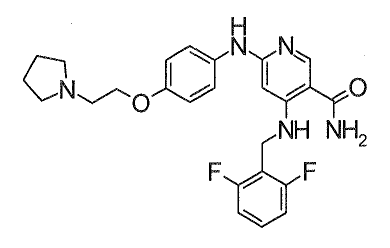 |
| 12-125 | 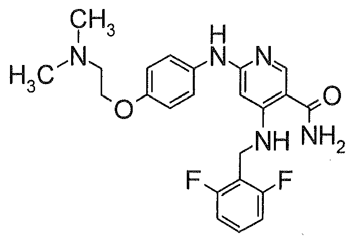 | 12-126 | 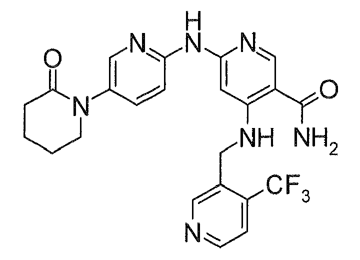 |
| 12-127 | 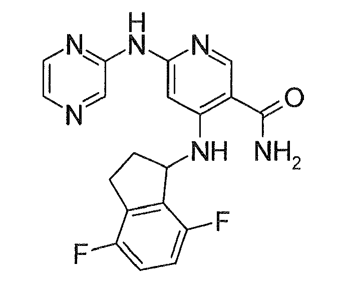 | 12-128 | 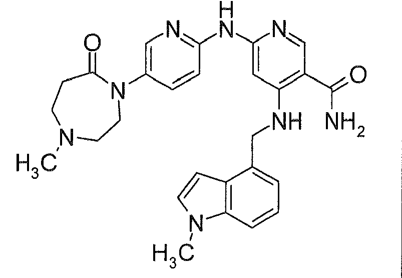 |
| 12-129 | 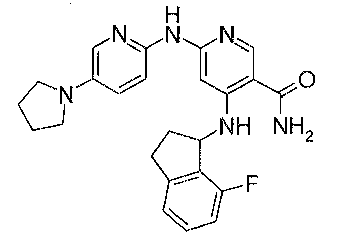 | 12-130 | 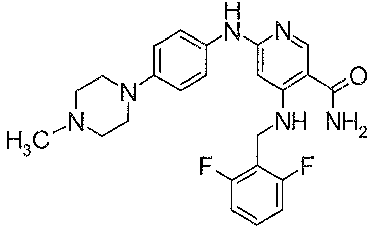 |
| 12-131 | 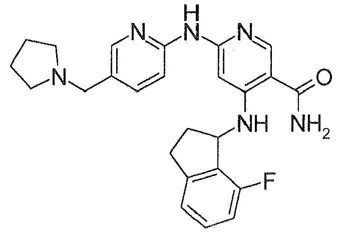 | 12-132 | 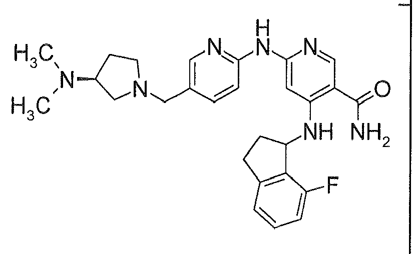 |

**Table 108**

| Ex | Structure | Ex | Structure |
|---|---|---|---|
| 12-133 | 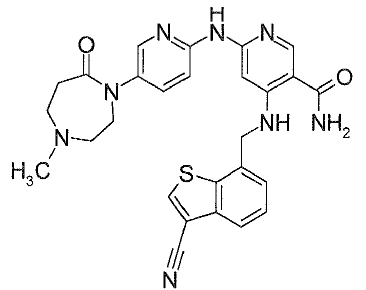 | 12-134 | 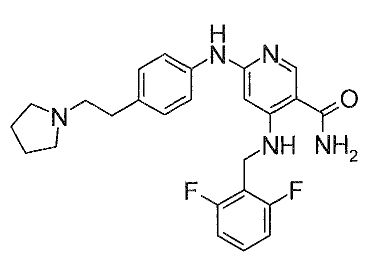 |
| 12-135 | 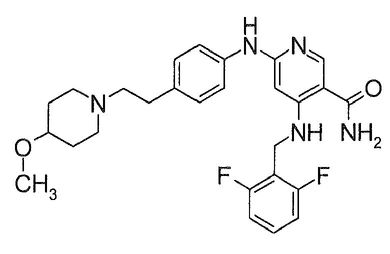 | 12-136 | 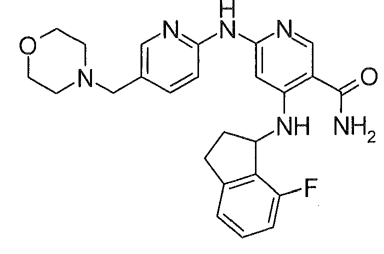 |
| 12-137 | 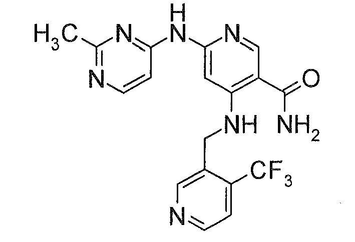 | 12-138 | 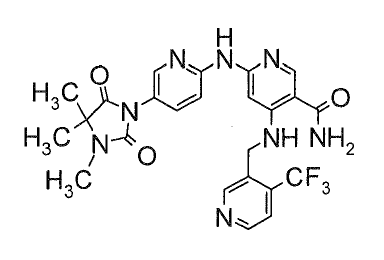 |
| 12-139 | 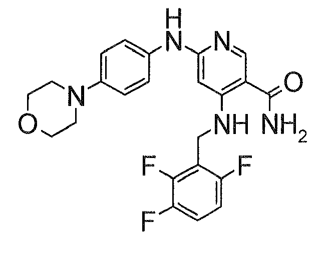 | 12-140 | 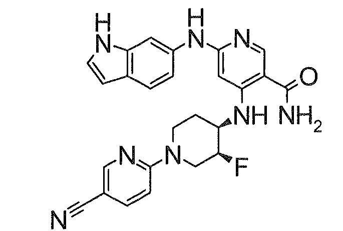 |
| 12-141 | 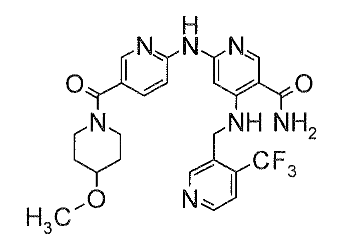 | 12-142 | 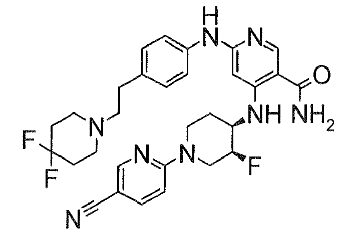 |

**[Table 109]**

| Ex | Structure | Ex | Structure |
|---|---|---|---|
| 12-143 | 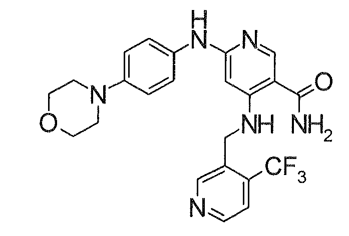 | 12-144 | 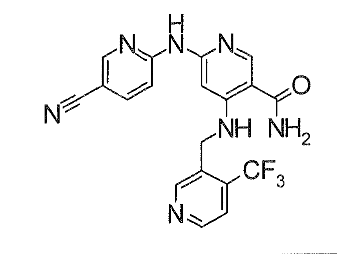 |
| 12-145 | 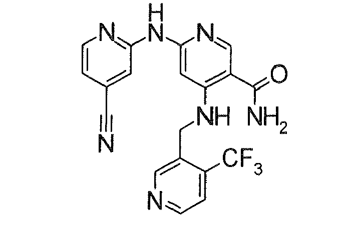 | 12-146 | 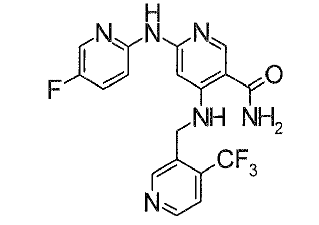 |
| 12-147 | 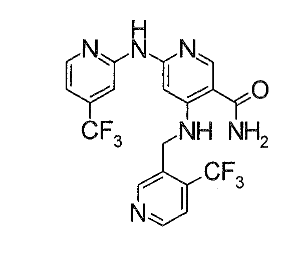 | 12-148 | 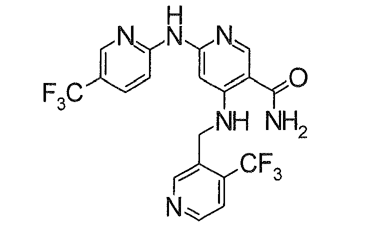 |
| 12-149 | 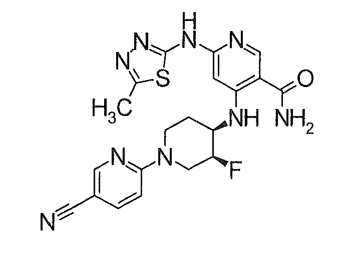 | 12-150 | 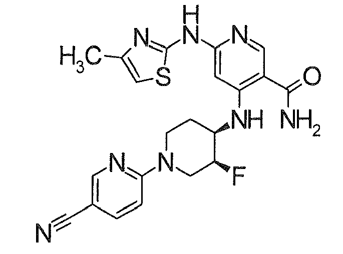 |
| 12-151 | 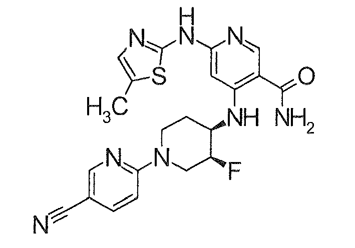 | 13 | 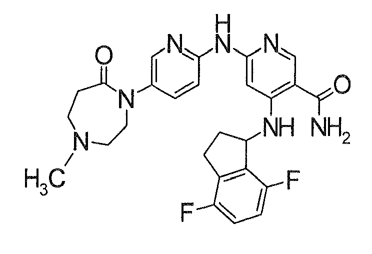 |

**[Table 110]**

| Ex | Structure |
|---|---|
| 14 | 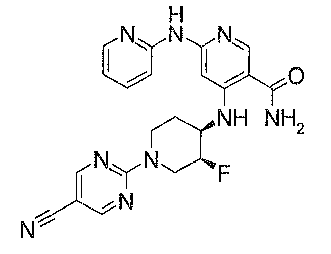 |

**[Table 111]**

| Ex | DATA |
|---|---|
| 1-4 | NMR-DMSO-d₆: 1.54-1.65 (1H, m), 1.96-2.03 (1H, m), 3.08-3.16 (1H, m), 3.37-3.49 (1H, m), 3.75-3.89 (1H, m), 4.52-4.56 (1H, m), 4.82-5.06 (2H, m), 5.02 (2H, brs), 6.14 (1H, s), 6.87 (1H, dd, J = 2.0, 8.8 Hz), 7.04 (1H, d, J = 9.2 Hz), 7.13 (1H, br), 7.18 (1H, d, J = 2.0 Hz), 7.22 (1H, d, J = 8.8 Hz), 7.82 (1H, br), 7.86 (1H, dd, J = 2.4, 9.2 Hz), 8.41 (1H, s), 8.49 (1H, d, J = 2.4 Hz), 8.91 (1H, d, J = 8.4 Hz), 9.14 (1H, s) ESI+ : 494 [M+Na]+ |
| 5-71 | NMR-DMSO-d₆: 1.58-1.69 (1H, m), 2.01-2.17 (7H, m), 2.29-2.34 (1H, m), 2.67 (2H, s), 3.13-3.21 (1H, m), 3.78-3.90 (1H, m), 4.55-4.60 (1H, m), 4.83-5.08 (2H, m), 7.05 (1H, d, J = 7.6 Hz), 7.11 (1H, br), 7.31 (1H, s), 7.54-7.62 (2H, m), 7.79 (1H, br), 7.86 (1H, dd, J = 2.4, 8.8 Hz), 8.12-8.13 (1H, m), 8.43 (1H, s), 8.49 (1H, d, J = 2.0 Hz), 8.93 (1H, d, J = 8.0 Hz), 9.49 (1H, s) ESI+ : 490 [M+H]+ |
| 5-72 | NMR-DMSO-d₆: 1.56-1.67 (1H, m), 2.01-2.08 (1H, m), 3.13-3.20 (1H, m), 3.35-3.48 (1H, m), 3.79-3.92 (1H, m), 4.55-4.61 (1H, m), 4.85-5.10 (2H, m), 6.88 (1H, m), 7.05 (1H, d, J = 8.8 Hz), 7.10 (1H, br), 7.27 (1H, m), 7.62-7.68 (2H, m), 7.79 (1H, br), 7.87 (1H, dd, J = 2.4, 8.8 Hz), 8.24 (1H, d, J = 4.4 Hz), 8.43 (1H, s), 8.49 (1H, d, J = 2.0 Hz), 8.92-8.95 (1H, m), 9.48 (1H, brs) ESI+ : 433 [M+H]+ |
| 5-73 | NMR-DMSO-d₆ 1.57-1.67 (1H, m), 2.01-2.07 (1H, m), 3.14-3.22 (1H, m), 3.27 (3H, s), 3.36-3.48 (1H, m), 3.77-3.92 (1H, m), 4.33 (2H, s), 4.55-4.63 (1H, m), 4.84-5.10 (2H, m), 7.06 (1H, d, J = 8.8 Hz), 7.11 (1H, br), 7.30-7.32 (1H, m), 7.60-7.65 (2H, m), 7.81 (1H, br), 7.87 (1H, dd, J = 2.4, 8.8 Hz), 8.20 (1H, s), 8.44 (1H, s), 8.49 (1H, d, J = 2.0 Hz), 8.94 (1H, d, J = 7.6 Hz), 9.55 (1H, brs) ESI+ : 477 [M+H]+ |
| 5-74 | NMR-DMSO-d₆: 1.34-1.46 (2H, m), 2.02-2.08 (2H, m), 2.11 (6H, s), 2.67 (2H, s), 3.55-3.64 (1H, m), 4.24-4.31 (2H, m), 6.02 (1H, s), 6.55 (1H, s), 6.99 (1H, d, J = 8.8 Hz), 7.01 (1H, br), 7.14 (2H, d, J = 8.8 Hz), 7.56 (2H, d, J = 8.8 Hz), 7.72 (1H, br), 7.85 (1H, dd, J = 2.4, 8.8 Hz), 8.37 (1H, s), 8.49 (1H, d, J = 2.0 Hz), 8.60 (1H, s), 8.69 (1H, d, J = 7.2 Hz), 8.91 (1H, s) ESI+: 471 [M+H]+ |
| 5-75 | NMR-DMSO-d₆: 1.57-1.69 (1H, m), 1.96-2.08 (1H, m), 2.27-2.38 (1H, m), 3.17-3.26 (1H, m), 3.88-4.02 (1H, m), 4.53-4.60 (1H, m), 4.83-5.11 (2H, m), 7.04 (1H, d, J = 9.2 Hz), 7.26-7.33 (1H, m), 7.67 (1H, s), 7.88 (1H, dd, J = 2.4, 9.2 Hz), 7.94-8.02 (1H, m), 8.49-8.50 (2H, m), 8.85-8.86 (2H, m), 9.06 (1H, d, J = 8.0 Hz), 10.50 (1H, s) ESI+ : 435 [M+H]+ |
| 5-76 | NMR-DMSO-d₆ 1.55-1.65 (1H, m), 1.95-2.03 (1H, m), 2.30-2.37 (4H, m), 2.53-2.57 (1H, m), 3.08-3.16 (1H, m), 3.37 (2H, s), 3.54-3.60 (4H, m), 3.75-3.88 (1H, m), 4.53-4.59 (1H, m), 4.82-5.05 (2H, m), 6.05 (1H, s), 7.00 (1H, br), 7.05 (1H, d, J = 9.2 Hz), 7.17 (2H, d, J = 8.8 Hz), 7.56 (2H, d, J = 8.8 Hz), 7.74 (1H, br), 7.86 (1H, dd, J = 2.4, 9.2 Hz), 8.38 (1H, s), 8.49 (1H, d, J = 2.0 Hz), 8.88 (1H, d, J = 8.0 Hz), 8.92 (1H, s) ESI+ : 531 [M+H]+ |
| 5-77 | NMR-DMSO-d₆: 1.54-1.66 (1H, m), 1.96-2.02 (1H, m), 2.15 (6H, s), 2.30-2.34 (1H, m), 2.67 (2H, s), 3.08-3.16 (1H, m), 3.74-3.88 (1H, m), 4.53-4.59 (1H, m), 4.81-5.05 (2H, m), 6.05 (1H, s), 7.02 (1H, br), 7.05 (1H, d, J = 8.8 Hz), 7.16 (2H, d, J = 8.4 Hz), 7.56 (2H, d, J = 8.4 Hz), 7.75 (1H, br), 7.86 (1H, dd, J = 2.4, 8.8 Hz), 8.39 (1H, s), 8.49 (1H, d, J = 2.0 Hz), 8.88 (1H, d, J = 8.4 Hz), 8.92 (1H, s) ESI+ : 489 [M+H]+ |

**[Table 112]**

| Ex | DATA |
|---|---|
| 5-78 | NMR-DMSO-d₆: 1.57-1.67 (1H, m), 2.02-2.08 (1H, m), 3.13-3.21 (1H, m), 3.35-3.50 (1H, m), 3.78-3.92 (1H, m), 4.55-4.61 (1H, m), 4.85-5.10 (2H, m), 6.86-6.91 (1H, m), 7.05 (1H, d, J = 9.2 Hz), 7.13 (1H, br), 7.23-7.28 (1H, m), 7.62-7.68 (2H, m), 7.81 (1H, br), 7.86 (1H, dd, J = 2.4, 9.2 Hz), 8.25 (1H, d, J = 9.2 Hz), 8.44 (1H, s), 8.49-8.50 (1H, m), 8.92-8.97 (1H, m), 9.50 (1H, brs) ESI+ : 433 [M+H]+ |
| 5-79 | NMR-DMSO-d₆: 1.54-1.66 (1H, m), 1.95-2.02 (1H, m), 2.37-2.47 (6H, m), 2.63-2.71 (2H, m), 3.08-3.16 (1H, m), 3.23-3.30 (2H, m), 3.53-3.63 (5H, m), 3.73-3.87 (1H, m), 4.53-4.59 (1H, m), 4.81-5.05 (2H, m), 6.03 (1H, s), 7.01 (1H, br), 7.04 (1H, d, J = 8.8 Hz), 7.10 (2H, d, J = 8.4 Hz), 7.74 (1H, br), 7.86 (1H, dd, J = 2.4, 8.8 Hz), 8.38 (1H, s), 8.49 (1H, d, J = 1.6 Hz), 8.85-8.89 (2H, m) ESI+ : 545 [M+H]+ |
| 5-80 | NMR-DMSO-d₆: 1.54-1.77 (6H, m), 1.96-2.02 (1H, m), 2.11-2.37 (2H, m), 3.07-3.18 (1H, m), 3.23-3.43 (2H, m), 3.52-3.65 (2H, m), 3.74-3.88 (1H, m), 4.52-4.59 (1H, m), 4.82-5.06 (2H, m), 6.05 (1H, s), 7.05 (1H, d, J = 8.8 Hz), 7.07 (1H, br), 7.19 (2H, d, J = 8.8 Hz), 7.57 (2H, d, J = 8.8 Hz), 7.74 (1H, br), 7.86 (1H, dd, J = 2.4, 8.8 Hz), 8.38 (1H, s), 8.49-8.50 (1H, m), 8.88 (1H, d, J = 8.4 Hz), 8.94 (1H, brs) ESI+ : 515 [M+H]+ |
| 5-81 | NMR-DMSO-d₆: 1.42-3.46 (8H, m), 3.62-3.70 (1H, m), 6.83-6.90 (1H, m), 7.07 (1H, brs), 7.26 (1H, s), 7.60-7.69 (2H, m), 7.81 (1H, brs), 8.20-8.24 (1H, m), 8.42 (1H, s), 8.49 (1H, d, J = 1.2 Hz), 8.56 (1H, d, J = 1.2 Hz), 8.75 (1H, d, J = 7.2 Hz), 9.49 (1H, s) ESI+ : 416 [M+H]+ |
| 5-82 | NMR-DMSO-d₆: 1.40-1.66 (2H, m), 1.80-2.01 (4H, m), 2.12-2.40 (2H, m), 2.66-2.81 (6H, m), 3.07-3.16 (1H, m), 3.17-3.27 (2H, m), 3.24 (3H, s), 3.74-3.88 (1H, m), 4.52-4.59 (1H, m), 4.81-5.05 (2H, m), 6.03 (1H, s), 7.04 (1H, d, J = 9.2 Hz), 7.06 (1H, br), 7.10 (2H, d, J = 8.8 Hz), 7.52 (2H, d, J = 8.8 Hz), 7.75 (1H, br), 7.86 (1H, dd, J = 2.4, 9.2 Hz), 8.37 (1H, s), 8.49 (1H, d, J = 2.4 Hz), 8.86-8.90 (2H, m) ESI+ : 573 [M+H]+ |
| 5-83 | NMR-DMSO-d₆: 1.54-1.65 (1H, m), 1.95-2.01 (1H, m), 2.66-2.71 (1H, m), 3.08-3.15 (1H, m), 3.74-3.88 (1H, m), 4.52-4.59 (1H, m), 4.81-5.05 (2H, m), 5.23 (2H, s), 6.05 (1H, s), 6.24-6.25 (1H, m), 7.04 (1H, d, J = 8.8 Hz), 7.07 (1H, br), 7.14 (2H, d, J = 8.8 Hz), 7.43-7.44 (1H, m), 7.58 (2H, d, J = 8.8 Hz), 7.74 (1H, br), 7.75-7.76 (1H, m), 7.86 (1H, dd, J = 2.4, 8.8 Hz), 8.38 (1H, s), 8.59 (1H, d, J = 2.4 Hz), 8.88 (1H, d, J = 8.4 Hz), 8.97 (1H, s) ESI+ : 512 [M+H]+ |
| 5-84 | NMR-DMSO-d₆: 1.23-1.34 (2H, m), 1.54-1.76 (4H, m), 1.83-2.01 (3H, m), 2.17 (3H, s), 2.76-2.82 (2H, m), 3.08-3.15 (1H, m), 3.33-3.45 (1H, m), 3.73-3.85 (3H, m), 4.51-4.58 (1H, m), 5.80-5.04 (2H, m), 5.96 (1H, s), 6.84 (2H, d, J = 9.2 Hz), 6.94 (1H, br), 7.04 (1H, d, J = 9.2 Hz), 7.46 (2H, d, J = 9.2 Hz), 7.70 (1H, br), 7.86 (1H, dd, J = 2.4, 9.2 Hz), 8.35 (1H, s), 8.49 (1H, d, J = 1.6 Hz), 8.72 (1H, s), 8.87 (1H, d, J = 8.0 Hz) ESI+ : 559 [M+H]+ |
| 5-85 | NMR-DMSO-d₆: 1.55-1.66 (1H, m), 1.96-2.04 (1H, m), 3.09-3.18 (1H, m), 3.34-3.44 (1H, m), 3.77-3.90 (1H, m), 4.53-4.61 (1H, m), 4.82-5.06 (2H, m), 6.05 (1H, s), 7.03-7.11 (4H, m), 7.23-7.25 (1H, m), 7.66 (2H, dd, J = 2.0, 6.8 Hz), 7.78 (1H, br), 7.87 (1H, dd, J = 2.4, 8.8 Hz), 8.39 (1H, s), 8.49 (1H, d, J = 2.4 Hz), 8.63 (2H, , J = 4.8 Hz), 8.90 (1H, d, J = 8.0 Hz), 9.02 (1H, s) ESI+ : 526 [M+H]+ |

**[Table 113]**

| Ex | DATA |
|---|---|
| 5-86 | NMR-DMSO-d₆: 1.73-1.98 (2H, m), 2.98-3.23 (1H, m), 3.26.3.66 (2H, m), 3.99-4.23 (2H, m), 4.48-4.58 (1H, m), 6.83-6.91 (1H, m), 7.09 (1H, d, J = 9.0 Hz), 7.02-7.20 (1H, br), 7.22-7.29 (1H, m), 7.61-7.68 (2H, m), 7.77-8.00 (1H, br), 7.93 (1H, dd, J = 2.4, 9.0 Hz), 8.19-8.24 (1H, m), 8.43-8.49 (1H, m), 8.54-8.58 (1H, m), 9.28-9.50 (1H, br), 9.49-9.63 (1H, m) ESI+ : 483 [M+H]+ |
| 5-87 | NMR-DMSO-d₆ : 1.53-1.68 (1H, m), 1.96-2.08 (1H, m), 3.08-3.21 (1H, m), 3.27-3.53 (1H, m), 3.74-3.93 (1H, m), 4.50-4.63 (1H, m), 4.81-5.12 (2H, m), 7.01 (1H, s), 7.03-7.07 (1H, m), 7.20 (1H, brs), 7.25-7.28 (1H, m), 7.84-7.89 (2H, m), 8.25 (1H, s), 8.43-8.52 (3H, m), 8.94-8.99 (1H, m), 9.95 (1H, s) ESI+ : 458 [M+H]+ |
| 5-88 | NMR-DMSO-d₆ 1.55-1.66 (1H, m), 1.97-2.03 (1H, m), 3.09-3.18 (1H, m), 3.34-3.44 (1H, m), 3.77-3.91 (1H, m), 4.53-4.61 (1H, m), 4.82-5.06 (2H, m), 6.05 (1H, s), 7.02-7.11 (4H, m), 7.18 (1H, dd, J = 0.8, 8.8 Hz), 7.66-7.70 (2H, m), 7.77 (1H, br), 7.87 (1H, dd, J = 2.4, 9.2 Hz), 8.29 (1H, dd, J = 2.4, 8.8 Hz), 8.40 (1H, s), 8.49 (1H, d, J = 2.0 Hz), 8.65 (1H, dd, J = 0.8, 2.4 Hz), 8.89 (1H, d, J = 8.4 Hz), 9.05 (1H, s) ESI+: 550 [M+H]+ |
| 5-89 | NMR-DMSO-d₆ : 1.56-1.67 (1H, m), 1.99-2.04 (1H, m), 3.09-3.18 (1H, m), 3.33-3.50 (1H, m), 3.77-3.91 (1H, m), 4.54-4.61 (1H, m), 4.83-5.08 (2H, m), 6.13 (1H, s), 7.05 (1H, d, J = 8.8 Hz), 7.11 (1H, br), 7.24-7.27 (1H, m), 7.76 (2H, d, J = 8.8 Hz), 7.8-7.89 (4H, m), 8.00 (2H, d, J = 8.8 Hz), 8.45 (1H, s), 8.49 (1H, d, J = 2.0 Hz), 8.59-8.61 (1H, m), 8.91 (1H, d, J = 8.4 Hz), 9.19 (1H, s) ESI+: 509 [M+H]+ |
| 5-90 | NMR-DMSO-d₆ : 1.52-1.69 (1H, m), 1.91-2.06 (1H, m), 3.05-3.19 (1H, m), 3.24-3.46 (1H, m), 3.76-3.94 (1H, m), 4.49-4.46 (1H, m), 4.78-5.09 (2H, m), 6.09 (1H, s), 7.01-7.08 (1H, m), 7.16 (1H, brs), 7.26-7.32 (1H, m), 7.41-7.48 (1H, m), 7.71-7.78 (1H, m), 7.81 (1H, brs), 7.83-7.90 (1H, m), 8.33-8.37 (1H, m), 8,46 (1H, s), 8.48-8.52 (1H, m), 8.90-8.97 (1H, m), 9.38 (1H, s) ESI+ : 457 [M+H]+ |
| 5-91 | NMR-DMSO-d₆: 1.65-1.75 (1H, m), 2.06-2.12 (1H, m), 3.21-3.27 (1H, m), 3.42-3.58 (1H, m), 3.85-3.99 (1H, m), 4.65-4.71 (1H, m), 4.89-5.15 (2H, m), 6.86-6.95 (1H, m), 7.22 (1H, br), 7.54 (1H, d, J = 9.6 Hz), 7.59-7.71 (3H, m), 7.83 (1H, d, J = 9.6 Hz), 7.89 (1H, br), 8.25-8.28 (1H, m), 8.44 (1H, s), 8.93-9.03 (1H, m), 9.50 (1H, brs) ESI+: 477 [M+H]+ |
| 5-92 | NMR-DMSO-d₆ : 1.56-1.67 (1H, m), 1.96-2.03 (1H, m), 3.08-3.17 (1H, m), 3.34-3.44 (1H, m), 3.77-3.90 (1H, m), 4.53-4.60 (1H, m), 4.82-5.06 (2H, m), 6.10 (1H, s), 7.05 (1H, d, J = 9.2 Hz), 7.12-7.20 (4H, m), 7.27-7.32 (2H, m), 7.37 (2H, d, J = 8.8 Hz), 7.74 (2H, d, J = 8.8 Hz), 7.79 (1H, br), 7.86 (1H, dd, J = 2.5, 9.2 Hz), 8.42 (1H, s), 8.49 (1H, d, J = 2.0 Hz), 8.91 (1H, d, J = 8.0 Hz), 9.20 (1H, s) ESI- : 538 [M-H]- |
| 5-93 | NMR-DMSO-d₆: 1.55-1.73 (1H, m), 1.96-2.09 (1H, m), 3.10-3.23 (1H, m), 3.25-3.51 (1H, m), 3.79-4.07 (1H, m), 4.52-4.65 (1H, m), 4.81-5.15 (2H, m), 6.79-8.35 (7H, m), 8.39-8.57 (2H, m), 8.69-8.75 (1H, m), 8.88-10.80 (2H, m) ESI+ : 458 [M+H]+ |

**[Table 114]**

| Ex | DATA |
|---|---|
| 5-94 | NMR-DMSO-d₆: 1.46-1.57 (2H, m), 2.15-2.21 (2H, m), 3.32 (3H, s), 3.33-3.39 (2H, m), 3.65-3.72 (1H, m), 4.39-4.46 (2H, m), 7.17 (1H, br), 7.35 (1H, d, J = 5.6 Hz), 7.46-7.49 (2H, m), 7.80 (1H, d, J = 5.6 Hz), 7.87 (1H, br), 8.29 (1H, d, J = 5.6 Hz), 8.45 (1H, s), 8.82 (1H, d, J = 6.8 Hz), 9.95 (1H, s) ESI+ : 474 [M+H]+ |
| 5-95 | NMR-DMSO-d₆ : 1.54-1.68 (1H, m), 1.96-2.02 (1H, m), 3.08-3.17 (1H, m), 3.34-3.44 (1H, m), 3.75-3.91 (1H, m), 4.53-4.60 (1H, m), 4.81-5.06 (2H, m), 6.04 (1H, s), 7.01-7.06 (3H, m), 7.09 (1H, br), 7.33-7.40 (2H, m), 7.67 (2H, d, J = 8.8 Hz), 7.76 (1H, br), 7.86 (1H, dd, J = 2.4, 8.8 Hz), 8.31 (1H, dd, J = 1.6, 8.4 Hz), 8.34 (1H, d, J = 2.4 Hz), 8.39 (1H, s), 8.49-8.50 (1H, m), 8.88-8.95 (1H, m), 9.03 (1H, s) ESI+ : 525 [M+H]+ |
| 5-96 | NMR-DMSO-d₆: 1.55-1.65 (1H, m), 1.95-2.02 (1H, m), 3.07-3.16 (1H, m), 3.34-3.43 (1H, m), 3.77-3.91 (1H, m), 4.53-4.59 (1H, m), 4.82-5.05 (2H, m), 6.16 (1H, s), 7.04 (1H, d, J = 9.2 Hz), 7.16 (1H, br), 7.57-7.67 (4H, m), 7.80-7.92 (7H, m), 8.44 (1H, s), 8.49 (1H, d, J = 2.4 Hz), 8.94 (1H, d, J = 8.4 Hz), 9.57 (1H, s) ESI- : 570 [M-H]- |
| 5-97 | NMR-DMSO-d₆ : 1.63-1.74 (1H, m), 2.04-2.10 (1H, m), 2.58-2.71 (1H, m), 3.42-3.58 (1H, m), 3.83-3.98 (1H, m), 4.64-4.71 (1H, m), 4.89-5.14 (2H, m), 7.18 (1H, s), 7.19 (1H, br), 7.54 (1H, d, J = 9.2 Hz), 7.83 (1H, d, J = 9.2 Hz), 7.87 (1H, br), 8.09 (1H, d, J = 2.4 Hz), 8.26 (1H, dd, J = 1.6, 2.8 Hz), 8.48 (1H, s), 9.00-9.03 (2H, m), 9.87 (1H, s) ESI+: 478 [M+H]+ |
| 5-98 | NMR-DMSO-d₆ 1.55-1.71 (1H, m), 2.04-2.15 (1H, m), 2.43 (3H, m), 3.11-3.24 (1H, m), 3.25-3.52 (1H, m), 3.83-4.03 (1H, m), 4.53-4.67 (1H, m), 4.81-5.14 (2H, m), 6.85-6.90 (1H, m), 7.02-7.07 (1H, m), 7.14 (1H, brs), 7.79-7.94 (3H, m), 8.40-8.46 (2H, m), 8.48-8.52 (1H, m), 9.02-9.07 (1H, m), 9.52 (1H, s) ESI+ : 448 [M+H]+ |
| 5-99 | NMR-DMSO-d₆ 1.59-1.70 (1H, m), 2.00-2.07 (1H, m), 3.12-3.20 (1H, m), 3.36-3.46 (1H, m), 3.77-3.91 (1H, m), 4.52-4.59 (1H, m), 4.81-4.90 (1H, m), 4.96-5.09 (1H, m), 6.86-6.90 (1H, m), 7.07 (1H, d, J = 9.2 Hz), 7.11 (1H, br), 7.25-7.29 (1H, m), 7.63-7.67 (2H, m), 7.80 (1H, dd, J = 2.4, 9.2 Hz), 7.84 (1H, br), 8.23-8.26 (1H, m), 8.41-8.42 (1H, m), 8.44 (1H, s), 8.92-8.96 (1H, m), 9.49 (1H, brs) ESI+ : 476 [M+H]+ |
| 5-100 | NMR-DMSO-d₆: 1.59-1.71 (1H, m), 2.05-2.12 (1H, m), 3.21-3.29 (1H, m), 3.41-3.58 (1H, m), 3.82-3.97 (1H, m), 4.60-4.68 (1H, m), 4.91-5.15 (2H, m), 6.86-6.92 (1H, m), 7.13 (1H, br), 7.21-7.30 (1H, m), 7.61-7.69 (2H, m), 7.83 (1H, br), 8.25 (1H, d, J = 5.2 Hz), 8.44 (1H, s), 8.57 (2H, dd, J = 1.4, 9.6 Hz), 8.93-8.99 (1H, m), 9.46-9.55 (1H, m) ESI+ : 434 [M+H]+ |
| 5-101 | NMR-DMSO-d₆ 1.55-1.67 (1H, m), 1.97-2.04 (1H, m), 3.09-3.18 (1H, m), 3.36-3.51 (1H, m), 3.72-3.86 (1H, m), 4.53-4.61 (1H, m), 4.83-5.08 (2H, m), 6.35 (1H, s), 6.98 (1H, d, J = 3.6 Hz), 7.05 (1H, d, J = 8.8 Hz), 7.18 (1H, br), 7.35 (1H, d, J = 3.6 Hz), 7.87 (1H, dd, J = 2.4, 8.8 Hz), 7.91 (1H, br), 8.49 (1H, d, J = 2.4 Hz), 8.51 (1H, s), 8.97 (1H, d, J = 8.0 Hz), 10.92 (1H, s) ESI+ : 461 [M+Na]+ |

**[Table 115]**

| **Ex** | **DATA** |
|---|---|
| **5-102** | NMR-DMSO-d₆: 1.59-1.70 (1H, m),1.97-2.05 (1H, m), 2.78 (3H, d, J = 5.2 Hz), 3.04-3.13 (1H, m), 3.20-3.28 (1H, m), 3.74-3.88 (1H, m), 4.43-4.49 (1H, m), 4.79-5.06 (2H, m), 5.84 (1H, s), 6.54-6.58 (1H, m), 6.85-6.93 (1H, m), 7.07-7.32 (2H, m), 7.59-7.69 (2H, m), 7.78-7.88 (1H, m), 8.06 (1H, s), 8.23-8.26 (1H, m), 8.42-8.44 (1H, m), 8.92-8.99 (1H, m), 9.45-9.53 (1H, m) ESI+ : 462 [M+H]⁺ |
| **5-103** | NMR-DMSO-d₆: 0.89 (3H, d, J = 6.8 Hz), 1.78-1.89 (2H, m), 2.30-2.33 (1H, m), 3.70-3.84 (4H, m), 3.93-4.01 (1H, m), 6.86 (1H, dd, J = 8.0, 4.8 Hz), 7.09 (1H, br), 7.26 (1H, s), 7.44 (1H, d, J = 10.0 Hz), 7.63-7.67 (2H, m), 7.82 (1H, br), 7.86 (1H, d, J = 10.0 Hz), 8.23 (1H, d, J = 4.8 Hz), 8.44 (1H, s), 9.08 (1H, d, J = 7.6 Hz), 9.50 (1H, s) ESI+ : 430 [M+H]+ |
| **5-104** | NMR-DMSO-d₆ : 1.60-1.71 (1H, m), 2.04-2.10 (1H, m), 3.21-3.29 (1H, m), 3.42-3.58 (1H, m), 3.83-3.98 (1H, m), 4.61-4.68 (1H, m), 4.91-5.14 (2H, m), 7.16 (1H, s), 7.18 (1H, br), 7.89 (1H, br), 8.09 (1H, d, J = 2.4 Hz), 8.25-8.27 (1H, m), 8.48 (1H, s), 8.56 (1H, d, J = 1.6 Hz), 8.58 (1H, d, J = 1.6 Hz), 9.00-9.03 (2H, m), 9.88 (1H, brs) ESI+ : 435 [M+H]⁺ |
| **5-105** | NMR-DMSO-d₆: 1.44-1.55 (2H, m), 2.09-2.16 (2H, m), 3.33-3.42 (2H, m), 3.62-3.71 (1H, m), 4.36-4.43 (2H, m), 7.19 (1H, br), 7.21 (1H, s), 7.86 (1H, d, J = 8.8 Hz), 7.88 (1H, br), 8.04 (1H, dd, J = 2.4, 8.8 Hz), 8.46 (1H, s), 8.49 (1H, d, J = 1.2 Hz), 8.56 (1H, d, J = 1.2 Hz), 8.67-8.68 (1H, m), 8.79 (1H, d, J = 7.2 Hz), 10.18 (1H, s) ESI+: 463 [M+Na]+ |
| **5-106** | NMR-DMSO-d₆: 1.59-1.70 (1H, m), 1.89-2.06 (1H, m), 3.18-3.25 (1H, m), 3.38-3.51 (1H, m), 3.85-3.99 (1H, m), 4.53-4.62 (1H, m), 4.84-5.11 (2H, m), 7.04 (1H, d, J = 9.2 Hz), 7.26 (1H, br), 7.67 (1H, s), 7.87 (1H, dd, J = 2.4, 9.2 Hz), 7.96 (1H, br), 8.47-8.51 (2H, m), 8.99-9.07 (3H, m), 10.66 (1H, s) ESI- : 457 [M-H]- |
| **5-107** | NMR-DMSO-d₆: 0.89 (3H, d, J = 6.8 Hz), 1.76-1.88 (2H, m), 2.26-2.33 (1H, m), 3.68-3.72 (4H, m), 3.90-3.96 (1H, m), 6.85-6.88 (1H, m), 7.10 (1H, br), 7.25 (1H, s), 7.63-7.66 (2H, m), 7.86 (1H, br), 8.21-8.23 (1H, m), 8.44 (1H, s), 8.51 (1H, d, J = 1.2 Hz), 8.55 (1H, d, J = 1.2 Hz), 9.06 (1H, d, J = 7.6 Hz), 9.50 (1H, s) ESI+ : 430 [M+H]⁺ |
| **6-1** | NMR-DMSO-d₆: 1.63-1.73 (1H, m), 1.90-1.97 (1H, m), 2.86 (3H, s), 2.93-3.01 (1H, m), 3.21-3.35 (2H, m), 3.65-3.79 (1H, m), 3.91-4.00 (1H, m), 4.11 (2H, d, J = 1.2 Hz), 4.84-4.96 (1H, m), 6.84-6.88 (1H, m), 7.10 (1H, br), 7.24 (1H, s), 7.63-7.66 (2H, m), 7.81 (1H, br), 8.22-8.24 (1H, m), 8.43 (1H, s), 8.92 (1H, d, J = 8.0 Hz), 9.47 (1H, s) ESI+ : 427 [M+H]+ |
| **6-2** | NMR-DMSO-d₆ : 1.64-1.76 (1H, m), 1.88-1.96 (1H, m), 2.71-2.77 (2H, m), 2.84-2.93 (5H, m), 3.30-3.36 (1H, m), 3.12-3.23 (1H, m), 3.39-3.48 (1H, m), 3.62-3.76 (2H, m), 3.81-3.89 (1H, m), 6.84-6.88 (1H, m), 7.11 (1H, br), 7.23 (1H, s), 7.63-7.66 (2H, m), 7.82 (1H, br), 8.21-8.24 (1H, m), 8.43 (1H, s), 8.93 (1H, d, J = 8.0 Hz), 9.47 (1H, s) ESI+ : 441 [M+H]+ |

**[Table 116]**

| **Ex** | **DATA** |
|---|---|
| **15** | NMR-DMSO-d₆: 0.92-1.04 (4H, m), 2.59-2.68 (1H, m), 2.85-2.90 (2H, m), 3.46-3.52 (2H, m), 4.36 (2H, s), 4.44 (2H, d, J = 6.0 Hz), 6.03 (1H, s), 6.96-7.55 (6H, m), 7.76 (1H, brs), 8.38 (1H, s), 8.94 (1H, brs), 9.05 (1H, t, J = 6.0 Hz) ESI+ : 532 [M+H]+ |
| **8-40** | ESI+ : 567 [M+H]+ |
| **8-41** | NMR-DMSO-d₆: 8.95 (1H, t, J = 6.0 Hz), 8.50 (1H, bs), 8.29 (1H, s), 7.83 (1H, dd, J = 8.4, 2.0 Hz), 7.44 (1H, d, J = 8.0 Hz), 7.31-7.21 (1H, m), 7.13 (1H, s), 6.94-6.88 (2H, m), 5.71 (2H, bs), 4.52 (2H, d, J = 6.0 Hz), 3.96-3.57 (3H, m), 3.49-3.42 (1H, m), 2.84-2.65 (1H, m), 2.32 (3H, s), 2.25 (3H, s), 2.21-2.08 (1H, m), 1.93-1.83 (1H, m) ESI+ : 496 [M+H]+ |
| **8-42** | NMR-DMSO-d₆: 8.92 (1H, bs), 8.54 (1H, d, J = 8.0 Hz), 8.26 (1H, s), 7.90 (1H, bs), 7.81 (1H, d, J = 8.8 Hz), 7.42-7.39 (1H, m), 7.28-7.19 (2H, m), 6.93-6.86 (2H, m), 5.85 (2H, bs), 4.53 (2H, d, J = 6.0 Hz), 3.96-3.57 (3H, m), 3.48-3.42 (1H, m), 2.82-2.64 (1H, m), 2.31 (3H, s), 2.24 (3H, s), 2.21-2.06 (1H, m), 1.92-1.81 (1H, m) ESI+ : 496 [M+H]+ |
| **8-43** | NMR-DMSO-d₆: 8.96 (1H, t, J = 5.6 Hz), 8.92 (1H, s), 8.35 (1H, s), 7.75 (1H, bs), 7.48-7.40 (3H, m), 7.18-7.09 (4H, m), 7.02 (1H, bs), 6.05 (1H, s), 4.38 (2H, d, J = 5.6 Hz), 3.54 (2H, s), 3.46 (2H, t, J = 6.8 Hz), 3.29 (2H, t, J = 6.8 Hz), 1.90-1.83 (2H, m), 1.79-1.72 (2H, m). ESI+: 466 [M+H]+ |
| **8-44** | NMR-DMSO-d₆: 8.96 (1H, t, J = 6.0 Hz), 8.93 (1H, s), 8.36 (1H, s), 7.75 (1H, bs), 7.48-7.40 (3H, m), 7.18-7.10 (4H, m), 7.03 (1H, bs), 6.05 (1H, s), 4.38 (2H, d, J = 6.0 Hz), 3.89-3.80 (1H, m), 3.72-3.65 (1H, m), 3.63 (2H, s), 3.39-3.34 (1H, m), 3.21 (3H, s), 3.20-3.16 (1H, m), 3.11-3.03 (1H, m), 1.80-1.62 (2H, m), 1.33-1.14 (2H, m) ESI+ : 510 [M+H]+ |
| **8-45** | NMR-DMSO-d₆: 8.96 (1H, t, J = 6.0 Hz), 8.92 (1H, s), 8.36 (1H, s), 7.73 (1H, bs), 7.48-7.40 (3H, m), 7.18-7.09 (4H, m), 7.01 (1H, bs), 6.06 (1H, s), 4.38 (2H, d, J = 6.0 Hz), 3.63 (2H, s), 3.48-3.42 (4H, m), 2.23-2.17 (4H, m), 2.14 (3H, s). ESI+ : 495 [M+H]+ |
| **8-46** | NMR-DMSO-d₆: 1.01-1.11 (2H, m), 1,38 (2H, q, J = 6.3 Hz), 1.58-1.76 (3H, m), 2.72 (1H, br s), 2.96 (1H, br s), 3.42-3.27 (2H, m), 3.62 (1H, br s), 4.36 (1H, t, J = 5.0 Hz), 4.43 (1H, br s), 4.64 (2H, d, J = 5.9 Hz), 5.81 (1H, s), 6.83 (1H, dt, J = 7.6, 1.2 Hz), 7.16 (1H, br s), 7.24 (1H, t, J = 7.9 Hz), 7.48-7.50 (1H, m), 7.67 (1H, t, J = 1.8 Hz), 7.79 (1H, d, J = 5.1 Hz), 7.83 (1H, br s), 8.43 (1H, s), 8.72 (1H, s), 8.80 (1H, d, J = 4.9 Hz), 9.04 (1H, s), 9.10 (1H, t, J=6.0Hz) ESI+ : 543 [M+H]+ |
| **8-47** | NMR-DMSO-d₆: 1.56 (1H, br s), 1.68 (1H, br s), 1.91 (2H, br s), 3.15 (1H, br s), 3.43 (1H, br s), 3.56 (1H, br s), 4.45 (1H, br s), 4.64 (2H, d, J = 5.7 Hz), 5.44 (1H, s), 5.82 (1H, s), 6.95 (1H, dt, J = 7.6,1.1 Hz), 7.18 (1H, br s), 7.26 (1H, t, J = 7.9 Hz), 7.48-7.51 (3H, m), 7.75 (1H, t, J = 1.8 Hz), 7.79 (1H, d, J = 4.9 Hz), 7.85 (1H, br s), 8.44 (1H, s), 8.51-8.53 (2H, m), 8.72 (1H, s), 8.79 (1H, d, J = 4.9 Hz), 9.05-9.10 (2H, m) ESI+ : 614 [M+Na]+ |
| **8-48** | NMR-DMSO-d₆: 1.75-1.85 (2H, m), 2.17 (2H, br s), 3.11-3.25 (2H, m), 3.49-3.57 (1H, m), 3.74 (1H, br s), 4.43 (1H, br s), 4.64 (2H, d, J = 5.6 Hz), 5.81 (1H, s), 6.90 (1H, dt, J = 7.6, 1.2 Hz), 7.16 (1H, br s), 7.27 (1H, t, J = 7.9 Hz), 7.50-7.52 (1H, m), 7.74 (1H, t, J = 1.7 Hz), 7.79 (1H, d, J = 5.1 Hz), 7.83 (1H, br s), 7.93-7.95 (2H, m), 8.44 (1H, s), 8.72 (1H, s), 8.79-8.82 (3H, m), 9.07-9.11 (2H, m) ESI+ : 666 [M+Na]+ |

**[Table 117]**

| **Ex** | **DATA** |
|---|---|
| **8-49** | NMR-DMSO-d₆: 1.52-1.64 (4H, m), 1.86-1.93 (2H, m), 2.22 (2H, dd, J = 8.3, 7.7 Hz), 2.79 (1H, br s), 3.09 (1H, br s), 3.29-3.31 (2H, m), 3.70 (1H, br s), 3.98-4.06 (1H, m), 4.55 (1H, br s), 4.64 (2H, d, J = 5.7 Hz), 5.81 (1H, s), 6.88 (1H, dt, J = 7.5, 1.2 Hz), 7.17 (1H, br s), 7.26 (1H, t, J = 7.8 Hz), 7.46-7.49 (1H, m), 7.74 (1H, t, J = 1.8 Hz), 7.79 (1H, d, J = 5.1 Hz), 7.84 (1H, br s), 8.43 (1H, s), 8.72 (1H, s), 8.80 (1H, d, J = 6.9 Hz), 9.06 (1H, s), 9.10 (1H, t, J=6.1 Hz) ESI- : 580 [M-H]- |
| **12-108** | NMR-DMSO-d₆: 1.82-1.90 (1H, m), 2.61-2.70 (1H, m), 2.88-3.06 (2H, m), 4.98-5.04 (1H, m), 7.13 (1H, br), 7.21-7.36 (5H, m), 7.88 (1H, br), 8.06 (1H, d, J = 2.8 Hz), 8.21 (1H, dd, J = 2.8,1.6 Hz), 8.48 (1H, s), 9.01 (1H, d, J = 1.6 Hz), 9.06 (1H, d, J = 7.2 Hz), 9.92 (1H, s) ESI+: 347 [M+H]+ |
| **12-109** | NMR-DMSO-d₆: 1.81-1.91 (1H, m), 2.62-2.69 (1H, m), 2.87-3.06 (2H, m), 4.97-5.04 (1H, m), 7.13 (1H, br), 7.20-7.36 (5H, m), 7.87 (1H, br), 8.06 (1H, d, J = 2.8 Hz), 8.22 (1H, dd, J = 2.8, 1.6 Hz), 8.48 (1H, s), 9.01 (1H, d, J = 1.6 Hz), 9.06 (1H, d, J = 6.8 Hz), 9.92 (1H, s) ESI+: 347 [M+H]+ |
| **12-110** | NMR-DMSO-d₆ : 1.88-1.97 (1H, m), 2.63-2.71 (1H, m), 2.84-3.03 (2H, m), 4.99-5.05 (1H, m), 7.08-7.15 (2H, m), 7.16 (1H, br), 7.28 (1H, s), 7.33-7.37 (1H, m), 7.89 (1H, br), 7.07 (1H, d, J = 2.8 Hz), 8.21 (1H, dd, J = 2.8, 1.6 Hz), 8.49 (1H, s), 8.99 (1H, d, J =1.6 Hz), 9.10 (1H, d, J = 2.8 Hz), 9.94 (1H, s) ESI+: 365 [M+H]+ |
| **12-111** | NMR-DMSO-d₆: 1.72-1.93 (9H, m), 3.95-3.98 (1H, m), 4.68 (2H, d, J = 5.6 Hz), 7.10 (1H, br), 7.15 (1H, s), 7.38-7.41 (2H, m), 7.46 (1H, d, J = 8.8 Hz), 7.51 (1H, d, J = 5.2 Hz), 7.56 (1H, dd, J = 2.4, 8.8 Hz), 7.76-7.83 (3H, m), 7.92 (1H, d, J = 2.4 Hz), 8.42 (1H, s), 9.26-9.30 (1H, m), 9.52 (1H, s) ESI+ : 499 [M+H]+ |
| **12-112** | NMR-DMSO-d₆: 1.67-1.78 (6H, m), 2.58-2.62 (2H, m), 3.64-3.69 (2H, m), 4.68 (2H, d, J = 5.6 Hz), 7.12 (1H, br), 7.17 (1H, s), 7.37-7.44 (3H, m), 7.50 (1H, d, J = 5.2 Hz), 7.76-7.82 (4H, m), 7.84 (1H, br), 8.42 (1H, s), 9.27-9.31 (1H, m), 9.53 (1H, s) ESI+ : 487 [M+H]+ |
| **12-113** | NMR-DMSO-d₆ : 1.81-1.89 (4H, m), 2.37-2.41 (2H, m), 3.57-3.61 (2H, m), 4.02 (3H, s), 4.78 (2H, d, J = 5.2 Hz), 6.43 (1H, d, J = 3.2 Hz), 6.95-6.99 (1H, m), 7.05 (1H, br), 7.13 (1H, d, J = 6.4 Hz), 7.23 (1H, s), 7.27 (1H, d, J = 3.2 Hz), 7.48-7.51 (1H, m), 7.56 (1H, dd, J = 8.8, 2.8 Hz), 7.69 (1H, d, J = 8.8 Hz), 7.82 (1H, br), 8.10 (1H, d, J = 2.8 Hz), 8.44 (1H, s), 8.93-8.99 (1H, m), 9.60 (1H, s) ESI+ : 470 [M+H]+ |
| **12-114** | NMR-DMSO-d₆: 2.29 (3H, s), 2.55-2.73 (6H, m), 3.74-3.79 (2H, m), 4.02 (3H, s), 4.78 (2H, d, J = 4.8 Hz), 6.43 (1H, s), 6.94-6.98 (1H, m), 7.05 (1H, br), 7.13 (1H, d, J = 6.4 Hz), 7.24 (1H, s), 7.27 (1H, d, J = 7.2 Hz), 7.47-7.52 (2H, m), 7.67 (1H, d, J = 8.8 Hz), 7.81 (1H, br), 8.02 (1H, d, J = 6.4 Hz), 8.44 (1H, s), 8.92-8.96 (1H, m), 9.61 (1H, s) ESI+ : 499 [M+H]+ |
| **12-115** | NMR-DMSO-d₆: 1.89-1.99 (1H, m), 2.66-2.74 (1H, m), 2.88-2.97 (1H, m), 3.03-3.11 (1H, m), 5.05-5.11 (1H, m), 7.09-7.13 (1H, m), 7.14 (1H, br), 7.19 (1H, d, J = 4.8 Hz), 7.26-7.32 (2H, m), 7.90 (1H, br), 8.06 (1H, d, J = 2.8 Hz), 8.20 (1H, dd, J = 1.6,2.8 Hz), 8.49 (1H, s), 8.99 (1H, d, J = 1.6 Hz), 9.11 (1H, d, J = 7.0 Hz), 9.93 (1H, s) ESI+ : 365 [M+H]+ |

**[Table 118]**

| **Ex** | **DATA** |
|---|---|
| **12-116** | NMR-DMSO-d₆: 9.89 (1H, s), 9.23 (1H, t, J = 6.0 Hz), 8.80 (1H, d, J = 1.2 Hz), 8.48 (1H, s), 8.01-7.99 (2H, m), 7.90 (1H, bs), 7.78 (1H, d, J = 8.0 Hz), 7.63 (1H, t, J = 7.6 Hz), 7.53-7.45 (2H, m), 7.21 (1H, bs), 7.03 (1H, s), 4.65 (2H, d, J = 6.0 Hz) ESI+: 389 [M+H]+ |
| **12-117** | NMR-DMSO-d₆: 1.78-1.89 (1H, m), 2.23 (3H, s), 2.55-2.73 (7H, m), 2.87-3.05 (2H, m), 3.74-3.79 (2H, m), 4.96-5.02 (1H, m), 7.06 (1H, br), 7.20-7.37 (5H, m), 7.50 (1H, dd, J = 2.8, 8.8 Hz), 7.67 (1H, d, J = 8.8 Hz), 7.82 (1H, br), 8.06 (1H, d, J = 2.8 Hz), 8.44 (1H, s), 8.99 (1H, d, J = 6.8 Hz), 9.63 (1H, s) ESI+ : 472 [M+H]+ |
| **12-118** | NMR-DMSO-d₆: 9.88 (1H, s), 9.17 (1H, t, J = 6.0 Hz), 8.86 (1H, d, J = 1.6 Hz), 8.47 (1H, s), 8.06 (1H, dd, J = 2.8, 1.6 Hz), 8.01 (1H, d, J = 2.8 Hz), 7.90 (1H, bs), 7.86 (1H, dd, J = 7.6, 1.2 Hz), 7.65 (1H, dt, J = 7.6, 1.2 Hz), 7.56-7.49 (2H, m), 7.20 (1H, bs), 7.01 (1H, s), 4.82 (2H, d, J = 6.0 Hz), 2.77 (6H, s) ESI+ : 428 [M+H]+ |
| **12-119** | NMR-DMSO-d₆: 9.92 (1H, s), 9.05 (1H, t, J = 5.6 Hz), 8.87 (1H, d, J = 1.6 Hz), 8.44 (1H, s), 8.27 (1H, dd, J = 2.4, 1.6 Hz), 8.07 (1H, d, J = 2.4 Hz), 7.82 (1H, bs), 7.52-7.44 (2H, m), 7.36-7.31 (1H, m), 7.29 (1H, s), 7.09 (1H, bs), 4.59 (1H, bs), 4.41 (1H, bs), 3.16 (3H, s), 3.09 (3H, s) ESI+ : 446 [M+H]+ |
| **12-120** | NMR-DMSO-d₆: 2.30 (3H, s), 2.55-2.64 (4H, m), .67-2.72 (2H, m), 3.70-3.76 (2H, m), 4.73 (2H, d, J = 6.0 Hz), 6.83-6.87 (1H, m), 7.08 (1H, s), 7.08-7.12 (2H, m), 7.43 (1H, dd, J = 2.8, 8.8 Hz), 7.57 (1H, d, J = 8.8 Hz), 7.60 (1H, d, J = 1.2 Hz), 7.73 (1H, d, J = 2.8 Hz), 7.82 (1H, br), 7.99 (1H, d, J = 1.2 Hz), 8.42 (1H, s), 8.45-8.48 (1H, m), 9.12-9.16 (1H, m), 9.51 (1H, s) ESI+ : 486 [M+H]+ |
| **12-121** | NMR-DMSO-d₆: 1.98-2.07 (1H, m), 2.28 (3H, s), 2.55-2.73 (7H, m), 2.91-2.99 (1H, m), 3.05-3.14 (1H, m), 3.75-3.80 (2H, m), 5.13-5.18 (1H, m), 7.01 (1H, br), 7.01-7.06 (1H, m), 7.18 (1H, d, J = 7.2 Hz), 7.32 (1H, s), .34-7.39 (1H, m), 7.50 (1H, dd, J = 2.8, 8.8 Hz), 7.65 (1H, d, J = 8.8 Hz), 7.80 (1H, br), 8.08 (1H, d, J = 2.8 Hz), 8.43 (1H, s), 8.94 (1H, d, J = 6.8 Hz), 9.63 (1H, s) ESI+ : 490 [M+H]+ |
| **12-122** | NMR-DMSO-d₆: 9.02 (1H, s), 8.79 (1H, t, J = 5.2 Hz), 8.36 (1H, s), 7.73 (1H, bs), 7.59-7.45 (4H, m), 7.38-7.31 (1H, m), 7.15-7.10 (2H, m), 6.99 (1H, bs), 6.07 (1H, s), 4.59-4.20 (2H, m), 3.57 (2H, t, J = 5.6 Hz), 3.17 (3H, s), 3.09 (3H, s), 2.37 (2H, t, J = 6.0 Hz), 1.89-1.79 (4H, m). ESI+ : 541 [M+H]+. |
| **12-123** | NMR-DMSO-d₆: 8.96 (1H, t, J = 6.0 Hz), 8.45 (1H, s), 8.29 (1H, s), 7.65 (1H, bs), 7.46-7.39 (1H, m), 7.19 (2H, d, J = 8.8 Hz), 7.12 (2H, t, J = 8.0 Hz), 6.90 (1H, bs), 6.51 (2H, d, J = 8.8 Hz), 5.86 (1H, s), 4.31 (2H, d, J = 6.0 Hz), 3.23-3.19 (4H, m), 1.98-1.93 (4H, m). ESI+ : 424 [M+H]+ |
| **12-124** | NMR-DMSO-d₆: 8.96 (1H, t, J = 6.0 Hz), 8.72 (1H, s), 8.32 (1H, s), 7.70 (1H, bs), 7.47-7.39 (1H, m), 7.36 (2H, dt, J = 8.8, 3.6 Hz), 7.17-7.10 (2H, m), 6.98 (1H, bs), 6.87 (2H, dt, J = 9.2, 3.2 Hz), 5.94 (1H, s), 4.35 (2H, d, J = 6.0 Hz), 4.03 (2H, t, J = 6.0 Hz), 2.77 (2H, t, J = 6.0 Hz), 2.59-2.43 (4H, m), 1.73-1.63 (4H, m). ESI+ : 468 [M+H]+ |

**[Table 119]**

| **Ex** | **DATA** |
|---|---|
| **12-125** | NMR-DMSO-d₆: 8.96 (1H, t, J = 6.0 Hz), 8.72 (1H, s), 8.32 (1H, s), 7.70 (1H, bs), 7.43 (1H, tt, J = 8.4, 6.8 Hz), 7.38-7.34 (2H, m), 7.17-7.10 (2H, m), 6.98 (1H, bs), 6.89-6.85 (2H, m), 5.94 (1H, s), 4.35 (2H, d, J = 6.0 Hz), 4.01 (2H, t, J = 5.6 Hz), 2.61 (2H, t, J = 5.6 Hz), 2.21 (6H, s). ESI+ : 442 [M+H]+ |
| **12-126** | NMR-DMSO-d₆: 1.81-1.89 (4H, m), 2.39 (2H, t, J = 6.2 Hz), 3.55-3.58 (2H, m), 4.68 (2H, d, J = 6.0 Hz), 7.08 (1H, s), 7.18 (1H, br s), 7.527-7.533 (2H, m), 7.78 (1H, d, J = 5.1 Hz), 7.86 (1H, br s), 7.98-7.99 (1H, m), 8.45 (1H, s), 8.76-8.78 (2H, m), 9.23 (1H, t, J = 6.2 Hz), 9.64 (1H, s) ESI+ : 486 [M+H]+ |
| **12-127** | NMR-DMSO-d₆ 2.05-2.14 (1H, m), 2.59-2.69 (1H, m), 2.93-3.02 (1H, m), 3.07-3.16 (1H, m), 5.21-5.27 (1H, m), 7.07-7.24 (4H, m), 7.87 (1H, br), 8.07 (1H, d, J= 2.8 Hz), 8.21 (1H, dd, J = 1.6,2.8 Hz), 8.48 (1H, s), 8.96 (1H, d, J = 1.6 Hz), 9.10 (1H, d, J = 7.2 Hz), 9.93 (1H, s) ESI+ : 383 [M+H]+ |
| **12-128** | NMR-DMSO-d₆: 2.29 (3H, s), 2.54-2.73 (6H, m), 3.73-3.78 (2H, m), 3.79 (3H, s), 4.61 (2H, d, J = 5.6 Hz), 6.56 (1H, d, J = 3.2 Hz), 7.02-7.14 (3H, m), 7.20 (1H, s), 7.32-7.27 (2H, m), 7.44 (1H, dd, J = 2.4, 8.8 Hz), 7.56 (1H, d, J = 8.8 Hz), 7.74 (1H, br), 7.94 (1H, d, J = 2.4 Hz), 8.40 (1H, s), 9.09-9.12 (1H, m), 9.53 (1H, s) ESI+ : 499 [M+H]+ |
| **12-129** | NMR-DMSO-d₆: 1.91-2.06 (5H, m), 2.53-2.58 (1H, m), 2.90-2.98 (1H, m), 3.05-3.13 (1H, m), 3.17-3.23 (4H, m), 5.10-5.15 (1H, m), 6.90 (1H, br), 6.98-7.05 (2H, m), 7.10 (1H, s), 7.18 (1H, d, J = 7.2 Hz), 7.32-7.38 (1H, m), 7.51 (1H, d, J = 7.2 Hz), 7.62 (1H, d, J = 2.8 Hz), 7.70 (1H, br), 8.36 (1H, s), 8.89 (1H, d, J = 7.2 Hz), 9.08 (1H, s) ESI+ : 433 [M+H]+ |
| **12-130** | NMR-DMSO-d₆: 8.95 (1H, t, J = 6.0 Hz), 8.65 (1H, s), 8.31 (1H, s), 7.68 (1H, bs), 7.47-7.39 (1H, m), 7.32-7.29 (2H, m), 7.17-7.10 (2H, m), 6.97 (1H, bs), 6.89-6.86 (2H, m), 5.94 (1H, s), 4.34 (2H, d, J = 6.0 Hz), 3.08-3.06 (4H, m), 2.48-2.44 (4H, m), 2.23 (3H, s). ESI+ : 453 [M+H]+ |
| **12-131** | NMR-DMSO-d₆: 1.66-1.71 (4H, m), 1.97-2.07 (1H, m), 2.39-2.44 (4H, m), 2.52-2.62 (1H, m), 2.89-3.14 (2H, m), 3.49 (2H, s), 5.13-5.19 (1H, m), 6.98 (1H, br), 7.01-7.06 (1H, m), 7.18 (1H, d, J = 7.6 Hz), 7.32-7.36 (2H, m), 7.57-7.58 (2H, m), 7.80 (1H, br), 8.12-8.13 (1H, m), 8.42 (1H, s), 8.94 (1H, d, J = 7.6 Hz), 9.52 (1H, s) ESI+ : 447 [M+H]+ |
| **12-132** | NMR-DMSO-d₆: 1.55-1.62 (1H, m), 1.79-1.86 (1H, m), 1.99-2.04 (1H, m), 2.06 (6H, s), 2.19-2.25 (1H, m), 2.34-2.44 (1H, m), 2.54-2.61 (2H, m), 2.63-2.68 (2H, m), 2.91-2.99 (1H, m), 3.06-3.14 (1H, m), 3.39-3.52 (2H, m), 5.13-5.19 (1H, m), 6.98 (1H, br), 7.01-7.06 (1H, m), 7.18 (1H, d, J = 7.6 Hz), 7.33-7.37 (2H, m), 7.54-7.59 (2H, m), 7.79 (1H, br), 8.11-8.12 (1H, m), 8.42 (1H, s), 8.94 (1H, d, J = 6.8 Hz), 9.53 (1H, s) ESI+ : 490 [M+H]+ |
| **12-133** | NMR-DMSO-d₆: 2.31 (3H, s), 2.57-2.65 (4H, m), 2.69-2.74 (2H, m), 3.72-3.76 (2H, m), 4.78 (2H, d, J = 5.6 Hz), 7.13 (1H, s), 7.17 (1H, br), 7.40-7.41 (2H, m), 7.61-7.63 (2H, m), 7.77-7.78 (1H, m), 7.82-7.87 (1H, m), 7.87 (1H, br), 8.42 (1H, s), 8.89 (1H, s), 9.33-9.37 (1H, m), 9.55 (1H, s) ESI+ : 527 [M+H]+ |

**[Table 120]**

| **Ex** | **DATA** |
|---|---|
| **12-134** | NMR-DMSO-d₆: 8.96 (1H, t, J = 6.0 Hz), 8.86 (1H, s), 8.35 (1H, s), 7.73 (1H, bs), 7.48-7.37 (3H, m), 7.18-7.09 (4H, m), 7.00 (1H, bs), 6.03 (1H, s), 4.37 (2H, d, J = 6.0 Hz), 2.70-2.64 (2H, m), 2.62-2.54 (2H, m), 2.48-2.44 (4H, m), 1.71-1.64 (4H, m). ESI+ : 452 [M+H]+ |
| **12-135** | NMR-DMSO-d₆: 8.96 (1H, t, J = 6.0 Hz), 8.85 (1H, s), 8.35 (1H, s), 7.72 (1H, bs), 7.48-7.38 (3H, m), 7.18-7.08 (4H, m), 7.01 (1H, bs), 6.03 (1H, s), 4.37 (2H, d, J = 6.0 Hz), 3.22 (3H, s), 3.18-3.12 (1H, m), 2.78-2.70 (2H, m), 2.67-2.63 (2H, m), 2.56-2.44 (2H, m), 2.13-2.07 (2H, m), 1.87-1.79 (2H, m), 1.44-1.36 (2H, m). ESI+ : 496 [M+H]+ |
| **12-136** | NMR-DMSO-d₆ : 1.99-2.07 (1H, m), 2.32-2.37 (4H, m), 2.54-2.62 (1H, m), 2.91-3.00 (1H, m), 3.06-3.15 (1H, m), 3.39 (2H, s), 3.55-3.58 (4H, m), 5.14-5.19 (1H, m), 6.99 (1H, br), 7.00-7.06 (1H, m), 7.18 (1H, d, J = 7.6 Hz), 7.33-7.38 (2H, m), 7.55-7.62 (2H, m), 7.79 (1H, br), 8.11-8.12 (1H, m), 8.42 (1H, s), 8.95 (1H, d, J = 6.8 Hz), 9.55 (1H, s) ESI+ : 463 [M+H]+ |
| **12-137** | NMR-DMSO-d₆: 2.29 (3H, s), 4.71 (2H, d, J = 6.0 Hz), 7.08 (1H, s), 7.28 (1H, br s), 7.40 (1H, d, J = 5.9 Hz), 7.79 (1H, d, J = 5.1 Hz), 7.95 (1H, br s), 8.26 (1H, d, J = 6.0 Hz), 8.50 (1H, s), 8.74 (1H, s), 8.79 (1H, d, J = 5.1 Hz), 9.23 (1H, t, J = 6.1 Hz), 10.00 (1H, s) ESI+ : 404 [M+H]+ |
| **12-138** | NMR-DMSO-d₆: 1.43 (6H, s), 2.88 (3H, s), 4.69 (2H, d, J = 6.0 Hz), 7.06 (1H, s), 7.19 (1H, br s), 7.61-7.67 (2H, m), 7.77 (1H, d, J = 6.2 Hz), 7.88 (1H, br s), 8.08 (1H, dd, J = 2.4, 0.8 Hz), 8.47 (1H, s), 8.76-8.78 (2H, m), 9.23 (1H, t, J = 6.1 Hz), 9.80 (1H, s) ESI+ : 529 [M+H]+ |
| **12-139** | NMR-DMSO-d₆ : 3.03-3.05 (4H, m), 3.73-3.75 (4H, m), 4.41 (2H, d, J = 6.0 Hz), 5.90 (1H, s), 6.88 (2H, d, J = 9.1 Hz), 6.98 (1H, br s), 7.18 (1H, tdd, J = 9.2, 3.7, 2.1 Hz), 7.31 (2H, d, J = 9.0 Hz), 7.49 (1H, ddd, J = 14.3, 9.4, 5.1 Hz), 7.69 (1H, br s), 8.32 (1H, s), 8.68 (1H, s), 9.05 (1H, t, J = 6.1 Hz) ESI+ : 458 [M+H]+ |
| **12-140** | NMR-DMSO-d₆ 1.56-1.66 (1H, m), 1.97-2.03 (1H, m), 3.06-3.15 (1H, m), 3.34-3.43 (1H, m), 3.73-3.83 (1H, m), 4.52-4.60 (1H, m), 4.81-5.06 (2H, m), 6.07 (1H, s), 6.33 (1H, s), 6.93-7.10 (3H, m), 7.20 (1H, s), 7.40 (1H, d, J = 8.4 Hz), 7.84-7.95 (3H, m), 8.37 (1H, s), 8.49 (1H, d, J = 1.6 H), 8.78-8.94 (2H, m), 10.90 (1H, brs) ESI+ : 471 [M+H]+ |
| **12-141** | N NMR-DMSO-d₆ : 1.42-1.50 (2H, m),1.83-1.90 (2H, m), 3.22-3.34 (6H, m), 3.42-3.48 (1H, m), 3.73 (1H, br s), 4.71 (2H, d, J = 6.2 Hz), 7.19-7.24 (2H, m), 7.45 (1H, d, J = 8.5 Hz), 7.66 (1H, dd, J = 8.6, 2.4 Hz), 7.78 (1H, d, J = 5.0 Hz), 7.89 (1H, br s), 8.10 (1H, dd, J = 2.4, 0.3 Hz), 8.46 (1H, s), 8.76 (1H, d, J = 5.0 Hz), 8.79 (1H, s), 9.27 (1H, t, J = 6.1 Hz), 9.84 (1H, s) ESI+ : 530 [M+H]+ |
| **12-142** | NMR-DMSO-d₆: 1.55-1.67 (1H, m), 1.89-2.00 (6H, m), 2.54-2.57 (4H, m), 2.65-2.69 (4H, m), 3.08-3.16 (1H, m), 3.74-3.86 (1H, m), 4.51-4.58 (1H, m), 4.82-5.04 (2H, m), 6.03 (1H, s), 7.01-7.06 (2H, m), 7.09 (2H, d, J = 8.8 Hz), 7.50 (2H, d, J = 8.8 Hz), 7.72 (1H, br), 7.86 (1H, dd, J = 2.4, 9.2 Hz), 8.37 (1H, s), 8.49 (1H, d, J = 2.4 Hz), 8.84-8.89 (2H, m) ESI+ : 579 [M+H]+ |
| **12-143** | NMR-DMSO-d₆ : 2.99-3.02 (4H, m), 3.72-3.74 (4H, m), 4.59 (2H, d, J = 5.7 Hz), 5.61 (1H, s), 6.79 (2H, d, J = 9.1 Hz), 7.05 (1H, br s), 7.19 (2H, d, J = 8.9 Hz), 7.76 (1H, br s), 7.78 (1H, d, J = 5.2 Hz), 8.37 (1H, s), 8.60 (1H, s), 8.69 (1H, s), 8.79 (1H, d, J = 5.0 Hz), 9.10 (1H, t, J = 6.0 Hz) ESI+ : 473 [M+H]+ |

**[Table 121]**

| **Ex** | **DATA** |
|---|---|
| **12-144** | NMR-DMSO-d₆ 4.70 (2H, d, J = 6.0 Hz), 7.06 (1H, s), 7.27 (1H, br s), 7.68 (1H, d, J = 8.7 Hz), 7.80 (1H, d, J = 5.2 Hz), 7.94 (1H, br s), 8.00 (1H, dd, J = 8.9, 2.3 Hz), 8.46 (1H, dd, J = 2.4, 0.6 Hz), 8.49 (1H, s), 8.77-8.78 (2H, m), 9.27 (1H, t, J = 6.2 Hz), 10.22 (1H, s) ESI+ : 414 [M+H]+ |
| **12-145** | NMR-DMSO-d₆: 4.69 (2H, d, J = 5.9 Hz), 6.92 (1H, s), 7.21-7.27 (2H, m), 7.79 (1H, d, J = 5.1 Hz), 7.91 (1H, br s), 8.01 (1H, s), 8.27 (1H, dd, J = 5.2, 0.6 Hz), 8.50 (1H, s), 8.76-8.77 (2H, m), 9.25 (1H, t, J = 6.1 Hz), 10.02 (1H, s) ESI+ : 414 [M+H]+ |
| **12-146** | NMR-DMSO-d₆ : 4.68 (2H, d, J = 6.2 Hz), 6.99 (1H, s), 7.06-7.30 (1H, br), 7.51-7.69 (2H, m), 7.77-7.95 (2H, m), 7.99 (1H, d, J = 2.8 Hz), 8.44 (1H, s), 8.74-8.82 (2H, m), 9.23 (1H, t, J = 6.2 Hz), 9.65 (1H, s) ESI+ : 407 [M+H]+ |
| **12-147** | NMR-DMSO-d₆ : 4.70 (2H, d, J = 6.0 Hz), 6.96 (1H, s), 7.14 (1H, dd, J = 1.2, 5.2 Hz), 7.15-7.35 (1H, br), 7.79 (1H, d, J = 5.2 Hz), 7.82-7.99 (1H, br), 8.04 (1H, s), 8.31 (1H, d, J = 5.2 Hz), 8.51 (1H, s), 8.74-8.80 (2H, m), 9.26 (1H, t, J = 6.0 Hz), 10.01 (1H, s) ESI+ : 457 [M+H]+ |
| **12-148** | N NMR-DMSO-d₆: 4.71 (2H, d, J = 6.0 Hz), 7.14 (1H, s), 7.18-7.33 (1H, br), 7.69 (1H, d, J = 8.8 Hz), 7.79 (1H, d, J = 5.0 Hz), 7.88-8.02 (1H, br), 7.96 (1H, dd, J = 2.8, 8.8 Hz), 8.36-8.41 (1H, m), 8.49 (1H, s), 8.77 (1H, d, J = 5.0 Hz), 8.80 (1H, s), 9.27 (1H, t, J = 6.0 Hz), 10.12 (1H, s) ESI+ : 457 [M+H]+ |
| **12-149** | NMR-DMSO-d₆: 1.55-1.66 (1H, m), 1.97-2.03 (1H, m), 2.56 (3H, s), 3.10-3.18 (1H, m), 3.34-3.46 (1H, m), 3.75-3.88 (1H, m), 4.53-4.60 (1H, m), 4.83-5.07 (2H, m), 6.34 (1H, s), 7.05 (1H, d, J = 9.2 Hz), 7.25 (1H, br), 7.87 (1H, dd, J = 2.4, 9.2 Hz), 7.93 (1H, br), 8.47 (1H, s), 8.49 (1H, d, J = 2.4 Hz), 9.02 (1H, d, J = 8.0 Hz), 11.15 (1H, s) ESI+: 476 [M+Na]+ |
| **12-150** | NMR-DMSO-d₆: 1.54-1.65 (1H, m), 1.95-2.04 (1H, m), 2.23 (3H, s), 3.09-3.18 (1H, m), 3.34-3.46 (1H, m), 3.72-3.86 (1H, m), 4.53-4.60 (1H, m), 4.83-5.07 (2H, m), 6.36-6.52 (1H, m), 7.06 (1H, d, J = 9.2 Hz), 7.14-7.39 (2H, m), 7.87 (1H, dd, J = 2.4, 9.2 Hz), 7.89 (1H, br), 8.47-8.50 (2H, m), 8.96 (1H, d, J = 8.0 Hz), 10.87 (1H, s) ESI+ : 453 [M+H]+ |
| **12-151** | NMR-DMSO-d₆: 1.54-1.65 (1H, m), 1.97-2.03 (1H, m), 2.30 (3H, s), 3.09-3.17 (1H, m), 3.36-3.46 (1H, m), 3.71-3.85 (1H, m), 4.54-4.60 (1H, m), 4.81-5.07 (2H, m), 6.32 (1H, s), 6.99 (1H, d, J = 1.6 Hz), 7.05 (1H, d, J = 9.2 Hz), 7.18 (1H, br), 7.87 (1H, dd, J = 2.4, 9.2 Hz), 7.89 (1H, br), 8.47 (1H, s), 8.49 (1H, d, J = 2.4 Hz), 8.95 (1H, d, J = 8.0 Hz), 10.70 (1H, s) ESI+ : 453 [M+H]+ |
| **13** | NMR-DMSO-d₆: 2.05-2.11 (2H, m), 2.28 (3H, s), 2.31-2.40 (1H, m), 2.55-2.73 (5H, m), 2.92-3.02 (1H, m), 3.06-3.15 (1H, m), 3.75-3.80 (2H, m), 5.19-5.27 (1H, m), 7.05 (1H, br), 7.06-7.12 (1H, m), 7.16-7.22 (1H, m), 7.30 (1H, s), 7.49 (1H, dd, J = 2.8, 8.8 Hz), 7.62 (1H, d, J = 8.8 Hz), 7.82 (1H, br), 8.43 (1H, s), 8.60 (1H, s), 9.03 (1H, d, J = 6.8 Hz), 9.64 (1H, s) ESI+: 508 [M+H]+ |
| **14** | NMR-DMSO-d₆ : 1.56-1.66 (1H, m), 2.05-2.12 (1H, m), 3.19-3.29 (1H, m), 3.35-3.54 (1H, m), 3.80-3.94 (1H, m), 4.83-4.90 (1H, m), 4.99-5.17 (2H, m), 6.85-6.89 (1H, m), 7.10 (1H, br), 7.27 (1H, s), 7.64-7.68 (2H, m), 7.83 (1H, br), 8.24 (1H, d, J = 5.2 Hz), 8.44 (1H, s), 8.78-8.79 (2H, s), 8.96 (1H, d, J = 8.0 Hz), 9.49 (1H, s) ESI+ : 434 [M+H]+ |

### Industrial Applicability

A 4,6-diaminonicotinamide compound which is the compound of the present invention has an excellent JAK3 inhibitory action, and thus is expected to be used as an agent for preventing or treating diseases caused by undesirable and/or abnormal cytokine signal transduction.

## Claims

1. A compound of the formula (I) or a salt thereof: 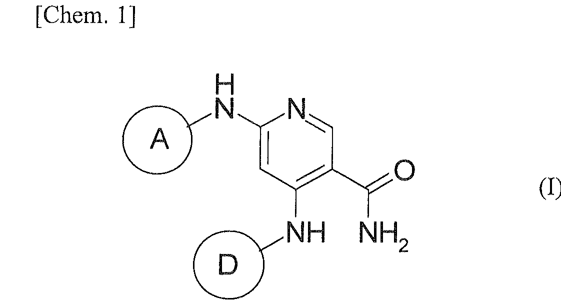 (wherein
A represents H, or lower alkyl, aryl, or a nitrogen-containing hetero ring, which may each be substituted,
D represents the following group: 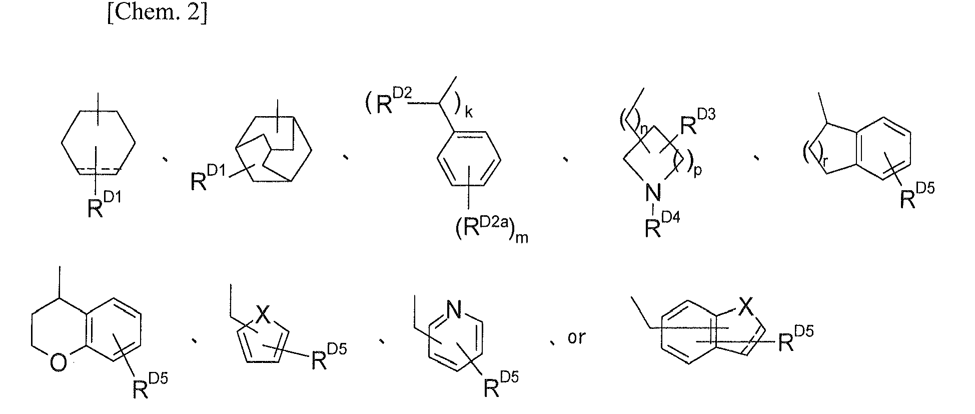
R^{D1} represents H, halogen, lower alkyl, OH, or CN; or -O-lower alkyl or a nitrogen-containing hetero ring, which may each be substituted,
R^{D2} may be the same as or different from each other and represent H, or lower alkyl,
R^{D2a} may be the same as or different from each other and represent H, halogen, OH, -S-lower alkyl, -S(=O)₂-lower alkyl, -N(lower alkyl)₂, -N(lower alkyl) -S(=O)₂-lower alkyl, -CN, or a nitrogen-containing hetero ring; or lower alkyl or -O-lower alkyl, which may each be substituted,
k represents 0, 1, or 2,
m represents 1, 2, or 3,
R^{D3} represents H, halogen, or lower alkyl which may be substituted,
R^{D4} represents H, CN, or protected carboxy; or lower alkyl, aryl, a nitrogen-containing hetero ring, -C(=O)-lower alkyl, or -S(=O)₂-aryl, which may each be substituted,
n represents 0, or 1,
p represents 1, 2, 3, or 4,
X represents NH, N-lower alkyl, or S,
R^{DS} represents H, halogen, -N(lower alkyl)₂, or lower alkyl which may be substituted, and
r represents 1 or 2.
wherein the broken line portion in the formula above together with an adjacent bond represents a single bond or a double bond).

2. A compound of the formula (Ia) or a salt thereof: 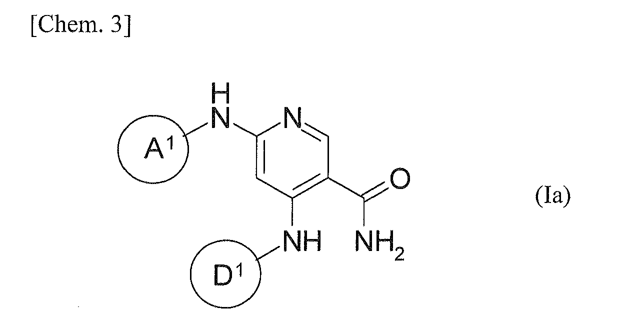 (wherein
A¹ represents H, or a nitrogen-containing aromatic 6-membered hetero ring, a nitrogen-containing unsaturated hetero ring having no aromatic property, or a nitrogen-containing saturated hetero ring, which may each be substituted,
D¹ represents the following group: 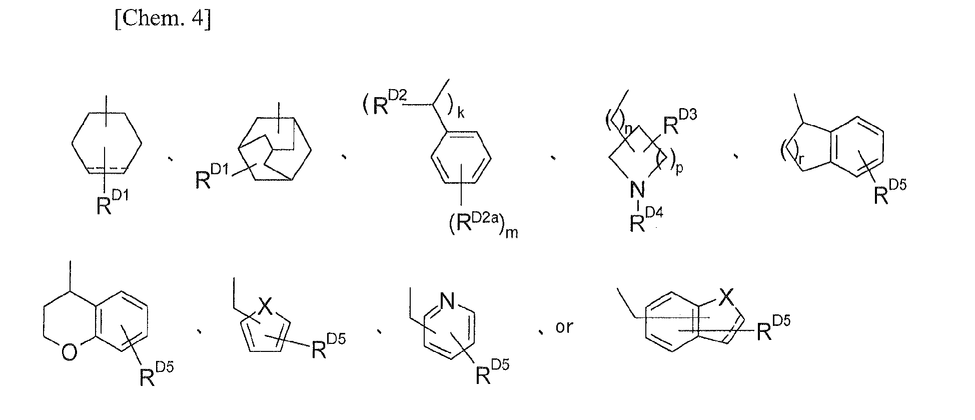
R^{D1} represents H, halogen, lower alkyl, OH, or CN; or -O-lower alkyl or a nitrogen-containing hetero ring, which may each be substituted,
R^{D2} may be the same as or different from each other and represent H or lower alkyl,
R^{D2a} may be the same as or different from each other and represent H, halogen, OH, -S-lower alkyl, -S(=O)₂-lower alkyl, -N(lower alkyl) -S(=O)₂-lower alkyl, or CN; or lower alkyl or -O-lower alkyl, which may each be substituted,
k represents 1 or 2,
m represents 1, 2, or 3,
R^{D3} represents H, halogen, or lower alkyl which may be substituted,
R^{D4} represents H, CN, or protected carboxy; or lower alkyl, aryl, a nitrogen-containing hetero ring, -C(=O)-lower alkyl, or -S(=O)₂-aryl, which may each be substituted,
n represents 0,
p represents 1, 2, 3, or 4,
X represents NH, N-lower alkyl, or S,
R^{D5} represents H, halogen, -N(lower alkyl)₂, or lower alkyl which may be substituted, and
r represents 1 or 2.
wherein the broken line portion in the formula above together with an adjacent bond represents a single bond or a double bond).

3. The compound or a salt thereof according to in claim 2, wherein A¹ represents a nitrogen-containing aromatic 6-membered hetero ring which may be substituted,
D¹ represents: 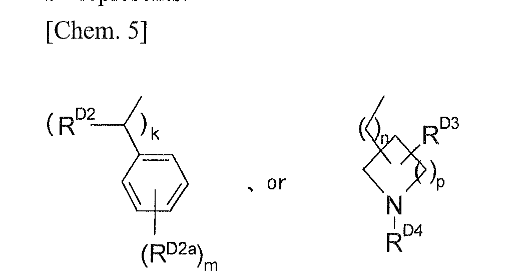 (wherein
R^{D2} may be the same as or different from each other and represent H or lower alkyl,
R^{D2a} may be the same as or different from each other and represent H, halogen, OH, -S-lower alkyl, -S(=O)₂-lower alkyl, -N(lower alkyl) -S(=O)₂-lower alkyl, or CN; or lower alkyl or -O-lower alkyl, which may each be substituted,
k represents 1 or 2,
m represents 1, 2, or 3,
R^{D3} represents H, halogen, or lower alkyl which may be substituted,
R^{D4} represents H, CN, or protected carboxy; or lower alkyl, aryl, a nitrogen-containing hetero ring, -C(=O)-lower alkyl, or -S(=O)₂-aryl, which may each be substituted,
n represents 0, and
p represents 1, 2, 3, or 4).

4. The compound or a salt thereof according to in claim 3, wherein D¹ represents: 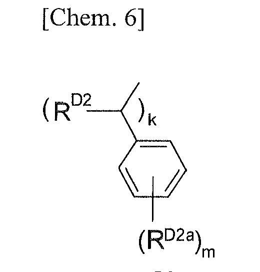
(wherein R^{D2} may be the same as or different from each other and represent H or lower alkyl,
R^{D2a} may be the same as or different from each other and represent H, halogen, OH, -S-lower alkyl, -S(=O)₂-lower alkyl, -N(lower alkyl) -S(=O)₂-lower alkyl, or CN; or lower alkyl or -O-lower alkyl, which may each be substituted,
k represents 1 or 2, and
m represents 1, 2, or 3).

5. The compound or a salt thereof according to in claim 3, wherein D¹ is 2,6-difluorobenzyl or 2,3,6-trifluorobenzyl.

6. The compound or a salt thereof according to in claim 3, wherein D¹ is 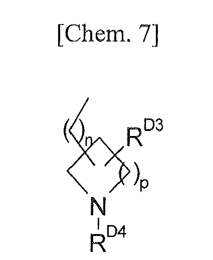
(wherein R^{D3} represents H, halogen, or lower alkyl which may be substituted,
R^{D4} represents H, CN, or protected carboxy; or lower alkyl, aryl, a nitrogen-containing hetero ring, -C(=O)-lower alkyl, or -S(=O)₂-aryl, which may each be substituted,
n represents 0, and
p represents 1, 2, 3, or 4).

7. The compound or a salt thereof according to in claim 3, wherein D¹ is 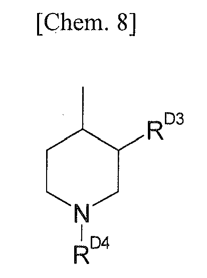
(wherein R^{D3} represents H, F, methoxy, methyl, or hydroxymethyl, and
R^{D4} represents aryl or a nitrogen-containing hetero ring, which may each be substituted with cyano, -O-benzyl, OH, or F).

8. The compound or a salt thereof according to in claim 3, wherein R^{D4} is 4-cyanophenyl, 4-benzyloxyphenyl, 4-hydroxyphenyl, 4-fluorophenyl, 5-[(cyanomethyl)carbamoyl]pyridin-2-yl, 5-[(cyanomethyl)(methyl)carbamoyl]pyridin-2-yl, 5-cyanopyridin-2-yl, 6-cyanopyridin-3-yl, 6-cyanopyridazin-3-yl, 4-cyanomethoxyphenyl, or 5-cyanopyrazin-2-yl.

9. The compound or a salt thereof according to in claim 3, wherein D¹ is 1-(5-cyanopyridin-2-yl)piperidin-4-yl, 1-(6-cyanopyridin-3-yl)piperidin-4-yl, 1-(6-cyanopyridazin-3-yl)piperidin-4-yl, 1-(5-cyanopyridin-2-yl)-3-methylpiperidin-4-yl, 1-(5-cyanopyridin-2-yl)-3-fluoropiperidin-4-yl, 1-(5-cyanopyridin-2-yl)-3-(hydroxymethyl)piperidin-4-yl, 1-(5-cyanopyridin-2-yl)-3-methoxypiperidin-4-yl, 1-(6-cyanopyridazin-3-yl)-3-fluoropiperidin-4-yl, 1-(5-cyanopyrazin-2-yl)-3-fluoropiperidin-4-yl, 1-{5-[(cyanomethyl)carbamoyl]pyridin-2-yl}piperidin-4-yl, or 1-{5-[(cyanomethyl)(methyl)carbamoyl]pyridin-2-yl}piperidin-4-yl.

10. The compound or a salt thereof according to in claim 2, which is
4-{[1-(5-cyanopyridin-2-yl)piperidin-4-yl]amino}-6-(pyridin-2-ylamino)nicotinamide,
4-{[1-(5-cyanopyridin-2-yl)piperidin-4-yl]amino}-6-(pyrazin-2-ylamino)nicotinamide,
4-{[1-(4-cyanophenyl)piperidin-4-yl]amino}-6-(pyrazin-2-yl)nicotinamide,
4-{[1-(5-cyanopyridin-2-yl)piperidin-4-yl]amino-6-(pyrimidin-4-ylamino)nicotinamide,
4-{[1-(5-cyanopyridin-2-yl)piperidin-4-yl]amino}-6-[(2-methylpyrimidin-4-yl)amino]nicotinamide,
4-{[(3S,4R)-1-(5-cyanopyridin-2-yl)-3-fluoropiperidin-4-yl]amino}-6-(pyrimidin-4-ylamino)nicotinamide,
4-{[(3S,4R)-1-(6-cyanopyridazin-3-yl)-3-fluoropiperidin-4-yl]amino}-6-(pyrazin-2-ylamino)nicotinamide,
4-{[1-(6-cyanopyridazin-3-yl)piperidin-4-yl]amino}-6-(pyrazin-2-ylamino)nicotinamide,
4-{[(3S,4R)-1-(5-cyanopyrazin-2-yl)-3-fluoropiperidin-4-yl]amino}-6-(pyrimidin-4-ylamino)nicotinamide,
4-{[1-(6-cyanopyridazin-3-yl)piperidin-4-yl]amino}-6-(pyrimidin-4-ylamino)nicotinamide,
4-{[1-(6-cyanopyridazin-3-yl)piperidin-4-yl]amino-6-[(5-methylpyrazin-2-yl)amino]nicotinamide,
4-{[1-(5-cyanopyridin-2-yl)piperidin-4-yl]amino}-6-[(5-methylpyrazin-2-yl)amino]nicotinamide,
4-({1-[4-(benzyloxy)phenyl]piperidin-4-yl}amino)-6-(pyrazin-2-ylamino)nicotinamide,
4-{[1-(4-hydroxyphenyl)piperidin-4-yl]amino}-6-(pyrazin-2-ylamino)nicotinamide,
4-{[1-(4-fluorophenyl)piperidin-4-yl]amino}-6-(pyrazin-2-ylamino)nicotinamide,
4-{[1-(6-cyanopyridin-3-yl)piperidin-4-yl]amino}-6-(pyrimidin-4-ylamino)nicotinamide,
4-{[(3S,4R)-1-(5-cyanopyridin-2-yl)-3-fluoropiperidin-4-yl]amino}-6-[(2-methylpyrimidin-4-yl)amino]nicotinamide,
4-{[(3S,4R)-1-(6-cyanopyridazin-3-yl)-3-fluoropiperidin-4-yl]amino}-6-[(2-methylpyrimidin-4-yl)amino]nicotinamide,
4-({1-[4-(cyanomethoxy)phenyl]piperidin-4-yl}amino)-6-(pyrazin-2-ylamino)nicotinamide,
4-[(1-{5-[(cyanomethyl)carbamoyl]pyridin-2-yl}piperidin-4-yl)amino]-6-(pyrazin-2-ylamino)nicotinamide,
6-(4-{[5-carbamoyl-2-(pyrazin-2-ylamino)pyridin-4-yl]amino}piperidin-1-yl)-N-(cyanomethyl)-N-methylnicotinamide,
4-{[1-(5-cyanopyridin-2-yl)piperidin-4-yl]amino}-6-{[5-(2-oxopyrrolidin-1-yl)pyridin-2-yl]amino}nicotinamide,
4-{[(3S,4R)-1-(5-cyanopyridin-2-yl)-3-(hydroxymethyl)piperidin-4-yl]amino}-6-(pyridin-2-ylamino)nicotinamide,
4-{[1-(4-cyanophenyl)piperidin-4-yl]amino}-6-{[5-(methoxymethyl)pyridin-2-yl]amino}nicotinamide,
4-{[(3S,4R)-1-(5-cyanopyridin-2-yl)-3-(hydroxymethyl)piperidin-4-yl]amino}-6-[(pyrazin-2-yl)]aminonicotinamide,
4-{[(3S,4R)-1-(5-cyanopyridin-2-yl)-3-(hydroxymethyl)piperidin-4-yl]amino}-6-{[5-(methoxymethyl)pyridin-2-yl]amino}nicotinamide,
4-{[(3S,4R)-1-(5-cyanopyridin-2-yl)-3-(hydroxymethyl)piperidin-4-yl]amino}-6-[(5-fluoropyridin-2-yl)amino]nicotinamide,
4-{[(3S,4R)-1-(5-cyanopyridin-2-yl)-3-(hydroxymethyl)piperidin-4-yl]amin}-6-{[5-(trifluoromethyl)pyridin-2-yl]amino}nicotinamide,
4-{[(3S,4R)-1-(5-cyanopyridin-2-yl)-3-fluoropiperidin-4-yl]amino}-6-{[5-(2-oxopiperidin-1-yl)pyridin-2-yl]amino}nicotinamide,
4-{[(3R,4S)-1-(5-cyanopyridin-2-yl)-3-fluoropiperidin-4-yl]amino}-6-{[5-(2-oxopiperidin-1-yl)pyridin-2-yl]amino}nicotinamide,
4-{[(3S,4R)-1-(5-cyanopyridin-2-yl)-3-fluoropiperidin-4-yl]amino}-6-{[5-(2-oxoazepan-1-yl)pyridin-2-yl]amino}nicotinamide,
4-{[(3R,4S)-1-(5-cyanopyridin-2-yl)-3-methylpiperidin-4-yl]amino}-6-{[5-(2-oxopiperidin-1-yl)pyridin-2-yl]amino}nicotinamide,
4-{[1-(5-cyanopyridin-2-yl)piperidin-4-yl]amino}-6-({5-[(1R, 4S)-3-oxo-2-azabicyclo[2.2.1]hept-2-yl]pyridin-2-yl}amino)nicotinamide,
4-{[1-(5-cyanopyridin-2-yl)piperidin-4-yl]amino}-6-({5-[(1S, 4R)-3-oxo-2-azabicyclo[2.2.1]hept-2-yl]pyridin-2-yl}amino)nicotinamide,
4-{[(3S,4R)-1-(5-cyanopyridin-2-yl)-3-fluoropiperidin-4-yl]amino}-6-(pyrazin-2-ylamino)nicotinamide,
4-{[1-(4-cyanophenyl)piperidin-4-yl]amino}-6-[(2-methylpyrimidin-4-yl)amino]nicotinamide,
6-(pyrazin-2-ylamino)-4-[(2,3,6-trifluorobenzyl)amino]nicotinamide,
6-[(2-methylpyrimidin-4-yl)amino]-4-[(2,3,6-trifluorobenzyl)amino]nicotinamide,
6-{[5-(2-oxopiperidin-1-yl)pyridin-2-yl]amino}-4-[(2,3,6-trifluorobenzyl)amino]nicotinamide,
6-{[5-(4-methyl-2-oxopiperazin-1-yl)pyridin-2-yl]amino}-4-[(2,3,6-trifluorobenzyl)amino]nicotinamide,
6-{[5-(4-methyl-7-oxo-1,4-diazepan-1-yl)pyridin-2-yl]amino}-4-[(2,3,6-trifluorobenzyl)amino]nicotinamide,
6-{[5-(3-oxomorpholin-4-yl)pyridin-2-yl]amino}-4-[(2,3,6-trifluorobenzyl)amino]nicotinamide,
6-({5-[(1S,4R)-3-oxo-2-azabicyclo[2.2.1]hept-2-yl]pyridin-2-yl}amino)-4-[(2,3,6-trifluorobenzyl)amino]nicotinamide,
6-{[5-(2-oxoazepan-1-yl)pyridin-2-yl]amino}-4-[(2,3,6-trifluorobenzyl)amino)]nicotinamide,
6-({5-[(1R,4S)-3-oxo-2-azabicyclo[2.2.1]hept-2-yl]pyridin-2-yl}amino)-4-[(2,3,6-trifluorobenzyl)amino]nicotinamide,
6-{[5-(5-oxo-1,4-oxyazepan-4-yl)pyridin-2-yl]amino}-4-[(2,3,6-trifluorobenzyl)amino]nicotinamide,
4-[(2,3,6-trifluorobenzyl)amino]-6-{[5-(3,4,4-trimethyl-2,5-dioxoimidazolidin-1-yl)pyridin-2-yl]amino}nicotinamide,
4-[(2,6-difluorobenzyl)amino]-6-{[5-(pyrrolidin-1-ylcarbonyl)pyridin-2-yl]amino nicotinamide,
4-[(2,6-difluorobenzyl)amino]-6-({5-[(4-methylpiperazin-1-yl)carbonyl]pyridin-2-yl}amino)nicotinamide,
4-[(2,6-difluorobenzyl)amino]-6-({(5-[(4-methyl-1,4-diazepan-1-yl)carbonyl]pyridin-2-yl}amino)nicotinamide,
4-[(2,6-difluorobenzyl)amino]-6-{[5-(4-methyl-7-oxo-1,4-diazepan-1-yl)pyridin-2-yl]amino}nicotinamide, or
4-[(2,6-difluorobenzyl)amino]-6-({5-[(4-methoxypiperidin-l-yl)carbonyl]pyridin-2-yl}amino)nicotinamide.

11. A pharmaceutical composition comprising the compound or a salt thereof according to in claim 1 and a pharmaceutically acceptable excipient.

12. A pharmaceutical composition for preventing or treating a disease caused by undesirable cytokine signal transduction or a disease caused by abnormal cytokine signal transduction, comprising the compound or a salt thereof according to in claim 1.

13. Use of the compound or a salt thereof according to in claim 1 for preparation of a pharmaceutical composition for preventing or treating a disease caused by undesirable cytokine signal transduction or a disease caused by abnormal cytokine signal transduction.

14. Use of the compound or a salt thereof according to in claim 1 for preventing or treating a disease caused by undesirable cytokine signal transduction or a disease caused by abnormal cytokine signal transduction.

15. A method for preventing or treating a disease caused by undesirable cytokine signal transduction or a disease caused by abnormal cytokine signal transduction, comprising administering to a patient an effective amount of the compound or a salt thereof according to in claim 1.
